# EUROPEAN PATENT SPECIFICATION

(11) **EP 4 605 403 B1**
(45) Date of publication and mention of the grant of the patent: **26.11.2025**
(21) Application number: 23792921.1
(22) Date of filing: 16.10.2023
(51) Int. Cl.: C07H 1/06, C07H 19/067, C07H 19/167, C07H 21/02

(54) **METHOD AND COMPOSITION FOR OLIGONUCLEOTIDE SYNTHESIS**
VERFAHREN UND ZUSAMMENSETZUNG ZUR SYNTHESE VON OLIGONUKLEOTIDEN
PROCÉDÉ ET COMPOSITION POUR LA SYNTHÈSE D'OLIGONUCLÉOTIDES

(30) Priority: 17.10.2022 EP 22202014
(43) Date of publication of application: 27.08.2025
(73) Proprietor: Bachem Holding AG, 4416 Bubendorf (CH)
(72) Inventor: SAITO, Masaharu, Yokohama Kanagawa 230-0045 (JP); TAKEDA, Kazutaka, Yokohama Kanagawa 230-0045 (JP); OKADA, Yohei, Tokyo 183-8509 (JP); LUTHER, Anatol, 4416 Bubendorf (CH)
(74) Representative: Jacobi, Markus Alexander
(86) International application number: PCT/EP2023/078683
(87) International publication number: WO 2024/083746

(56) References cited:
- EP-A1- 3 925 964
- US-A1- 2013 267 697
- SHOKAKU KIM ET AL: "Liquid-Phase RNA Synthesis by Using Alkyl-Chain-Soluble Support", CHEMISTRY - A EUROPEAN JOURNAL, JOHN WILEY & SONS, INC, DE, vol. 19, no. 26, 9 May 2013 (2013-05-09), pages 8615 - 8620, XP071836831, ISSN: 0947-6539, DOI: 10.1002/CHEM.201300655

## Description

The present invention generally pertains to the field of oligonucleotide synthesis at an industrial or laboratory scale. Improved methods for the synthesis of oligonucleotides are disclosed.

Efficient methods for the manufacture of oligonucleotides at an industrial or laboratory scale as well as solvents for use in these methods are of significant pharmaceutical and commercial interest.

The synthesis of oligonucleotides commonly relies on iterative coupling cycles, in which oligomeric or monomeric building blocks are added to a growing oligonucleotide chain. Solid-phase synthesis is characterized in that a growing oligonucleotide chain is assembled on a solid support, typically made of polymer resins or controlled pore glass. Solid-phase synthesis processes using the so-called phosphoramidite method have been optimized and automated. Thus, such solid-phase synthesis processes may be advantageous in terms of speed and are used most widely. However, the upscaling of solid-phase synthesis processes is difficult for several reasons, including excessive consumption of reagents and raw materials. A further disadvantage of solid-phase synthesis processes is that it is difficult to follow the progress of the reaction in real time and to analyze the structure of intermediates.

More recently, liquid-phase synthesis processes have been developed, in which the growing oligonucleotide chain is bonded to a soluble support, also referred to as pseudo solid-phase protecting group or tag. These pseudo solid-phase protecting groups are typically hydrophobic and alter the solubility of the oligonucleotides. In consequence, oligonucleotides bonded to one or more such pseudo solid-phase protecting groups, herein also referred to as conjugates of oligonucleotides and pseudo solid-phase protecting groups, may be readily soluble in the organic solvents commonly used for oligonucleotide synthesis.

In solid-phase synthesis, where the solid support is insoluble in the reaction solution(s) at all times, excess reagents and dissolved side-products or by-products may easily be removed by draining the reaction solution through a filter or frit or membrane of suitable pore sizes, thereby retaining the solid support carrying the growing oligonucleotide chains inside the reaction vessel or column. After draining of the liquids, new solvents and/or reagents and/or synthetic building blocks may be added.

In contrast, in liquid-phase oligonucleotide synthesis using pseudo solid-phase protecting groups, these groups are typically selected so that the conjugates of the growing oligonucleotide chains and the pseudo solid-phase protecting groups are soluble in the reaction solvent(s). Thus, solid-liquid separation, typically filtration, may not readily be performed, and requires prior precipitation of the said conjugates. Such precipitation is typically achieved by addition of one or more polar solvents (e.g. water, acetonitrile, propionitrile, methanol, ethanol, 1-propanol, 2-propanol, and the like) and/or by concentrating the respective solution (e.g. *in vacuo*) and/or by decreasing the temperature of the respective solution. Each such sequence of precipitation and solid-liquid separation may be tedious, in particular since precipitating oligonucleotides in filterable form may not always be straight-forward. It may thus be desirable to reduce the number of such precipitation and solid-liquid separation steps required during liquid-phase oligonucleotide synthesis, e.g. to one such step per coupling cycle. Examples of precipitation and solid-liquid separation approaches to oligonucleotide synthesis using pseudo solid-phase protecting groups are e.g. disclosed in: US2015112053A1, EP3825300A1, EP2711370A1, EP3398955A1, US2018291056A1, EP3925964A1, EP2921499A1, EP3733680A1, EP3378869A1, WO2020227618A2, EP3263579A1, EP3950698A1, EP4006045A1, and EP3015467A1.

Alternatively, or additionally, liquid-phase oligonucleotide synthesis using pseudo solid-phase protecting groups may rely on one or more (liquid-liquid) extraction steps, typically aqueous extraction steps, to remove excess reagents and/or side-products and/or by-products, thereby avoiding the need for precipitation. This strategy typically exploits the fact that the pseudo solid-phase protecting groups are usually quite hydrophobic. Thus, the conjugates of such pseudo solid-phase protecting groups and the growing oligonucleotide chains are typically (mostly) retained in an organic phase during aqueous extraction(s). Also in such approaches, at least one solid-liquid separation, typically filtration, step is usually performed per coupling cycle, either after evaporation to dryness and re-dissolution to achieve further purification, or prior to evaporation to dryness, if a drying agent such as sodium sulfate has been added, e.g. to the organic phase obtained from aqueous extraction. Examples of such approaches are e.g. disclosed in: M.C. de Koning et al., Organic Process Research & Development 2006, 10, 1238-1245; A. Schwenger et al., European Journal of Organic Chemistry 2017, 5852-5864; S. Kim et al., Chemistry - A European Journal 2013, 19, 8615-8620.

An oligonucleotide synthesis process, which does not comprise any solid-liquid separation during and in between at least two consecutive coupling cycles, may be referred to as one-pot process. Such one-pot processes may be preferred, but have been reported very scarcely, namely in US2013267697A1, US2015080565A1.

Both of these applications disclose inter alia methods for the synthesis of oligonucleotides, where a first nucleoside or oligonucleotide comprising a 3'-phosphoramidite moiety and a protected 5'-hydroxyl moiety is condensed with a second nucleoside or oligonucleotide comprising a free 5'-hydroxyl group and a protected 3'-hydroxyl moiety. The pseudo solid phase protecting groups are placed either on one or more bases of the first or second nucleoside or oligonucleotide and/or on the 3'-hydroxyl moiety of the second nucleoside or oligonucleotide. Two different strategies for a one-pot synthesis of oligonucleotides longer than 5 nucleotides are disclosed: Either, more than one pseudo solid-phase protecting group is used to increase the lipophilicity (i.e. solubility in organic solvents) of the growing oligonucleotide strand (cf, e.g., Example 18). In the alternative, the halogenated solvent DCM is used (cf., e.g., Example 26).

It is well-known that halogenated solvents such as DCM or chloroform are capable of dissolving even long oligonucleotides bonded to a single pseudo solid-phase protecting group (e.g. ≥ 20mers, see for example: EP3825300A1, EP2711370A1, EP3398955A1, US2018291056A1, EP3263579A1, and EP3950698A1). However, the use of halogenated solvents such as chloroform or DCM may be undesirable, in particular on industrial scale, due to ecological and economic concerns. The use of essentially halogen-free solvents is clearly preferred.

Likewise, using more than one pseudo solid-phase protecting group is undesirable in terms of atom economy and commercial aspects.

There is still a need for further methods for the liquid-phase synthesis of oligonucleotides using pseudo solid-phase protecting groups. Ideally, such methods would involve no or a minimum number of solid-liquid separations. Such methods will rely on one or more aqueous extractions. In general, optimized conditions, in particular optimized solvents, for the aqueous extraction of solutions comprising nucleosides or oligonucleotides bound to a pseudo-solid phase protecting group are desirable. Moreover, the methods would ideally work with a single pseudo solid-phase protecting group per oligonucleotide molecule to be synthesized and allow for the synthesis of oligonucleotides comprising at least 5 nucleoside subunits, e.g., 5-18 nucleoside subunits. It is generally preferable to use as little halogenated solvents as possible.

The present invention provides methods and compositions addressing the above needs.

One aspect of the invention pertains to a method for the synthesis of a target oligonucleotide O^{T}, said method comprising a step of subjecting a solution comprising a component C selected from the group consisting of a nucleoside and an oligonucleotide to one or more aqueous extractions, wherein the organic phase comprises said component C, and wherein
- said component C is covalently bonded to a pseudo solid-phase protecting group PG-s, and
- each of said one or more aqueous extractions is carried out in the presence of one or more amide solvents S^{A}, wherein each amide solvent S^{A} is an amide solvent comprising one or more alkyl groups, wherein these one or more alkyl groups together comprise in total 6-48 carbon atoms, preferably 6-24 carbon atoms, in particular 6-16 or 6-15 carbon atoms.

One aspect of the invention pertains to a method for the synthesis of a target oligonucleotide O^{T}, said method comprising a step of subjecting a solution comprising a component C to one or more aqueous extractions, wherein the organic phase comprises said component C and wherein
- said component C is an oligonucleotide, preferably an oligonucleotide comprising equal to or more than 5 nucleoside subunits, in particular an oligonucleotide comprising 5-18 nucleoside subunits;
- said component C is covalently bonded to a pseudo solid-phase protecting group PG-s, and
- each of said one or more aqueous extractions is carried out in the presence of one or more amide solvents S^{A}, wherein each amide solvent S^{A} is an amide solvent comprising one or more alkyl groups, wherein these one or more alkyl groups together comprise in total 6-48 carbon atoms.

One aspect of the invention pertains to a method for the synthesis of a target oligonucleotide O^{T}, said method comprising a step of subjecting a solution comprising a component C to one or more aqueous extractions, wherein the organic phase comprises said component C and wherein
- said component C is an oligonucleotide, preferably an oligonucleotide comprising equal to or more than 5 nucleoside subunits, in particular an oligonucleotide comprising 5-18 nucleoside subunits;
- said component C is covalently bonded to a pseudo solid-phase protecting group PG-s, and
- each of said one or more aqueous extractions is carried out in the presence of one or more amide solvents S^{A}, wherein each amide solvent S^{A} is an amide solvent comprising one or more alkyl groups, wherein these one or more alkyl groups together comprise in total 6-24 carbon atoms.

One aspect of the invention pertains to a method for the synthesis of a target oligonucleotide O^{T}, said method comprising a step of subjecting a solution comprising a component C to one or more aqueous extractions, wherein the organic phase comprises said component C and wherein
- said component C is an oligonucleotide, preferably an oligonucleotide comprising equal to or more than 5 nucleoside subunits, in particular an oligonucleotide comprising 5-18 nucleoside subunits;
- said component C is covalently bonded to a pseudo solid-phase protecting group PG-s, and
- each of said one or more aqueous extractions is carried out in the presence of one or more amide solvents S^{A}, wherein each amide solvent S^{A} is an amide solvent comprising one or more alkyl groups, wherein these one or more alkyl groups together comprise in total 6-16 carbon atoms, preferably 6-15 carbon atoms.

One aspect of the invention pertains to a method for the synthesis of a target oligonucleotide O^{T}, said method comprising a step of subjecting a solution comprising a component C to one or more aqueous extractions, wherein the organic phase comprises said component C and wherein
- said component C is an oligonucleotide, preferably an oligonucleotide comprising equal to or more than 5 nucleoside subunits, in particular an oligonucleotide comprising 5-18 nucleoside subunits;
- said component C is covalently bonded to exactly one pseudo solid-phase protecting group, which is the pseudo solid-phase protecting group PG-s, and
- each of said one or more aqueous extractions is carried out in the presence of one or more amide solvents S^{A}, wherein each amide solvent S^{A} is an amide solvent comprising one or more alkyl groups, wherein these one or more alkyl groups together comprise in total 6-48 carbon atoms.

One aspect of the invention pertains to a method for the synthesis of a target oligonucleotide O^{T}, said method comprising a step of subjecting a solution comprising a component C to one or more aqueous extractions, wherein the organic phase comprises said component and wherein
- said component C is an oligonucleotide, preferably an oligonucleotide comprising equal to or more than 5 nucleoside subunits, in particular an oligonucleotide comprising 5-18 nucleoside subunits;
- said component C is covalently bonded to exactly one pseudo solid-phase protecting group, which is the pseudo solid-phase protecting group PG-s, and
- each of said one or more aqueous extractions is carried out in the presence of one or more amide solvents S^{A}, wherein each amide solvent S^{A} is an amide solvent comprising one or more alkyl groups, wherein these one or more alkyl groups together comprise in total 6-24 carbon atoms.

One aspect of the invention pertains to a method for the synthesis of a target oligonucleotide O^{T}, said method comprising a step of subjecting a solution comprising a component C to one or more aqueous extractions, wherein the organic phase comprises said component C and wherein
- said component C is an oligonucleotide, preferably an oligonucleotide comprising equal to or more than 5 nucleoside subunits, in particular an oligonucleotide comprising 5-18 nucleoside subunits;
- said component C is covalently bonded to exactly one pseudo solid-phase protecting group, which is the pseudo solid-phase protecting group PG-s, and
- each of said one or more aqueous extractions is carried out in the presence of one or more amide solvents S^{A}, wherein each amide solvent S^{A} is an amide solvent comprising one or more alkyl groups, wherein these one or more alkyl groups together comprise in total 6-16 carbon atoms, preferably 6-15 carbon atoms.

The term "aqueous extraction" may, in the context of the aforementioned aspects of the invention, be understood in the broadest sense as any liquid-liquid extraction operation during which said solution comprising said component C is extracted with water or an aqueous solution in general. As defined herein, an "aqueous extraction" is further characterized in that it results in a mixture comprising at least one aqueous phase (preferably an aqueous layer) and at least one organic phase (preferably an organic layer), wherein the at least one aqueous phase (preferably layer) is then separated from the at least one organic phase (preferably layer). In case of more than one organic phases (either from a single aqueous extraction or from back-extraction of one or more aqueous phase), these more than one organic phases may be combined to obtain "the organic phase". In general, the means of performing aqueous extractions are well-known to the skilled person.

In the context of the above-mentioned aspects of the invention, to state that "each of said one or more aqueous extractions is carried out in the presence of one or more amide solvents S^{A}" means that at some point in time during each aqueous extraction, one or more amide solvents S^{A} are present in the vessel or separatory funnel or other receptacle, in which the respective extraction is carried out. One or more amide solvents S^{A} may preferably already be contained in said solution comprising the component C. Additionally, or alternatively, one or more amide solvents S^{A} may be added during the respective extraction. Preferably, to state that "each of said one or more aqueous extractions is carried out in the presence of one or more amide solvents S^{A}" means that in each aqueous extraction, one or more amide solvents S^{A} are comprised in the mixture from which an organic phase and an aqueous phase are to be obtained. In other words: The one or more amide solvents S^{A} are preferably present before the phase separation is completed.

As used herein, an aqueous solution is a solution comprising water, preferably at least 10 vol-%, 20 vol-%, 30 vol-%, 40 vol-%, 50 vol-%, or 60 vol-% of water. Hence, water is herein also considered a specific aqueous solution, since it comprises more than 10 vol-%, 20 vol-%, 30 vol-%, 40 vol-%, 50 vol-%, or 60 vol-% of water.

An aqueous solution may optionally comprise one or more water-miscible organic solvents. Non-limiting examples of such water-miscible organic solvents comprise:
- alcohol solvents such as methanol, ethanol, and isopropyl alcohol (propan-2-ol, IPA),
- nitrile solvents such as acetonitrile and propionitrile,
- ketone solvents such as acetone and 2-butanone,
- polar ether solvents such as tetrahydrofuran and 1,4-dioxane,
- polar amide solvents such as N,N-dimethylformamide, N,N-dimethylacetamide, N-methyl-2-pyrrolidone, and N-methyl-2-piperidone,
- sulfoxide solvents such as dimethyl sulfoxide, and
- mixtures thereof.

An aqueous solution may optionally also comprise one or more dissolved components. Non-limiting examples of such dissolved components comprise:
- inorganic salts such as alkali halides, in particular sodium chloride (e.g., typically used to facilitate and/or accelerate phase separation),
- water-soluble protic acids such as acetic acid,
- water-soluble organic or inorganic bases such as N-methylmorpholine (NMM), pyridine, sodium or potassium or ammonium carbonate or hydrogen carbonate, and sodium or potassium or ammonium phosphate or hydrogen phosphate or dihydrogen phosphate, and
- mixtures thereof.

Further non-limiting examples of aqueous solutions are provided below in the context of steps of steps (c-1), (c-2), (c-x), (g-1), (g-2), and (g-x) and also in the synthetic Examples provided herein.

The expression "the organic phase comprises the component C" may be understood in the broadest sense to mean that some of the molecules of said component C are dissolved in the organic phase. It is preferred that most molecules (e.g., more than 50 %, 60 %, 70 %, 80 %, or 90 % of the molecules) of the said component C are comprised in the organic phase (and thus not in the aqueous phase).

Said "solution comprising a component C" may, for example, be a reaction mixture or it may, for example, be obtained from a reaction mixture by addition of one or more compounds and/or solvents. Examples of such compounds are bases, preferably bases having a pKa-value (determined in water, i.e., an aqueous solution of the base, at 25 °C) equal to or smaller than 9.0 or 8.0 or 7.0 or 6.5 or 6.0 or 5.5. Pyridine is an example of such a base. The protonated from of pyridine has a pKa-value smaller than 5.5. Pyridine is a weak base compared to, e.g., triethylamine whose protonated from has a pKa-value larger than 9.0. Examples of solvents which may be added to a reaction mixture comprising the component C are non-polar solvents. Alternatively, or additionally, such non-polar solvents may also be added during or in between the one or more aqueous extractions. Non-limiting examples of non-polar solvents which may be added comprise:
- ether solvents such as, e.g., alkylated derivatives of tetrahydropyran or tetrahydrofuran, in particular 4-methyltetrahydropyran (MTHP) and 2-methyltetrahydrofuran, cyclopentyl methyl ether (CPME), *tert*-butyl methyl ether, diethyl ether, and anisole;
- aromatic solvents such as, e.g., benzene, toluene, *o*- or *m*- or *p*-xylene, and mesitylene;
- aliphatic hydrocarbon solvents such as, e.g., pentanes, hexanes, heptanes, octanes, nonanes, and cyclic derivatives thereof such as cyclohexane;
- ester solvents such as, e.g., ethyl acetate and isopropyl acetate;
- halogenated aliphatic hydrocarbon solvents such as, e.g., dichloromethane, 1,2-dichloroethane, and chloroform; and
- mixtures thereof.

4-Methyltetrahydropyran (MTHP) is a preferred non-polar ether solvent which may be added prior to or during or in between the aqueous extraction(s). Additionally, or alternatively, one or more amide solvents S^{A} may be added prior to or during or in between the aqueous extraction(s).

In some preferred embodiments of the method of the above-mentioned aspects of the invention, the one or more aqueous extractions are carried out in essentially halogen-free solvents. As used herein, the term "essentially halogen-free solvent" refers to a solvent which contains in total equal to or less than 3.0 vol-%, 2.0 vol-%, 1.0 vol-%, 0.1 vol-%, 0.01 vol-%, or 0.001 vol-% of halogenated solvents. As used herein, the term "halogenated solvent" refers to any solvent comprising in its chemical structure at least one halogen atom. Examples of halogenated solvents comprise dichloromethane, chloroform, 1,1-dichloroethane, and 1,2-dichloroethane. To state that "the one or more aqueous extractions are carried out in essentially halogen-free solvents" means that:
- the solution comprising the component C comprises in total equal to or less than 3.0 vol-%, 2.0 vol-%, 1.0 vol-%, 0.1 vol-%, 0.01 vol-%, or 0.001 vol-% of halogenated solvents; and
- only essentially halogen-free solvents may be added prior to, during or in between the one or more aqueous extractions.

In some preferred embodiments of the method of the invention, each organic phase and each aqueous phase of the one or more aqueous extractions comprises in total less than 3.0 vol-%, 2.0 vol-%, 1.0 vol-%, 0.1 vol-%, 0.01 vol-%, or 0.001 vol-% of halogenated solvents.

As used herein, the expression "in total less than 3.0 vol-%, 2.0 vol-%, 1.0 vol-%, 0.1 vol-%, 0.01 vol-%, or 0.001 vol-% of halogenated solvents" means that, if more than one halogenated solvents (e.g., dichloromethane and chloroform) are comprised, their vol-% are to be summed up to obtain the total vol-% which is to be less than 3.0 vol-%, 2.0 vol-%, 1.0 vol-%, 0.1 vol-%, 0.01 vol-%, or 0.001 vol-%.

In some embodiments of the method of the above-mentioned aspects of the invention, said one or more aqueous extractions comprise a first aqueous extraction comprising the following steps (Ex-1) to (Ex-4):

| | |
|---|---|
| (Ex-1) | Combining the solution comprising the component C with a first aqueous solution AS-1; |
| (Ex-2) | Agitating the mixture of step (Ex-1); |
| (Ex-3) | Allowing the phases to separate, so as to obtain a first organic phase OP-1 and a first aqueous phase AP-1, wherein the first organic phase OP-1 comprises the component C; and |
| (Ex-4) | Removing the first aqueous phase AP-1 from the first organic phase OP-1 or vice versa; |

wherein the solution comprising the component C of step (Ex-1) comprises one or more amide solvents S^{A} and/or one or more amide solvents S^{A} are added during any one of steps (Ex-1) and (Ex-2) and/or in between these steps.

In some embodiments of the method of the above-mentioned aspects of the invention, said one or more aqueous extractions comprise a first aqueous extraction comprising the aforementioned steps (Ex-1) to (Ex-4), and a second aqueous extraction comprising the following steps (Ex-5) to (Ex-8):

| | |
|---|---|
| (Ex-5) | Combining the first organic phase OP-1 with a second aqueous solution AS-2; |
| (Ex-6) | Agitating the mixture of step (Ex-5); |
| (Ex-7) | Allowing the phases to separate, so as to obtain a second organic phase OP-2 and a second aqueous phase AP-2, wherein the second organic phase OP-2 comprises the component C; and |
| (Ex-8) | Removing the second aqueous phase AP-2 from the second organic phase OP-2 or vice versa; |

wherein the first organic phase OP-1 comprises one or more amide solvents S^{A} and, optionally, one or more amide solvents S^{A} are added during any one of steps (Ex-5) and (Ex-6) and/or in between these steps.

In some embodiments of the method of the above-mentioned aspects of the invention, said one or more aqueous extractions comprise a first aqueous extraction comprising the aforementioned steps (Ex-1) to (Ex-4), a second aqueous extraction comprising the aforementioned steps (Ex-5) to (Ex-8), and a third aqueous extraction comprising the following steps (Ex-9) to (Ex-12):

| | |
|---|---|
| (Ex-9) | Combining the second organic phase OP-2 with a third aqueous solution AS-3; |
| (Ex-10) | Agitating the mixture of step (Ex-9); |
| (Ex-11) | Allowing the phases to separate, so as to obtain a third organic phase OP-3 and a third aqueous phase AP-3, wherein the third organic phase OP-3 comprises the component C; and |
| (Ex-12) | Removing the third aqueous phase AP-3 from the third organic phase OP-3 or vice versa; |

wherein the second organic phase OP-2 comprises one or more amide solvents S^{A} and, optionally, one or more amide solvents S^{A} are added during any one of steps (Ex-9) and (Ex-10) and/or in between these steps.

The skilled artisan understands that one or more further aqueous extractions similar to the first, second, and third aqueous extraction comprising steps (Ex-1) to (Ex-4) or steps (Ex-5) to (Ex-8) or steps (Ex-9) to (Ex-12), respectively, may be carried out in addition. Such further aqueous extractions preferably comprise the following steps (Ex-A) to (Ex-D):

| | |
|---|---|
| (Ex-A) | Combining the organic phase obtained from the previous aqueous extraction with an aqueous solution; |
| (Ex-B) | Agitating the mixture of step (Ex-A); |
| (Ex-C) | Allowing the phases to separate, so as to obtain an organic phase and an aqueous phase, wherein the organic phase comprises the component C; and |
| (Ex-D) | Removing the aqueous phase from the organic phase or vice versa; |

wherein the organic phase obtained from the previous aqueous extraction comprises one or more amide solvents S^{A} and, optionally, one or more amide solvents S^{A} are added during any one of steps (Ex-A) and (Ex-B) and/or in between these steps.

The skilled artisan understands that the aqueous solutions of two or more aqueous extractions, e.g., the aqueous solutions AS-1, AS-2, and AS-3, need not be identical, i.e., they may or may not comprise the same species/components/solvents and the amounts/volumes of said species/components/solvents may be the same or different, unless indicated differently in specific embodiments.

In some embodiments of the method of the above-mentioned aspects of the invention, the aqueous phase of at least one, preferably each, of said one or more aqueous extractions has a pH-value equal to or smaller than 7, preferably in the range of 4-7. As used herein, the pH-value of the aqueous phase of an aqueous extraction is to be determined from the respective aqueous phase after the aqueous extraction (i.e., after the phase separation, preferably after removing the aqueous phase from the organic phase or vice versa) and at a temperature in the range of 23-28 °C, preferably 25 °C.

As used herein, the term "aqueous phase" of an aqueous extraction is distinct from the aqueous solution, which is initially combined with the solution comprising the component C, followed by extraction. The skilled artisan understands that the "aqueous phase" typically comprises species which have been extracted from the solution comprising the component C, e.g., water-soluble species. Hence, to say that the aqueous phase of an aqueous extraction has a certain pH-value is not the same as to say that the aqueous solution employed in the aqueous extraction has a certain pH-value. For example, pure water or any other aqueous solution may be used for an aqueous extraction, and during the aqueous extraction one or more protic acids may partition from said solution comprising the component C into the aqueous phase, so that the aqueous phase then has a pH-value which is lower than the pH-value of the water or other aqueous solution employed.

In some embodiments of the method of the above-mentioned aspects of the invention, said one or more aqueous extractions comprise a first aqueous extraction comprising the following steps (Ex-1) to (Ex-4):

| | |
|---|---|
| (Ex-1) | Combining the solution comprising the component C with a first aqueous solution AS-1; |
| (Ex-2) | Agitating the mixture of step (Ex-1); |
| (Ex-3) | Allowing the phases to separate, so as to obtain a first organic phase OP-1 and a first aqueous phase AP-1, wherein the first organic phase OP-1 comprises the component C; and |
| (Ex-4) | Removing the first aqueous phase AP-1 from the first organic phase OP-1 or vice versa; |

wherein
- the solution comprising the component C of step (Ex-1) comprises one or more amide solvents S^{A} and/or one or more amide solvents S^{A} are added during any one of steps (Ex-1) and (Ex-2) and/or in between these steps; and
- the first aqueous phase AP-1 has a pH-value equal to or smaller than 7, preferably in the range of 1.0-7.0, 2.0-7.0, 3.0-7.0, 3.5-7.0, 4.0-7.0, 4.5-7.0, 4.5-6.5, 4.5-6.0, or 4.5-5.5.

In some embodiments of the method of the above-mentioned aspects of the invention, said one or more aqueous extractions comprise a first aqueous extraction comprising the aforementioned steps (Ex-1) to (Ex-4), and a second aqueous extraction comprising the following steps (Ex-5) to (Ex-8):

| | |
|---|---|
| (Ex-5) | Combining the first organic phase OP-1 with a second aqueous solution AS-2; |
| (Ex-6) | Agitating the mixture of step (Ex-5); |
| (Ex-7) | Allowing the phases to separate, so as to obtain a second organic phase OP-2 and a second aqueous phase AP-2, wherein the second organic phase OP-2 comprises the component C; and |
| (Ex-8) | Removing the second aqueous phase AP-2 from the second organic phase OP-2 or vice versa; |

wherein
- the first organic phase OP-1 comprises one or more amide solvents S^{A} and, optionally, one or more amide solvents S^{A} are added during any one of steps (Ex-5) and (Ex-6) and/or in between these steps; and
- the second aqueous phase AP-2 has a pH-value in the range of 4.0-7.0, 4.5-7.0, 5.0-7.0, 5.0-6.5, or 5.0-6.0.

In some embodiments of the method of the above-mentioned aspects of the invention, said one or more aqueous extractions comprise a first aqueous extraction comprising the aforementioned steps (Ex-1) to (Ex-4), a second aqueous extraction comprising the aforementioned steps (Ex-5) to (Ex-8), and a third aqueous extraction comprising the following steps (Ex-9) to (Ex-12):

| | |
|---|---|
| (Ex-9) | Combining the second organic phase OP-2 with a third aqueous solution AS-3; |
| (Ex-10) | Agitating the mixture of step (Ex-9); |
| (Ex-11) | Allowing the phases to separate, so as to obtain a third organic phase OP-3 and a third aqueous phase AP-3, wherein the third organic phase OP-3 comprises the component C; and |
| (Ex-12) | Removing the third aqueous phase AP-3 from the third organic phase OP-3 or vice versa; |

wherein
- the second organic phase OP-2 comprises one or more amide solvents S^{A} and, optionally, one or more amide solvents S^{A} are added during any one of steps (Ex-9) and (Ex-10) and/or in between these steps; and
- the third aqueous phase AP-3 has a pH-value in the range of 5.0-7.0, 5.0-6.5, or 5.0-6.0.

The skilled artisan understands that one or more further aqueous extractions similar to the first, second, and third aqueous extractions comprising steps (Ex-1) to (Ex-4) or steps (Ex-5) to (Ex-8) or steps (Ex-9) to (Ex-12), respectively, may be carried out in addition. Such further aqueous extractions preferably comprise the following steps (Ex-A) to (Ex-D):

| | |
|---|---|
| (Ex-A) | Combining the organic phase obtained from the previous aqueous extraction with an aqueous solution; |
| (Ex-B) | Agitating the mixture of step (Ex-A); |
| (Ex-C) | Allowing the phases to separate, so as to obtain an organic phase and an aqueous phase, wherein the organic phase comprises the component C; and |
| (Ex-D) | Removing the aqueous phase from the organic phase or vice versa; |

wherein
- the organic phase obtained from the previous aqueous extraction comprises one or more amide solvents S^{A} and, optionally, one or more amide solvents S^{A} are added during any one of steps (Ex-A) and (Ex-B) and/or in between these steps; and
- the aqueous phase obtained in each of steps (Ex-C) has a pH-value equal to or smaller than 7, preferably in the range of 4.0-7.0, in particular in the range of 5.0-7.0, 5.0-6.5, or 5.0-6.0

The expressions "the first organic phase OP-1 comprises the component C" and "the second organic phase OP-2 comprises the component C" and "the third organic phase OP-3 comprises the component C" will be understood based on the above explanation of the term "the organic phase comprises the component C", since each of OP-1, OP-2, and OP-3 is an organic phase.

In some embodiments of the method of the invention, in which said one or more aqueous extractions comprise said first aqueous extraction comprising steps (Ex-1) to (Ex-4), the first aqueous solution AS-1 has a pH-value in the range of 5.0-8.0, 5.5-7.5, 6.0-7.5, or 6.5-7.5. In some embodiments of the method of the invention, in which said one or more aqueous extractions comprise said first aqueous extraction comprising steps (Ex-1) to (Ex-4), the first aqueous solution AS-1 does not comprise a compound having a pKa-value equal to or larger than 9.0, 8.5, 8.0, 7.5, 7.0, 6.5, 6.0 or 5.5. The pKa-value is to be determined in water (i.e., in an aqueous solution of the respective compound) at 25 °C. For example, pyridine is a base and its protonated form has a pKa value of 5.2 (cf., e.g., A. Fischer et al., J. Chem. Soc. 1964, 3591-3596; https://doi.org/10.1039/JR9640003591). Thus, pyridine is not a compound having a pKa-value equal to or larger than 5.5. As another example, 4-aminopyridine is also a base and its protonated form has a pKa-value of 9.1 (cf., e.g., A. Fischer et al., J. Chem. Soc. 1964, 3591-3596; https://doi.org/10.1039/JR9640003591). Hence, 4-aminopyridine is a compound having a pKa-value equal to or larger than 9.0.

In some embodiments of the method of the invention, in which said one or more aqueous extractions comprise said first aqueous extraction comprising steps (Ex-1) to (Ex-4), and said second aqueous extraction comprising steps (Ex-5) to (Ex-8), each of the first aqueous solution AS-1 and the second aqueous solution AS-2 has a pH-value in the range of 5.0-8.0, 5.5-7.5, 6.0-7.5, or 6.5-7.5. In some embodiments of the method of the invention, in which said one or more aqueous extractions comprise said first aqueous extraction comprising steps (Ex-1) to (Ex-4), and said second aqueous extraction comprising steps (Ex-5) to (Ex-8), none of the first aqueous solution AS-1 and the second aqueous solution AS-2 comprises a compound having a pKa-value equal to or larger than 9.0, 8.5, 8.0, 7.5, 7.0, 6.5, 6.0 or 5.5. The pKa-value is to be determined in water (i.e., in an aqueous solution of the respective compound) at 25 °C.

In some embodiments of the method of the invention, in which said one or more aqueous extractions comprise said first aqueous extraction comprising steps (Ex-1) to (Ex-4), and said second aqueous extraction comprising steps (Ex-5) to (Ex-8), and said third aqueous extraction comprising steps (Ex-9) to (Ex-12), each of the first aqueous solution AS-1, the second aqueous solution AS-2, and the third aqueous solution AS-3 has a pH-value in the range of 5.0-8.0, 5.5-7.5, 6.0-7.5, or 6.5-7.5. In some embodiments of the method of the invention, in which said one or more aqueous extractions comprise said first aqueous extraction comprising steps (Ex-1) to (Ex-4), and said second aqueous extraction comprising steps (Ex-5) to (Ex-8), and said third aqueous extraction comprising steps (Ex-9) to (Ex-12), none of the first aqueous solution AS-1, the second aqueous solution AS-2, and the third aqueous solution AS-3 comprises a compound having a pKa-value equal to or larger than 9.0, 8.5, 8.0, 7.5, 7.0, 6.5, 6.0 or 5.5. The pKa-value is to be determined in water (i.e., in an aqueous solution of the respective compound) at 25 °C.

Step (Ex-1) is: Combining the solution comprising the component C with a first aqueous solution AS-1. This means that the solution comprising the component C and the first aqueous solution AS-1 are combined in the vessel or funnel or other receptacle, in which the agitation of step (Ex-2) is to be performed. Step (Ex-5) is: Combining the first organic phase OP-1 with a second aqueous solution AS-2. This means that the first organic phase OP-1 and the second aqueous solution AS-2 are combined in the vessel or funnel or other receptacle, in which the agitation of step (Ex-6) is to be performed. Step (Ex-9) is: Combining the second organic phase OP-2 with a third aqueous solution AS-3. This means that the second organic phase OP-2 and the third aqueous solution AS-3 are combined in the vessel or funnel or other receptacle, in which the agitation of step (Ex-10) is to be performed. Step (Ex-A) is: Combining the organic phase obtained from the previous aqueous extraction with an aqueous solution. This means that the organic phase from the previous aqueous extraction and the aqueous solution are combined in the vessel or funnel or other receptacle, in which the agitation of step (Ex-B) is to be performed.

The term "agitating" in steps (Ex-2), (Ex-6), (Ex-10), and (Ex-B) may be understood in the broadest sense to refer to any operation of inducing a movement of the respective mixture. This typically facilitates the extraction process. Suitable means of agitation are known to those skilled in the art and comprise, e.g., shaking, stirring, e.g., mechanical stirring, bubbling of an inert has such as nitrogen, and inversion of the vessel or funnel or other receptacle in which the one or more aqueous extractions may be carried out. More than one such means of agitation may be used in combination. On an industrial scale, a reaction vessel made of a suitable material, e.g., glass or stainless steel, equipped with a mechanical stirrer may preferably be used for the agitation step (Ex-2), (Ex-6), (Ex-10) or (Ex-B). In practice, it is usually most convenient, to carry out all steps of an aqueous extraction in the same vessel or funnel or other receptacle.

Under the agitation of step (Ex-2), (Ex-6), (Ex-10), and (Ex-B) the respective organic phase and the respective aqueous phase usually form a dispersion. Under such conditions, one phase may typically not be removed from the other. For this purpose, phase separation is usually required. Hence:
- Step (Ex-3) comprises "Allowing the phases to separate, so as to obtain a first organic phase OP-1 and a first aqueous phase AP-1";
- Step (Ex-7) comprises "Allowing the phases to separate, so as to obtain a second organic phase OP-2 and a second aqueous phase AP-2";
- Step (Ex-11) comprises "Allowing the phases to separate, so as to obtain a third organic phase OP-3 and a third aqueous phase AP-3"; and
- Step (Ex-C) comprises "Allowing the phases to separate, so as to obtain an organic phase and an aqueous phase."

As used herein, the term "allowing the phases to separate so as to obtain an organic phase and an aqueous phase" may be understood in the broadest sense as establishing conditions under which the organic phase forms one or more, preferably one, layer (i.e., organic layer) and the aqueous phase forms one or more, preferably one, layer (i.e., aqueous layer). This definition likewise applies to the first, second, and third organic phase OP-1, OP-2, and OP-3 and the respective first, second, and third aqueous phase AP-1, AP-2, and AP-3. The skilled artisan understands that an organic layer formed from an organic phase may typically not be completely free of an aqueous phase and that an aqueous layer formed from an aqueous phase may typically not be completely free of an organic phase. Hence, an organic layer is mostly but not necessarily completely composed of an organic phase and an aqueous layer is mostly but not necessarily completely composed of an aqueous phase.

"Establishing conditions under which the organic phase forms one or more, preferably one, layer (i.e., organic layer) and the aqueous phase forms one or more, preferably one, layer (i.e., aqueous layer)" may usually comprise stopping the agitation. This means that the shaking and/or stirring and/or inert gas bubbling and/or inversion of the vessel or funnel or other receptacle is stopped. It will be understood that some movement of the liquids may still occur.

Hence, in some preferred embodiments, step (Ex-3) is: Stopping the agitation and allowing the phases to separate, so as to obtain a first organic phase OP-1 and a first aqueous phase AP-1, wherein the first organic phase OP-1 comprises the component C. In some preferred embodiments, step (Ex-7) is: Stopping the agitation and allowing the phases to separate, so as to obtain a second organic phase OP-2 and a second aqueous phase AP-2, wherein the second organic phase OP-2 comprises the component C. In some preferred embodiments, step (Ex-11) is: Stopping the agitation and allowing the phases to separate, so as to obtain a third organic phase OP-3 and a third aqueous phase AP-3, wherein the third organic phase OP-3 comprises the component C. In some preferred embodiments, step (Ex-C) is: Stopping the agitation and allowing the phases to separate, so as to obtain an organic phase and an aqueous phase, wherein the organic phase comprises the component C.

Typically, one may simply wait for the phases to separate. Phase separation may optionally be speeded up by addition of aqueous solutions comprising dissolved ions, e.g., by addition of brine (i.e., an aqueous solution of sodium chloride), as known to those skilled in the art. The expression "the organic phase comprises the component C" has been explained above.

In steps (Ex-4), (Ex-8), (Ex-12), and (Ex-D), the respective aqueous phase is removed from the respective organic phase or vice versa. This means that at least one aqueous layer is physically removed from at least one organic layer or vice versa. This will be understood by the skilled artisan. This removal may typically comprise draining of one layer, typically the bottom layer, e.g., the aqueous layer from the vessel or funnel or other receptacle, in which the agitation has been carried out. It will be understood that this removal is not necessarily complete. In other words, "removing" in this context is not to mean "completely removing" but preferably means "mostly removing". In this context, removing one phase from another phase may mean removing at least 51 vol-%, 60 vol-%, 70 vol-%, 80 vol-%, 90 vol-%, 95 vol-%, 96 vol-%, 97 vol-%, 98 vol-% or 99 vol-% of the respective phase to be removed. In practice, there will usually be a clearly recognizable phase boundary between the organic layer and the aqueous layer, so that removing the one from the other may simply be achieved by draining the bottom layer until the phase boundary is reached.

In some embodiments of the method of the above-mentioned aspects of the invention, the component C is a compound of Formula I below, wherein, PG-0 is absent, so that the hydroxyl moiety otherwise protected by PG-0 is a free hydroxyl group. In such embodiments, the integer m in Formula I preferably is equal to or larger than 4, in particular an integer in the range of 4-17. The component C preferably is the component (C-0)^{#} as defined below.

One aspect of the invention pertains to a method for the synthesis of a target oligonucleotide O^{T}, wherein the target oligonucleotide O^{T} comprises a first cycle oligonucleotide O-1, and the method comprises the following step (a-1), and a first coupling cycle comprising the following steps:

| | |
|---|---|
| (a-1) | providing a component C-0 selected from the group consisting of a nucleoside and an oligonucleotide, wherein the component C-0 is covalently bonded to a pseudo solid-phase protecting group PG-s and comprises a backbone hydroxyl moiety protected by a protecting group PG-0 removable under acidic conditions; |
| (b-1) | incubating the component C-0 of step (a-1) with a deprotection mixture M-(b-1), thereby cleaving the protecting group PG-0 from the component C-0, so as to obtain a component (C-0)^{#} having a free backbone hydroxyl group; |
| (c-1) | subjecting a solution comprising the component (C-0)^{#} to one or more aqueous extractions, wherein the organic phase comprises the component (C-0)^{#}; |
| (d-1) | optionally, reducing the water content of the organic phase comprising the component (C-0)^{#}; |
| (e-1) | reacting the component (C-0)^{#} with a building block B-1, wherein said building block B-1 is selected from the group consisting of a nucleoside and an oligonucleotide and comprises |
| | - a backbone hydroxyl moiety protected by a protecting group PG-1 removable under acidic conditions, and |
| | - a phosphorus moiety covalently bonded via its phosphorus atom to an oxygen atom of the backbone of the building block B-1, under conditions suitable to form a covalent bond between said free backbone hydroxyl group of the component (C-0)^{#} and the phosphorus atom of said phosphorus moiety of the building block B-1, thereby obtaining a first cycle oligonucleotide O-1; |
| (f-1) | optionally, incubating the first cycle oligonucleotide O-1 with an oxidizing or sulfurizing agent, thereby converting any P (III) atoms within said first cycle oligonucleotide O-1 to P (V) atoms; |
| (g-1) | optionally, subjecting a solution comprising the first cycle oligonucleotide O-1 to one or more aqueous extractions, wherein the organic phase comprises the first cycle oligonucleotide O-1; |
| (h-1) | if step (g-1) has been carried out, optionally reducing the water content of the organic phase comprising the first cycle oligonucleotide O-1; |

wherein during and in between steps (b-1) to (h-1) (as far as present), no solid-liquid separation is performed and steps (c-1) and (g-1) are carried out in the presence of one or more amide solvents S^{A}, wherein each amide solvent S^{A} is an amide solvent comprising one or more alkyl groups, wherein these one or more alkyl groups together comprise in total 6-48 or 6-24 or 6-16 or 6-15 carbon atoms.

Some embodiments of the present invention relate to a method for the synthesis of a target oligonucleotide O^{T}, wherein the target oligonucleotide O^{T} comprises a first cycle oligonucleotide O-1, and the method comprises the above step (a-1), and a first coupling cycle comprising the above steps (b-1) to (h-1) (as far as present), wherein:
no solid-liquid separation of the first cycle oligonucleotide O-1 or of any oligonucleotidic educts or intermediates involved in the synthesis of the first cycle oligonucleotide O-1 is performed during and in between steps (b-1) to (h-1) (as far as present), and steps (c-1) and (g-1) are carried out in the presence of one or more amide solvents S^{A}, wherein each amide solvent S^{A} is an amide solvent comprising one or more alkyl groups, wherein these one or more alkyl groups together comprise in total 6-48 or 6-24 or 6-16 or 6-15 carbon atoms.

In some embodiments of the method of the invention, the target oligonucleotide O^{T} comprises a second cycle oligonucleotide O-2, and the method further comprises performing a second coupling cycle comprising the following steps:

| | |
|---|---|
| (b-2) | incubating the first cycle oligonucleotide O-1 obtained in the first coupling cycle with a deprotection mixture M-(b-2), thereby cleaving the protecting group PG-1 from the first cycle oligonucleotide O-1, so as to obtain a first cycle oligonucleotide (O-1)^{#} having a free backbone hydroxyl group; |
| (c-2) | subjecting a solution comprising the first cycle oligonucleotide (O-1)^{#} to one or more aqueous extractions, wherein the organic phase comprises the first cycle oligonucleotide (O-1)^{#}; |
| (d-2) | optionally, reducing the water content of the organic phase comprising the first cycle oligonucleotide (O-1)^{#}; |
| (e-2) | reacting the first cycle oligonucleotide (O-1)^{#} with a building block B-2, wherein said building block B-2 is selected from the group consisting of a nucleoside and an oligonucleotide and comprises |
| | - a backbone hydroxyl moiety protected by a protecting group PG-2 removable under acidic conditions, and |
| | - a phosphorus moiety covalently bonded via its phosphorus atom to an oxygen atom of the backbone of the building block B-2, under conditions suitable to form a covalent bond between said free backbone hydroxyl group of the first cycle oligonucleotide (O-1)^{#} and the phosphorus atom of said phosphorus moiety of the building block B-2, thereby obtaining a second cycle oligonucleotide O-2; |
| (f-2) | optionally, incubating the second cycle oligonucleotide O-2 with an oxidizing or sulfurizing agent, thereby converting any P (III) atoms within said second cycle oligonucleotide O-2 to P (V) atoms; |
| (g-2) | optionally, subjecting a solution comprising the second cycle oligonucleotide O-2 to one or more aqueous extractions, wherein the organic phase comprises the second cycle oligonucleotide O-2; |
| (h-2) | if step (g-2) has been carried out, optionally reducing the water content of the organic phase comprising the second cycle oligonucleotide O-2; |

wherein during and in between steps (b-1) to (h-2) (as far as present) no solid-liquid separation is performed and steps (c-1), (g-1), (c-2), and (g-2) are carried out in the presence of one or more amide solvents S^{A}, wherein each amide solvent S^{A} is an amide solvent comprising one or more alkyl groups, wherein these one or more alkyl groups together comprise in total 6-48 or 6-24 or 6-16 or 6-15 carbon atoms.

In some embodiments of the method of the invention, the target oligonucleotide O^{T} comprises a second cycle oligonucleotide O-2, and the method further comprises performing a second coupling cycle comprising the above steps (b-2) to (h-2) (as far as present), wherein:
no solid-liquid separation of the second cycle oligonucleotide O-2 or of any oligonucleotidic educts or intermediates involved in the synthesis of the second cycle oligonucleotide O-2 is performed during and in between steps (b-1) to (h-2) (as far as present) and steps (c-1), (g-1), (c-2), and (g-2) are carried out in the presence of one or more amide solvents S^{A}, wherein each amide solvent S^{A} is an amide solvent comprising one or more alkyl groups, wherein these one or more alkyl groups together comprise in total 6-48 or 6-24 or 6-16 or 6-15 carbon atoms. It will be readily understood in this context that, e.g. the first cycle oligonucleotide O-1 is an oligonucleotidic educt or intermediate involved in the in the synthesis of the second cycle oligonucleotide O-2.

In some embodiments of the method of the invention, the target oligonucleotide O^{T} comprises a n-th cycle oligonucleotide O-n, and the method further comprises performing performing (n-2) iterations of a coupling cycle comprising the following steps (b-x) to (h-x) (as far as present), wherein n is an integer in the range of 3 to 99, which denotes the total number of coupling cycles performed to obtain the n-th cycle oligonucleotide O-n, and each individual coupling cycle comprising the following steps (b-x) to (h-x) (as far as present) is identified by a serial number x, which runs in steps of 1 from 3 to n:

| | |
|---|---|
| (b-x) | incubating the (x-1)-th cycle oligonucleotide O-(x-1) obtained in the previous coupling cycle with a deprotection mixture M-(b-x), thereby cleaving the protecting group PG-(x-1) from the (x-1)-th cycle oligonucleotide O-(x-1), so as to obtain a (x-1)-th cycle oligonucleotide (O-(x-1))^{#} having a free backbone hydroxyl group; |
| (c-x) | subjecting a solution comprising the (x-1)-th cycle oligonucleotide (O-(x-1))^{#} to one or more aqueous extractions, wherein the organic phase comprises the (x-1)-th cycle oligonucleotide (O-(x-1))^{#}; |
| (d-x) | optionally, reducing the water content of the organic phase comprising the (x-1)-th cycle oligonucleotide (O-(x-1))^{#}; |
| (e-x) | reacting the (x-1)-th cycle oligonucleotide (O-(x-1))^{#} with a building block B-x, wherein said building block B-x is selected from the group consisting of a nucleoside and an oligonucleotide and comprises |
| | - a backbone hydroxyl moiety protected by a protecting group PG-x removable under acidic conditions, and |
| | - a phosphorus moiety covalently bonded via its phosphorus atom to an oxygen atom of the backbone of the building block B-x, under conditions suitable to form a covalent bond between said free backbone hydroxyl group of the (x-1)-th cycle oligonucleotide (O-(x-1))^{#} and the phosphorus atom of said phosphorus moiety of the building block B-x, thereby obtaining a x-th cycle oligonucleotide O-x; |
| (f-x) | optionally, incubating the x-th cycle oligonucleotide O-x with an oxidizing or sulfurizing agent, thereby converting any P (III) atoms within said x-th cycle oligonucleotide O-x to P (V) atoms; |
| (g-x) | optionally, subjecting a solution comprising the x-th cycle oligonucleotide O-x to one or more aqueous extractions, wherein the organic phase comprises the x-th cycle oligonucleotide O-x; |
| (h-x) | if step (g-x) has been carried out, optionally reducing the water content of the organic phase comprising the x-th cycle oligonucleotide O-x; |

wherein during and in between steps (b-1) to (h-n) (as far as present) no solid-liquid separation is performed, and steps (c-1), (g-1), (c-2), and (g-2), as well as each iteration of steps (c-x) and (g-x) is carried out in the presence of one or more amide solvents S^{A}, wherein each amide solvent S^{A} is an amide solvent comprising one or more alkyl groups, wherein these one or more alkyl groups together comprise in total 6-48 or 6-24 or 6-16 or 6-15 carbon atoms.

In some embodiments of the method of the invention, the target oligonucleotide O^{T} comprises a n-th cycle oligonucleotide O-n, and the method further comprises performing performing (n-2) iterations of a coupling cycle comprising the above steps (b-x) to (h-x) (as far as present), wherein n is an integer in the range of 3 to 99, which denotes the total number of coupling cycles performed to obtain to obtain the n-th cycle oligonucleotide O-n, and each individual coupling cycle comprising the above steps (b-x) to (h-x) (as far as present) is identified by a serial number x, which runs in steps of 1 from 3 to n, wherein:
no solid-liquid separation of the n-th cycle oligonucleotide O-n or of any oligonucleotidic educts or intermediates involved in the synthesis of the n-th cycle oligonucleotide O-n is performed during and in between steps (b-1) to (h-n) (as far as present), and steps (c-1), (g-1), (c-2), and (g-2), as well as each iteration of steps (c-x) and (g-x) is carried out in the presence of one or more amide solvents S^{A}, wherein each amide solvent S^{A} is an amide solvent comprising one or more alkyl groups, wherein these one or more alkyl groups together comprise in total 6-48 or 6-24 or 6-16 or 6-15 carbon atoms. It will be readily understood in this context that, e.g. any previous cycle oligonucleotide is an oligonucleotidic educt or intermediate involved in the synthesis of the n-th cycle oligonucleotide O-n.

As used in the context of the present invention, expressions like "target oligonucleotide O^{T}" may be understood synonymously with "target oligonucleotide (O^{T})". This means "O^{T}" and "(O^{T})" may be understood as reference marks, which do not imply any further limitation, unless indicated differently. The same applies for similar expressions such as the first cycle oligonucleotides O-1 and (O-1)^{#}, the second cycle oligonucleotides O-2 and (O-2)^{#}, the (x-1)-th cycle oligonucleotides O-(x-1) and (O-(x-1))^{#}, the x-th cycle oligonucleotides O-x and (O-x)^{#}, and the n-th cycle oligonucleotides O-n and (O-n)^{#}. The number sign (i.e. hashtag, #) in superscript, i.e. "^{#}" is merely used to denote the absence of the protecting group PG-0, PG-1, PG-2, PG-(x-1), PG-x, or PG-n and thus the presence of a free backbone hydroxyl group. The numbers or integers 1, 2, (x-1), x, and n are merely used to denote the coupling cycle, in which the respective oligonucleotide is obtained or employed. The deprotection mixtures are referred to by a reference mark "M" followed by an indicator of the step in which the respective deprotection mixture is used. For example, the deprotection mixture used in step (b-1) is referred to as deprotection mixture M-(b-1). The same rationale applies to the deprotection mixtures M-(b-2) (used in step (b-2)), M-(b-x) (used in step (b-x)), M-(b-n) (used in step (b-x) of the last coupling cycle comprising steps (b-x) to (h-x), as far as present), M-(k-1) (used in step (k-1)), M-(k-2) (used in step (k-2)), and M-(k-n) (used in step (k-n)). In the building block B-1, "B" is a mere reference mark and "1" indicates the number of the coupling cycle in which said building block is used, e.g. the first coupling cycle in the case of B-1. The same rationale applies to the building blocks B-2, B-x, and B-n. The component C-0 is a specific component (a nucleoside or oligonucleotide) used as starting material in the method of the invention. Since it is provided in step (a-1), which is performed prior to the first coupling cycle, a "0" has been added to its reference mark. This is not to be construed to be limiting in any kind. The component C is also a specific component (a nucleoside or oligonucleotide). In some embodiments, the component C is structurally identical to the component (C-0)^{#} as defined herein. In such embodiments, any definitions and explanations provided for the component (C-0)^{#} may likewise apply to the component C.

As used herein, the indefinite articles "a" and "an" may mean "one or more", unless indicated differently.

As used herein, the term "target oligonucleotide O^{T}" refers to any specific oligonucleotide which is to be synthesized by the method of the invention. In other words, the "target oligonucleotide O^{T}" is generally the final oligonucleotide product of the method of the invention. For example, the "target oligonucleotide O^{T} may have the same sequence as the n-th cycle oligonucleotide O-n, or may comprise one or more further nucleoside moieties, and/or may be obtained by conjugating the n-th cycle oligonucleotide O-n to another compound.

Herein, the term "oligonucleotide" is used in a most general way to relate to any oligomers comprising at least two nucleoside subunits interconnected via an internucleosidic linkage group of any one of Formulae A and B: 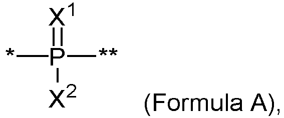 wherein in Formula A:
X¹ is selected from the group consisting of O and S;
X² is selected from the group consisting of O-R¹, S-R¹, and H; and
R¹ may be any conceivable residue, and is preferably selected from the group consisting of H and a protecting group (preferably a protecting group removable under alkaline conditions, in particular the 2-cyanoethyl group, i.e. CH₂-CH₂-CN)); 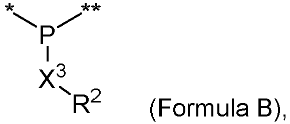 wherein in Formula B:
   X³ is selected from the group consisting of O and S; and
   R² is a protecting group, preferably a protecting group removable under alkaline conditions, in particular the 2-cyanoethyl group; and
   wherein in Formulae A and B each of * and ** independently of each other denotes a binding site (i.e. the point of attachment) of a nucleoside subunit, which is to say that each of * and ** represents the atom of the respective nucleoside subunit with which said nucleoside subunit binds to the internucleosidic linkage group, wherein said atom represented by * and said atom represented by ** are both O.

It will be understood that the internucleosidic linkage groups of Formulae A or B may be protonated (e.g. at a carbonyl or thiocarbonyl group) or deprotonated (e.g. hydroxyl or sulfhydryl groups may be deprotonated), without this being indicated specifically in Formulae A and B. Likewise, Formulae A and B will be understood to embrace any salts, stereoisomeric and tautomeric forms of the respective internucleosidic linkage groups, without this being indicated specifically in Formulae A and B.

Internucleosidic linkage groups, also referred to as internucleoside linkage groups or linkage groups, may be classified depending on the oxidation state of the respective phosphorus (P) atom. Internucleosidic linkage groups typically comprise a P (III) atom or a P (V) atom. Throughout this text, the terms P (III) or phosphorus (III) atom are used interchangeably to denote a P atom of a certain oxidation state, namely with the oxidation state III (i.e. +3). Along the same lines, the terms P (V) or phosphorus (V) atom are used interchangeably to denote a P atom of a certain oxidation state, namely with the oxidation state V (i.e. +5). Internucleosidic linkage groups whose P atom is a P (III) atom are herein referred to as P (III) or phosphorus (III) linkage groups. Internucleosidic linkage groups whose P atom is a P (V) atom are herein referred to as P (V) or phosphorus (V) linkage groups.

Examples of P (V) linkage groups are:
- phosphate diester (i.e. a phosphodiester) groups (i.e. groups of Formula A with X¹ being O and X² being OH),
- thiophosphate diester (i.e. a phosphorothioate) groups (i.e. groups of Formula A with X¹ being S and X² being OH),
- dithiophosphate diester (i.e. a phosphorodithioate) groups (i.e. groups of Formula A with X¹ being S and X² being SH),
- phosphate triester groups (i.e. groups of Formula A with X¹ being O and X² being OR¹, where R¹ is not H, and preferably is a protecting group such as the 2-cyanoethyl group),
- thiophosphate triester groups (i.e. groups of Formula A with X¹ being S and X² being OR¹, where R¹ is not H, and preferably is a protecting group such as the 2-cyanoethyl group), and
- dithiophosphate triester groups (i.e. groups of Formula A with X¹ being S and X² being SR¹, where R¹ is not H, and preferably is a protecting group such as the 2-cyanoethyl group).

Examples of P (III) linkage groups are:
- phosphite triester groups (i.e. groups of Formula B with X³ being O),
- thiophosphite triester groups (i.e. groups of Formula B with X³ being S), and
- H-phosphonate diester groups (i.e. groups of Formula A with X¹ being O and X² being H).

It will be understood by those skilled in the art, that, if an oligonucleotide comprises more than one internucleosidic linkage groups, these internucleosidic linkage groups may be the same or different (i.e. have the same or different chemical structures). An oligonucleotide may also comprise one or more P(III) linkage groups and one or more P(V) linkage groups. As known to the skilled artisan, internucleosidic linkage groups may be modified in the course of oligonucleotide synthesis. For example, P(III) linkage groups may be converted to P (V) linkage groups by incubation with an oxidizing or sulfurizing agent, and/or protecting groups may be removed.

Herein, the term "substituent" may be understood in the broadest sense and may denote any chemical residue or moiety. Thus, herein, unless indicated differently, the terms "substituent", "residue" and "moiety" may be used interchangeably.

The term "group" is also used to denote certain substituents (i.e. residues or moieties). However, the terms "substituent", "moiety" or "residue" may be broader than the term "group" in the cases laid out below:
Herein, the term hydroxyl group refers to a OH residue. In other words, a hydroxyl group is always a free hydroxyl group. However, the term hydroxyl moiety additionally encompasses a hydroxyl residue, which results from a reaction of a hydroxyl group with another chemical group, e.g. a hydroxyl residue involved in an internucleosidic linkage group or a hydroxyl residue bound to a protecting group. The expressions sulfhydryl group, SH, and sulfhydryl moiety are used analogously. Herein, the terms amino group and amine group are used interchangeably to denote a substituent bonded to the respective chemical structure via a nitrogen atom, wherein said nitrogen atom is further bonded to two or three residues selected from the group consisting of H, alkyl, heteroalkyl, alkenyl, heteroalkenyl, alkynyl, heteroalkynyl, aryl, and heteroaryl. These residues may be further substituted and may be further specified, for example when denoting the amino group as di(C₁-C₆-alkyl)amino group (e.g. N(C₁-C₆-alkyl)₂). Said di(C₁-C₆-alkyl)amino group may also be a cyclic amino group in which formally two alkyl residues are bonded to each other to form a cyclic structure. Examples of such cyclic amino groups comprise a pyrrolidine group and a piperidine group. The term amine moiety additionally encompasses an amine residue, which results from a reaction of an amine group with another chemical group, e.g. an amine residue involved in a bond to a protecting group.

As used herein, the term "optionally substituted" may be understood in the broadest sense to mean that in the respective structure, which is optionally substituted, one or more hydrogen residues may optionally and independently of each other be substituted by another residue, also referred to as substituent. If a "substituent" (i.e. residue or moiety) is not further specified it may be any conceivable stable atom or atom group and may preferably be selected from the group consisting of an alkyl residue, O-alkyl, a halogen residue (F, Cl, Br, I), a cyano (i.e. CN) residue, a heteroalkyl residue, an alkenyl residue, a heteroalkenyl residue, an alkynyl residue, a heteroalkynyl residue, an aryl residue, a heteroaryl residue, a ketone residue (in particular C(O)alkyl), an aldehyde residue (CHO), a carboxylic acid residue or ester (in particular C(O)O-alkyl) or amide thereof, an amine group or moiety, a boryl residue (i.e. a substituent bonded via a B atom), a silyl residue (i.e. a substituent bonded via a silicon atom), a hydroxyl group or moiety, a sulfhydryl group or moiety, and combinations thereof. Unless indicated differently, these substituents may be further substituted, i.e. one or more hydrogen atoms in these substituents may be substituted by further substituents.

As used throughout the present invention, the term "alkyl" may be understood in the broadest sense and may be used to denote any aliphatic group (in other words: residue, moiety or substituent) which is composed of atoms of the chemical elements carbon (C) and hydrogen (H), but does not comprise any heteroatoms. Additionally, an alkyl group as defined herein may be characterized in that it comprises at least one carbon atom and in that the one or more carbon atoms may only be bonded to each other via direct single bonds. Preferably, unless indicated differently in the context of specific embodiments, an alkyl group may comprise in total 1 to 40 carbon atoms (i.e. in total not less than 1 and not more than 40 carbon atoms). Unless stated differently, the term "alkyl" may generally embrace unbranched, branched, and cyclic groups. Non-limiting examples of alkyl groups comprise methyl, ethyl, *n*-propyl (propane-1-yl), isopropyl (propane-2-yl), *n*-butyl (butan-1-yl), *sec*-butyl (butan-2-yl), *tert*-butyl (2-methylpropan-2-yl), isobutyl (2-methylpropan-1-yl), cyclohexyl, cyclopentyl, and longer alkyl chains, in particular in pseudo solid-phase protecting groups, e.g. the pseudo solid-phase protecting group PG-s and amide solvents S^{A}.

As used throughout the present invention, the term "heteroalkyl" may be understood in the broadest sense and may be used to denote any alkyl group, in which one or more carbon or hydrogen atoms are substituted by a heteroatom, with the proviso that a heteroalkyl group must comprise at least one carbon atom. A heteroatom in this context may be any atom of a chemical element other than carbon and hydrogen, and, unless indicated differently in the context of specific embodiments, may preferably be an atom which is at each occurrence independently selected from the group consisting of N (nitrogen), O, (oxygen), S (sulfur), P (phosphorus), F (fluorine), Cl (chlorine), Br (bromine), I (iodine), and Si (silicon). Preferably, unless indicated differently in the context of specific embodiments, a heteroalkyl group may comprise in total 1 to 40, 1 to 19, 1 to 6, or 1 to 5 carbon atoms. Unless stated differently, the term "heteroalkyl" may generally embrace unbranched, branched, and cyclic groups. Non-limiting examples of heteroalkyl groups comprise halogenated alkyl groups such as trifluoromethyl groups, oxygenated alkyl groups (i.e. alkyloxy groups), aminated alkyl groups (i.e. alkylamine groups, e.g. dimethylamine groups), and cyclic groups such as e.g. piperidine groups, pyrrolidine groups or morpholine groups. A heteroalkyl-substituent may typically be bonded via one or more of its carbon atoms.

As used throughout the present invention, the term "alkenyl" may be understood in the broadest sense and may be used to denote any aliphatic group which may be derived from an alkyl group by introducing one or more C=C-double bonds between carbon atoms. The term "heteroalkenyl" may be understood in the broadest sense and may be used to denote any aliphatic group which may be derived from a heteroalkyl group by introducing one or more C=C-double bonds between carbon atoms. Herein, unless stated differently in the context of specific embodiments, the terms "alkenyl" and "heteroalkenyl" generally embrace both, the (E)- and the (Z)-isomers, and mixtures thereof. As used throughout the present invention, the term "alkynyl" may be understood in the broadest sense and may be used to denote any aliphatic group which may be derived from an alkyl group by introducing one or more C=C-triple bonds between carbon atoms. The term "heteroalkynyl" may be understood in the broadest sense and may be used to denote any aliphatic group which may be derived from a heteroalkyl group by introducing one or more C=C-triple bonds between carbon atoms. It will be understood that an alkenyl group, a heteroalkenyl group, an alkynyl group, and a heteroalkynyl group comprise at least two carbon atoms.

As used throughout the present invention, the term "aryl" may be understood in the broadest sense and may be used to denote any mono- or polycyclic aromatic group, with the proviso that all aromatic ring atoms are carbon atoms. Unless indicted differently in the context of specific embodiments, an aryl group may preferably comprise in total 6 to 30, 6 to 20, or 6 to 15, in particular 6, aromatic carbon atoms. Benzene (i.e. the phenyl group, Ph) may be a preferred example of a monocyclic aromatic (i.e. aryl) group. It will be understood by those skilled in the art that the expression "polycyclic" in this regard may refer to condensed aromatic ring systems in which two or more aromatic rings are fused together. This is to say that the condensed aromatic rings share at least one bond between aromatic carbon atoms, so that this shared bond as well as the carbon atoms forming the bond are part of two or more monocyclic aromatic groups which build up the polycyclic aromatic group. Non-limiting examples of polycyclic aromatic (i.e. aryl) groups are naphthalene (i.e. napthyl), anthracene (i.e. anthracenyl), and phenanthrene (i.e. phenanthryl).

As used throughout the present invention, the term "heteroaryl" may be understood in the broadest sense and may be used to denote any mono- or polycyclic heteroaromatic group, which differs from an aromatic (i.e. aryl) group in that at least one, preferably 1 to 5 or 1 to 3, aromatic ring atoms are heteroatoms. A heteroatom in this context is any atom of a chemical element other than carbon and hydrogen, and may, unless indicated differently in the context of specific embodiments, preferably be an atom which is at each occurrence independently selected from the group consisting of N, O, and S. Non-limiting examples of monocyclic heteroaromatic (i.e. heteroaryl) groups comprise pyridine (i.e. pyridyl), pyridazine (i.e. pyridazinyl), pyrimidine (i.e. pyrimidinyl), pyrazine (i.e. pyrazinyl), 1,2,3- triazine (i.e. 1,2,3-triazinyl), 1,2,4- triazine (i.e. 1,2,4-triazinyl), 1,3,5- triazine (i.e. 1,3,5-triazinyl), 1,2,3,4- tetrazine (i.e. 1,2,3,4-tetrazinyl), 1,2,4,5- tetrazine (i.e. 1,2,4,5-tetrazinyl), pyrrole (i.e. pyrrolyl), pyrazole (i.e. pyrazolyl), imidazole (i.e. imidazolyl), 1,2,3-triazole (i.e. 1,2,3-triazolyl), 1,2,4-triazole (i.e. 1,2,4-triazolyl), thiophene (i.e. thiophenyl), and furan (i.e. furanyl). Non-limiting examples of polycyclic heteroaromatic (i.e. heteroaryl) groups comprise quinoline (i.e. quinolinyl), quinazoline (i.e. quinazolinyl), quinoxaline (i.e. quinoxalinyl), benzofuran (i.e. benzofuranyl), benzothiophene (i.e. benzothiophenyl), benzimidazole (i.e. benzimidazolyl), benzothiazole (i.e. benzothiazolyl), benzoxazole (i.e. benzoxazolyl), dibenzofuran (i.e. dibenzofuranyl), and acridine (i.e. acridinyl).

As used herein, the term "aliphatic" may be used to denote any residues or compounds which are not aromatic, i.e. which do not comprise an aromatic or heteroaromatic ring system. It is understood that such aliphatic compounds may be unbranched, branched or cyclic, unless indicated differently in the context of specific embodiments. For example, an aliphatic cyclic amine moiety may, e.g., be a piperidine moiety, a pyrrolidine moiety or a morpholine moiety, but may not be a pyridine moiety, which instead is a heteroaromatic amine moiety or, more general, a heteroaryl moiety or heteroaromatic moiety. Non-limiting examples of aliphatic moieties are aliphatic hydrocarbon groups. As used herein, the term "aliphatic hydrocarbon" may be understood in the broadest sense and may be used to denote any aliphatic residue which is composed of atoms of the chemical elements carbon (C) and hydrogen (H), but does not comprise any heteroatoms. The term "aliphatic hydrocarbon" embraces alkyl groups, alkenyl groups, and alkynyl groups as defined herein, unless indicated differently in the context of specific embodiments.

In line with common practice, the names of substituents (i.e. residues or moieties and also groups) may herein typically be derived from the chemical name and indicated by the last syllable "-yl". However, herein, this wording may be used interchangeably with the chemical name of the respective chemical atom or atom group as if it was not a substituent, with common abbreviations, and with the element symbol(s) themselves. For example, a CH₃-substituent may also be referred to as methyl or Me, a Cl-substituent may also be referred to as chlorine or simply Cl, a phenyl substituent may also be denoted as Ph, and a cyano group may also be referred to as CN. If a substituent is denoted using element symbols, e.g. CN, O(C₁-C₆-alkyl), C(O)(C₁-C₆-alkyl), C(O)O(C₁-C₆-alkyl) and comprises more than one atom, the point of attachment of the respective residue (i.e. the atom from which the chemical bond to the parent structure originates) is herein generally the atom denoted to the very left, e.g. the carbon atom in CN and the oxygen atom in O(C₁-C₆-alkyl), unless indicated differently. The same rationale applies if a substituent is denoted using generic residues R such as in O-R^{z-1}, where the oxygen atom constitutes the point of attachment of the residue to the parent structure. Herein, unless stated differently in the context of specific embodiments, when referring to a specific substituent in general terms such as "butyl", this wording embraces all isomers having a chemical structure falling under the respective general substituent name such as "butyl", including for example regioisomers (such as *n*-butyl, *sec*-butyl, *tert*-butyl, isobutyl, and the like), diastereomers, and enantiomers, where applicable.

As used throughout the present invention, the number of carbon atoms of residues (also moieties or substituents or groups) is occasionally indicated in the form of subscript numbers, for example in the form of C₁-C₂₄-alkyl or C₆-C₂₀-aryl. These subscript numbers refer to the range of allowable numbers of carbon atoms for the specific residue and in the context of the specific embodiment. When referring to aryl or heteroaryl residues, these subscript numbers refer to aromatic ring carbon atoms. For example, a C₁-C₂₄-alkyl group comprises in total 1 to 24 carbon atoms, but not less than 1 or more than 24 carbon atoms. Along the same lines, a C₆-C₂₀-aryl group comprises in total 6 to 20 aromatic ring carbon atoms, but not less than 6 or more than 20 aromatic ring carbon atoms.

Herein, unless stated differently in the context of specific embodiments, when referring to an oligonucleotide, said oligonucleotide may generally be present as a mixture of isomers, in particular as a mixture of stereoisomers. A person skilled in the art understands that a molecule of an oligonucleotide may (typically) only be present in a single (stereo)isomeric form at a certain point in time. Thus, when referring to an oligonucleotide as optionally being present as a mixture of (stereo)isomers, this may refer to a population of oligonucleotide molecules having essentially the same nucleoside sequence, wherein the molecules of said population may be present in different (stereo)isomeric forms. Thus, said population of oligonucleotide molecules may be a mixture of numerous discrete stereoisomers, or may be enriched in one specific stereoisomeric form, or may essentially consist of molecules of a specific stereoisomeric form.

It will be understood that certain groups, for example OH and SH groups within an oligonucleotide may be deprotonated. Likewise, it will be understood that certain groups, for example amino groups, within an oligonucleotide may be protonated. It will be understood by a person skilled in the art that an oligonucleotide as defined herein may optionally bear any counter ions known in the art, in particular cations such as sodium cations, potassium cations, magnesium cations, ammonium cations as well as in general cations derived from amines such as trimethylamine (TEA), diisopropylamine (DIPEA) or derived from heteroaromatics such as pyridine or collidine and/or anions such as chloride anions, bromide anions, acetate anions, trifluoroacetate anions, carbonate anions, hydrocarbonate anions, phosphate anions, hydrogen phosphate anions, dihydrogen phosphate anions, perchlorate anions, and combinations thereof. Deprotonation or protonation of an oligonucleotide as well as the (non-covalent) attachment of one or more cations and/or anions may for example occur during the synthesis, isolation, and purification of an oligonucleotide. In general, it will be understood by those skilled in the art that an oligonucleotide as defined herein may be non-covalently bonded or associated to/with species with whom the oligonucleotide was contacted in the course of its synthesis, isolation or purification, for example cations and/or anions or residuals of protecting groups as well as traces of one or more cation scavengers, such as, e.g., thiols or silanes. It will also be understood that such non-covalently bonded or associated species will typically not be depicted in chemical formulas, especially generic formulas, referring to the oligonucleotide or oligonucleotides.

Non-limiting examples of oligonucleotides of the invention are 2'-deoxynucleic acids (DNA), ribonucleic acids (RNA), locked nucleic acids (LNA), constrained ethyl nucleic acid analogs (cET), bridged nucleic acids (BNA), tricycloDNA, unlocked nucleic acids (UNA), small interfering RNA (siRNA), microRNA, antisense oligonucleotides (ASO), gapmers, glycerol nucleic acids, phosphorothioate oligonucleotides, phosphorodithioate oligonucleotides, as well as derivatives and analogs thereof, all of which are known to those skilled in the art.

An oligonucleotide preferably is a linear sequence of nucleoside subunits, wherein any two adjacent nucleoside subunits are interconnected by an internucleosidic linkage group as defined above. Such a linear sequence may also be referred to as an oligonucleotide strand. In line with common practice, the number of nucleoside subunits in an oligonucleotide may be denoted by a number (an integer equal to or larger than 2) followed by the syllable "mer" or by using a suitable prefix (e.g. di for 2, tri for 3, and the like). For example, an oligonucleotide comprising exactly two nucleoside subunits may be denoted as a 2mer or 2mer oligonucleotide or a dinucleotide and an oligonucleotide comprising exactly 20 nucleoside subunits may be denoted as a 20mer or a 20mer oligonucleotide.

Such an oligonucleotide strand will comprise a first terminal nucleoside subunit and a second terminal nucleoside subunit, both of which only have exactly one adjacent nucleoside subunit. In case of a dinucleotide (i.e. a 2mer oligonucleotide) the two terminal nucleoside subunits are interconnected by a first and only internucleosidic linkage group. An oligonucleotide comprising more than two nucleoside subunits, will comprise exactly two terminal nucleoside subunits and one or more non-terminal nucleoside subunits, wherein non-terminal nucleoside subunits are characterized in that they have exactly two adjacent (i.e. one antecedent and one following) nucleoside subunits. The term "adjacent" when referring to nucleoside subunits of an oligonucleotide (strand) refers to any two nucleoside subunits which are interconnected by an internucleosidic linkage group.

It is understood that a nucleoside moiety which forms part of an oligonucleotide is herein referred to as a nucleoside subunit of said oligonucleotide. Herein, the term nucleoside moiety embraces both, nucleosides as such and nucleoside subunits of an oligonucleotide as defined herein. The term "nucleoside" (without the addition "moiety" or "subunit") is however only used to denote a mononucleoside, i.e. a nucleoside moiety which is not a nucleoside subunit of an oligonucleotide. The possible chemical structures of nucleoside moieties are known to those skilled in the art.

A nucleoside moiety may be composed of:
- a carbohydrate moiety (in other words: a sugar moiety), preferably a monosaccharide moiety, more preferably a pentose moiety, in particular a ribose moiety (such as typically present in RNA) or a 2'-deoxyribose moiety (such as typically present DNA); and
- a nucleobase,
wherein said carbohydrate moiety and said nucleobase may typically be covalently bonded to each other via a direct covalent bond, typically an N-glycosidic bond. The nature and the chemistry of carbohydrates and nucleobases form part of the common knowledge of those skilled in the art, who are able to select a carbohydrate moiety as well as a nucleobase for a nucleoside moiety depending on the structure of the oligonucleotide to be synthesized.

Herein, unless indicated differently in the context of specific embodiments, the term "nucleoside moiety" may comprise naturally occurring nucleoside moieties as well as non-natural nucleoside moieties, in which the carbohydrate moiety and/or the nucleobase have been chemically modified or in which the nucleobase may even be absent (i.e. an abasic site). Non-limiting examples of naturally occurring nucleoside moieties may comprise adenosine, 2'-deoxyadenosine, guanosine, 2'-deoxyguanosine, cytidine, 2'-deoxycytidine, uridine, (2'-deoxy)thymidine, ribothymidine, inosine, and methylated derivatives thereof, all of which are known to those skilled in the art. Further examples may comprise queuosine, archaeosine, wybutosine, lysidine, and N⁶-threonylcarbamoyladenosine.

The term "derivative" as used herein may be understood in the broadest sense and may refer to a compound obtainable from a first compound (i.e. a parent compound) by means of one or more, preferably one, chemical reaction. As a result, a derivative may differ from the first (parent) compound for example with regards to the substitutional pattern or with regards to the presence or absence of one or more atom, atom groups, functional groups, or protecting groups.

For example, chemical modifications of (non-natural) nucleoside moieties may comprise modifications of the carbohydrate, in particular of the ribose or 2'-deoxyribose moiety. Such carbohydrate-modifications may exemplarily be selected from the group consisting of:
- introducing an O-CH₃ (i.e. O-methyl or OMe) group, an O-CH₂-CH₂-O-CH₃ (i.e. O-methoxyethyl or O-MOE) group or an F-substituent (i.e. a fluorine substituent) at the 2'-carbon atom;
- introducing a methylene or ethylene bridge between the 2'-oxygen and 4'-carbon ("locked" derivatives) of a ribose moiety;
- interconnecting the 3'-carbon atom and the 5'-carbon atom of a ribose or 2'-deoxyribose moiety (e.g. in tricycloDNA, tcDNA);
- replacing the ribose or 2'-deoxyribose moiety by another pentose such as arabinose or a hexose such as mannose; and
- using the L-enantiomer of the carbohydrate instead of the naturally occurring D-enantiomer;
and/or combinations of these modifications.

Herein, the term "nucleobase" encompasses both non-natural nucleobases as well as naturally occurring nucleobases such as adenine, guanine, cytosine, thymine, and uracil. A person skilled in the art knows how to select a non-natural nucleobase suitable to replace a naturally occurring nucleobase in a nucleoside moiety, so that the non-natural nucleobase may still be capable of a specific interaction with a complementary nucleobase. The interaction between complementary nucleobases may be mediated by hydrogen bonds (cf. Watson-Crick base pairing). Non-natural nucleobases may preferably be derivatives of purine or pyrimidine, which are capable of a specific interaction with another nucleobase.

Non-natural nucleoside moieties may for example be formed from a naturally occurring carbohydrate and a non-natural nucleobase or from a non-natural carbohydrate and a naturally occurring nucleobase or from a non-natural carbohydrate and a non-natural nucleobase.

Examples of nucleoside moieties are nucleoside moieties of Formula C: 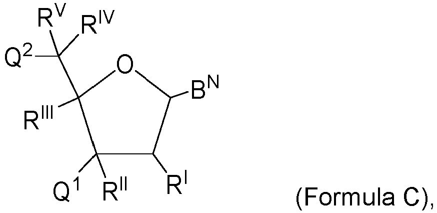 wherein in Formula C:
B^{N} is a nucleobase which may carry one or more protecting groups;
Q¹ is selected from the group consisting of OR³ (if the nucleoside moiety is the 3'-terminal nucleoside moiety of an oligonucleotide or if the nucleoside moiety is a nucleoside) and an oxygen atom covalently bonded to an internucleosidic linkage group (if the respective nucleoside moiety is part of an oligonucleotide and not the 3'-terminal nucleoside moiety thereof);
R³ is selected from the group consisting of H, a protecting group, and a conjugated moiety which is not a nucleoside, nucleotide or oligonucleotide;
Q² is selected from the group consisting of OR⁴ (if the nucleoside moiety is the 5'-terminal nucleoside moiety of an oligonucleotide or if the nucleoside moiety is a nucleoside) and an oxygen atom covalently bonded to an internucleosidic linkage group (if the respective nucleoside moiety is part of an oligonucleotide and not the 5'-terminal nucleoside moiety thereof);
R⁴ is selected from the group consisting of H, a protecting group, and a conjugated moiety which is not a nucleoside, nucleotide or oligonucleotide;
R^{I} is selected from the group consisting of H, F, and OR⁵, where R⁵ may be H, a protecting group, any conceivable substituent, or a conjugated moiety which is not a nucleoside, nucleotide or oligonucleotide;
R^{III} is H or R^{III} and R^{I} are bonded together to form a cyclic structure;
R^{II}, R^{IV}, and R^{V} may independently of each other be any conceivable substituent, wherein two or three of these residues may optionally be bonded together to form a cyclic structure.

As laid out above, unless indicated differently in the context of specific embodiments, an oligonucleotide may herein generally be present as a mixture of isomers. No stereochemical information may be deduced from Formula C. In some preferred embodiments, a nucleoside moiety of Formula C is a nucleoside moiety of the following Formula C-a: 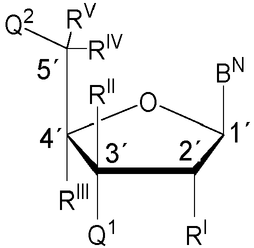 (Formula C-a; carbon atoms labelled from 1' to 5'), wherein in Formula C-a, the carbon atoms have been numbered (in line with common practice) from 1' to 5', which merely serves illustrative purposes and should not be construed to be limiting in any kind. In Formula C-a, Q¹, Q², B^{N}, R^{I}, R^{II}, R^{III}, R^{IV}, R^{V}, R³, R⁴, and R⁵ are defined as for Formula C. It is understood that the carbon atom numbers will be identical in any ribose or 2'-deoxyribose based nucleoside moieties.

From what has been laid out above, it will be understood that any nucleoside subunits comprising a ribose or 2'-deoxyribose moiety, e.g. nucleoside moieties of Formula C and/or C-a, will preferably all be incorporated into an oligonucleotide in 3' → 5' direction or 5' → 3'direction (all in the same direction). Such nucleoside subunits will bond to one (if it is a terminal nucleoside subunit) or two (if it is a non-terminal nucleoside subunit) internucleosidic linkage groups, wherein, preferably, bonding to said internucleosidic linkage group(s) occurs through the 3'-hydroxyl moiety (i.e. the hydroxyl moiety bonded to the 3'-carbon atom) and/or the 5'-hydroxyl moiety (i.e. the hydroxyl moiety bonded to the 5'-carbon atom) exclusively. Since all nucleoside subunits are preferably incorporated in the same direction, any internucleosidic linkage groups between nucleoside subunits comprising a ribose or 2'-deoxyribose moiety are preferably bonded to the 5'-hydroxyl moiety of one nucleoside subunit and the 3'-hydroxyl moiety of another nucleoside subunit. Obviously, the 3'-hydroxyl moiety of the 3'-terminal nucleoside subunit does not engage in bonding to an internucleosidic linkage group. Likewise, the 5'-hydroxyl moiety of the 5'-terminal nucleoside subunit does not engage in bonding to an internucleosidic linkage group.

It is understood from what has been laid out above, that an oligonucleotide comprises two or more nucleoside subunits (nucleoside moieties). It will further be understood that these two or more nucleoside subunits of an oligonucleotide may be the same or different (i.e. have the same or different chemical structures).

As used herein, the term "nucleotide" may be understood in the broadest sense and may preferably refer to a conjugate of a nucleoside moiety and a phosphate group or derivative thereof, wherein a hydroxyl moiety of said nucleoside moiety bonds via its oxygen atom to the phosphorus atom of the phosphate moiety or derivative thereof.

As will be understood by those skilled in the art, the internucleosidic linkage groups and any carbohydrate moieties, e.g. ribose- or 2'-deoxyribose moieties, are herein referred to as the "backbone" of an oligonucleotide. Thus, the term "backbone" of an oligonucleotide excludes the nucleobases. It will also be understood that in a (mono-)nucleoside, the backbone is the carbohydrate moiety, e.g. the ribose- or 2'-deoxyribose moiety, since a nucleoside does not contain any internucleosidic linkage groups.

It forms part of the common knowledge of those skilled in the art that oligonucleotides may be conjugated to additional moieties, which are not a nucleoside, nucleotide or oligonucleotide as defined herein, for various purposes. In particular, free OH-groups (e.g. 3'-OH and/or 5'-OH groups of ribose or 2'-deoxyribose moieties), preferably at the terminal nucleosides of an oligonucleotide strand, may engage in such conjugation. For example, the 5'-OH group of the antisense strand of siRNA (small interfering RNA, known to those skilled in the art) may be modified by a vinyl phosphonate to inhibit cellular degradation and improve potency. A further example is conjugation to N-acetylgalactosamine (GalNAc), which may be of interest for targeted oligonucleotide delivery to hepatocytes. GalNAc structures, often branched to accommodate 3 or 4 GalNAc moieties, may be conjugated, e.g. to the 5'-OH group of an oligonucleotide (cf., e.g., WO2016055601), to the 3'-OH group (cf., e.g., WO2009073809), or monovalent GalNAc moieties may be conjugated via a linker to the 2'-position of subsequent ribose moieties within the oligonucleotide strand (cf., e.g., WO2019075419). For example, the target oligonucleotide O^{T} may be an oligonucleotide conjugate, unless indicated differently in the context of specific embodiments. A used herein, the term "oligonucleotide conjugate" may refer to any oligonucleotide comprising at least one nucleoside-subunit which is covalently bonded to another moiety, which is not a nucleoside, nucleotide or oligonucleotide, e.g. to a moiety comprising a peptide, protein, lipid, carbohydrate, or a hydrocarbon moiety, among which carbohydrate moieties may be preferred. In some embodiments of the method of the invention, the target oligonucleotide O^{T} is not an oligonucleotide conjugate.

The expression "the target oligonucleotide O^{T} comprises a first cycle oligonucleotide O-1" herein means that the target oligonucleotide O^{T} comprises the nucleoside sequence of said first cycle oligonucleotide O-1. The expression "the target oligonucleotide O^{T} comprises a second cycle oligonucleotide O-2" herein means that the target oligonucleotide O^{T} comprises the nucleoside sequence of said second cycle oligonucleotide O-2. The expression "the target oligonucleotide O^{T} comprises a n-th cycle oligonucleotide O-n" herein means that the target oligonucleotide O^{T} comprises the nucleoside sequence of said n-th cycle oligonucleotide O-n.

As used herein, the term "nucleoside sequence" refers to an array of the nucleoside subunits within an oligonucleotide. For a linear oligonucleotide, the nucleoside sequence is usually given starting from a first terminal nucleoside subunit, optionally continuing with one or more non-terminal nucleoside subunits, and ending with a second terminal nucleoside subunit. The nucleoside sequence of an oligonucleotide may be referred to as "sequence" of the oligonucleotide herein. Unless otherwise noted, the sequence of oligonucleotides with a phosphoribose backbone is written herein from the 5' end (left) to the 3' end (right). As used herein, the term "nucleoside sequence" does not specify one or more internucleosidic linkage groups and does not take into account the presence or absence of any protecting groups. Thus, two oligonucleotides comprising the same nucleoside subunits in the same order are herein considered to comprise the same nucleoside sequence, regardless of whether or not the internucleosidic linkage groups interconnecting these nucleoside subunits are the same or not (i.e. have the same chemical structure or not) and regardless of whether or not any atoms or functional groups within the carbohydrate moieties, the nucleobases, and the internucleosidic linkage groups are protected. A pseudo solid-phase protecting group is herein regarded a (permanent) protecting group and thus comprised in the general term "protecting group", unless indicated differently in the context of specific embodiments. For example, the internucleosidic linkage groups may differ with regards to the oxidation state of the phosphorus atom and/or the presence or absence of protecting groups such as the 2-cyanoethyl group. As another example, the exocyclic amino groups of nucleobases such as cytosine, 5-methylcytosine, guanine, and adenine may be protected or may not be protected, and a 5'-terminal hydroxyl moiety may or may not be protected without having any impact on what is herein referred to as the nucleoside sequence.

It will be understood that, unless indicated differently in the context of specific embodiments, the target oligonucleotide O^{T} may comprise more nucleoside subunits than the first cycle oligonucleotide O-1, for example, if the first coupling cycle comprising steps (b-1) to (h-1) (as far as present) is followed by further steps including further coupling cycles. It will be understood that, unless indicated differently in the context of specific embodiments, the target oligonucleotide O^{T} may comprise more nucleoside subunits than the second cycle oligonucleotide O-2, for example, if the second coupling cycle comprising steps (b-2) to (h-2) (as far as present) is followed by further steps including further coupling cycles. It will be understood that, unless indicated differently in the context of specific embodiments, the target oligonucleotide O^{T} may comprise more nucleoside subunits than the n-th cycle oligonucleotide O-n, for example, if said (n-2) iterations of the coupling cycle comprising steps (b-x) to (h-x) (as far as present) are followed by further steps including further coupling cycles. It will be understood that the term "further coupling cycles" embraces fragment coupling approaches, since a fragment coupling may be considered a coupling cycle in which two fragments are coupled.

Additionally, or alternatively, the target oligonucleotide O^{T} may also be a conjugate as defined herein, i.e. covalently bonded to a moiety which is not a nucleoside, nucleotide or oligonucleotide, for example, to a carbohydrate moiety. Such conjugation may, for example, be achieved after the final iteration of the coupling cycle has been carried out.

In some embodiments of the method of the invention, the target oligonucleotide O^{T} consists of the same nucleoside sequence as the first cycle oligonucleotide O-1. In such embodiments, the target oligonucleotide O^{T} may only differ from the first cycle oligonucleotide O-1 with regards to the internucleosidic linkage groups and the absence or presence of protecting groups. In some embodiments of the method of the invention, the target oligonucleotide O^{T} consists of the same nucleoside sequence as the second cycle oligonucleotide O-2. In such embodiments, the target oligonucleotide O^{T} may only differ from the second cycle oligonucleotide O-2 with regards to the internucleosidic linkage groups and the absence or presence of protecting groups. In some embodiments of the method of the invention, the target oligonucleotide O^{T} consists of the same nucleoside sequence as the n-th cycle oligonucleotide O-n. In such embodiments, the target oligonucleotide O^{T} may only differ from the n-th cycle oligonucleotide O-n with regards to the internucleosidic linkage groups and the absence or presence of protecting groups. For example, the target oligonucleotide may preferably only comprise phosphorus (V) linkage groups while the first cycle oligonucleotide O-1, the second cycle oligonucleotide O-2, or the n-th cycle oligonucleotide O-n may comprise one or more phosphorus (III) linkage groups. For example, the target oligonucleotide O^{T} may preferably not comprise any protecting groups, while the first cycle oligonucleotide O-1, the second-cycle oligonucleotide O-2, and the n-th cycle oligonucleotide O-n typically comprise several protecting groups and are at least covalently linked to a pseudo solid-phase protecting group, which is herein considered a (permanent) protecting group.

In some embodiments of the method of the invention, the target oligonucleotide O^{T} comprises 2-200, 2-150, 2-100, 2-90, 2-80, 2-70, 2-60, 2-50, 3-50, 4-50, 5-50, 5-40, 5-30, 5-25, 5-20, 6-20 or 6-18 nucleoside subunits.

As used herein, the term "protecting group" may be understood in the broadest sense as any group which is introduced into a molecule by means of chemical modification (i.e. through one or more, typically one, chemical reaction) of an atom or functional group (i.e. the atom or functional group which is to be protected) and which, once introduced, prevents said atom or functional group from engaging in one or more chemical reactions in subsequent process steps. The terms "protected" and "carrying a protecting group" are herein used interchangeably to denote an atom or functional group covalently bonded to a protecting group. A protecting group used for protecting an amine moiety may herein be referred to as an amine protecting group or amino protecting group. A protecting group used for protecting a hydroxyl moiety may herein be referred to as a hydroxyl protecting group. It will be understood by those skilled in the art that a protecting group may substitute a hydrogen residue from the atom or functional group to be protected. For example, a hydroxyl protecting group may covalently bind to the oxygen atom of the hydroxyl moiety to be protected, thereby substituting the hydrogen residue. As another example, an amine protecting group may covalently bind to the nitrogen atom of the amine moiety to be protected, thereby substituting a hydrogen residue. It will be understood that pseudo solid-phase protecting groups are also protecting groups as defined herein and embraced by the term "protecting group", unless indicated differently in the context of specific embodiments.

Oligonucleotide synthesis typically comprises one or more coupling steps (i.e. condensing steps or condensation steps), during each of which a free (i.e. unprotected) hydroxyl group of a first nucleoside or oligonucleotide is reacted with a phosphorus moiety of second nucleoside or oligonucleotide, which typically comprises a protected backbone hydroxyl moiety. Protection of this backbone hydroxyl moiety is required to avoid double insertion of said second nucleoside or oligonucleotide and/or multimerization. Prior to the next coupling step, this hydroxyl moiety is typicall deprotected to then engage in the next condensation reaction with the phosphorus moiety of a third nucleoside or oligonucleotide, and so on. The (hydroxyl) protecting groups cleaved prior to each condensation reaction (i.e. once per coupling cycle) are commonly referred to as temporary protecting groups. Typically, functional groups (e.g. exocyclic amino groups) in the nucleobases and other functional groups within the carbohydrate (e.g. ribose or 2-deoxyribose) moieties or internucleosidic linkage groups are also protected during chemical oligonucleotide synthesis. However, since these protecting groups are typically only removed once all coupling cycles have been performed, they are commonly referred to as permanent protecting groups. It will be understood that a pseudo solid-phase protecting group preferably is a permanent protecting group, since it shall typically influence the solubility of the growing oligonucleotide strand until it has been fully assembled (i.e. until all desired coupling cycles have been carried out). The temporary and the permanent protecting groups are typically orthogonal to each other, meaning that one type can be removed under conditions, which do not affect the other type of protecting group. Typically, the permanent protecting groups are removable (i.e. cleavable) under alkaline conditions and the temporary protecting groups are removable (i.e. cleavable) under acidic conditions.

Herein, the terms "removing a protecting group", "cleaving a protecting group", and "deprotecting" a functional group are used interchangeably to denote a process, preferably a chemical reaction, of removing a protecting group from an atom or functional group, e.g. from a hydroxyl or amine moiety, so that the latter is again available in free form, e.g. as (free) hydroxyl group or (free) amine group. To expressly denote that a specific atom or functional group, e.g. a hydroxyl group, does not carry a protecting group, the adjective "free" may be used. Herein, the process of removing a protecting group comprising an optionally substituted triarylmethyl residue, e.g. a DMT group, may be referred to as "detritylation". Herein, the terms "cleavage of a protecting group", "removal of a protecting group", and "deprotection" of a functional group are used interchangeably and also refer to a process, preferably a chemical reaction, of removing a protecting group from an atom or functional group.

In the context of the invention, the term "pseudo solid-phase protecting group" refers to a protecting group, preferably a hydroxyl protecting group or an amine protecting group, in particular a hydroxyl protecting group, characterized in that it comprises one or more aliphatic hydrocarbon residues, with the proviso that all aliphatic hydrocarbon residues comprised in a pseudo solid-phase protecting group together comprise in total 18-200 carbon atoms. It will be understood by those skilled in the art, that such pseudo solid-phase protecting groups are typically quite non-polar and soluble in organic solvents commonly used in oligonucleotide synthesis. Their introduction to an otherwise quite polar nucleoside or oligonucleotide may thus increase the solubility of said nucleoside or oligonucleotide in typical organic solvents and decrease the solubility in water or aqueous solutions. Unless indicated differently in specific embodiments, a pseudo solid-phase protecting group is not limited with regard to the atom or functional group which it is bonded to (in other words: which it protects).

As used herein, the term "oligonucleotide synthesis using pseudo solid-phase protecting groups" may be understood in the broadest sense to refer to any oligonucleotide synthesis characterized in that during at least one, preferably each, coupling cycle, a nucleoside or oligonucleotide to be elongated (i.e. which is coupled with another nucleoside or oligonucleotide) is covalently bonded to at least one pseudo solid-phase protecting group.

As used herein, the term "coupling cycle" may be understood in the broadest sense and may refer to a sequence of two or more process steps, including a deprotection step used to provide a free hydroxyl group at a nucleoside or oligonucleotide, and a subsequent coupling step (also referred to as condensation step), in which said free hydroxyl group engages in a bond forming reaction with a phosphorus moiety of another nucleoside or oligonucleotide. Several iterations (i.e. rounds of) coupling cycles may be carried out. During each coupling cycle, an elongated oligonucleotide is obtained. A coupling cycle may be further characterized in aspects and embodiments of the method of the invention. For example, in the method of the invention, the first coupling cycle comprises steps (b-1) to (h-1) (as far as present), i.e. steps (b-1), (c-1), (d-1), (e-1), (f-1), (g-1), and (h-1), which may preferably be carried out in this order, wherein any one of steps (d-1), (f-1), (g-1), and (h-1) is optional (i.e. may or may not be carried out), unless indicated differently in the context of specific embodiments. It will be understood that stating that the first coupling cycle comprises steps (b-1) to (h-1) (as far as present) does not alter the fact that any one of steps (d-1), (f-1), (g-1), and (h-1) is optional. It will be understood that stating that the first coupling cycle comprises steps (b-1) to (h-1) does not alter the fact that any one of steps (d-1), (f-1), (g-1), and (h-1) is optional.

In some embodiments of the method of the invention, the first coupling cycle is followed by a second coupling cycle characterized in that it comprises the steps (b-2) to (h-2) (as far as present), i.e. steps (b-2), (c-2), (d-2), (e-2), (f-2), (g-2), and (h-2) as far as present, which may preferably be carried out in this order, wherein any one of steps (d-2), (f-2), (g-2), and (h-2) is optional (i.e. may or may not be carried out), unless indicated differently in the context of specific embodiments. It will be understood that stating that the second coupling cycle comprises steps (b-2) to (h-2) does not alter the fact that any one of steps (d-2), (f-2), (g-2), and (h-2) is optional.

In some embodiments of the method of the invention, said second coupling cycle comprising steps (b-2) to (h-2) (as far as present) is followed by (n-2) iterations of a coupling cycle comprising steps (b-x) to (h-x) (as far as present), i.e. steps (b-x), (c-x), (d-x), (e-x), (f-x), (g-x), and (h-x), which may preferably be carried out in this order, wherein any one of steps (d-x), (f-x), (g-x), and (h-x) is optional (i.e. may or may not be carried out), unless indicated differently in the context of specific embodiments. It will be understood that said (n-2) iterations of a coupling cycle comprising steps (b-x) to (h-x) (as far as present) need not be identical with regards to which of the optional steps are carried out or are not carried out. For example, one or more of the optional steps may be carried out in one of said (n-2) iterations and may not be carried out in another of said (n-2) iterations.

It will further be understood that a coupling cycle may comprise further steps, which may be inserted before, between, or after the steps specified.

The "n" in the term "(n-2) iterations" is an integer in the range of 3 to 99. In some embodiments of the method of the invention, n is an integer in the range of 3-89, 3-79, 3-69, 3-59, 3-49, 3-39, 3-29, 3-19, 3-18, 3-17, 3-16, 3-15, 3-14, 3-13, 3-12, 3-11, 3-10, 3-9, 3-8, 3-7, 3-6, 3-5, or 3-4. It will be understood that to state that an integer is in the range of 3-5 means that said integer may be 3 or 4 or 5. Said integer n denotes the total number of coupling cycles performed to obtain the n-th cycle oligonucleotide O-n. This total number of performed coupling cycles denoted by the integer n includes the first coupling cycle comprising steps (b-1) to (h-1) (as far as present), the second coupling cycle comprising steps (b-2) to (h-2) (as far as present), as well as the (n-2) iterations of the coupling cycle comprising steps (b-x) to (h-x) (as far as present), but does not include any coupling cycles optionally performed, e.g. to prepare component C-0 or any of the building blocks used. Each coupling cycle comprising steps (b-x) to (h-x) (as far as present) is herein identified by a serial number x, which runs in steps of 1 from 3 to n, with n being said integer representing the total number of coupling cycles performed to obtain the n-th cycle oligonucleotide O-n.

It will be understood that for, e.g. n=3, the method of the invention comprises a total of 3 coupling cycles: The first coupling cycle comprising the steps (b-1) to (h-1) (as far as present), the second coupling cycle comprising the steps (b-2) to (h-2) (as far as present), and (n-2), i.e. 3-2=1, i.e. one, iteration of the coupling cycle comprising the steps (b-x) to (h-x) (as far as present) the latter being numbered with x=3=n. For n=30, the method of the invention comprises a total of 30 coupling cycles: The first coupling cycle comprising the steps (b-1) to (h-1) (as far as present), the second coupling cycle comprising the steps (b-2) to (h-2) (as far as present), and (n-2), i.e. 30-2=28, iterations of the coupling cycle comprising the steps (b-x) to (h-x) (as far as present), the latter being numbered with consecutive integers x running from 3 to 30.

The final iteration of the coupling cycle comprising steps (b-x) to (h-x), (as far as present) is referred to as the the n-th cycle, i.e. x=n. The serial number x is herein used to identify any specific coupling cycle comprising the steps (b-x) to (h-x) (as far as present), and the building blocks employed in, as well as the oligonucleotides obtained from said coupling cycle. It will be understood that, for example, said building block B-x is, e.g., the building block B-5 in the fifth coupling cycle (x=5). It will also be understood that, for example, said x-th cycle oligonucleotide O-x is, e.g., the fifth cycle oligonucleotide O-5 in the fifth coupling cycle (x=5). One will readily understand that, as the first coupling cycle comprising the steps (b-1) to (h-1) (as far as present) as well as the second coupling cycle comprising the steps (b-2) to (h-2) (as far as present) are counted in the total number of coupling cycles, the fifth coupling cycle (x=5) is the third iteration of the coupling cycle comprising the steps (b-x) to (h-x) (as far as present). Thus, the serial number x does not denote the *number of iterations* of the coupling cycle comprising the steps (b-x) to (h-x) (as far as present). Instead, x is simply used to consecutively number the coupling cycles comprising the steps (b-x) to (h-x) (as far as present). In other words: x is used to consecutively number the coupling cycles from the third coupling cycle onwards.

It will further be understood that, because x starts at 3 and runs in steps of 1, the coupling cycle previous to any given coupling cycle x (or x-th cycle) may be referred to as the coupling cycle (x-1) (or (x-1)-th cycle) and the oligonucleotide obtained in this previous coupling cycle may be referred to as the (x-1)-th cycle oligonucleotide O-(x-1). Thus, the x-th coupling cycle will start with removing the protecting group PG-(x-1) from the (x-1)-th-cycle oligonucleotide O-(x-1) of the previous coupling cycle. The x-th cycle oligonucleotide O-x of the coupling cycle x will then be obtained by reacting the deprotected (x-1)-th cycle oligonucleotide (O-(x-1))^{#} with the building block B-x. For example, in the third coupling cycle, the (x-1)-th cycle oligonucleotide O-(x-1) will be the second cycle oligonucleotide O-2 obtained in the second coupling cycle. As another example, in the fourth coupling cycle, the (x-1)-th cycle oligonucleotide O-(x-1) will be the third cycle oligonucleotide O-3 obtained in the third coupling cycle. It will further be understood that the n-th cycle is the final coupling cycle yielding the n-th cycle oligonucleotide O-n.

Step (a-1) of the method of the invention is not part of a coupling cycle and is carried out prior to the first coupling cycle comprising steps (b-1) to (h-1) (as far as present).

Step (a-1) of the method of the invention is: providing a component C-0 selected from the group consisting of a nucleoside and an oligonucleotide, wherein the component C-0 is covalently bonded to a pseudo solid-phase protecting group PG-s and comprises a backbone hydroxyl moiety protected by a protecting group PG-0 removable under acidic conditions.

The terms "nucleoside" and "oligonucleotide" have been explained above. In some embodiments of the method of the invention, the component C-0 is a nucleoside. In some embodiments of the method of the invention, the component C-0 is an oligonucleotide. In some embodiments of the method of the invention, the component C-0 is an oligonucleotide comprising 2-50, 2-40, 2-30, 2-25, 2-20, 2-19, 2-18, 2-17, 2-16, 2-15, 2-14, 2-13, 2-12, 2-11, 2-10, 2-9, 2-8, 2-7, 2-6, or 2-5 nucleoside subunits. In some embodiments of the method of the invention, the component C-0 is an oligonucleotide comprising 5-50, 5-40, 5-30, 5-25, 5-20, 5-19, 5-18, 5-17, 5-16, 5-15, 5-14, 5-13, 5-12, 5-11, or 5-10 nucleoside subunits. In some preferred embodiments of the invention, the component C-0 is an oligonucleotide comprising equal to or more than 5 nucleoside subunits, in particular 5-18 nucleoside subunits or 5-17 nucleoside subunits.

The term "backbone hydroxyl moiety" refers to a hydroxyl moiety which is part of the backbone of the component C-0 and will be understood based on the above explanations of the terms "hydroxyl moiety" and "backbone". Preferably, said hydroxyl moiety is a hydroxyl moiety of a carbohydrate moiety, in particular of an optionally substituted and/or protected ribose or 2'-deoxyribose moiety. Preferably, said backbone hydroxyl moiety is comprised in a terminal nucleoside subunit of the component C-0. In some preferred embodiments, the component C-0 comprises exactly one protecting group PG-0.

The protecting group PG-0 is a protecting group "removable under acidic conditions". As used herein, a protecting group is "removable under acidic conditions", if it can be cleaved by treatment with a protic acid. As used herein, the term "protic acid" may be understood in the broadest sense and may refer to any Bronsted acid, also referred to as Brønsted-Lowry acid. The terms "protic acid" and "Brønsted acid" (or "Brønsted-Lowry acid") are well-known to those skilled in the art.

Preferred examples protecting groups removable under acidic conditions are protecting groups comprising an optionally substituted triarylmethyl residue. As used herein, the two interchangeable terms "protecting group comprising an optionally substituted triarylmethyl residue" and "triarylmethyl type protecting group" may be understood in the broadest sense as any protecting group which covalently binds to the atom or functional group to be protected (e.g. to the oxygen atom of the hydroxyl moiety to be protected) via a carbon atom to which three optionally substituted aryl moieties are bonded. The basic triarylmethyl type protecting group is the triphenylmethyl group (i.e. the trityl group). As known to those skilled in the art, substituents, e.g. alkyl or alkoxy substituents, capable of stabilizing a triarylmethyl-cation formed during acid-catalyzed removal of a triarylmethyl type protecting group, may facilitate acid-catalyzed cleavage of the respective protecting group. Examples of triarylmethyl type protecting groups are the trityl group, the (p-methylphenyl)diphenylmethyl group (i.e. the 4-methyltrityl group), the di(p-methylphenyl)phenylmethyl group (i.e. the 4,4'-dimethyltrityl group), the tri(p-methylphenyl)methyl group (i.e. the 4,4',4"-trimethyltrityl group), the (p-methoxyphenyl)diphenylmethyl group (i.e. the MMT group), the di(p-methoxyphenyl)phenylmethyl group (i.e. the DMT or DMTr group), the tri(p-methoxyphenyl)methyl group (i.e. the TMT group), the 4,4'-dimethoxy-3"-[N-(imidazolylmethyl)]trityl group (i.e. the IDT group), the 4,4'-dimethoxy-3"-[N-(imidazolylethyl)carbamoyl]trityl group (i.e. the IET group), the bis(4-methoxyphenyl)-1'-pyrenylmethyl group (i.e. the Bmpm group), and the 4-(17-tetrabenzo[a,c,g,i]fluorenylmethyl)-4',4"-dimethoxytrityl group (i.e. the Tbf-DMT group). The trityl group, the MMT group, and the DMT group may be preferred. The DMT group is herein most preferred as temporary hydroxyl protecting group. As used herein, the terms DMT and DMTr are used interchangeably to denote a di(p-methoxyphenyl)phenylmethyl group.

It will be understood that said protecting group PG-x may be the same or different (i.e. have the same or a different chemical structure) in each iteration x of the coupling cycle, unless indicated differently in the context of specific embodiments.

In some embodiments of the method of the invention, said protecting group PG-0 is a protecting group comprising an optionally substituted triarylmethyl residue. In some embodiments of the method of the invention, said protecting group PG-0 is selected from the group consisting of the triphenylmethyl group (i.e. the trityl group), the (*p*-methylphenyl)diphenylmethyl group (i.e. the 4-methyltrityl group), the di(*p*-methylphenyl)phenylmethyl group (i.e. the 4,4'-dimethyltrityl group), the tri(*p*-methylphenyl)methyl group (i.e. the 4,4',4"-trimethyltrityl group), the (*p*-methoxyphenyl)diphenylmethyl group (i.e. the MMT group), and the di(*p*-methoxyphenyl)phenylmethyl group (i.e. the DMT group). In some embodiments of the method of the invention, said protecting group PG-0 is selected from the group consisting of the triphenylmethyl group, the (*p*-methoxyphenyl)diphenylmethyl group, and the di(*p*-methoxyphenyl)phenylmethyl group. In some embodiments of the method of the invention, said protecting group PG-0 is a di(*p*-methoxyphenyl)phenylmethyl group. In some preferred embodiments, the component C-0 comprises exactly one backbone hydroxyl moiety protected by a protecting group comprising an optionally substituted triarylmethyl residue, which is said protecting group PG-0. In some preferred embodiments, the component C-0 comprises exactly one protecting group comprising an optionally substituted triarylmethyl residue, which is said protecting group PG-0.

The term "pseudo solid-phase protecting group" has been defined above and will be exemplified in more detail in a later section of this text. The expression "the component C-0 is covalently bonded to a pseudo solid-phase protecting group PG-s" means that there are one or more, preferably exactly one, covalent chemical bonds interconnecting the component C-0 and said pseudo solid-phase protecting group PG-s. It will be understood that the name "PG-s" has been assigned for illustrative purposes only and should not be construed to be limiting in any kind, unless indicated differently in the context of specific embodiments. In some embodiments of the method of the invention, the component C-0 is covalently bonded to exactly one pseudo solid-phase protecting group, which is said pseudo solid-phase protecting group PG-s.

As stated above, a pseudo solid-phase protecting group as defined herein, which includes said pseudo solid-phase protecting group PG-s, is not limited with regard to the atom or functional group of the respective nucleoside moiety to which it is covalently bonded, unless indicated differently in the context of specific embodiments. For example, a pseudo solid-phase protecting group, e.g. said pseudo solid-phase protecting group PG-s, may be covalently bonded to
- a hydroxyl moiety of a carbohydrate moiety of a nucleoside moiety, preferably the 2'- or 3'-, in particular the 3'-hydroxyl moiety, of a ribose- or 2'-deoxyribose-type nucleoside moiety (e.g. of any one of Formulae C and C-a), or
- an amine moiety of a nucleobase (e.g. an exocyclic amine moiety of adenine, guanine, cytosine or 5-methylcytosine or to the endocyclic amine (or rather amide) nitrogen atom in 3-position of thymine or uracil)
of said component C-0 and of said component C.

In some embodiments of the method of the invention, said pseudo solid-phase protecting group PG-s is covalently bonded to a hydroxyl moiety of a nucleoside moiety of said component C-0, preferably to the 3'-hydroxyl moiety of an optionally modified ribose or 2'-deoxyribose moiety (e.g. of any one of the aforementioned Formulae C and C-a). In some embodiments of the method of the invention, said pseudo solid-phase protecting group PG-s is covalently bonded to a hydroxyl moiety of a nucleoside moiety of said component C, preferably to the 3'-hydroxyl moiety of an optionally modified ribose or 2'-deoxyribose moiety (e.g., of any one of the aforementioned Formulae C and C-a).

In some embodiments of the method of the invention, the component C-0 is a compound of the following Formula I: 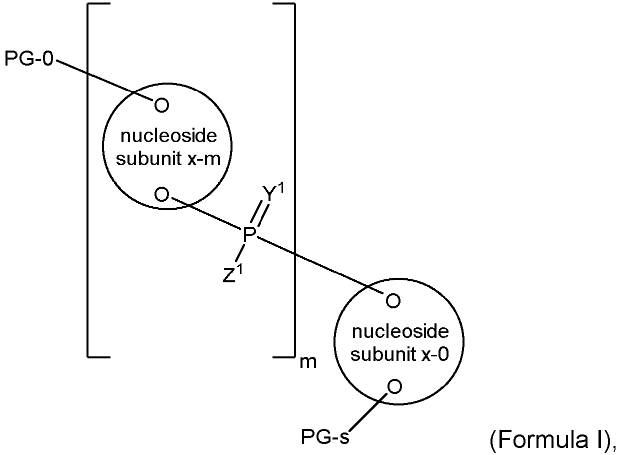 wherein in Formula I:
each oxygen atom (O) depicted within each nucleoside subunit x-0 to x-m represents the oxygen atom of a hydroxyl moiety of the respective nucleoside subunit;
each of the nucleoside subunits x-0 to x-m may be the same or different (i.e. have the same or a different chemical structure);
m is an integer equal to or larger than 0;
PG-0 is a protecting group removable under acidic conditions;
Y¹ is selected independently for each repetitive unit m from the group consisting of O and S;
Z¹ is selected independently for each repetitive unit m from the group consisting of O-R^{z-1}, S-R^{z-1}, and H;
R^{z-1} is a protecting group, which may be the same or different for each repetitive unit m; and
PG-s is a pseudo solid-phase protecting group.

In some embodiments of the method of the invention, the component C-0 of Formula I, is a compound of the following Formula I-a: 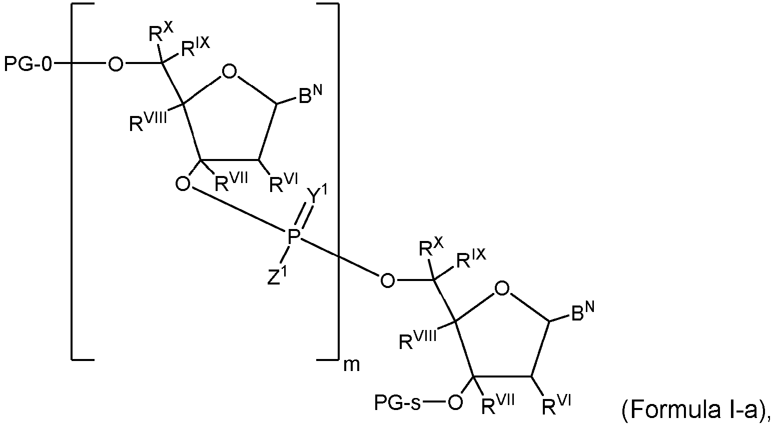 wherein in Formula I-a:
m, Y¹, Z¹, R^{z-1}, PG-0, and PG-s are defined as for Formula I;
B^{N} is a nucleobase, which may be the same or different at each occurrence;
R^{VI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{VIII} is independently at each occurrence H or R^{VIII} and R^{VI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++,
where + is the point of attachment to the 4'-carbon atom (i.e. the carbon atom to which R^{VIII} is bonded) and ++ is the point of attachment to the 2'-carbon (i.e. the carbon atom to which R^{VI} is bonded);
for each nucleoside subunit independently, R^{VII}, R^{IX}, and R^{X} are either all H or they are bonded together so that the respective nucleoside subunit has a structure of the following Formula I-a-tc: 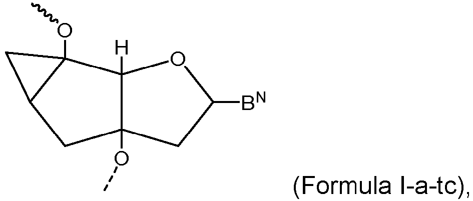
wherein in Formula I-a-tc:
   the oxygen atom from which the dashed line originates represents the oxygen atom bonded to the 3'-carbon atom, i.e. the carbon atom to which R^{VII} is bonded in Formula I-a;
   the dashed line indicates the covalent chemical bond interconnecting the respective oxygen atom of the nucleoside subunit of Formula I-a-tc and the pseudo solid-phase protecting group PG-s or P(Y¹)(Z¹) in Formula I-a;
   the oxygen atom from which the wavy line originates represents the oxygen atom bonded to the 5'-carbon atom, i.e. the carbon atom to which -C(R^{IX})(R^{X}) is bonded in Formula I-a; and
   the wavy line indicates the covalent chemical bond interconnecting the respective oxygen atom of the nucleoside subunit of Formula I-a-tc and either PG-0 or P(Y¹)(Z¹) in Formula I-a.

In some embodiments of the method of the invention, the component C-0 of any one of Formulae **I,** and I-a, is a compound of the following Formula I-b: 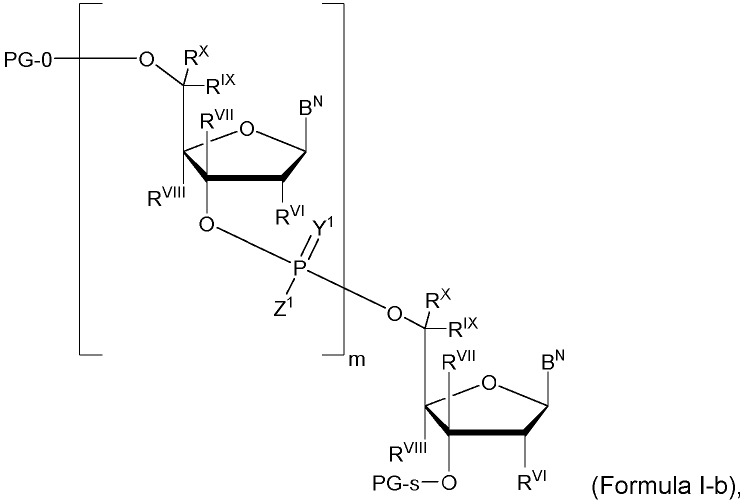 wherein in Formula I-b:
m, PG-0, PG-s, Y¹, Z¹, R^{z-1}, B^{N}, R^{VI}, and R^{VIII} are defined as for Formula I-a, and
for each nucleoside subunit independently, R^{VII}, R^{IX}, and R^{X} are either all H or they are bonded together so that the respective nucleoside subunit has a structure of the following Formula I-b-tc: 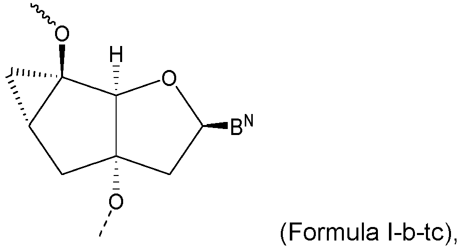
wherein in Formula I-b-tc:
   the oxygen atom from which the dashed line originates represents the oxygen atom bonded to the 3'-carbon atom, i.e. the carbon atom to which R^{VII} is bonded in Formula I-b;
   the dashed line indicates the covalent chemical bond interconnecting the respective oxygen atom of the nucleoside subunit of Formula I-b-tc and the pseudo solid-phase protecting group PG-s or P(Y¹)(Z¹) in Formula I-b;
   the oxygen atom from which the wavy line originates represents the oxygen atom bonded to the 5'-carbon atom, i.e. the carbon atom to which C(R^{IX})(R^{X}) is bonded in Formula I-b; and
   the wavy line indicates the covalent chemical bond interconnecting the respective oxygen atom of the nucleoside subunit of Formula I-b-tc and either PG-0 or P(Y¹)(Z¹) in Formula I-b.

In some embodiments, in the component C-0 of any one of Formulae I, I-a, and I-b, R^{z-1} is a protecting group removable under alkaline conditions, wherein R^{z-1} may be the same or different at each occurrence. As used herein, a protecting group is "removable under alkaline conditions", if it can be cleaved by treatment with a base (wherein "a base" is not to be construed to mean any base, but specific bases adapted to the protecting group to be removed based on the common knowledge of those skilled in the art and routine experimentation). The term "base" may in this context be understood as proton acceptor in the sense of the Brønsted-Lowry theory. Examples of such a base include ammonia, in particular an aqueous solution of ammonia (i.e. an aqueous ammonium hydroxide solution), methylamine, *tert-*butylamine, diethylamine, triethylamine, diisopropylethylamine, alkali hydroxides, and the like. In some embodiments, in the component C-0 of any one of Formulae I, I-a, and I-b, R^{z-1} is for each repetitive unit m independently a protecting group of the chemical structure CH₂-CH₂-EWG, where EWG is an electron withdrawing group, preferably a cyano group. The electron withdrawing group may for example be selected from the group consisting of a cyano group, a halogen atom such as a chlorine, fluorine, or bromine atom, a formyl group, a keto group, a carboxyester group, and a carboxamide group. In some preferred embodiments, in the component C-0 of any one of Formulae I, I-a, and I-b, R^{z-1} is for each repetitive unit m a 2-cyanoethyl group (i.e. CH₂-CH₂-CN).

In some embodiments, in the component C-0 of any one of Formulae I, I-a, and I-b, Z¹ is selected independently for each repetitive unit m from the group consisting of O-R^{z-1} and S-R^{z-1}. In some embodiments, in the component C-0 of any one of Formulae I, I-a, and I-b, Z¹ is selected independently for each repetitive unit m from the group consisting of O-R^{z-1} and S-R^{z-1}, where R^{z-1} is for each repetitive unit m a 2-cyanoethyl group. In such embodiments, Z¹ is selected independently for each repetitive unit m from the group consisting of O-CH₂-CH₂-CN and S-CH₂-CH₂-CN. In some embodiments, in the component C-0 of any one of Formulae I, I-a, and I-b, Z¹ is for each repetitive unit m O-R^{z-1}, where R^{z-1} is for each repetitive unit m a 2-cyanoethyl group. In such embodiments, Z¹ is for each repetitive unit m O-CH₂-CH₂-CN.

In some embodiments, in the component C-0 of any one of Formulae I, I-a, and I-b, Y¹ is for each repetitive unit m O. In some embodiments, in the component C-0 of any one of Formulae I, I-a, and I-b, Y¹ is for each repetitive unit m S.

In some embodiments, in the component C-0 of any one of Formulae I-a, and I-b:
R^{VI} is selected independently at each occurrence from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert*-butyl(dimethyl)silyloxy), and O-CH₂-O-Si(C(CH₃)₃)₃ (i.e. ((triisopropylsilyl)oxy)-methyloxy);
R^{VIII} is independently at each occurrence H or R^{VIII} and R^{VI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{VIII} is bonded) and ++ is the point of attachment to the 2'-carbon (i.e. the carbon atom to which R^{VI} is bonded); and
for each nucleoside subunit independently, R^{VII}, R^{IX}, and R^{X} are either all H or they are bonded together so that the respective nucleoside subunit has a structure of the aforementioned Formula I-a-tc (in a component C-0 of Formula I-a) or Formula I-b-tc (in a component C-0 of Formula I-b).

In some embodiments, in the component C-0 of any one of Formulae I-a, and I-b: R^{VI} is selected independently at each occurrence from the group consisting of H, F, O-CH₃ (i.e. methoxy), and O-CH₂-CH₂-O-CH₃ (i.e. 2-methoxyethyl-1-oxy); and each of R^{VII}, R^{VIII}, R^{IX} , and R^{X} is at each occurrence H.

In some embodiments, in the component C-0 of any one of Formulae I, I-a, and I-b, the integer m is an integer in the range of 0-49, 0-39, 0-29, 0-24, 0-19, 0-18, 0-17, 0-16, 0-15, 0-14, 0-13, 0-12, 0-11, 0-10, 0-9, 0-8, 0-7, 0-6, 0-5 or 0-4. It will be understood that to state than an integer is in the range of e.g. 0-5 means that said integer may be 0, 1, 2, 3, 4, or 5. In some embodiments, in the component C-0 of any one of Formulae I, I-a, and I-b, the integer m is an integer in the range of 4-49, 4-39, 4-29, 4-24, 4-19, 4-18, 4-17, 4-16, 4-15, 4-14, 4-13, 4-12, 4-11, 4-10, or 4-9. In some preferred embodiments, in the component C-0 of any one of Formulae I, I-a, and I-b, the integer m is an integer equal to or larger than 5, in particular an integer in the range of 5-18 or 5-17. In some embodiments, in the component C-0 of any one of Formulae I, I-a, and I-b, the integer m is 0.

The skilled person will understand that any kind of nucleobase B^{N} may be present in the component C-0 of any one of Formulae I, I-a, and I-b. Reference is e.g. made to the explanations relating to the nucleobase given above. In some embodiments, in the component C-0 of any one of Formulae I, I-a, and I-b, B^{N} is a nucleobase and at each occurrence independently selected from the group consisting of adenine, guanine, cytosine, 5-methylcytosine, thymine, and uracil. It will be understood by those skilled in the art, that any nucleobase B^{N} in any one of Formulae I, I-a, and I-b may optionally be protected, i.e. carry one or more protecting groups, without this being indicated specifically. Thus, when, for example, stating that B^{N} is adenine, guanine, cytosine, 5-methylcytosine, thymine, or uracil, this embraces the aforementioned nucleobases in protected form and in free form (i.e. with and without any protecting groups). The same rationale applies to nucleobases in general.

The skilled artisan is familiar with nucleobase protecting groups and knows how to select, introduce, and remove them. In particular, the exocyclic amino groups in nucleobases such as in adenine, guanine, cytosine, and 5-methylcytosine may be protected. Non-limiting examples of protecting groups for exocyclic amino groups of nucleobases comprise the acetyl group, the benzoyl group, the isobutyryl group, the pivaloyl group, the pivaloyloxymethyl group, the trifluoroacetyl group, the phenoxyacetyl group, the 4-isopropylphenoxyacetyl group, the 4*-tert-*butylphenoxyacetyl group, and the dimethylformamidinyl group. Carbonyl groups present in nucleobases such as in thymine, uracil, and guanine may also be protected, for example, by reaction with phenol, 2,5-dichlorophenol, 3-chlorophenol, 3,5-dichlorophenol, 2-formylphenol, 2-naphthol, 4-methoxyphenol, 4-chlorophenol, 2-nitrophenol, 4-nitrophenol, 4-acetylaminophenol, pentafluorophenol, 4-pivaloyloxybenzyl alcohol, 4-nitrophenethyl alcohol, 2-(methylsulfonyl)ethanol, 2-(phenylsulfonyl)ethanol, 2-cyanoethanol, 2-(trimethylsilyl)ethanol, dimethylcarbamoyl chloride, diethylcarbamoyl chloride, ethylphenylcarbamoyl chloride, 1-pyrrolidinecarbonyl chloride, 4-morpholinecarbonyl chloride, diphenylcarbamoyl chloride, and the like. Examples of preferred nucleobase protecting groups are compiled in the following Table T-1.

**Table T-1: Non-limiting overview of preferred nucleobase protecting groups.**

| protecting group | typically used for |
|---|---|
| Bz or bz = benzoyl; iBu or ibu = isobutyryl; PAC = phenoxyacetyl | exocyclic amino group of adenine |
| Ac = acetyl; Bz = benzoyl | exocyclic amino group of cytosine or 5-methylcytosine |
| iBu or ibu = isobutyryl; i-Pr-PAC = 4-isopropylphenoxyacetyl; dimethylformamidino | exocyclic amino group of guanine |

In some embodiments of the method of the invention, each nucleobase of the component C-0, in particular each nucleobase B^{N} of the component C-0 of any one of Formulae I, I-a, and I-b is independently selected from the group consisting of
- adenine, in which the exocyclic amino group is protected;
- guanine, in which the exocyclic amino group is protected;
- cytosine, in which the exocyclic amino group is protected;
- 5-methylcytosine, in which the exocyclic amino group is protected;
- thymine; and
- uracil.

In some embodiments of the method of the invention, each nucleobase of the component C-0, in particular each nucleobase B^{N} of the component C-0 of any one of Formulae I, I-a, and I-b is independently selected from the group consisting of
- adenine, in which the exocyclic amino group is protected by a benzoyl group, an isobutyryl group or a phenoxyacetyl group;
- guanine, in which the exocyclic amino group is protected by an isobutyryl group, a 4-isopropylphenoxyacetyl group or a dimethylformamidino group;
- cytosine, in which the exocyclic amino group is protected by an acetyl group or a benzoyl group;
- 5-methylcytosine, in which the exocyclic amino group is protected by an acetyl group or a benzoyl group;
- thymine; and
- uracil.

In some embodiments, in the component C-0 of Formula I:
m is an integer in the range of 0-49, 0-39, 0-29, 0-24, 0-19, 0-18, 0-17, 0-16, 0-15, 0-14, 0-13, 0-12, 0-11, 0-10, 0-9, 0-8, 0-7, 0-6, 0-5 or 0-4 or in the range of 4-49, 4-39, 4-29, 4-24, 4-19, 4-18, 4-17, 4-16, 4-15, 4-14, 4-13, 4-12, 4-11, 4-10, or 4-9;
PG-0 is a protecting group comprising an optionally substituted triarylmethyl residue, preferably a di(p-methoxyphenyl)phenylmethyl (DMT) protecting group;
Y¹ is selected independently for each repetitive unit m from the group consisting of O and S;
Z¹ is selected independently for each repetitive unit m from the group consisting of O-R^{z-1}, S-R^{z-1}, and H;
R^{z-1} is for each repetitive unit m a 2-cyanoethyl group; and
PG-s is a pseudo solid-phase protecting group.

In some embodiments, in the component C-0 of Formula I:
m is an integer in the range of 0-49, 0-39, 0-29, 0-24, 0-19, 0-18, 0-17, 0-16, 0-15, 0-14, 0-13, 0-12, 0-11, 0-10, 0-9, 0-8, 0-7, 0-6, 0-5 or 0-4 or in the range of 4-49, 4-39, 4-29, 4-24, 4-19, 4-18, 4-17, 4-16, 4-15, 4-14, 4-13, 4-12, 4-11, 4-10, or 4-9;
PG-0 is a protecting group comprising an optionally substituted triarylmethyl residue, preferably a di(p-methoxyphenyl)phenylmethyl (DMT) protecting group;
Y¹ is selected independently for each repetitive unit m from the group consisting of O and S;
Z¹ is selected independently for each repetitive unit m from the group consisting of O-R^{z-1} and S-R^{z-1};
R^{z-1} is for each repetitive unit m a 2-cyanoethyl group; and
PG-s is a pseudo solid-phase protecting group.

In some embodiments, in the component C-0 of Formula I:
m is an integer in the range of 0-49, 0-39, 0-29, 0-24, 0-19, 0-18, 0-17, 0-16, 0-15, 0-14, 0-13, 0-12, 0-11, 0-10, 0-9, 0-8, 0-7, 0-6, 0-5 or 0-4 or in the range of 4-49, 4-39, 4-29, 4-24, 4-19, 4-18, 4-17, 4-16, 4-15, 4-14, 4-13, 4-12, 4-11, 4-10, or 4-9;
PG-0 is a protecting group comprising an optionally substituted triarylmethyl residue, preferably a di(p-methoxyphenyl)phenylmethyl (DMT) protecting group;
Y¹ is selected independently for each repetitive unit m from the group consisting of O and S;
Z¹ is for each repetitive unit m O-R^{z-1};
R^{z-1} is for each repetitive unit m a 2-cyanoethyl group; and
PG-s is a pseudo solid-phase protecting group.

In some embodiments, in the component C-0 of any one of Formulae I-a and I-b:
m is an integer in the range of 0-49, 0-39, 0-29, 0-24, 0-19, 0-18, 0-17, 0-16, 0-15, 0-14, 0-13, 0-12, 0-11, 0-10, 0-9, 0-8, 0-7, 0-6, 0-5 or 0-4 or in the range of 4-49, 4-39, 4-29, 4-24, 4-19, 4-18, 4-17, 4-16, 4-15, 4-14, 4-13, 4-12, 4-11, 4-10, or 4-9;
PG-0 is a protecting group comprising an optionally substituted triarylmethyl residue, preferably a di(p-methoxyphenyl)phenylmethyl (DMT) protecting group;
Y¹ is selected independently for each repetitive unit m from the group consisting of O and S;
Z¹ is selected independently for each repetitive unit m from the group consisting of O-R^{z-1}, S-R^{z-1}, and H;
R^{z-1} is for each repetitive unit m a 2-cyanoethyl group;
PG-s is a pseudo solid-phase protecting group;
B^{N} is a nucleobase, which may be the same or different at each occurrence;
R^{VI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{VIII} is independently at each occurrence H or R^{VIII} and R^{VI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{VIII} is bonded) and ++ is the point of attachment to the 2'-carbon (i.e. the carbon atom to which R^{VI} is bonded); and
for each nucleoside subunit independently, R^{VII}, R^{IX}, and R^{X} are either all H or they are bonded together so that the respective nucleoside subunit has a structure of the aforementioned Formula I-a-tc (in a component C-0 of Fomula I-a) or the aforementioned Formula I-b-tc (in a component C-0 of Fomula I-b).

In some embodiments, in the component C-0 of any one of Formulae I-a and I-b:
m is an integer in the range of 0-49, 0-39, 0-29, 0-24, 0-19, 0-18, 0-17, 0-16, 0-15, 0-14, 0-13, 0-12, 0-11, 0-10, 0-9, 0-8, 0-7, 0-6, 0-5 or 0-4 or in the range of 4-49, 4-39, 4-29, 4-24, 4-19, 4-18, 4-17, 4-16, 4-15, 4-14, 4-13, 4-12, 4-11, 4-10, or 4-9;
PG-0 is a protecting group comprising an optionally substituted triarylmethyl residue, preferably a di(p-methoxyphenyl)phenylmethyl (DMT) protecting group;
Y¹ is selected independently for each repetitive unit m from the group consisting of O and S;
Z¹ is selected independently for each repetitive unit m from the group consisting of O-R^{z-1}, and S-R^{z-1};
R^{z-1} is for each repetitive unit m a 2-cyanoethyl group;
PG-s is a pseudo solid-phase protecting group;
B^{N} is a nucleobase, which may be the same or different at each occurrence;
R^{VI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{VIII} is independently at each occurrence H or R^{VIII} and R^{VI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{VIII} is bonded) and ++ is the point of attachment to the 2'-carbon (i.e. the carbon atom to which R^{VI} is bonded); and
for each nucleoside subunit independently, R^{VII}, R^{IX}, and R^{X} are either all H or they are bonded together so that the respective nucleoside subunit has a structure of the aforementioned Formula I-a-tc (in a component C-0 of Fomula I-a) or the aforementioned Formula I-b-tc (in a component C-0 of Fomula I-b).

In some embodiments, in the component C-0 any one of Formulae I-a and I-b:
m is an integer in the range of 0-49, 0-39, 0-29, 0-24, 0-19, 0-18, 0-17, 0-16, 0-15, 0-14, 0-13, 0-12, 0-11, 0-10, 0-9, 0-8, 0-7, 0-6, 0-5 or 0-4 or in the range of 4-49, 4-39, 4-29, 4-24, 4-19, 4-18, 4-17, 4-16, 4-15, 4-14, 4-13, 4-12, 4-11, 4-10, or 4-9;
PG-0 is a protecting group comprising an optionally substituted triarylmethyl residue, preferably a di(p-methoxyphenyl)phenylmethyl (DMT) protecting group;
Y¹ is selected independently for each repetitive unit m from the group consisting of O and S;
Z¹ is for each repetitive unit m O-R^{z-1};
R^{z-1} is for each repetitive unit m a 2-cyanoethyl group;
PG-s is a pseudo solid-phase protecting group;
B^{N} is a nucleobase, which may be the same or different at each occurrence;
R^{VI} is selected independently at each occurrence from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. tert-butyl(dimethyl)silyloxy), and O-CH₂-O-Si(C(CH₃)₃)₃ (i.e. ((triisopropylsilyl)oxy)methyloxy); and
each of R^{VII}, R^{VIII}, R^{IX}, and R^{X} is at each occurrence H.

The term "providing a component C-0" in step (a-1) may be understood in the broadest sense to refer to any means of obtaining a component C-0. The component C-0 may, for example, be obtained commercially, in particular, if it is a nucleoside. Alternatively, the component C-0 may be provided by means of chemical synthesis. Such means are well known to the skilled artisan and comprise, for example, solid-phase and liquid-phase oligonucleotide synthesis. As non-limiting examples, the following documents disclose means of providing components C-0 for use in the method of the invention: PCT/EP2022/059528, US2015112053A1, , EP3825300A1, EP2711370A1, EP3398955A1, US2018291056A1, EP3925964A1, EP2921499A1, EP3733680A1, EP3378869A1, WO2020227618A2, EP3263579A1, EP3950698A1, EP3015467A1, M.C. de Koning et al., Organic Process Research & Development 2006, 10, 1238-1245; A. Schwenger et al., European Journal of Organic Chemistry 2017, 5852-5864; S. Kim et al., Chemistry - A European Journal 2013, 19, 8615-8620, US2015080565A1, US2013267697A1,and X. Shi et al., J. Org. Chem. 2022, 87(4), 20872110.

Step (b-1) of the methods of the invention is: incubating the component C-0 of step (a-1) with a deprotection mixture M-(b-1), thereby cleaving the protecting group PG-0 from the component C-0, so as to obtain a component (C-0)^{#} having a free backbone hydroxyl group.

Step (b-2) of some methods of the invention is: incubating the first cycle oligonucleotide O-1 obtained in the first coupling cycle with a deprotection mixture M-(b-2), thereby cleaving the protecting group PG-1 from the first cycle oligonucleotide O-1, so as to obtain a first cycle oligonucleotide (O-1)^{#} having a free backbone hydroxyl group.

Step (b-x) of some methods of the invention is: incubating the (x-1)-th cycle oligonucleotide O-(x-1) obtained in the previous coupling cycle with a deprotection mixture M-(b-x), thereby cleaving the protecting group PG-(x-1) from the (x-1)-th cycle oligonucleotide O-(x-1), so as to obtain a (x-1)-th cycle oligonucleotide (O-(x-1))^{#} having a free backbone hydroxyl group.

The component C-0 and the protecting group PG-0 have been defined. The first cycle oligonucleotide O-1 is obtained in the first coupling cycle. It will be undertood that, since the building block B-1 used to prepare said first cycle oligonucleotide O-1 comprises a backbone hydroxyl moiety protected by a protecting group PG-1 removable under acidic conditions, the first cycle oligonucleotide O-1 also comprises a backbone hydroxyl moiety protected by a protecting group PG-1 removable under acidic conditions. As laid out above, the term (x-1)-th cycle oligonucleotide O-(x-1) refers to the x-th cycle oligonucleotide obtained in the previous coupling cycle, and, in the case of the first coupling cycle comprising steps (b-x) to (h-x) (as far as present) (i.e. x = 3) refers to the second cycle oligonucleotide O-2. It will be understood that, since the building block B-2 used to prepare the second cycle oligonucleotide O-2 comprises a backbone hydroxyl moiety protected by a protecting group PG-2 removable under acidic conditions, the second cycle oligonucleotide O-2 also comprises a backbone hydroxyl moiety protected by a protecting group PG-2 removable under acidic conditions. It will be understood that, since each building block B-x comprises a backbone hydroxyl moiety protected by a protecting group PG-x removable under acidic conditions, each (x-1)-th cycle oligonucleotide O-(x-1) also comprises a backbone hydroxyl moiety protected by a protecting group PG-(x-1) removable under acidic conditions.

The term "removable under acidic conditions" has been defined above in the context of the protecting group PG-0 and likewise applies to all protecting groups PG-1, PG-2, PG-x, PG-(x-1), and PG-n.

In some embodiments of the method of the invention, each of the protecting groups PG-1, PG-2, PG-x, PG-(x-1), and PG-n is independently of each other a protecting group comprising an optionally substituted triarylmethyl residue. In some embodiments of the method of the invention, each of the protecting groups PG-1, PG-2, PG-x, PG-(x-1), and PG-n is independently of each other selected from the group consisting of the triphenylmethyl group (i.e. the trityl group), the (p-methylphenyl)diphenylmethyl group (i.e. the 4-methyltrityl group), the di(p-methylphenyl)phenylmethyl group (i.e. the 4,4'-dimethyltrityl group), the tri(p-methylphenyl)methyl group (i.e. the 4,4',4"-trimethyltrityl group), the (p-methoxyphenyl)diphenylmethyl group (i.e. the MMT group), and the di(p-methoxyphenyl)phenylmethyl group (i.e. the DMT group). In some embodiments of the method of the invention, each of the protecting groups PG-1, PG-2, PG-x, PG-(x-1), and PG-n is independently of each other selected from the group consisting of the triphenylmethyl group, the (p-methoxyphenyl)diphenylmethyl group, and the di(p-methoxyphenyl)phenylmethyl group. In some embodiments of the method of the invention, each of the protecting groups PG-1, PG-2, PG-x, PG-(x-1), and PG-n is a di(p-methoxyphenyl)phenylmethyl group.

In some preferred embodiments, the first cycle oligonucleotide O-1 comprises exactly one backbone hydroxyl moiety protected by a protecting group comprising an optionally substituted triarylmethyl residue, which is said protecting group PG-1. In some preferred embodiments, the first cycle oligonucleotide O-1 comprises exactly one protecting group comprising an optionally substituted triarylmethyl residue, which is said protecting group PG-1. In some preferred embodiments, the second cycle oligonucleotide O-2 comprises exactly one backbone hydroxyl moiety protected by a protecting group comprising an optionally substituted triarylmethyl residue, which is said protecting group PG-2. In some preferred embodiments, the second cycle oligonucleotide O-2 comprises exactly one protecting group comprising an optionally substituted triarylmethyl residue, which is said protecting group PG-2. In some preferred embodiments, each (x-1)-th cycle oligonucleotide O-(x-1) comprises exactly one backbone hydroxyl moiety protected by a protecting group comprising an optionally substituted triarylmethyl residue, which is said protecting group PG-(x-1). In some preferred embodiments, each (x-1)-th cycle oligonucleotide O-(x-1) comprises exactly one protecting group comprising an optionally substituted triarylmethyl residue, which is said protecting group PG-(x-1).

In some preferred embodiments of the method of the invention:
- the component (C-0)^{#} differs from the component C-0 only in that the backbone hydroxyl moiety protected by the protecting group PG-0 in the component C-0 is a free backbone hydroxyl group in the component (C-0)^{#};
- the first cycle oligonucleotide (O-1)^{#} differs from the first cycle oligonucleotide O-1 only in that the backbone hydroxyl moiety protected by the protecting group PG-1 in the first cycle oligonucleotide O-1 is a free backbone hydroxyl group in the first cycle oligonucleotide (O-1)^{#};
- the second cycle oligonucleotide (O-2)^{#} differs from the second cycle oligonucleotide O-2 only in that the backbone hydroxyl moiety protected by the protecting group PG-2 in the second cycle oligonucleotide O-2 is a free backbone hydroxyl group in the second cycle oligonucleotide (O-2)^{#};
- each (x-1)-th cycle oligonucleotide (O-(x-1))^{#} differs from the respective (x-1)-th cycle oligonucleotide O-(x-1) only in that the backbone hydroxyl moiety protected by a protecting group PG-(x-1) in the (x-1)-th cycle oligonucleotide O-(x-1) is a free backbone hydroxyl group in the (x-1)-th cycle oligonucleotide (O-(x-1))^{#}*.*

The term "free backbone hydroxyl group" refers to a free hydroxyl group, which is part of the backbone of the respective nucleoside or oligonucleotide.

The term "deprotection mixture M-(b-1)" refers to any mixture which may be used to effect cleavage of the protecting group PG-0 from the component C-0. The term "deprotection mixture M-(b-2)" refers to any mixture which may be used to effect cleavage of the protecting group PG-1 from the first cycle oligonucleotide O-1. The term "deprotection mixture M-(b-3)" refers to any mixture which may be used to effect cleavage of the protecting group PG-2 from the second cycle oligonucleotide O-2. The term "deprotection mixture M-(b-x)" refers to any mixture which may be used to effect cleavage of the protecting group PG-(x-1) from the (x-1)-th cycle oligonucleotide O-(x-1). It will be understood that said deprotection mixture M-(b-x) may be the same or different (i.e. comprise the same or different components in the same or different ratios and/or concentrations) between different iterations of step (b-x).

Per definition, each of the protecting groups PG-0, PG-1, PG-2, PG-(x-1), PG-x, and PG-n is a protecting group "removable under acidic conditions", which has been explained above to mean that each of these protecting groups can be cleaved (i.e. removed) by treatment with a protic acid, also referred to as Brønsted-Lowry acid.

It is known to the skilled artisan that the acid strength, simply speaking, the tendency of a Brønsted-Lowry acid to donate a proton, may be expressed in terms of a pKa value, wherein a strong acid has a lower (i.e. smaller) pKa value than a weak acid. The term "pKa value" as such and the means of determining the pKa value of a protic acid form part of the common knowledge of the skilled artisan. Additionally, pKa values for commonly used Brønsted-Lowry acids (and many more) are available in tabulated form from the literature. As used herein, the pKa value is such determinable in water (i.e. aqueous solution) at 25 °C, unless indicated differently.

In some embodiments of the method of the invention, the deprotection mixture M-(b-1), the deprotection mixture M-(b-2), and each deprotection mixture M-(b-x) comprise a protic acid. In some embodiments of the method of the invention, the deprotection mixture M-(b-1), the deprotection mixture M-(b-2), and each deprotection mixture M-(b-x) comprise a protic acid having a pKa equal to or smaller than 5, equal to or smaller than 4, equal to or smaller than 3.5, equal to or smaller than 3, equal to or smaller than 2.5, or equal to or smaller than 2. In some embodiments of the method of the invention, the deprotection mixture M-(b-1), the deprotection mixture M-(b-2), and each deprotection mixture M-(b-x) comprise a protic acid having a pKa in the range of 0-5, 0-4, 0-3.5, 0-3, 0-2.5, or 0-2. The protic acid is not particularly limited in terms of its chemical structure. For example, the protic acid may be a carboxylic acid, a sulfonic acid, a mineral acid or mixtures thereof.

Examples of carboxylic acids comprise trifluoroacetic acid (TFA), dichloroacetic acid (DCA), trichloroacetic acid (TCA), and acetic acid (AcOH). A carboxylic acid such as acetic acid may, for example, also be employed in combination with trimethylsilyl chloride (TMSCI). Examples of sulfonic acids comprise methanesulfonic acid and p-toluenesulfonic acid. Examples of mineral acids comprise hydrochloric acid and sulfuric acid.

It will be understood that the molar amount of the protic acid in each deprotection mixture M-(b-1), M-(b-2), and M-(b-x) is preferably higher than the molar amount of the respective component or oligonucleotide from which the respective protecting group is to be cleaved. For example, the total molar amount of the protic acid comprised in the respective deprotection mixture may be in the range of 1-100 mol, 1-90 mol, 1-80 mol, 1-70 mol, 1-60 mol, 1-50 mol, 1-40 mol, or 1-30 mol per 1 mol of the respective component or oligonucleotide from which the respective protecting group is to be cleaved.

Besides the protic acid, each of the deprotection mixtures M-(b-1), M-(b-2), and M-(b-x) may further comprise one or more carbocation scavengers. As used herein, the term "carbocation scavenger" relates to a nucleophilic compound, which may be used to bind a carbocation or to consume a carbocation by formal donation of a hydride anion, thereby preventing unwanted side reactions of the carbocation. Typical examples of such carbocations are carbocations formed during the cleavage of protecting groups comprising an optionally substituted triarylmethyl residue. For example, the cleavage of a di(p-methoxyphenyl)phenylmethyl (DMT) protecting group may result in a DMT cation (i.e. a di(*p*-methoxyphenyl)phenylmethyl cation). Non-limiting examples of carbocation scavengers comprise:
- compounds comprising one or more sulfhydryl groups (SH) and one or more carboxyl groups (C(O)OH or C(O)O⁻) such as, e.g., glutathione, thiomalic acid, 3-mercaptopropionic acid, cysteine, cysteinyl-glutamic acid, and cysteinyl-aspartic acid, wherein the N-terminal amino group in any one of cysteine, cysteinyl-glutamic acid, and cysteinyl aspartic acid may optionally be protected by a protecting group removable under alkaline conditions such as, e.g., the 9-fluorenylmethyloxycarbonyl (Fmoc) protecting group,
- compounds comprising an indole residue and one or more carboxyl groups (C(O)OH or C(O)O⁻), such as, e.g., 1-(1*H*-indol-5-yl) butanedicarboxylate (CAS-RN: 1905473-95-1), tryptophan, tryptophanyl-glutamic acid, tryptophanyl-aspartic acid, wherein the *N*-terminal amino group in any one of tryptophan, tryptophanyl-glutamic acid, and tryptophanyl-aspartic acid may optionally be protected by a protecting group removable under alkaline conditions such as, e.g., the Fmoc protecting group,
- pyrrole or derivatives thereof, in which one or more hydrogen residue have been substituted for a C₁-C₆-alkyl group, preferably a methyl group (CH₃), such as, e.g., pyrrole, 2-methylpyrrole, 3-methylpyrrole, 2,3-dimethylpyrrole, and 2,4-dimethylpyrrole,
- indole or derivatives thereof, in which one or more hydrogen residue have been substituted for a C₁-C₆-alkyl group, preferably a methyl group (CH₃), such as, e.g., indole, 2-methylindole, 3-methylindole, 4-methylindole, 5-methylindole, 6-methylindole, 7-methylindole, 5,6-dimethylindole, and 6,7-dimethylindole,
- water,
- aliphatic alcohols comprising 1 to 6 carbon atoms such as methanol, ethanol, propanol, and isopropyl alcohol,
- phenol or derivatives thereof such as o-cresol, *m*-cresol, and *p*-cresol,
- phenol ethers such as, e.g., anisole, 1,3-dimethoxybenzene, and 1,3,5-trimethoxybenzene,
- thioethers such as, e.g., dimethyl sulfide, methyl ethyl sulfide, and thioanisole,
- silanes such as, e.g., triisopropylsilane (TIS) and triethylsilane (TES),
- thiols and thiophenols such as, e.g., 1,2-ethanedithiol (EDT), 1,4-dithioerythrol (DTE), 1,4-dithiothreitol (DTT), 3,6-dioxa-1,8-octanedithiol (DODT), 1,4-benzenedimethanthiol (BDMT), 1,4-butanedithiol, 2-mercaptoethanol, thiophenol, and *p*-thiocresol,
- polyalkylbenzenes such as, e.g., 1,3,5-trimethylbenzene and pentamethylbenzene, and
- mixtures thereof.

The above-mentioned compounds comprising one or more sulfhydryl groups (SH) and one or more carboxyl groups (C(O)OH or C(O)O⁻) as well as the above-mentioned compounds comprising an indole residue and one or more carboxyl groups (C(O)OH or C(O)O⁻) may be preferred carbocation scavengers, and glutathione, thiomalic acid, and 3-mercaptopropionic acid may be a particularly preferred carbocation scavenger. Glutathione is most preferred.

It will be understood that the molar amount of the optional carbocation scavenger in each deprotection mixture M-(b-1), M-(b-2), and M-(b-x) is preferably higher than the molar amount of the respective component or oligonucleotide from which the respective protecting group is to be cleaved. For example, the total molar amount of the carbocation scavenger optionally comprised in each deprotection mixture M-(b-1), M-(b-2), and M-(b-x) may be in the range of 1-60 mol, 1-50 mol, 1-40 mol, 1-35 mol, 1-30 mol, 5-30 mol, 10-30 mol, or 15-30 mol, per 1 mol of of the respective component or oligonucleotide from which the respective protecting group is to be cleaved. With glutathione as carbocation scavenger, 1-10 mol or 2-9 mol or 3-8 mol or 3-7 mol or 4-6 mor or 5 mol of glutathione per 1 mol of the respective component or oligonucleotide from which the respective protecting group is to be cleaved may preferably be used. TFA as protic acid may preferably be used alongside glutathione as carbocation scavenger. The skilled artisan understands that in the course of an iterative (i.e., stepwise) oligonucleotide synthesis, it may be unpractical to determine the molar amount of the component or oligonucleotide from which the protecting group is to be cleaved in each coupling cycle. Hence, in practice, one may assume that the molar amount of the respective component or oligonucleotide from which the respective protecting group is to be cleaved is essentially constant throughout the synthesis and equal to the molar amount of the component C-0 as provided in step (a-1).

In some embodiments of the method of the invention, each deprotection mixture M-(b-1), M-(b-2), and M-(b-x) comprises:
- a protic acid, preferably a protic acid having a pKa equal to or smaller than 5, 4, 3.5, 3, 2.5 or 2,
- one or more carbocation scavengers (as defined herein), and
- optionally trimethylsiyl chloride.

In some embodiments of the method of the invention, each deprotection mixture M-(b-1), M-(b-2), and M-(b-x) comprises:
- a protic acid, preferably a protic acid having a pKa equal to or smaller than 5, 4, 3.5, 3, 2.5 or 2, and
- one or more carbocation scavengers (as defined herein).

In some embodiments of the method of the invention, each deprotection mixture M-(b-1), M-(b-2), and M-(b-x) comprises:
- a protic acid, preferably a protic acid having a pKa equal to or smaller than 5, 4, 3.5, 3, 2.5 or 2,
- one or more carbocation scavengers selected from the group consisting of compounds comprising one or more sulfhydryl groups (SH) and one or more carboxyl groups (C(O)OH or C(O)O⁻) and compounds comprising one indole residue and one or more carboxyl groups (C(O)OH or C(O)O⁻), and
- optionally trimethylsiyl chloride.

In some embodiments of the method of the invention, each deprotection mixture M-(b-1), M-(b-2), and M-(b-x) comprises:
- a protic acid, preferably a protic acid having a pKa equal to or smaller than 5, 4, 3.5, 3, 2.5 or 2, and
   - one or more carbocation scavengers selected from the group consisting of compounds comprising one or more sulfhydryl groups (SH) and one or more carboxyl groups (C(O)OH or C(O)O⁻) and compounds comprising one indole residue and one or more carboxyl groups (C(O)OH or C(O)O⁻).

In some embodiments of the method of the invention, each deprotection mixture M-(b-1), M-(b-2), and M-(b-x) comprises:
- a protic acid selected from the group consisting of a carboxylic acid, a sulfonic acid, a mineral acid, and mixtures thereof,
- one or more carbocation scavengers, each of which is a compound comprising one sulfhydryl group (SH) and one or two carboxyl groups (C(O)OH or C(O)O⁻), and
- optionally, trimethylsiyl chloride.

In some embodiments of the method of the invention, each deprotection mixture M-(b-1), M-(b-2), and M-(b-x) comprises:
- a protic acid selected from the group consisting of a carboxylic acid, a sulfonic acid, a mineral acid, and mixtures thereof, and
- one or more carbocation scavengers, each of which is a compound comprising one sulfhydryl group (SH) and one or two carboxyl groups (C(O)OH or C(O)O⁻).

In some embodiments of the method of the invention, each deprotection mixture M-(b-1), M-(b-2), and M-(b-x) comprises:
- a protic acid having a pKa equal to or smaller than 5, 4, 3.5, 3, 2.5 or 2 and being selected from the group consisting of a carboxylic acid, a sulfonic acid, a mineral acid, and mixtures thereof, and
- one or more carbocation scavengers, each of which is a compound comprising one sulfhydryl group (SH) and one or two carboxyl groups (C(O)OH or C(O)O⁻).

In some embodiments of the method of the invention, each deprotection mixture M-(b-1), M-(b-2), and M-(b-x) comprises:
- a protic acid selected from the group consisting of trifluoroacetic acid, dichloroacetic acid, trichloroacetic acid, acetic acid, hydrochloric acid, sulfuric acid, methanesulfonic acid, p-toluenesulfonic acid, and mixtures thereof,
- one or more carbocation scavengers, each of which is a compound comprising one sulfhydryl group (SH) and one or two carboxyl groups (C(O)OH or C(O)O⁻), and
- optionally, trimethylsiyl chloride.

In some embodiments of the method of the invention, each deprotection mixture M-(b-1), M-(b-2), and M-(b-x) comprises:
- a protic acid selected from the group consisting of trifluoroacetic acid, dichloroacetic acid, trichloroacetic acid, acetic acid, hydrochloric acid, sulfuric acid, methanesulfonic acid, benzenesulfonic acid, *p*-toluenesulfonic acid, and mixtures thereof, and
- one or more carbocation scavengers, each of which is a compound comprising one sulfhydryl group (SH) and one or two carboxyl groups (C(O)OH or C(O)O⁻).

In some embodiments of the method of the invention, each deprotection mixture M-(b-1), M-(b-2), and M-(b-x) comprises:
- a protic acid selected from the group consisting of trifluoroacetic acid, dichloroacetic acid, trichloroacetic acid, acetic acid, hydrochloric acid, sulfuric acid, methanesulfonic acid, *p*-toluenesulfonic acid, and mixtures thereof,
- one or more carbocation scavengers selected from the group consisting of thiomalic acid 3-mercaptopropionic acid, cysteine, cysteinyl-glutamic acid, and cysteinyl-aspartic acid, wherein the *N*-terminal amino group in any one of cysteine, cysteinyl-glutamic acid, and cysteinyl aspartic acid may optionally be protected by a protecting group removable under alkaline conditions such as, e.g., the 9-fluorenylmethyloxycarbonyl (Fmoc) protecting group, and
- optionally, trimethylsiyl chloride.

In some embodiments of the method of the invention, each deprotection mixture M-(b-1), M-(b-2), and M-(b-x) comprises:
- a protic acid selected from the group consisting of trifluoroacetic acid, dichloroacetic acid, trichloroacetic acid, hydrochloric acid, sulfuric acid, methanesulfonic acid, benzenesulfonic acid, *p*-toluenesulfonic acid, and mixtures thereof, and
- one or more carbocation scavengers selected from the group consisting of glutathione, thiomalic acid, 3-mercaptopropionic acid, cysteine, cysteinyl-glutamic acid, and cysteinyl-aspartic acid, wherein the *N*-terminal amino group in any one of cysteine, cysteinyl-glutamic acid, and cysteinyl aspartic acid may optionally be protected by a protecting group removable under alkaline conditions such as, e.g., the 9-fluorenylmethyloxycarbonyl (Fmoc) protecting group.

In some embodiments of the method of the invention, each deprotection mixture M-(b-1), M-(b-2), and M-(b-x) comprises:
- a protic acid selected from the group consisting of trifluoroacetic acid, dichloroacetic acid, and acetic acid,
- thiomalic acid, and
- optionally, trimethylsiyl chloride, with the proviso that a deprotection mixture M-(b-1), M-(b-2) or M-(b-x) comprising acetic acid also comprises trimethylsilyl chloride.

In some embodiments of the method of the invention, each deprotection mixture M-(b-1), M-(b-2), and M-(b-x) comprises:
- a protic acid selected from the group consisting of trifluoroacetic acid, dichloroacetic acid, and trichloroacetic acid, and
- a carbocation scavenger selected from the group consisting of glutathione, thiomalic acid, and 3-mercaptopropionic acid.

In some preferred embodiments of the method of the invention, each deprotection mixture M-(b-1), M-(b-2), and M-(b-x) comprises trifluoroacetic acid and glutathione.

Each deprotection mixture M-(b-1), M-(b-2), and M-(b-x) may be a solution, i.e. may further comprise a solvent or mixed solvent capable of dissolving said protic acid, and, if present, also said one or more carbocation scavengers and/or said one or more further additives. Said solvent or mixed solvent may preferably comprise one or more non-polar solvents. Non-limiting examples of non-polar solvents comprise:
- ether solvents such as, e.g., alkylated derivatives of tetrahydropyran or tetrahydrofuran, in particular 4-methyltetrahydropyran (MTHP) and 2-methyltetrahydrofuran, cyclopentyl methyl ether, *tert*-butyl methyl ether, diethyl ether, and anisole;
- aromatic solvents such as, e.g., benzene, toluene, *o*- or *m*- or *p*-xylene, and mesitylene;
- aliphatic hydrocarbon solvents such as, e.g., pentanes, hexanes, heptanes, octanes, nonanes, and cyclic derivatives thereof such as cyclohexane;
- ester solvents such as, e.g., ethyl acetate and isopropyl acetate;
- halogenated aliphatic hydrocarbon solvents such as, e.g., dichloromethane, 1,2-dichloroethane, and chloroform; and
mixtures thereof.

Optionally, said one or more non-polar solvents may be mixed with one or more polar solvents. Non-limiting examples of such polar solvents comprise:
- nitrile solvents such as, e.g., acetonitrile and propionitrile;
- amide solvents such as, e.g., *N-N*-dimethylformamide, *N-N*-dimethylacetamine, *N*-methyl-2-piperidone, and *N*-methyl-2-pyrrolidone;
- ketone solvents such as, e.g., acetone and 2-butanone;
- polar ether solvents such as, e.g., 1,4-dioxane and tetrahydrofuran;
- sulfoxide solvents such as, e.g., dimethylsulfoxide; and
- mixtures thereof.

Alternatively, any one of the deprotection mixtures M-(b-1), M-(b-2), and M-(b-x) may be formed by adding said protic acid, and optionally the one or more carbocation scavengers and/or further additives directly into the respective reaction mixture or organic phase of the preceding step of the coupling cycle.

The term "incubating" in steps (b-1), (b-2), and (b-x) may be understood in the broadest sense to refer to any process of contacting the respective component or oligonucleotide from which the respective protecting group is to be cleaved and the respective deprotection mixture, preferably inside the same reaction vessel or reactor. Typically, the respective component or oligonucleotide from which the respective protecting group is to be cleaved may already be contained in a reaction vessel or reactor, to which the respective deprotection mixture is then added. Alternatively, the components of the respective deprotection mixture may be added individually to the reaction vessel or reactor containing the respective component or oligonucleotide from which the respective protecting group is to be cleaved, so that the respective deprotection mixture may be formed directly within said reaction vessel or reactor containing the respective component or oligonucleotide from which the respective protecting group is to be cleaved. In other words: The term "incubating" does not imply that a preformed deprotection mixture is employed. Alternatively, the respective deprotection mixture may already be contained in a reaction vessel or reactor, to which the respective component or oligonucleotide from which the respective protecting group is to be cleaved is then added.

Any one of steps (b-1), (b-2), and (b-x) may, for example, be performed at a temperature in the range of -15 to 90 °C, -10 to 90 °C, 0 to 90 °C, 5 to 90 °C, 10 to 70 °C, 15 to 60 °C, or 15 to 50 °C. For convenience, any one of steps (b-1), (b-2), and (b-x) may simply be performed at room temperature. Increased temperatures may result in shorter reaction times. The reaction time may also depend on the chemical structure of the reactants and will routinely be selected by a skilled person, for example based on reaction monitoring using, e.g., thin-layer chromatography and/or high performance liquid chromatography (HPLC), optionally coupled to mass spectrometry.

After performing the deprotection reaction of any one of steps (b-1), (b-2), and (b-x), a base may be added to the reaction mixture to completely or partly neutralize any excess of the protic acid. Such neutralization may typically be achieved by addition of one or more bases or a solution thereof directly into the reaction mixture of the respective step (b-1), (b-2), and (b-x). Non-limiting examples of bases which may be used for such neutralization comprise pyridine, benzimidazole, 1,2,4-triazole, *N-*phenylimidazole, 2-amino-4,6-dimethylpyrimidine, 1,10-phenanthroline, imidazole, *N*-methylimidazole, 2-chlorobenzimidazole, 2-bromobenzimidazole, 2-methylimidazole, 2-phenylbenzimidazole, *N*-phenylbenzimidazole, and 5-nitrobenzimidazole, among which pyridine may be preferred. It will be understood that such neutralization may additionally or alternatively be achieved, if at least one of the one or more aqueous extractions of the subsequent step (c-1), (c-2), or (c-x) is performed with an alkaline aqueous solution.

Step (c-1) of the methods of the invention is: subjecting a solution comprising the component (C-0)^{#} to one or more aqueous extractions, wherein the organic phase comprises the component (C-0)^{#}.

Step (c-2) of some methods of the invention is: subjecting a solution comprising the first cycle oligonucleotide (O-1)^{#} to one or more aqueous extractions, wherein the organic phase comprises the first cycle oligonucleotide (O-1)⁴.

Step (c-x) of some methods of the invention is: subjecting a solution comprising the (x-1)-th cycle oligonucleotide (O-(x-1))^{#} to one or more aqueous extractions, wherein the organic phase comprises the (x-1)-th cycle oligonucleotide (O-(x-1))^{#}.

Said solution comprising the component (C-0)^{#} of step (c-1) may, for example, be the reaction mixture obtained from carrying step (b-1). Said solution comprising the first cycle oligonucleotide (O-1)^{#} of step (c-2) may, for example, be the reaction mixture obtained from carrying step (b-2). Said solution comprising the (x-1)-th cycle oligonucleotide (O-(x-1))^{#} of step (c-x) may, for example, be the reaction mixture obtained from carrying step (b-x) of the same coupling cycle. Optionally, said solutions comprising the component (C-0)^{#} or the first cycle oligonucleotide (O-1)^{#} or the (x-1)-th cycle oligonucleotide (O-(x-1))^{#} may be obtained from the respective reaction mixture by addition of one or more non-polar solvents, for example in order to facilitate the phase separation. Alternatively, or additionally, such non-polar solvents may also be added during or in between the one or more aqueous extractions of any one of steps (c-1), (c-2), and (c-x). Non-limiting examples of non-polar solvents which may be added comprise:
- ether solvents such as, e.g., alkylated derivatives of tetrahydropyran or tetrahydrofuran, in particular 4-methyltetrahydropyran (MTHP) and 2-methyltetrahydrofuran, cyclopentyl methyl ether, tert-butyl methyl ether, diethyl ether, and anisole;
- aromatic solvents such as, e.g., benzene, toluene, *o*- or *m*- or *p*-xylene, and mesitylene;
- aliphatic hydrocarbon solvents such as, e.g., pentanes, hexanes, heptanes, octanes, nonanes, and cyclic derivatives thereof such as cyclohexane;
- ester solvents such as, e.g., ethyl acetate and isopropyl acetate;
- halogenated aliphatic hydrocarbon solvents such as, e.g., dichloromethane, 1,2-dichloroethane, and chloroform; and
- mixtures thereof.

4-Methyltetrahydropyran (MTHP) may be a preferred non-polar ether solvent which may be added prior to or during or in between the aqueous extraction(s) of any one of steps (c-1), (c-2), and (c-x). Additionally, or alternatively, one or more amide solvents S^{A} may be added prior to or during or in between the aqueous extraction(s) of any one of steps (c-1), (c-2), and (c-x).

The term "aqueous extraction" in steps (c-1), (c-2), and (c-x) of the method of the invention may be understood in the broadest sense as any liquid-liquid extraction operation during which the respective solution comprising the component (C-0)^{#} or the first cycle oligonucleotide (O-1)^{#} or the (x-1)-th cycle oligonucleotide (O-(x-1))^{#} is extracted with water or an aqueous solution. As used herein, an aqueous solution is a solution comprising water, preferably at least 10 vol-%, 20 vol-%, 30 vol-%, 40 vol-%, 50 vol-%, or 60 vol-% of water. Hence, water is herein also considered a specific aqueous solution, since it comprises more than 10 vol-%, 20 vol-%, 30 vol-%, 40 vol-%, 50 vol-%, or 60 vol-% of water.

An aqueous solution may optionally comprise one or more water-miscible organic solvents. Non-limiting examples of such water-miscible organic solvents comprise:
- alcohol solvents such as methanol, ethanol, and isopropyl alcohol (propan-2-ol, IPA),
- nitrile solvents such as acetonitrile and propionitrile,
- ketone solvents such as acetone and 2-butanone,
- polar ether solvents such as tetrahydrofuran and 1,4-dioxane,
- polar amide solvents such as *N,N*-dimethylformamide, *N,N*-dimethylacetamide, *N*-methyl-2-pyrrolidone, and *N*-methyl-2-piperidone,
- sulfoxide solvents such as dimethyl sulfoxide, and
- mixtures thereof.

An aqueous solution may optionally also comprise one or more dissolved components. Non-limiting examples of such dissolved components comprise:
- inorganic salts such as alkali halides, in particular sodium chloride (e.g. typically used to facilitate and/or accelerate phase separation),
- water-soluble protic acids (as defined herein) such as acetic acid,
- water-soluble organic or inorganic bases such as *N*-methylmorpholine (NMM), pyridine, sodium or potassium or ammonium carbonate or hydrogen carbonate, and sodium or potassium or ammonium phosphate or hydrogen phosphate or dihydrogen phosphate, and
- mixtures thereof.

As one example, an aqueous solution of *N*-methylmorpholine (e.g. 1 mol/L) may be used as aqueous solution for the aqueous extraction(s) of any one of steps (c-1), (c-2), and (c-x). As one example, a mixture of acetone, an aqueous solution of *N*-methylmorpholine (e.g. 1 mol/L), and *N*-methylmorpholine (e.g. 5:20:14, v:v:v) may be used as aqueous solution for the aqueous extraction(s) of any one of steps (c-1), (c-2), and (c-x). As another example, a mixture of *N*-methylmorpholine, acetone, and 2-20 wt-% aqueous sodium chloride solution (brine) (e.g. 9:15:40 or 3:3:8, v:v:v) may be used as aqueous solution for the aqueous extraction(s) of any one of steps (c-1), (c-2), and (c-x). As another example, a mixture of 2-20 wt-% aqueous sodium chloride solution (brine), acetone, an aqueous solution of *N*-methylmorpholine (e.g. 1 mol/L), and *N*-methylmorpholine (e.g. 5:5:5:2, v:v:v) may be used as aqueous solution for the aqueous extraction(s) of any one of steps (c-1), (c-2), and (c-x). As another example, a mixture of isopropyl alcohol, *N*-methylmorpholine, and 2-20 wt-% aqueous sodium chloride solution (brine) (e.g. 1:5:6, v:v:v) may be used as aqueous solution for the aqueous extraction(s) of any one of steps (c-1), (c-2), and (c-x). As another example, a mixture of isopropyl alcohol, *N*-methylmorpholine, 2-20 wt-% aqueous sodium chloride solution (brine), and acetone (e.g. 1:5:6:1.5 or 1:5:6:1, v:v:v:v) may be used as aqueous solution for the aqueous extraction(s) of any one of steps (c-1), (c-2), and (c-x). As another example, a solution (e.g. 0.5-1.0 mol/L) of *N*-methylmorpholine in a mixture of water and acetone (e.g. 1:1, v:v) may be used as aqueous solution for the aqueous extraction(s) of any one of steps (c-1), (c-2), and (c-x). As another example, a mixture of a 2-20 wt-% aqueous sodium chloride solution (brine), *N*-methylmorpholine, and acetone (e.g. 1:1:1 or 1:2:2, v:v:v) may be used as aqueous solution for the aqueous extraction(s) of any one of steps (c-1), (c-2), and (c-x). As another example, an aqueous solution of sodium hydrogen carbonate (e.g. 0.5 mol/L) may be used for the aqueous extraction(s) of any one of steps (c-1), (c-2), and (c-x). As another example, a mixture of an aqueous solution of sodium hydrogen carbonate (e.g. 0.5 mol/L), *N*-methylmorpholine, and acetone (e.g. 5:1:5, v:v:v) may be used for the aqueous extraction(s) of any one of steps (c-1), (c-2), and (c-x). As another example, (pure) water may be used for the aqueous extraction(s) of any one of steps (c-1), (c-2), and (c-x). As another example, a mixture of water and acetone (e.g. 1:1 or 2:1 or 3:1 or 3:2, v:v) may be used as aqueous solution for the aqueous extraction(s) of any one of steps (c-1), (c-2), and (c-x). As another example, a mixture of 2-20 wt-% aqueous sodium chloride solution (brine) and acetone (e.g. 1:1 or 2:1, v:v) may be used as aqueous solution for the aqueous extraction(s) of any one of steps (c-1), (c-2), and (c-x). As another example, a mixture of water, 2-20 wt-% aqueous sodium chloride solution (brine), and acetone (e.g. 1:1:1, v:v:v) may be used as aqueous solution for the aqueous extraction(s) of any one of steps (c-1), (c-2), and (c-x). As another example, a solution of acetic acid in water may be used as aqueous solution for the aqueous extraction(s) of any one of steps (c-1), (c-2), and (c-x). As another example, a solution of acetic acid in a mixture of water and acetone (e.g. 1:1, v:v) may be used as aqueous solution for the aqueous extraction(s) of any one of steps (c-1), (c-2), and (c-x). As another example, a solution of acetic acid in a mixture of water, acetone, and 2-20 wt-% aqueous sodium chloride solution (brine) (e.g. 2:2:1, v:v:v) may be used as aqueous solution for the aqueous extraction(s) of any one of steps (c-1), (c-2), and (c-x).

It will be understood that for each of the one or more aqueous extractions of a step (c-1) or (c-2) or (c-x), the aqueous solution may be the same or different (i.e. comprise the same or different components in the same or different ratios). It will also be understood that the aqueous solutions may be the same or different for different steps (c-x) of different coupling cycles.

As used herein, an "aqueous extraction" is further characterized in that it results in a mixture comprising at least one aqueous phase (preferably an aqueous layer) and at least one organic phase (preferably an organic layer), wherein the at least one aqueous phase (preferably layer) is then separated from the at least one organic phase (preferably layer). In case of more than one organic phases (either from a single aqueous extraction or from back-extraction of one or more aqueous phase), these more than one organic phases may be combined to obtain "the organic phase" mentioned in steps (c-1), (c-2), and (c-x). In general, the means of performing aqueous extractions are well-known to the skilled person.

The expression "the organic phase comprises the component (C-0)^{#}" in step (c-1) may be understood in the broadest sense to mean that some of the molecules of said component (C-0)^{#} are dissolved in the organic phase. The expression "the organic phase comprises the first cycle oligonucleotide (O-1)^{#}" in step (c-2) may be understood in the broadest sense to mean that some of the molecules of said first cycle oligonucleotide (O-1)^{#} are dissolved in the organic phase. The expression "the organic phase comprises the (x-1)-th cycle oligonucleotide (O-(x-1))^{#}" in step (c-x) may be understood in the broadest sense to mean that some of the molecules of said (x-1)-th cycle oligonucleotide (O-(x-1 ))^{#} are dissolved in the organic phase. It is preferred that most molecules (e.g. more than 50 %, 60 %, 70 %, 80 %, or 90 % of the molecules) of the said component (C-0)^{#} or said first cycle oligonucleotide (O-1)^{#}or said (x-1)-th cycle oligonucleotide (O-(x-1))^{#} are comprised in the organic phase (and thus not in the aqueous phase).

In some embodiments of the method of the invention, in each of the one or more aqueous extractions of step (c-1), the aqueous phase has a pH-value equal to or smaller than 7, preferably in the range of 4-7. In some embodiments of the method of the invention, in each of the one or more aqueous extractions of step (c-2), the aqueous phase has a pH-value equal to or smaller than 7, preferably in the range of 4-7. In some embodiments of the method of the invention, in each of the one or more aqueous extractions of each step (c-x), the aqueous phase has a pH-value equal to or smaller than 7, preferably in the range of 4-7.

In some embodiments of the method of the invention:
- in each of the one or more aqueous extractions of step (c-1), the aqueous phase has a pH-value equal to or smaller than 7, preferably in the range of 4-7;
- if a second coupling cycle comprising steps (b-2) to (h-2) is performed, in each of the one or more aqueous extractions of step (c-2), the aqueous phase has a pH-value equal to or smaller than 7, preferably in the range of 4-7; and
- if (n-2) iterations of the coupling cycle comprising steps (b-x) to (h-x) are performed, in each of the one or more aqueous extractions of each step (c-x), the aqueous phase has a pH-value equal to or smaller than 7, preferably in the range of 4-7.

As used herein, the pH-value of the aqueous phase of an aqueous extraction is to be determined from the respective aqueous phase after the aqueous extraction (i.e., after the phase separation, preferably after removing the aqueous phase from the organic phase or vice versa) and at a temperature in the range of 23-28 °C, preferably 25 °C.

As used herein, the term "aqueous phase" of an aqueous extraction is distinct from the aqueous solution, which is initially combined with the solution comprising the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)), followed by extraction. The skilled artisan understands that the "aqueous phase" typically comprises species which have been extracted from the solution comprising the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)), e.g., water-soluble species. Hence, to say that the aqueous phase of an aqueous extraction has a certain pH-value is not the same as to say that the aqueous solution employed in the aqueous extraction has a certain pH-value. For example, pure water or any other aqueous solution may be used for an aqueous extraction, and during the aqueous extraction one or more protic acids may partition from said solution comprising the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)) into the aqueous phase, so that the aqueous phase then has a pH-value which is lower than the pH-value of the water or other aqueous solution employed.

In some embodiments of the method of the invention, said one or more aqueous extractions of steps (c-1), (c-2), and (c-x) comprise a first aqueous extraction comprising the following steps (Ex-I) to (Ex-IV):

| | |
|---|---|
| (Ex-I) | Combining the solution comprising the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)) with a first aqueous solution AS-I; |
| (Ex-II) | Agitating the mixture of step (Ex-I); |
| (Ex-III) | Allowing the phases to separate, so as to obtain a first organic phase OP-I and a first aqueous phase AP-I, wherein the first organic phase OP-I comprises the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)); and |
| (Ex-IV) | Removing the first aqueous phase AP-I from the first organic phase OP-I or vice versa; |

wherein the solution comprising the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)) of step (Ex-I) comprises one or more amide solvents S^{A} and/or one or more amide solvents S^{A} are added during any one of steps (Ex-I) and (Ex-II) and/or in between these steps. In such embodiments, the first aqueous phase AP-I preferably has a pH-value equal to or smaller than 7, preferably in the range of 1.0-7.0, 2.0-7.0, 3.0-7.0, 3.5-7.0, 4.0-7.0, 4.5-7.0, 4.5-6.5, 4.5-6.0, or 4.5-5.5.

In some embodiments of the method of the invention, said one or more aqueous extractions of steps (c-1), (c-2), and (c-x) comprise a first aqueous extraction comprising the aforementioned steps (Ex-I) to (Ex-IV), and a second aqueous extraction comprising the following steps (Ex-V) to (Ex-VIII):

| | |
|---|---|
| (Ex-V) | Combining the first organic phase OP-I with a second aqueous solution AS-II; |
| (Ex-VI) | Agitating the mixture of step (Ex-V); |
| (Ex-VII) | Allowing the phases to separate, so as to obtain a second organic phase OP-II and a second aqueous phase AP-II, wherein the second organic phase OP-II comprises the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)); and |
| (Ex-VIII) | Removing the second aqueous phase AP-II from the second organic phase OP-II or vice versa; |

wherein the first organic phase OP-I comprises one or more amide solvents S^{A} and, optionally, one or more amide solvents S^{A} are added during any one of steps (Ex-V) and (Ex-VI) and/or in between these steps. In such embodiments, the second aqueous phase AP-II preferably has a pH-value in the range of 4.0-7.0, 4.5-7.0, 5.0-7.0, 5.0-6.5, or 5.0-6.0.

In some embodiments of the method of the invention, said one or more aqueous extractions of steps (c-1), (c-2), and (c-x) comprise a first aqueous extraction comprising the aforementioned steps (Ex-I) to (Ex-IV), a second aqueous extraction comprising the aforementioned steps (Ex-V) to (Ex-VIII), and a third aqueous extraction comprising the following steps (Ex-IX) to (Ex-XII):

| | |
|---|---|
| (Ex-IX) | Combining the second organic phase OP-II with a third aqueous solution AS-III; |
| (Ex-X) | Agitating the mixture of step (Ex-IX); |
| (Ex-XI) | Allowing the phases to separate, so as to obtain a third organic phase OP-III and a third aqueous phase AP-III, wherein the third organic phase OP-III comprises the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)); and |
| (Ex-XII) | Removing the third aqueous phase AP-III from the third organic phase OP-III or vice versa; |

wherein the second organic phase OP-II comprises one or more amide solvents S^{A} and, optionally, one or more amide solvents S^{A} are added during any one of steps (Ex-IX) and (Ex-X) and/or in between these steps. In such embodiments, the third aqueous phase AP-III preferably has a pH-value in the range of 5.0-7.0, 5.0-6.5, or 5.0-6.0.

The skilled artisan understands that one or more further aqueous extractions similar to the first, second, and third aqueous extraction comprising steps (Ex-I) to (Ex-IV) or steps (Ex-V) to (Ex-VIII) or steps (Ex-IX) to (Ex-XII), respectively, may be carried out in addition in any one of steps (c-1), (c-2), and (c-x). Such further aqueous extractions preferably comprise the following steps (Ex-A') to (Ex-D'):

| | |
|---|---|
| (Ex-A') | Combining the organic phase obtained from the previous aqueous extraction with an aqueous solution; |
| (Ex-B') | Agitating the mixture of step (Ex-A'); |
| (Ex-C') | Allowing the phases to separate, so as to obtain an organic phase and an aqueous phase, wherein the organic phase comprises the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)); and |
| (Ex-D') | Removing the aqueous phase from the organic phase or vice versa; |

wherein the organic phase obtained from the previous aqueous extraction comprises one or more amide solvents S^{A} and, optionally, one or more amide solvents S^{A} are added during any one of steps (Ex-A') and (Ex-B') and/or in between these steps. In such embodiments, the aqueous phase obtained in each step (Ex-C') preferably has a pH-value equal to or smaller than 7, preferably in the range of 4.0-7.0, in particular in the range of 5.0-7.0, 5.0-6.5, or 5.0-6.0

The skilled artisan understands that the aqueous solutions of two or more aqueous extractions, e.g., the aqueous solutions AS-I, AS-I, and AS-III, need not be identical, i.e., they may or may not comprise the same species/components/solvents and the amounts/volumes of said species/components/solvents may be the same or different, unless indicated differently in specific embodiments.

In some embodiments of the method of the invention, in which said one or more aqueous extractions of any one of steps (c-1), (c-2), and (c-x) comprise said first aqueous extraction comprising steps (Ex-I) to (Ex-IV), the first aqueous solution AS-I has a pH-value in the range of 5.0-8.0, 5.5-7.5, 6.0-7.5, or 6.5-7.5. In some embodiments of the method of the invention, in which said one or more aqueous extractions of any one of steps (c-1), (c-2), and (c-x) comprise said first aqueous extraction comprising steps (Ex-I) to (Ex-IV), the first aqueous solution AS-I does not comprise a compound having a pKa-value equal to or larger than 9.0, 8.5, 8.0, 7.5, 7.0, 6.5, 6.0 or 5.5. The pKa-value is to be determined in water (i.e., in an aqueous solution of the respective compound) at 25 °C.

In some embodiments of the method of the invention, in which said one or more aqueous extractions of any one of steps (c-1), (c-2), and (c-x) comprise said first aqueous extraction comprising steps (Ex-I) to (Ex-IV), and said second aqueous extraction comprising steps (Ex-V) to (Ex-VIII), each of the first aqueous solution AS-I and the second aqueous solution AS-II has a pH-value in the range of 5.0-8.0, 5.5-7.5, 6.0-7.5, or 6.5-7.5. In some embodiments of the method of the invention, in which said one or more aqueous extractions of any one of steps (c-1), (c-2), and (c-x) comprise said first aqueous extraction comprising steps (Ex-I) to (Ex-IV), and said second aqueous extraction comprising steps (Ex-V) to (Ex-VIII), none of the first aqueous solution AS-I and the second aqueous solution AS-II comprises a compound having a pKa-value equal to or larger than 9.0, 8.5, 8.0, 7.5, 7.0, 6.5, 6.0 or 5.5. The pKa-value is to be determined in water (i.e., in an aqueous solution of the respective compound) at 25 °C.

In some embodiments of the method of the invention, in which said one or more aqueous extractions of any one of steps (c-1), (c-2), and (c-x) comprise said first aqueous extraction comprising steps (Ex-I) to (Ex-IV), and said second aqueous extraction comprising steps (Ex-V) to (Ex-VIII), and said third aqueous extraction comprising steps (Ex-IX) to (Ex-XII), each of the first aqueous solution AS-I, the second aqueous solution AS-II, and the third aqueous solution AS-III has a pH-value in the range of 5.0-8.0, 5.5-7.5, 6.0-7.5, or 6.5-7.5. In some embodiments of the method of the invention, in which said one or more aqueous extractions of any one of steps (c-1), (c-2), and (c-x) comprise said first aqueous extraction comprising steps (Ex-I) to (Ex-IV), and said second aqueous extraction comprising steps (Ex-V) to (Ex-VIII), and said third aqueous extraction comprising steps (Ex-IX) to (Ex-XII), none of the first aqueous solution AS-I, the second aqueous solution AS-II, and the third aqueous solution AS-III comprises a compound having a pKa-value equal to or larger than 9.0, 8.5, 8.0, 7.5, 7.0, 6.5, 6.0 or 5.5. The pKa-value is to be determined in water (i.e., in an aqueous solution of the respective compound) at 25 °C.

Step (Ex-I) is: Combining the solution comprising the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)) with a first aqueous solution AS-I. This means that the solution comprising the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)) and the first aqueous solution AS-I are combined in the vessel or funnel or other receptacle, in which the agitation of step (Ex-II) is to be performed. Step (Ex-V) is: Combining the first organic phase OP-I with a second aqueous solution AS-II. This means that the first organic phase OP-I and the second aqueous solution AS-II are combined in the vessel or funnel or other receptacle, in which the agitation of step (Ex-VI) is to be performed. Step (Ex-IX) is: Combining the second organic phase OP-II with a third aqueous solution AS-III. This means that the second organic phase OP-II and the third aqueous solution AS-III are combined in the vessel or funnel or other receptacle, in which the agitation of step (Ex-X) is to be performed. Step (Ex-A') is: Combining the organic phase obtained from the previous aqueous extraction with an aqueous solution. This means that the organic phase from the previous aqueous extraction and the aqueous solution are combined in the vessel or funnel or other receptacle, in which the agitation of step (Ex-B') is to be performed.

The term "agitating" in steps (Ex-II), (Ex-VI), (Ex-X), and (Ex-B') may be understood in the broadest sense to refer to any operation of inducing a movement of the respective mixture. This typically facilitates the extraction process. Suitable means of agitation are known to those skilled in the art and comprise, e.g., shaking, stirring, e.g., mechanical stirring, bubbling of an inert has such as nitrogen, and inversion of the vessel or funnel or other receptacle in which the one or more aqueous extractions may be carried out. More than one such means of agitation may be used in combination. On an industrial scale, a reaction vessel made of a suitable material, e.g., stainless steel, equipped with a mechanical stirrer may preferably be used for the agitation step (Ex-2), (Ex-6), (Ex-X) or (Ex-B'). In practice, it is usually most convenient, to carry out all steps of an aqueous extraction in the same vessel.

Under the agitation of step (Ex-II), (Ex-VI), (Ex-X), and (Ex-B') the respective organic phase and the respective aqueous phase usually form a dispersion. Under such conditions, one phase may typically not be removed from the other. For this purpose, phase separation is usually required. Hence:
- Step (Ex-III) comprises "Allowing the phases to separate, so as to obtain a first organic phase OP-I and a first aqueous phase AP-I";
- Step (Ex-VII) comprises "Allowing the phases to separate, so as to obtain a second organic phase OP-II and a second aqueous phase AP-II";
- Step (Ex-XI) comprises "Allowing the phases to separate, so as to obtain a third organic phase OP-III and a third aqueous phase AP-III"; and
- Step (Ex-C') comprises "Allowing the phases to separate, so as to obtain an organic phase and an aqueous phase."

As used herein, the term "allowing the phases to separate so as to obtain an organic phase and an aqueous phase" may be understood in the broadest sense as establishing conditions under which the organic phase forms one or more, preferably one, layer (i.e., organic layer) and the aqueous phase forms one or more, preferably one, layer (i.e., aqueous layer). The skilled artisan understands that an organic layer formed from an organic phase may typically not be completely free of an aqueous phase and that an aqueous layer formed from an aqueous phase may typically not be completely free of an organic phase. Hence, an organic layer is mostly but not necessarily completely composed of an organic phase and an aqueous layer is mostly but not necessarily completely composed of an aqueous phase.

"Establishing conditions under which the organic phase forms one or more, preferably one, layer (i.e., organic layer) and the aqueous phase forms one or more, preferably one, layer (i.e., aqueous layer)" may usually comprise stopping the agitation. This means that the shaking and/or stirring and/or inert gas bubbling and/or inversion of the vessel or funnel or other receptacle is stopped. It will be understood that some movement of the liquids may still occur.

Hence, in some preferred embodiments, step (Ex-III) is: Stopping the agitation and allowing the phases to separate, so as to obtain a first organic phase OP-I and a first aqueous phase AP-I, wherein the first organic phase OP-I comprises the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)). In some preferred embodiments, step (Ex-VII) is: Stopping the agitation and allowing the phases to separate, so as to obtain a second organic phase OP-II and a second aqueous phase AP-II, wherein the second organic phase OP-II comprises the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)). In some preferred embodiments, step (Ex-XI) is: Stopping the agitation and allowing the phases to separate, so as to obtain a third organic phase OP-III and a third aqueous phase AP-III, wherein the third organic phase OP-III comprises the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)). In some preferred embodiments, step (Ex-C') is: Stopping the agitation and allowing the phases to separate, so as to obtain an organic phase and an aqueous phase, wherein the organic phase comprises the component (C-0)^{#} (in step (c-1)) or the first cycle oligonucleotide (O-1)^{#} (in step (c-2)) or the (x-1)-th cycle oligonucleotide (O-(x-1))^{#} (in a step (c-x)).

Typically, one may simply wait for the phases to separate. Phase separation may optionally be speeded up by addition of aqueous solutions comprising dissolved ions, e.g., by addition of brine (i.e., an aqueous solution of sodium chloride), as known to those skilled in the art. The expression "the organic phase comprises the component C" has been explained above.

In steps (Ex-IV), (Ex-VIII), (Ex-XII), and (Ex-D'), the respective aqueous phase is removed from the respective organic phase or vice versa. This means that at least one aqueous layer is physically removed from at least one organic layer or vice versa. This removal may typically comprise draining of one layer, typically the bottom layer, e.g., the aqueous layer. It will be understood that this removal is not necessarily complete. In other words, "removing" in this context is not to mean "completely removing" but preferably means "mostly removing". In this context, removing one phase from another phase may mean removing at least 51 vol-%, 60 vol-%, 70 vol-%, 80 vol-%, 90 vol-%, 95 vol-%, 96 vol-%, 97 vol-%, 98 vol-% or 99 vol-% of the respective phase to be removed. In practice, there will usually be a clearly recognizable phase boundary between the organic layer and the aqueous layer, so that removing the one from the other may simply be achieved by draining the bottom layer until the phase boundary is reached.

Step (d-1) of the methods of the invention is: optionally, reducing the water content of the organic phase comprising the component (C-0)^{#}.

Step (d-2) of some of the methods of the invention is: optionally, reducing the water content of the organic phase comprising the first cycle oligonucleotide (O-1)^{#}.

Step (d-x) of some of the methods of the invention is: optionally, reducing the water content of the organic phase comprising the (x-1)-th cycle oligonucleotide (O-(x-1))^{#}*.*

The term "optionally" in steps (d-1), (d-2), and (d-x) denotes that the respective step may or may not be carried out in a given coupling cycle, unless indicated differently in the context of specific embodiments. In some embodiments of the method of the invention, step (d-1) is carried out (i.e. is not optional). In some embodiments of the method of the invention, in the second coupling cycle, step (d-2) is carried out (i.e. is not optional). In some embodiments of the method of the invention, in at least one, or each, coupling cycle comprising steps (b-x) to (h-x) (as far as present), step (d-x) is carried out (i.e. is not optional).

The term "the organic phase" in steps (d-1), (d-2), and (d-x) refers to "the organic phase" of the respective step (c-1) or (c-2) or (c-x) of the same coupling cycle.

The term "reducing the water content" may be understood in the broadest sense as any operation during which the concentration of water, preferably measured via standard Karl Fischer titration at 25 °C, is reduced. The set up may, for example, be as follows: The titration cell may be filled with HYDRANAL^{™} -Coulomat Oil for anolyte reagent, and the inner burette may be filled with HYDRANAL^{™} -Coulomat CG for catholyte reagent. After pretitration for dehydration, sample (100 µL) may be added to the titration sell and the measurement may be started. In line with common practice, the water content of solutions is herein reported in parts per million (ppm).

Said reduction of the water content may be characterized by its end point. Preferably, the water content is reduced so that (i.e. until) it is equal to or less than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm. In some embodiments of the method of the invention, step (d-1) is: optionally, reducing the water content of the organic phase comprising the component (C-0)^{#}, wherein the water content is adjusted to be equal to or less than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm. In some embodiments of the method of the invention, step (d-2) is: optionally, reducing the water content of the organic phase comprising the first cycle oligonucleotide (O-1)^{#}, wherein the water content is adjusted to be equal to or less than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm. In some embodiments of the method of the invention, step (d-x) is: optionally, reducing the water content of the organic phase comprising the (x-1)-th cycle oligonucleotide (O-(x-1))^{#}, wherein the water content is adjusted to be equal to or less than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm.

In some embodiments of the method of the invention:
- step (d-1) is carried out (i.e. is not optional), if the organic phase comprising the component (C-0)^{#} of step (c-1) comprises more than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm of water;
- step (d-2) is carried out (i.e. is not optional), if the organic phase comprising the first cycle oligonucleotide (O-1)^{#} of step (c-2) comprises more than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm of water; and
- step (d-x) is carried out (i.e. is not optional), if the organic phase comprising the (x-1)-th cycle oligonucleotide (O-(x-1))^{#} of step (c-x) of the same coupling cycle comprises more than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm of water.

Any means of reducing the water content may be employed, preferred examples of which comprise azeotropic distillation and the use of a drying agent. In some embodiments of the method of the invention, in each of steps (d-1), (d-2), and (d-x), reducing the water content is achieved by means of azeotropic distillation (i.e. by subjecting said organic phase comprising the component (C-0)^{#} or the first cycle oligonucleotide (O-1)^{#} or the (x-1)-th cycle oligonucleotide (O-(x-1))^{#} to azeotropic distillation) and/or by means of contacting said organic phase comprising the component (C-0)^{#} or the first cycle oligonucleotide (O-1)^{#} or the (x-1)-th cycle oligonucleotide (O-(x-1))^{#} with a drying agent. In some embodiments of the method of the invention, in at least one or each of steps (d-1), (d-2), and (d-x), reducing the water content is achieved by means of azeotropic distillation.

In some embodiments of the method of the invention, in at least one or each of steps (d-1), (d-2), and (d-x), reducing the water content is achieved by means of contacting said organic phase comprising the component (C-0)^{#} or the first cycle oligonucleotide (O-1)^{#} or the (x-1)-th cycle oligonucleotide (O-(x-1))^{#} with a drying agent. It will be understood that in any one of steps (d-1), (d-2), and (d-x), one or more solvents and/or further components may be added to said organic phase comprising the component (C-0)^{#} or the first cycle oligonucleotide (O-1)^{#} or the (x-1)-th cycle oligonucleotide (O-(x-1 ))^{#} prior to, during, and/or after reduction of the water content.

The term "azeotropic distillation" may be understood in the broadest sense to refer to a distillation, in which an azeotrope of water and one or more organic solvents is distilled off. Said one or more organic solvents may already be comprised in the organic phase and/or may be added to the organic phase prior to and/or during azeotropic distillation. Preferably, said one or more organic solvents are selected so that the azeotrope with water has a lower boiling point than water itself and any one of the one or more organic solvents used to form said azeotrope. Preferred examples of said one or more organic solvents comprise toluene, benzene, and ether solvents, e.g. ether solvents S^{E} as defined herein (vide infra), in particular 4-methyltetrahydropyran (MTHP). A plethora of azeotropic data is available to the skilled artisan, e.g., in tabulated form, enabling him to select one or more suitable organic solvents for an azeotropic distillation.

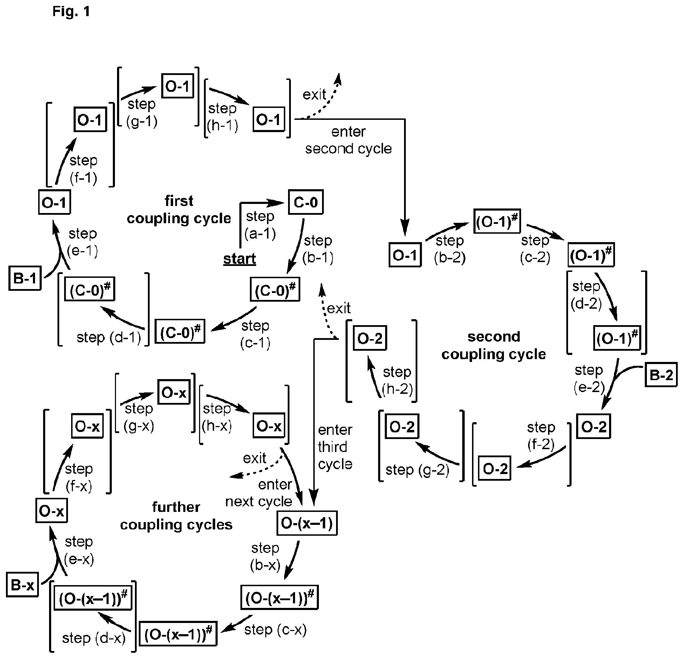

As used herein, the term "drying agent" may be understood in the broadest sense to refer to any salt or compound or macroscopic structure used to remove water from one or more organic solvents or solutions comprising one or more organic solvents. Any drying agent used for reducing the water content of organic solvents or solutions comprising organic solvents may be used in any one of steps (d-1), (d-2), and (d-x) of the method of the invention. Non-limiting examples of suitable drying agents comprise molecular sieves, in particular 3 Å (i.e. 3 angstrom) or 4 Å molecular sieves, sulfuric acid, and inorganic salts such as sodium sulfate, magnesium sulfate, and calcium chloride. For example, the drying agent may be added to the organic phase, the water content of which is to be reduced. Alternatively, the organic phase, the water content of which is to be reduced, may be passed through a compartment, e.g. a column, packed with a drying agent, e.g. molecular sieves, as, for example, disclosed in X. Shi et al., The Journal of Organic Chemistry 2022, 87(4), 20872110 (cf., e.g., Figure 1 of the cited reference and explanations pertaining thereto).

Step (e-1) of the methods of the invention is: reacting the component (C-0)^{#} with a building block B-1, wherein said building block B-1 is selected from the group consisting of a nucleoside and an oligonucleotide and comprises
- a backbone hydroxyl moiety protected by a protecting group PG-1 removable under acidic conditions, and
- a phosphorus moiety covalently bonded via its phosphorus atom to an oxygen atom of the backbone of the building block B-1,
under conditions suitable to form a covalent bond between said free backbone hydroxyl group of the component (C-0)^{#} and the phosphorus atom of said phosphorus moiety of the building block B-1, thereby obtaining a first cycle oligonucleotide O-1.

Step (e-2) of some of the methods of the invention is: reacting the first cycle oligonucleotide (O-1)^{#} with a building block B-2, wherein said building block B-2 is selected from the group consisting of a nucleoside and an oligonucleotide and comprises
- a backbone hydroxyl moiety protected by a protecting group PG-2 removable under acidic conditions, and
- a phosphorus moiety covalently bonded via its phosphorus atom to an oxygen atom of the backbone of the building block B-2,
under conditions suitable to form a covalent bond between said free backbone hydroxyl group of the first cycle oligonucleotide (O-1)^{#} and the phosphorus atom of said phosphorus moiety of the building block B-2, thereby obtaining a second cycle oligonucleotide O-2.

Step (e-x) of some of the methods of the invention is: reacting the (x-1)-th cycle oligonucleotide (O-(x-1))^{#} with a building block B-x, wherein said building block B-x is selected from the group consisting of a nucleoside and an oligonucleotide and comprises
- a backbone hydroxyl moiety protected by a protecting group PG-x removable under acidic conditions, and
- a phosphorus moiety covalently bonded via its phosphorus atom to an oxygen atom of the backbone of the building block B-x,
under conditions suitable to form a covalent bond between said free backbone hydroxyl group of the (x-1)-th cycle oligonucleotide (O-(x-1))^{#} and the phosphorus atom of said phosphorus moiety of the building block B-x, thereby obtaining a x-th cycle oligonucleotide O-x.

The terms "nucleoside and oligonucleotide" have been explained above. In some embodiments of the method of the invention, each of the building blocks B-1, B-2, and B-x is selected from the group consisting of a nucleoside and an oligonucleotide comprising not more than 50, 40, 30, 25, 20, 15, 10, or 5 nucleoside subunits. In some embodiments of the method of the invention, each of the building blocks B-1, B-2, and B-x is a nucleoside. In some embodiments of the method of the invention, each of the building blocks B-1, B-2, and B-x is an oligonucleotide. In some embodiments of the method of the invention, each of the building blocks B-1, B-2, and B-x is an oligonucleotide comprising not more than 50, 40, 30, 25, 20, 15, 10, or 5 nucleoside subunits.

The term "backbone hydroxyl moiety" refers to a hydroxyl moiety which is part of the backbone of the respective building block B-1, B-2 or B-x and will be understood based on the above explanations of the terms "hydroxyl moiety" and "backbone". The terms "protecting group" and "removable under acidic conditions" have been defined. The protecting groups PG-1, PG-2, and PG-x have been explained. It will be understood that said protecting group PG-x may be the same or different (i.e. have the same or a different chemical structure) in each coupling cycle comprising steps (b-x) to (h-x) (as far as present), unless indicated differently in the context of specific embodiments.

Per definition, each of the building blocks B-1, B-2, and B-x further comprises a phosphorus moiety covalently bonded via its phosphorus atom to an oxygen atom of the backbone of the respective building block B-1, B-2, and B-x. The term "an oxygen atom of the backbone" refers to an oxygen atom which is part of the backbone of the respective building block B-1, B-2 or B-x and will be understood based on the above explanation of the term "backbone".

In some embodiments, each of the building blocks B-1, B-2, and B-x comprises:
- exactly one backbone hydroxyl moiety protected by a protecting group PG-1 or PG-2 or PG-x removable under acidic conditions, and
- exactly one phosphorus moiety covalently bonded via its phosphorus atom to an oxygen atom of the backbone of the respective building block B-1 or B-2 or B-x.

In some embodiments of the method of the invention, each of the building blocks B-1, B-2, and B-x comprises:
- exactly one protecting group removable under acidic conditions, which is said protecting group PG-1 or PG-2 or PG-x, and
- exactly one phosphorus moiety covalently bonded via its phosphorus atom to an oxygen atom of the backbone of the respective building block B-1 or B-2 or B-x.

In some embodiments of the method of the invention, each of the building blocks B-1, B-2, and B-x comprises:
- exactly one protecting group comprising an optionally substituted triarylmethyl residue, which is said protecting group PG-1 or PG-2 or PG-x, and
- exactly one phosphorus moiety covalently bonded via its phosphorus atom to an oxygen atom of the backbone of the respective building block B-1 or B-2 or B-x.

In some embodiments of the method of the invention, each of the building blocks B-1, B-2, and B-x is a compound of the following Formula II: 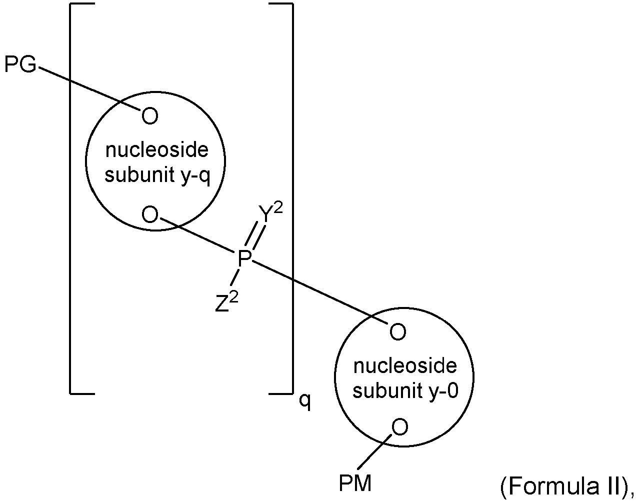 wherein in Formula II:
each oxygen atom (O) depicted within each nucleoside subunit y-0 to y-q represents the oxygen atom of a hydroxyl moiety of the respective nucleoside subunit;
each nucleoside subunit y-0 to y-q may be the same or different (i.e. have the same or a different chemical structure);
PM is a phosphorus moiety;
PG is the protecting group PG-1 (for building block B-1) or PG-2 (for building block B-2) or PG-x (for a building block B-x) and is a protecting group removable under acidic conditions;
q is an integer equal to or larger than 0;
Y² is selected independently for each repetitive unit q from the group consisting of O and S;
Z² is selected independently for each repetitive unit q from the group consisting of O-R^{z-2}, and S-R^{z-2}; and
R^{z-2} is a protecting group, which may be the same or different for each repetitive unit q.

In some embodiments of the method of the invention, each of the building blocks B-1, B-2, and B-x is a compound of the following Formula II-a: 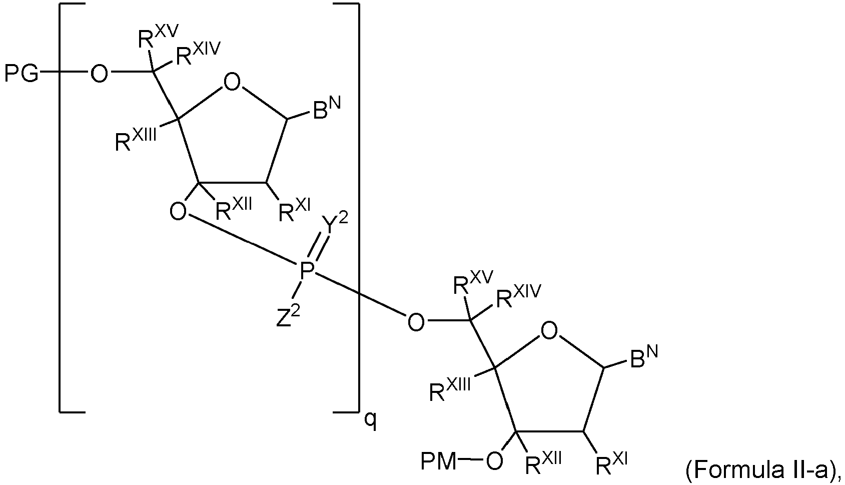 wherein in Formula II-a:
q, PG, Y², Z², R^{z-2}, and PM are defined as for Formula II;
B^{N} is a nucleobase, which may be the same or different (i.e. have the same or a different chemical structure) at each occurrence;
R^{XI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and
O-CH₂-O-Si(C₁-C₅-alkyl)₃;
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++,
where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the following Formula II-a-tc: 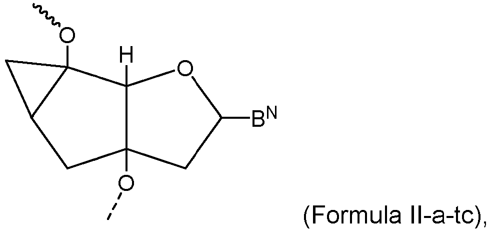
wherein in Formula II-a-tc:
   the oxygen atom from which the dashed line originates represents the oxygen atom bonded to the 3'-carbon atom, i.e. the carbon atom to which R^{XII} is bonded in Formula II-a;
   the dashed line indicates the covalent chemical bond interconnecting the respective oxygen atom of the nucleoside subunit of Formula II-a-tc and the phosphorus moiety PM or P(Y²)(Z²) in Formula II-a;
   the oxygen atom from which the wavy line originates represents the oxygen atom bonded to the 5'-carbon atom, i.e. the carbon atom to which C(R^{XIV})(R^{XV}) is bonded in Formula II-a; and
   the wavy line indicates the covalent chemical bond interconnecting the respective oxygen atom of the nucleoside subunit of Formula II-a-tc and either PG or P(Y²)(Z²) in Formula II-a.

In some embodiments of the method of the invention each of the building blocks B-1, B-2, and B-x is a compound of the following Formula II-b: 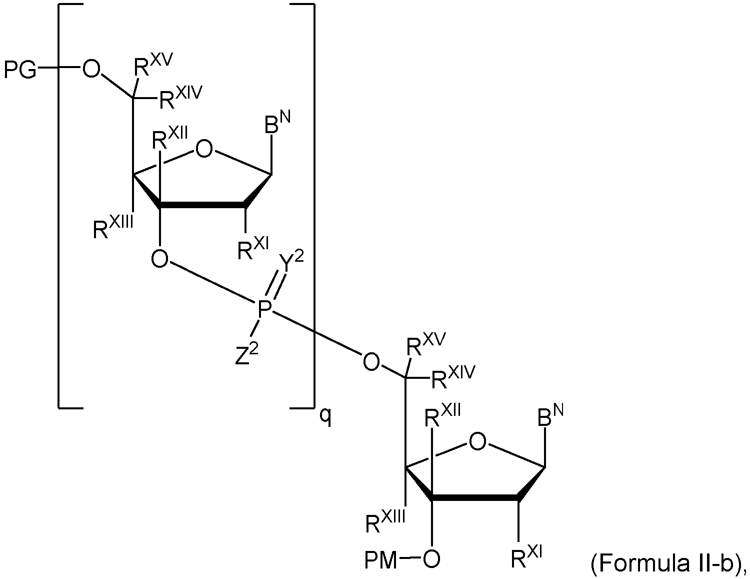 wherein in Formula II-b:
q, PG, Y², Z², R^{z-2}, PM, B^{N}, R^{XI}, and R^{XIII} are defined as for Formula II-a, and
for each nucleoside subunit independently, R^{XII}, R^{XIV}, and R^{XV} are either all H or they are bonded together so that the respective nucleoside subunit has a structure of the following Formula II-b-tc: 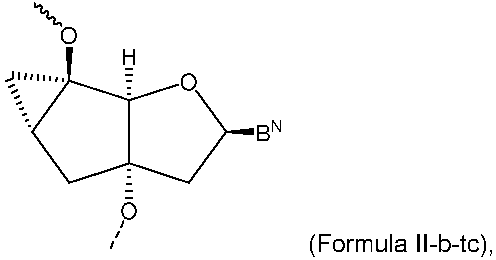
wherein in Formula II-b-tc:
   the oxygen atom from which the dashed line originates represents the oxygen atom bonded to the 3'-carbon atom, i.e. the carbon atom to which R^{XII} is bonded in Formula II-b;
   the dashed line indicates the covalent chemical bond interconnecting the respective oxygen atom of the nucleoside subunit of Formula II-b-tc and either the phosphorus moiety PM or P(Y²)(Z²) in Formula II-b;
   the oxygen atom from which the wavy line originates represents the oxygen atom bonded to the 5'-carbon atom, i.e. the carbon atom to which C(R^{XIV})(R^{XV}) is bonded in Formula II-b; and
   the wavy line indicates the covalent chemical bond interconnecting the respective oxygen atom of the nucleoside subunit of Formula II-b-tc and either PG or P(Y²)(Z²) in Formula II-b.

As used herein, the term "phosphorus moiety" may be understood in the broadest sense and may refer to any atom group comprising at least one, preferably exactly one, phosphorus atom, wherein the term "atom group" may refer to two or more atoms. In such an atom group, it is not required that each atom is covalently bonded to each further atom of said atom group, but that each atom is covalently bonded to at least one further atom of said atom group. From steps (e-1), (e-2), and (e-x) it is clear that said phosphorus moiety covalently bonded via its phosphorus atom to an oxygen atom of the backbone of the respective building block B-1 or B-2 or B-x, e.g. the phosphorus moiety PM of any one of Formulae II, II-a, and II-b, is a phosphorus moiety capable of engaging in a bond forming (condensation) reaction with a free hydroxyl group. The skilled artisan is aware of phosphorus moieties which fulfill this requirement and is able to select a suitable phosphorus moiety without undue experimentation. It is further known to those skilled in the art, that the phosphorus moiety which engages in such a bond forming reaction will typically form an internucleosidic linkage group of the elongated oligonucleotide formed via said bond forming reaction. Said phosphorus moiety covalently bonded via its phosphorus atom to an oxygen atom of the backbone of the respective building block B-1 or B-2 or B-x, e.g. the phosphorus moiety PM of any one of Formulae II, II-a, and II-b, may be a phosphorus (III) moiety, also referred to as P (III) moiety, i.e. a phosphorus moiety comprising a P (III) atom as defined herein. Non-limiting examples of such P (III) moieties are phosphoramidite moieties, as e.g. disclosed in X. Wei et al., Tetrahedron 2013, 69, 3615-3637, and H-phosphonate monoester moieties, as e.g. disclosed in J. Stawinski and R. Strömberg (2005), Di- and Oligonucleotide Synthesis Using H-Phosphonate Chemistry, in Methods in Molecular Biology, vol. 288: Oligonucleotide Synthesis: Methods and Applications, edited by P. Herdewijn, Humana Press Inc., Totowa NJ, https://doi.org/10.1385/1-59259-823-4:081. Alternatively, said phosphorus moiety covalently bonded via its phosphorus atom to an oxygen atom of the backbone of the respective building block B-1 or B-2 or B-x, e.g. the phosphorus moiety PM of any one of Formulae II, II-a, and II-b, may be a phosphorus (V) moiety, also referred to as P (V) moiety, i.e. a phosphorus moiety comprising a P (V) atom as defined herein. Examples of such P (V) moieties comprise classical aryl phosphate diester moieties as e.g. exemplified by H. Lönneberg (Beilstein Journal of Organic Chemistry, 2017, 13, 1368-1387) and the P (V) moieties disclosed by Baran et al. (Science 2018, 361, 12341238 and ACS Central Science 2021, 7, 1473-1485).

In some embodiments of the method of the invention, each of the building blocks B-1, B-2, and B-x is a compound of the following Formula II-1: 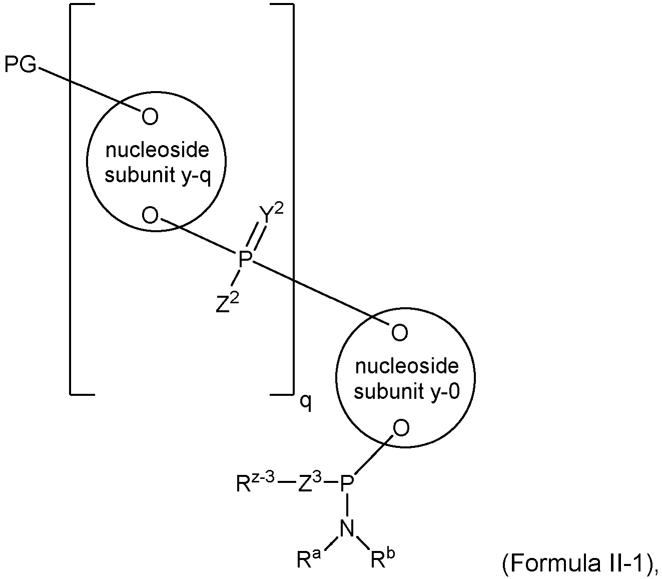 wherein in Formula II-1:
each oxygen atom (O) depicted within each nucleoside subunit y-0 to y-q represents the oxygen atom of a hydroxyl moiety of the respective nucleoside subunit;
each nucleoside subunit y-0 to y-q may be the same or different;
PG is the protecting group PG-1 (for the building block B-1) or PG-2 (for the building block B-2) or PG-x (for a building block B-x) and is a protecting group removable under acidic conditions;
q is an integer equal to or larger than 0;
Y² is selected independently for each repetitive unit q from the group consisting of O and S;
Z² is selected independently for each repetitive unit q from the group consisting of O-R^{z-2} and S-R^{z-2};
R^{z-2} is a protecting group, which may be the same or different for each repetitive unit q;
Z³ is selected from the group consisting of O and S; and
R^{z-3} is a protecting group;
each of R^{a} and R^{b} is a C₁-C₆-alkyl group, wherein R^{a} and R^{b} may be the same or different and may also bond to each other to form a 5-membered or 6-membered aliphatic cyclic amine moiety together with the nitrogen atom to which R^{a} and R^{b} are bonded;
and wherein step (f-1) or (f-2) or (f-x) is carried out in each coupling cycle.

In some embodiments of the method of the invention, each of the building blocks B-1, B-2, and B-x is a compound of the following Formula II-1-a: 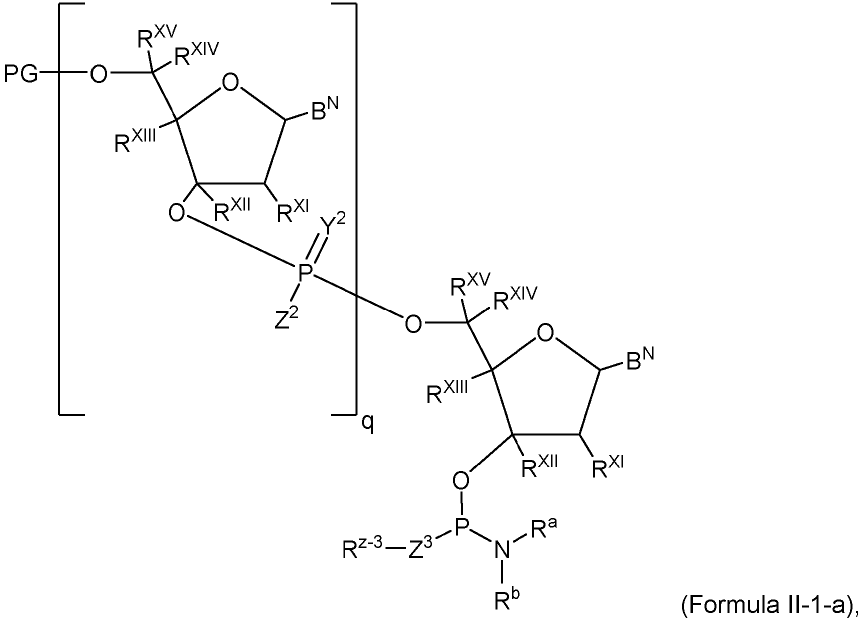 wherein in Formula II-1-a:
q, PG, Y², Z², R^{z-2}, Z³, R^{z-3}, R^{a}, and R^{b} are defined as for Formula II-1; and
B^{N}, R^{XI}, R^{XII}, R^{XIII}, R^{XIV}, and R^{XV} are defined as for Formula II-a, and
wherein step (f-1) or (f-2) or (f-x) is carried out in each coupling cycle.

In some embodiments of the method of the invention, each of the building blocks B-1, B-2, and B-x is a compound of the following Formula II-1-b: 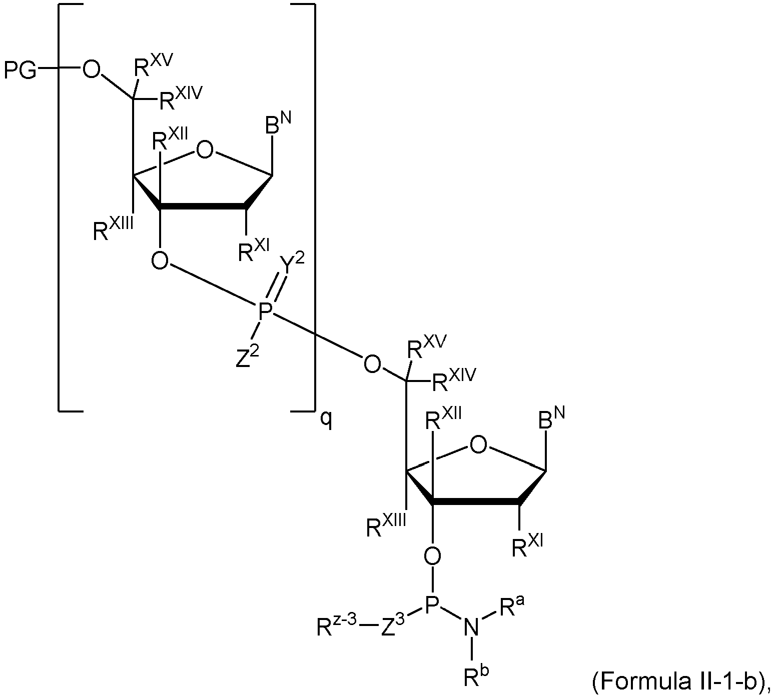 wherein in Formula II-1-b:
q, PG, Y², Z², R^{z-2}, Z³, R^{z-3}, R^{a}, and R^{b} are defined as for Formula II-1,
B^{N}, R^{XI}, R^{XII}, R^{XIII}, R^{XIV}, and R^{XV} are defined as for Formula II-b, and
wherein step (f-1) or (f-2) or (f-x) is carried out in each coupling cycle.

In some embodiments of the method of the invention, each of the building blocks B-1, B-2, and B-x is a compound of the following Formula II-2: 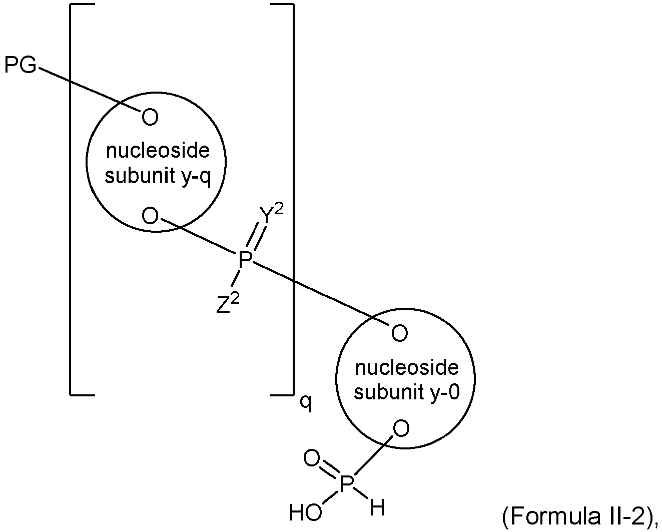 wherein in Formula II-2:
each oxygen atom (O) depicted within each nucleoside subunit y-0 to y-q represents the oxygen atom of a hydroxyl moiety of the respective nucleoside subunit;
each nucleoside subunit y-0 to y-q may be the same or different (i.e. have the same or a different chemical structure);
PG is the protecting group PG-1 (for building block B-1) or PG-2 (for building block B-2) or PG-x (for a building block B-x) and is a protecting group removable under acidic conditions;
q is an integer equal to or larger than 0;
Y² is selected independently for each repetitive unit q from the group consisting of O and S;
Z² is selected independently for each repetitive unit q from the group consisting of H, O-R^{z-2}, and S-R^{z-2}; and
R^{z-2} is a protecting group, which may be the same or different for each repetitive unit q; and
wherein at least in the final coupling cycle, step (f-1) or (f-2) or (f-x) is carried out.

In some embodiments of the method of the invention, each of the building blocks B-1, B-2, and B-x is a compound of the following Formula II-2-a: 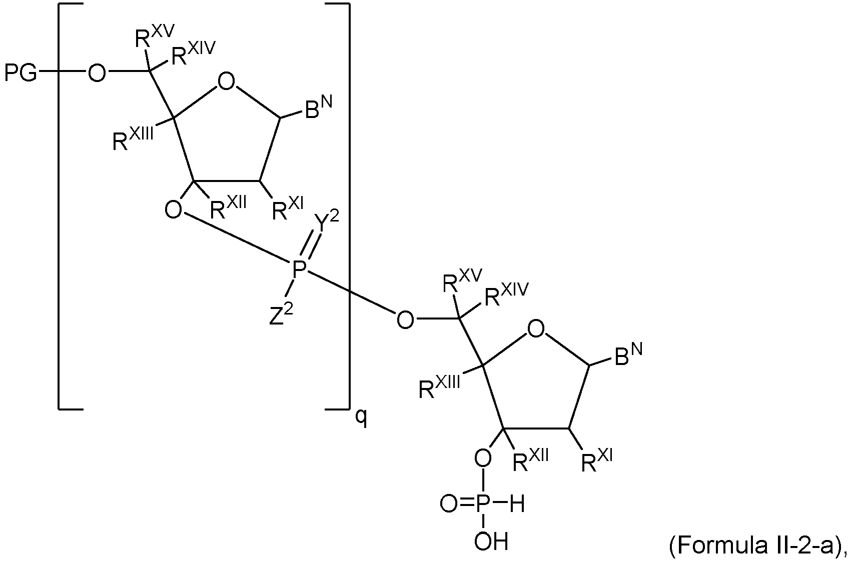 wherein in Formula II-2-a:
q, PG, Y², Z², and R^{z-2} are defined as for Formula II-2;
B^{N}, R^{XI}, R^{XII}, R^{XIII}, R^{XIV}, and R^{XV} are defined as for Formula II-a; and
wherein at least in the final coupling cycle, step (f-1) or (f-2) or (f-x) is carried out.

In some embodiments of the method of the invention, each of the building blocks B-1, B-2, and B-x is a compound of the following Formula II-2-b: 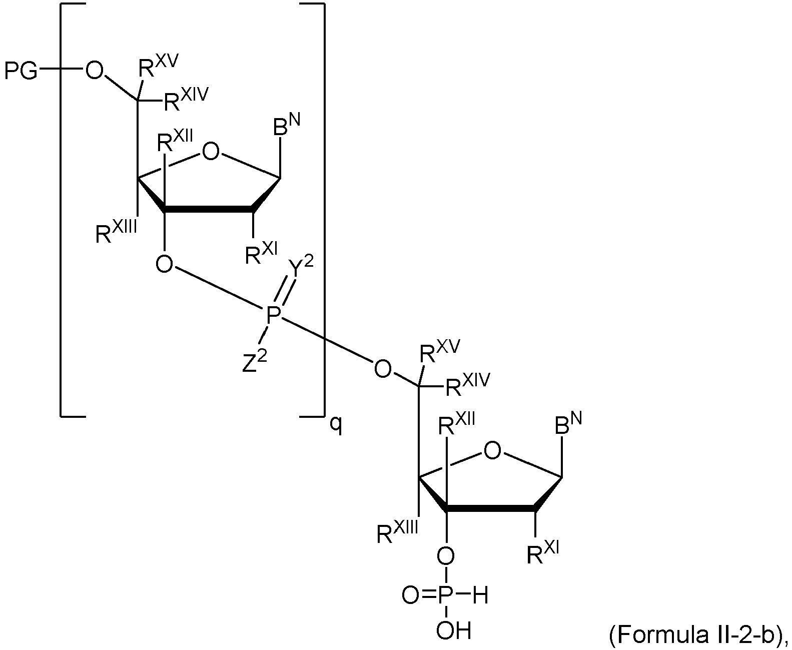 wherein in Formula II-2-b:
q, PG, Y², Z², and R^{z-2} are defined as for Formula II-2;
B^{N}, R^{XI}, R^{XII}, R^{XIII}, R^{XIV}, and R^{XV} are defined as for Formula II-b, and
wherein at least in the final coupling cycle, step (f-1) or (f-2) or (f-x) is carried out.

In some embodiments, in the building block B-1 or B-2 or B-x of any one of Formulae II, II-a, II-b, II-1, II-1-a, II-1-b, II-2, II-2-a, and II-2-b, the integer q is an integer in the range of 0-50, 0-35, 0-30, 0-25, 0-20, 0-15, 0-10, 0-5, 0-3, 0-2, 0-1 or q is 0. In some embodiments, in the building block B-1 or B-2 or B-x of any one of Formulae II, II-a, II-b, II-1, II-1-a, II-1-b, II-2, II-2-a, and II-2-b, the integer q is 0. It will be understood that, if the integer q in any one of Formulae II, II-a, II-b, II-1, II-1-a, II-1-b, II-2, II-2-a, and II-2-b is 0, the protecting group PG is bonded to the respective hydroxyl moiety of the then remaining nucleoside subunit carrying the phosphorus moiety (e.g. nucleoside subunit y-0 in Formulae II, II-1 or II-2).

In some embodiments, in the building block B-1 or B-2 or B-x of any one of Formulae II, II-a, II-b, II-1, II-1-a, II-1-b, II-2, II-2-a, and II-2-b, the protecting group PG is a protecting group comprising an optionally substituted triarylmethyl residue. In some embodiments, in the building block B-1 or B-2 or B-x of any one of Formulae II, II-a, II-b, II-1, 11-1-a, II-1-b, II-2, II-2-a, and II-2-b, the protecting group PG is selected from the group consisting of the triphenylmethyl group (i.e. the trityl group), the (p-methylphenyl)diphenylmethyl group (i.e. the 4-methyltrityl group), the di(p-methylphenyl)phenylmethyl group (i.e. the 4,4'-dimethyltrityl group), the tri(p-methylphenyl)methyl group (i.e. the 4,4',4"-trimethyltrityl group), the (p-methoxyphenyl)diphenylmethyl group (i.e. the MMT group), and the di(p-methoxyphenyl)phenylmethyl group (i.e. the DMT group). In some embodiments, in the building block B-1 or B-2 or B-x of any one of Formulae II, II-a, II-b, II-1, II-1-a, II-1-b, II-2, II-2-a, and II-2-b, the protecting group PG is selected from the group consisting of the triphenylmethyl group, the (*p*-methoxyphenyl)diphenylmethyl group, and the di(*p*-methoxyphenyl)phenylmethyl group. In some embodiments, in the building block B-1 or B-2 or B-x of any one of Formulae II, II-a, II-b, II-1, 11-1-a, 11-1-b, II-2, II-2-a, and II-2-b, the protecting group PG is the di(*p*-methoxyphenyl)phenylmethyl group.

In some embodiments, in the building block B-1 or B-2 or B-x of any one of Formulae II, II-a, II-b, II-1, II-1-a, II-1-b, II-2, II-2-a, and II-2-b, Y² is at each occurrence O. In some embodiments, in the building block B-1 or B-2 or B-x of any one of Formulae II, II-a, II-b, 11-1, 11-1-a, II-1-b, II-2, II-2-a, and II-2-b, Y² is at each occurrence S.

In some embodiments, in the building block B-1 or B-2 or B-x of any one of Formulae II, II-a, II-b, 11-1, II-1-a, II-1-b, II-2, II-2-a, and II-2-b, R^{z-2} is a protecting group removable under alkaline conditions, wherein R^{z-2} may be the same or different at each occurrence. The term "removable under alkaline conditions" has been explained above. In some embodiments, in the building block B-1 or B-2 or B-x of any one of Formulae II, II-a, II-b, II-1, II-1-a, II-1-b, II-2, II-2-a, and II-2-b, R^{z-2} is for each repetitive unit q independently a protecting group of the chemical structure CH₂-CH₂-EWG, where EWG is an electron withdrawing group, preferably a cyano group. The electron withdrawing group may for example be selected from the group consisting of a cyano group, a halogen atom such as a chlorine, fluorine, or bromine atom, a formyl group, a keto group, a carboxyester group, or a carboxamide group. In some embodiments, in the building block B-1 or B-2 or B-x of any one of Formulae II, II-a, II-b, 11-1, 11-1-a, II-1-b, II-2, II-2-a, and II-2-b, R^{z-2} is for each repetitive unit q a 2-cyanoethyl group (i.e. CH₂-CH₂-CN).

In some embodiments, in the building block B-1 or B-2 or B-x of any one of Formulae II, II-a, II-b, 11-1, II-1-a, II-1-b, II-2, II-2-a, and II-2-b, Z² is selected independently for each repetitive unit q from the group consisting of O-R^{z-2} and S-R^{z-2}. In some embodiments, in the building block B-1 or B-2 or B-x of any one of Formulae II, II-a, II-b, II-1, II-1-a, II-1-b, II-2, II-2-a, and II-2-b, Z² is selected independently for each repetitive unit q from the group consisting of O-R^{z-2} and S-R^{z-2}, where R^{z-2} is for each repetitive unit q a 2-cyanoethyl group (i.e. CH₂-CH₂-CN). In such embodiments, Z² is selected independently for each repetitive unit q from the group consisting of O-CH₂-CH₂-CN and S-CH₂-CH₂-CN. In some embodiments, in the building block B-1 or B-2 or B-x of any one of Formulae II, II-a, II-b, II-1, II-1-a, II-1-b, II-2, II-2-a, and II-2-b, Z² is for each repetitive unit q O-R^{z-2}, where R^{z-2} is for each repetitive unit q a 2-cyanoethyl group (i.e. CH₂-CH₂-CN). In such embodiments, Z² is for each repetitive unit q O-CH₂-CH₂-CN.

In some embodiments, in the building block B-1 or B-2 or B-x of any one of Formulae II, II-a, II-b, II-2, II-2-a, and II-2-b, Z² is for each repetitive unit q H. In some embodiments, in the building block B-1 or B-2 or B-x of any one of Formulae II, II-a, II-b, II-2, II-2-a, and II-2-b, Y² is for each repetitive unit q O and Z² is for each repetitive unit q H.

The skilled person will understand that any kind of nucleobase B^{N} may be present in a building block B-1 or B-2 or B-x of any one of Formulae II-a, II-b, II-1-a, II-1-b, II-2-a, and II-2-b. In some embodiments, in the building block B-1 or B-2 or B-x of any one of Formulae II-a, II-b, II-1-a, II-1-b, II-2-a, and II-2-b, B^{N} is a nucleobase and at each occurrence independently selected from the group consisting of adenine, guanine, cytosine, 5-methylcytosine, thymine, and uracil. It will be understood by those skilled in the art, that any nucleobase B^{N} in any one of Formulae II-a, II-b, 11-1-a, II-1-b, II-2-a, and II-2-b may optionally be protected, i.e. carry one or more protecting groups, without this being indicated specifically. Thus, when, for example, stating that B^{N} is adenine, guanine, 5-methylcytosine, cytosine, thymine, or uracil, this embraces the aforementioned nucleobases in protected form and in free form (i.e. with and without any protecting groups). Nucleobase protecting groups are known to those skilled in the art and have also been explained above, including, for example, the preferred nucleobase protecting groups compiled in Table T-1.

In some embodiments, each nucleobase of each building block B-1, B-2 and B-x, in particular each nucleobase B^{N} of each building block B-1, B-2 and B-x of any one of Formulae II-a, II-b, 11-1-a, 11-1-b, II-2-a, and II-2-b, is independently selected from the group consisting of
- adenine, in which the exocyclic amino group is protected;
- guanine, in which the exocyclic amino group is protected;
- cytosine, in which the exocyclic amino group is protected;
- 5-methylcytosine, in which the exocyclic amino group is protected;
- thymine; and
- uracil.

In some embodiments, each nucleobase of each building block B-1, B-2 and B-x, in particular each nucleobase B^{N} of each building block B-1, B-2 and B-x of any one of Formulae II-a, II-b, 11-1-a, 11-1-b, II-2-a, and II-2-b, is independently selected from the group consisting of
- adenine, in which the exocyclic amino group is protected by a benzoyl group, an isobutyryl group or a phenoxyacetyl group;
- guanine, in which the exocyclic amino group is protected by an isobutyryl group, a 4-isopropylphenoxyacetyl group or a dimethylformamidino group;
- cytosine, in which the exocyclic amino group is protected by an acetyl group or a benzoyl group;
- 5-methylcytosine, in which the exocyclic amino group is protected by an acetyl group or a benzoyl group;
- thymine; and
- uracil.

In some embodiments, in each building block B-1, B-2, and B-x of any one of Formulae II-a, II-b, II-1-a, II-1-b, II-2-a, and II-2-b:
R^{XI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1 or B-2 or B-x of any one of Formulae II-a, II-1-a, and II-2-a) or Formula II-b-tc (in a building block B-1 or B-2 or B-x of any one of Formula II-b, 11-1-b, and II-2-b).

In some embodiments, in each building block B-1, B-2, and B-x of any one of Formulae II-a, II-b, II-1-a, II-1-b, II-2-a, and II-2-b:
R^{XI} is at each occurrence independently selected from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert-*butyl(dimethyl)silyloxy), and O-CH₂-O-Si(C(CH₃)₃)₃ (i.e. ((triisopropylsilyl)oxy)-methyloxy);
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1 or B-2 or B-x of any one of Formulae II-a, II-1-a, and II-2-a) or Formula II-b-tc (in a building block B-1 or B-2 or B-x of any one of Formula II-b, 11-1-b, and II-2-b).

In some embodiments, in each building block B-1, B-2, and B-x of any one of Formulae II-a, II-b, II-1-a, II-1-b, II-2-a, and II-2-b:
R^{XI} is selected independently at each occurrence from the group consisting of H, F, O-CH₃ (i.e. methoxy), and O-CH₂-CH₂-O-CH₃ (i.e. 2-methoxyethyl-1-oxy); and
each of R^{XII}, R^{XIII}, R^{XIV}, and R^{XV} is H at each occurrence.

In some embodiments, in each building block B-1, B-2, and B-x of any one of Formulae II-1, II-1-a, and II-1-b, Z³ is O. In some embodiments, in each building block B-1, B-2, and B-x of any one of Formulae 11-1, II-1-a, and II-1-b, Z³ is S.

In some embodiments, in each building block B-1, B-2, and B-x of any one of Formulae II-1, II-1-a, and II-1-b, R^{z-3} is a protecting group removable under alkaline conditions. The term "removable under alkaline conditions" has been explained above. In some embodiments, in each building block B-1, B-2, and B-x of any one of Formulae II-1, II-1-a, and II-1-b, R^{z-3} is a protecting group of the chemical structure CH₂-CH₂-EWG, where EWG is an electron withdrawing group, preferably a cyano group. The electron withdrawing group may for example be selected from the group consisting of a cyano group, a halogen atom such as a chlorine, fluorine, or bromine atom, a formyl group, a keto group, a carboxyester group, or a carboxamide group. In some preferred embodiments, in each building block B-1, B-2, and B-x of any one of Formulae II-1, II-1-a, and II-1-b, R^{z-3} is a 2-cyanoethyl group (i.e. CH₂-CH₂-CN).

In some embodiments, in each building block B-1, B-2, and B-x of any one of Formulae II-1, II-1-a, and II-1-b, each of R^{a} and R^{b} is a C₁-C₆-alkyl group, wherein R^{a} and R^{b} may be the same or different, and wherein R^{a} and R^{b} may not bond to each other. In some embodiments, in each building block B-1, B-2, and B-x of any one of Formulae II-1, 11-1-a, and II-1-b, each of R^{a} and R^{b} is an isopropyl group (i.e. CH(CH₃)₂).

In some embodiments, in each building block B-1, B-2, and B-x of Formula II:
PM is a phosphorus moiety, preferably a phosphorus moiety selected from the group consisting of a phosphoramidite moiety and a H-phosphonate monoester moiety;
PG is the protecting group PG-1 (for building block B-1) or PG-2 (for building block B-2) or PG-x (for a building block B-x) and is a di(p-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is selected independently for each repetitive unit q from the group consisting of O-R^{z-2} , S-R^{z-2}, and H; and
R^{z-2} is at each occurrence a 2-cyanoethyl group.

In some embodiments, in each building block B-1, B-2, and B-x of Formula II:
PM is a phosphoramidite moiety;
PG is the protecting group PG-1 (for building block B-1) or PG-2 (for building block B-2) or PG-x (for a building block B-x) and is a di(p-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is selected independently for each repetitive unit q from the group consisting of O-R^{z-2} and S-R^{z-2}; and
R^{z-2} is at each occurrence a 2-cyanoethyl group.

In some embodiments, in each building block B-1, B-2, and B-x of Formula II:
PM is a phosphoramidite moiety;
PG is the protecting group PG-1 (for building block B-1) or PG-2 (for building block B-2) or PG-x (for a building block B-x) and is a di(p-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is for each repetitive unit q O-R^{z-2}; and
R^{z-2} is at each occurrence a 2-cyanoethyl group.

In some embodiments, in each building block B-1, B-2, and B-x of Formula II:
PM is a H-phosphonate monoester moiety;
PG is the protecting group PG-1 (for building block B-1) or PG-2 (for building block B-2) or PG-x (for a building block B-x) and is a di(p-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S, and preferably is O; and
Z² is for each repetitive unit q H.

In some embodiments, in each building block B-1, B-2, and B-x of any one of Formulae II-a and II-b:
PM is a phosphorus moiety, preferably a phosphorus moiety selected from the group consisting of a phosphoramidite moiety and a H-phosphonate monoester moiety;
PG is the protecting group PG-1 (for building block B-1) or PG-2 (for building block B-2) or PG-x (for a building block B-x) and is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is selected independently for each repetitive unit q from the group consisting of O-R^{z-2}, S-R^{z-2}, and H;
R^{z-2} is at each occurrence a 2-cyanoethyl group;
R^{XI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1, B-2 or B-x of Formula II-a) or Formula II-b-tc (in a building block B-1, B-2 or B-x of Formula II-b).

In some embodiments, in each building block B-1, B-2, and B-x of any one of Formulae II-a and II-b:
PM is a phosphorus moiety, preferably a phosphorus moiety selected from the group consisting of a phosphoramidite moiety and a H-phosphonate monoester moiety;
PG is the protecting group PG-1 (for building block B-1) or PG-2 (for building block B-2) or PG-x (for a building block B-x) and is a di(p-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is selected independently for each repetitive unit q from the group consisting of O-R^{z-2}, S-R^{z-2}, and H;
R^{z-2} is at each occurrence a 2-cyanoethyl group;
R^{XI} is at each occurrence independently selected from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. tert-butyl(dimethyl)silyloxy), and O-CH₂-O-Si(C(CH₃)₃)₃ (i.e. ((triisopropylsilyl)oxy)-methyloxy);
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1, B-2 or B-x of Formula II-a) or Formula II-b-tc (in a building block B-1, B-2 or B-x of Formula II-b).

In some embodiments, in each building block B-1, B-2, and B-x of any one of Formulae II-a and II-b:
PM is a phosphoramidite moiety;
PG is the protecting group PG-1 (for building block B-1) or PG-2 (for building block B-2) or PG-x (for a building block B-x) and is a di(p-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is for each repetitive unit q O-R^{z-2};
R^{z-2} is at each occurrence a 2-cyanoethyl group;
R^{XI} is selected independently at each occurrence from the group consisting of H, F, O-CH₃ (i.e. methoxy), and O-CH₂-CH₂-O-CH₃ (i.e. 2-methoxyethyl-1-oxy); and each of R^{XII}, R^{XIII}, R^{XIV}, and R^{XV} is H at each occurrence.

In some embodiments, in each building block B-1, B-2, and B-x of Formula 11-1: PG is the protecting group PG-1 (for building block B-1) or PG-2 (for building block B-2) or PG-x (for a building block B-x) and is a di(p-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is selected independently for each repetitive unit q from the group consisting of O-R^{z-2} and S-R^{z-2}, and Z² preferably is for each repetitive unit q O-R^{z-2};
R^{z-2} is at each occurrence a 2-cyanoethyl group;
Z³ is selected from the group consisting of O and S, and preferably is O;
R^{z-3} is a 2-cyanoethyl group; and
each of R^{a} and R^{b} is a C₁-C₆-alkyl group, preferably an isopropyl group.

In some embodiments, in each building block B-1, B-2, and B-x of Formula 11-1: PG is the protecting group PG-1 (for building block B-1) or PG-2 (for building block B-2) or PG-x (for a building block B-x) and is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is for each repetitive unit q O-R^{z-2};
R^{z-2} is at each occurrence a 2-cyanoethyl group;
Z³ is O;
R^{z-3} is a 2-cyanoethyl group; and
each of R^{a} and R^{b} is an isopropyl group.

In some embodiments, in each building block B-1, B-2, and B-x of any one of Formula II-1-a and 11-1-b:
PG is the protecting group PG-1 (for building block B-1) or PG-2 (for building block B-2) or PG-x (for a building block B-x) and is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is selected independently for each repetitive unit q from the group consisting of O-R^{z-2} and S-R^{z-2}, and Z² preferably is for each repetitive unit q O-R^{z-2};
R^{z-2} is at each occurrence a 2-cyanoethyl group;
Z³ is selected from the group consisting of O and S, and preferably is O;
R^{z-3} is a 2-cyanoethyl group;
each of R^{a} and R^{b} is a C₁-C₆-alkyl group, preferably an isopropyl group;
R^{XI} is at each occurrence independently selected from the group consisting of H, F, O-(C₁-C₅-alkyl), O-(C₁-C₅-alkyl)-O-(C₁-C₅-alkyl), O-Si(C₁-C₅-alkyl)₃, and O-CH₂-O-Si(C₁-C₅-alkyl)₃;
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1, B-2 or B-x of Formula II-1-a) or Formula II-b-tc (in a building block B-1, B-2 or B-x of Formula 11-1-b).

In some embodiments, in each building block B-1, B-2, and B-x of any one of Formula II-1-a and 11-1-b:
PG is the protecting group PG-1 (for building block B-1) or PG-2 (for building block B-2) or PG-x (for a building block B-x) and is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S;
Z² is selected independently for each repetitive unit q from the group consisting of O-R^{z-2} and S-R^{z-2}, and Z² preferably is for each repetitive unit q O-R^{z-2};
R^{z-2} is at each occurrence a 2-cyanoethyl group;
Z³ is selected from the group consisting of O and S and preferably is O;
R^{z-3} is a 2-cyanoethyl group;
each of R^{a} and R^{b} is a C₁-C₆-alkyl group, preferably an isopropyl group;
R^{XI} is at each occurrence independently selected from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert*-butyl(dimethyl)silyloxy), and O-CH₂-O-Si(C(CH₃)₃)₃ (i.e. ((triisopropylsilyl)oxy)-methyloxy);
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a block B-1, B-2 or B-x of Formula II-1-a) or Formula II-b-tc (in a B-1, B-2 or B-x of Formula II-1-b).

In some embodiments, in each building block B-1, B-2, and B-x of any one of Formula II-1-a and 11-1-b:
PG is the protecting group PG-1 (for building block B-1) or PG-2 (for building block B-2) or PG-x (for a building block B-x) and is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
B^{N} is a nucleobase, which may be the same or different at each occurrence;
Y² is selected independently for each repetitive unit q from the group consisting of O and S;
Z² is selected independently for each repetitive unit q from the group consisting of O-R^{z-2} and S-R^{z-2}, and Z² preferably is for each repetitive unit q O-R^{z-2};
R^{z-2} is at each occurrence a 2-cyanoethyl group;
Z³ is selected from the group consisting of O and S and preferably is O;
R^{z-3} is a 2-cyanoethyl group;
each of R^{a} and R^{b} is a C₁-C₆-alkyl group, preferably an isopropyl group;
R^{XI} is selected independently at each occurrence from the group consisting of H, F, O-CH₃ (i.e. methoxy), and O-CH₂-CH₂-O-CH₃ (i.e. 2-methoxyethyl-1-oxy); and each of R^{XII}, R^{XIII}, R^{XIV}, and R^{XV} is H at each occurrence.

In some embodiments, in each building block B-1, B-2, and B-x of Formula II-2:
PG is the protecting group PG-1 (for building block B-1) or PG-2 (for building block B-2) or PG-x (for a building block B-x) and is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S, and preferably is O; and
Z² is for each repetitive unit q H.

In some embodiments, in each building block B-1, B-2, and B-x of any one of Formulae II-2-a and II-2-b:
PG is the protecting group PG-1 (for building block B-1) or PG-2 (for building block B-2) or PG-x (for a building block B-x) and is a di(*p*-methoxyphenyl)phenylmethyl (DMT) protecting group;
q is an integer in the range of 0-50, 0-35, 0-30, 0-25, 0-20, 0-15, 0-10, or 0-5, or q is 0;
Y² is selected independently for each repetitive unit q from the group consisting of O and S, and preferably is O;
Z² is for each repetitive unit q H;
R^{XI} is at each occurrence independently selected from the group consisting of H, F, O-CH₃ (i.e. methoxy), O-CH₂-CH₂-O-CH₃ (i.e. 2-methoxyethyl-1-oxy), O-Si(CH₃)₃ (i.e. trimethylsilyloxy), O-Si(CH₃)₂(C(CH₃)₃) (i.e. *tert*-butyl(dimethyl)silyloxy), and O-CH₂-O-Si(C(CH₃)₃)₃ (i.e. ((triisopropylsilyl)oxy)-methyloxy);
R^{XIII} is independently at each occurrence H or R^{XIII} and R^{XI} of the same nucleoside subunit (i.e. bonded to the 4'- and 2'-C atom of the same carbohydrate moiety) together form a structure +-CH₂-O-++, +-CH(CH₃)-O-++, or +-CH₂-CH₂-O-++, where + is the point of attachment to the 4'-carbon atom (i.e. the carbon atom to which R^{XIII} is bonded) and ++ is the point of attachment to the 2'-carbon (i.e. the carbon atom to which R^{XI} is bonded); and
for each nucleoside subunit independently, R^{XII}, R^{XIV}, and R^{XV} are either all H or they are bonded together so that the respective nucleoside subunit has a structure of the aforementioned Formula II-a-tc (in a building block B-1, B-2 or B-x of Formula II-2-a) or Formula II-b-tc (in a building block B-1, B-2 or B-x of Formula II-2-b).

In the method of the invention, the building block B-x may be the same or different (i.e. have the same or a different chemical structure) for each coupling cycle comprising steps (b-x) to (h-x) (as far as present), unless indicated differently in the context of specific embodiments.

A building block B-1, B-2 or B-x for use in the method of the invention may, for example, be obtained commercially, in particular, if said phosphorus moiety is a phosphoramidite moiety or a H-phosphonate monoester moiety. Alternatively, a building block B-1, B-2 or B-x for use in the method of the invention may be obtained by means of chemical synthesis. The person skilled in the art is aware of methods of synthesizing such compounds, wherein the synthesis route will obviously depend on the chemical structure of said phosphorus moiety. Building blocks B-1, B-2 or B-x, e.g. of any one of Formulae II, II-a, and II-b, in which said phosphorus moiety, e.g. said phosphorus moiety PM, is a phosphoramidite moiety, in particular building blocks B-1, B-2 or B-x of any one of Formulae II-1, II-1-a, and II-1-b, may, for example, be synthesized as disclosed in, in K.V. Gothelf et al., Nature Communications 2021, 12, 2760, and in X. Wei et al., Tetrahedron 2013, 69, 3615-3637. Building blocks B-1, B-2 or B-x, e.g. of any one of Formulae II, II-a, and II-b, in which said phosphorus moiety, e.g. said phosphorus moiety PM, is a H-phosphonate monoester moiety, in particular building blocks B-1, B-2 or B-x of any one of Formulae II-2, II-2-a, and II-2-b, may, for example, be synthesized as disclosed in J. Stawinski and R. Strömberg (2005), Di- and Oligonucleotide Synthesis Using H-Phosphonate Chemistry, in Methods in Molecular Biology, vol. 288: Oligonucleotide Synthesis: Methods and Applications, edited by P. Herdewijn, Humana Press Inc., Totowa NJ, https://doi.org/10.1385/1-59259-823-4:081. Building blocks B-1, B-2 or B-x, e.g. of any one of Formulae II, II-a, and II-b, in which said phosphorus moiety, e.g. said phosphorus moiety PM, is a P (V) moiety suitable for chiral phosphorothioate synthesis may, for example, be synthesized as disclosed in P.S. Baran et al., Science 2018, 361, 1234-1238 (also see Supplementary Materials for this reference).

The term "reacting" in step (e-1) may be understood in the broadest sense as any operation during which the component (C-0)^{#} and the building block B-1 are present in the same reaction vessel or reactor and engage in the bond forming reaction of step (e-1). The term "reacting" in step (e-2) may be understood in the broadest sense as any operation during which the first cycle oligonucleotide (O-1)^{#} and the building block B-2 are present in the same reaction vessel or reactor and engage in the bond forming reaction of step (e-2). The term "reacting" in step (e-x) may be understood in the broadest sense as any operation during which the (x-1)-th cycle oligonucleotide (O-(x-1))^{#} and the building block B-x are present in the same reaction vessel or reactor and engage in the bond forming reaction of step (e-x). Typically, the component (C-0)^{#}, the first cycle oligonucleotide (O-1)^{#} or the (x-1)-th cycle oligonucleotide (O-(x-1))^{#} may already be contained in a reaction vessel or reactor, to which the building block B-1, B-2 or B-x is then added. Alternatively, the building block B-1, B-2 or B-x or a solution thereof may already be contained in a reaction vessel or reactor, to which the component (C-0)^{#}, the first cycle oligonucleotide (O-1)^{#} or the (x-1)-th cycle oligonucleotide (O-(x-1))^{#} is then added.

During each of steps (e-1), (e-2), and (e-x), a covalent (chemical) bond is formed between said free backbone hydroxyl group of the component (C-0)^{#}, the first cycle oligonucleotide (O-1)^{#} or the (x-1)-th cycle oligonucleotide (O-(x-1))^{#} and the phosphorus atom of said phosphorus moiety of the building block B-1, B-2 or B-x. The bond forming reaction of steps (e-1), (e-2), and (e-x) is herein also referred to as coupling or coupling reaction or condensation or condensation reaction, and steps (e-1), (e-2), and (e-x) are also referred to as coupling steps or condensation steps. The product obtained from the bond forming reaction of step (e-1) is the first cycle oligonucleotide O-1, which comprises the nucleoside sequence of the component (C-0)^{#} and of the building block B-1, wherein these two are now interconnected by an internucleosidic linkage group derived from the phosphorus moiety of the building block B-1. The product obtained from the bond forming reaction of step (e-2) is the second cycle oligonucleotide O-2, which comprises the nucleoside sequence of the first cycle oligonucleotide (O-1)^{#} and of the building block B-2, wherein these two are now interconnected by an internucleosidic linkage group derived from the phosphorus moiety of the building block B-2. The product obtained from the bond forming reaction of step (e-x) is the x-th cycle oligonucleotide O-x, which comprises the nucleoside sequence of the (x-1)-th cycle oligonucleotide (O-(x-1))^{#} and of the building block B-x, wherein these two are now interconnected by an internucleosidic linkage group derived from the phosphorus moiety of the building block B-x.

The "conditions suitable" to form a covalent bond between said free backbone hydroxyl group of the component (C-0)^{#}, the first cycle oligonucleotide (O-1)^{#} or the (x-1)-th cycle oligonucleotide (O-(x-1))^{#} and the phosphorus atom of said phosphorus moiety of the building block B-1, B-2 or B-x form part of the common knowledge of those skilled in the art. These conditions may, for example, depend on the chemical structure of said phosphorus moiety which is to engage in the bond forming reaction.

A bond forming reaction of step (e-1), (e-2) or (e-x), in which the phosphorus moiety engaging in said reaction is a phosphoramidite moiety, as e.g. present in building blocks B-1, B-2 or B-x of any one of Formulae II-1, II-1-a, and II-1-b, is herein also referred to as phosphoramidite coupling (reaction). Such phosphoramidite couplings may preferably be performed in the presence of an activator. Any activator used in oligonucleotide synthesis by the so-called phosphoramidite method may be used in the method of the invention. The activator may, for example, be selected from the group consisting of:
- a tetrazole type activator such as 1*H*-tetrazole, 5-ethylthio-1*H*-tetrazole (ETT), 5-benzylthio-1*H*-tetrazole (BTT), 5-methylthio-1*H*-tetrazole (MTT), 1-methyl-5-mercaptotetrazole, 1-phenyl-5-mercaptotetrazole, and 5-(4-nitrophenyl)-1*H*-tetrazole,
- an imidazole type activator such as 4,5-dicyanoimidazole (DCI) and 2-bromo-4,5-dicyanoimidacole (2-Br-DCI),
- a 1-hydroxybenzotriazole type activator such as 1-hydroxybenzotriazole, 1-hydroxy-6-trifluorobenzotriazole, and 1-hydroxy-6-trifluoro-4-nitrobenzotriazole,
- a pyridinium salt type activator such as pyridinium hydrochloride, pyridinium *p*-toluenesulfonate, and pyridinium trifluoroacetate, and
- a saccharin type activator, i.e. salts obtained from reacting saccharin with an organic base such as pyridine, collidine, lutidine, picoline, *N*-methylimidazole, and triethylamine.

5-Benzylthio-1*H*-tetrazole (BTT), 5-ethylthio-1*H*-tetrazole (ETT), 1*H*-tetrazole, and 4,5-dicyanoimidacole (DCI) may be preferred. N-methylimidazole (NMI) or pyridine may be added alongside the activator, which may help to adjust the acidity of the solution. For example, 1.0-100.0 mol, 1.0-50.0 mol, 1.0-40.0 mol, 1.0-30.0 mol, 1.0-20.0 mol, 1.0-15.0 mol, 1.0-10.0 mol, or 2.5-10.0 mol of the activator may be used per 1 mol of the building block B-1, B-2 or B-x engaging in the respective coupling reaction with its phosphoramidite moiety. As another example, NMI may be used alongside a protic acid, for example a carboxylic acid such as trifluoroacetic acid, without an additional activator. Acetonitrile may also be added. For example, a mixture of acetonitrile, NMI, and TFA (e.g. 7.5:2:2 v/v) may be added for the coupling reaction. In some preferred embodiments of the method of the invention, steps (e-1), (e-2), and (e-x) are carried out using a tetrazole-type activator, preferably ETT, most preferably in combination with a base, in particular pyridine. It will be understood that this does not imply that a step (e-2) and/or (e-x) must be performed.

A bond forming reaction of step (e-1), (e-2) or (e-x), in which the phosphorus moiety engaging in said reaction is a H-phosphonate monoester moiety, as e.g. present in building blocks B-1, B-2 or B-x of any one of Formulae II-2, II-2-a, and II-2-b, is herein also referred to as H-phosphonate coupling (reaction). Such H-phosphonate couplings may typically be performed using a condensing agent. Any condensing agent used in oligonucleotide synthesis by the so-called H-phosphonate method may be used in the method of the invention. The condensing agent may, for example, be selected from the group consisting of pivaloyl chloride (PvCl), 1-adamantanecarbonyl chloride (AdCl), 2,2-dimethylbutyryl chloride, isobutyryl chloride, diphenyl chlorophosphate, 2,4,6-triisopropylbenzenesulfonyl chloride, bis(pentafluorophenyl) carbonate, 2-chloro-5,5-dimethyl-1,3,2-dioxaphosphorinane 2-oxide (also referred to as 5,5-dimethyl-2-oxo-2-chloro-1,3,2-dioxaphosphinane, DMOCP), and bis(2-oxo-3-oxazolidinyl)phosphinic chloride (OXP or BOP-CI). The building block B-1, B-2 or B-x comprising said H-phosphonate monoester moiety may be pre-activated with (i.e. incubated with) said condensing agent, and then be combined with the component (C-0)^{#}, the first cycle oligonucleotide (O-1)^{#} or the (x-1)-th cycle oligonucleotide (O-(x-1))^{#}. Besides the condensing agent, a nucleophile such as pyridine may be added. For example, EP3378869A1 discloses suitable reaction conditions.

A bond forming reaction of step (e-1), (e-2) or (e-x), in which the phosphorus moiety engaging in said reaction is an arylphosphate diester moiety, is herein also referred to as phosphotriester coupling (reaction). Briefly, the arylphosphate diester moieties may typically be activated with an arylsulfonyl chloride activator, such as mesitylene-2-sulfonyl chloride (MsCl), usually in the presence of an auxiliary nucleophile such as 1-methylimidazole. Alternatively, pre-formed or in-situ generated 1-hydroxybenzotriazole-phosphotriesters may, for example, be used as phosphorus moiety instead of an aryl phosphate diester moiety in a phosphotriester coupling, again in the presence of an auxiliary nucleophile such as 1-methylimidazole.

A bond forming reaction of step (e-1), (e-2) or (e-x), in which the phosphorus moiety engaging in said reaction is a P (V) moiety allowing for chiral phosphorothioate synthesis may, for example, be performed as disclosed in Baran et al.,Science 2018, 361, 1234-1238 (see also the Supplementary Materials of said publication) and ACS Central Science 2021, 7, 1473-1485), or in a similar fashion.

Preferably, the solutions in which steps (e-1), (e-2), and (e-x) are carried out are substantially anhydrous, which may mean that they preferably comprise equal to or less than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm of water. The water content may be determined by means of standard Karl Fischer titration at 25 °C, as known to those skilled in the art. The set up may, for example, be as follows: The titration cell may be filled with HYDRANAL^{™} -Coulomat Oil for anolyte reagent, and the inner burette may be filled with HYDRANAL^{™} - Coulomat CG for catholyte reagent. After pretitration for dehydration, sample (100 µL) may be added to the titration sell and the measurement may be started. In line with common practice, the water content of solutions is herein reported in parts per million (ppm).

For example, 1.0-20.0 mol, 1.0-10.0 mol, 1.0-5.0 mol, 2.0-5.0 mol, or 2.0-4.0 mol of the building block B-1, B-2 or B-x may be used per mol of the component (C-0)^{#}, the first cycle oligonucleotide (O-1)^{#} or the (x-1)-th cycle oligonucleotide (O-(x-1))^{#}.

Steps (e-1), (e-2), and (e-x) may, for example, be performed at a temperature in the range of 0-90 °C, 10-70 °C, 15-60 °C, 15-50 °C, or 20-50 °C. For convenience, steps (e-1), (e-2), and (e-x) may simply be performed at room temperature. Increased temperatures may result in shorter reaction times. The reaction time may also depend on the chemical structure of the reactants and will routinely be selected by a skilled person, for example based on reaction monitoring using, e.g., thin-layer chromatography and/or high performance liquid chromatography (HPLC), optionally coupled to mass spectrometry.

If the internucleosidic linkage group formed in a bond forming reaction of a step (e-1), (e-2) or (e-x) is a P(III) linkage group as defined herein, e.g. a phosphite triester linkage group (as e.g. obtained from a phosphoramidite coupling), and supposed to be sulfurized (by incubation with a sulfurizing agent) to a P (V) linkage group, e.g. a phosphorothioate linkage group, in the subsequent step (f-1), (f-2) or (f-x), the respective step (e-1), (e-2) or (e-x) may be carried out in the presence of an antioxidant . Said antioxidant may suppress unwanted oxidation of a formed P(III) linkage group to a P (V) linkage group, which may not be amenable to sulfurization in the subsequent step. Examples of suitable antioxidants are, e.g., disclosed in EP3950698A1 and include inter alia triphenylphosphine, methyldiphenylphosphine, triethyl phosphite, ethoxydiphenylphosphine, diethoxyphenylphosphine, 9,10-dihydro-9-oxa-10-phosphaphenanthrene 10-oxide, and isobutylene sulfide. Alternatively, the solvents used may be controlled so that they contain no or a low level of oxidizing species.

After the bond-forming reaction of step (e-1), (e-2) or (e-x) has been carried out, a quencher may optionally be added, wherein said quencher may be used to consume (i.e. react with) the phosphorus moiety of unreacted (excess) molecules of the building block B-1, B-2 or B-x employed. The quencher will typically be a nucleophilic compound capable of reacting with the phosphorus moiety. Water may, for example, be used as a quencher.

Step (f-1) of the methods of the invention is: optionally, incubating the first cycle oligonucleotide O-1 with an oxidizing or sulfurizing agent, thereby converting any P (III) atoms within said first cycle oligonucleotide O-1 to P (V) atoms.

Step (f-2) of some of the methods of the invention is: optionally, incubating the second cycle oligonucleotide O-2 with an oxidizing or sulfurizing agent, thereby converting any P (III) atoms within said second cycle oligonucleotide O-2 to P (V) atoms.

Step (f-x) of some of the methods of the invention is: optionally, incubating the x-th cycle oligonucleotide O-x with an oxidizing or sulfurizing agent, thereby converting any P (III) atoms within said x-th cycle oligonucleotide O-x to P (V) atoms.

The terms "P (III) atom" and "P (V) atom" have been defined above. As used throughout this text, the "oxidation state" may be "the charge of an atom after its homonuclear bonds have been divided equally and heteronuclear bonds assigned to the bond partners according to Allen electronegativity, except when the electronegative atom is bonded reversibly as a Lewis-acid ligand, in which case it does not obtain that bonds electrons", as described in the IUPAC Recommendations 2016 (P. Karen et al., Pure and Applied Chemistry 2016, 88(8), 831-839). The Allen electronegativities given in said reference may be used and the P-H bond electron pair is assigned to H. As an example, the phosphorus atom of the H-phosphonate monoester moiety in any one of Formulae II-2, II-2-a, and II-2-b has the oxidation state III. As another example, the phosphorus atom of the phosphoramidite moiety in any one of Formulae II-1, II-1-a, and II-1-b has the oxidation state III. As a third example, the phosphorus atoms in the phosphodiester linkage groups of DNA and RNA have the oxidation state V.

The term "optionally" in step (f-1), (f-2), and (f-x) denotes that the respective step may or may not be carried out in a given coupling cycle, unless indicated differently in the context of specific embodiments.

As known to those skilled in the art, most or even all phosphorus atoms of an oligonucleotide may typically be comprised in the backbone of said oligonucleotide, usually in the internucleosidic linkage group(s). The terms "P (III) linkage group" and "P (V) linkage group" have been defined above. As also known to those skilled in the art, oligonucleotides comprising one or more P (III) linkage groups are typically less stable than related oligonucleotides comprising only P (V) linkage groups (as e.g. present in DNA and RNA). In some embodiments of the method of the invention, the target oligonucleotide O^{T} comprises only P (V) linkage groups.

If the first cycle oligonucleotide O-1 obtained in step (e-1) does not comprise any P (III) atoms, in particular not any P (III) linkage groups, it may not be necessary to carry out step (f-1). If the second cycle oligonucleotide O-2 obtained in step (e-2) does not comprise any P (III) atoms, in particular not any P (III) linkage groups, it may not be necessary to carry out step (f-2). If the x-th cycle oligonucleotide O-x obtained in step (e-x) of a coupling cycle does not comprise any P (III) atoms, in particular not any P (III) linkage groups, it may not be necessary to carry out step (f-x) in said coupling cycle.

It is understood by those skilled in the art, that the oxidation state of the phosphorus atom within an internucleosidic linkage group formed in the bond forming reaction of a step (e-1), (e-2) or (e-x) will typically depend on the chemical structure of said phosphorus moiety of said building block B-1, B-2 or B-x, which engaged in the respective bond forming reaction. Typically, the oxidation state of the phosphorus atom within such a phosphorus moiety will be preserved in the course of the bond forming reaction of step (e-1), (e-2) or (e-x). For example, if a phosphotriester coupling as defined herein is carried out in a step (e-1), (e-2) or (e-x), the phosphorus atom of the resulting phosphotriester internucleosidic linkage group will typically be present as P (V) atom, i.e. the phosphotriester linkage group is a P (V) linkage group. The same principle applies, e.g., to the P (V) chemistry utilized by Baran et al. (Science 2018, 361, 1234-1238 and ACS Central Science 2021, 7, 1473-1485).

On the other hand, if the phosphorus moiety of a component C-x engaging in a bond forming reaction of a step (e-1), (e-2) or (e-x) comprises a P (III) atom, the resulting internucleosidic linkage group will typically also comprise a P (III) atom, i.e. be a P (III) linkage group. For example, the bond forming reaction of a step (e-1), (e-2) or (e-x) may typically afford a phophite triester product (comprising a phosphite triester internucleosidic linkage group, i.e. a P (III) linkage group), if said phosphorus moiety covalently bonded via its phosphorus atom to an oxygen atom of the backbone of the building block B-1, B-2 or B-x, e.g. the phosphorus moiety PM of any one of Formulae II, II-a, and II-b, is a phosphoramidite moiety such as, e.g., present in any one of Formulae II-1, 11-1-a, and 11-1-b, while a H-phosphonate diester product (comprising a H-phosphonate diester internucleosidic linkage group, i.e. a P (III) linkage group) may typically be obtained, if said phosphorus moiety covalently bonded via its phosphorus atom to an oxygen atom of the backbone of the building block B-1, B-2 or B-x, e.g. the phosphorus moiety PM of any one of Formulae II, II-a, and II-b, is a H-phosphonate monoester moiety such as, e.g., present in any one of Formulae II-2, II-2-a, and II-2-b. H-phosphonate diester linkage groups may be more stable, e.g. under the conditions of steps (b-1), (b-2) or (b-x), than phosphite triester linkage groups, so that a step (f-1) or (f-2) or (f-x) may not need to be performed in each coupling cycle, in which said phosphorus moiety covalently bonded via its phosphorus atom to an oxygen atom of the backbone of the building block B-1, B-2 or B-x, e.g. the phosphorus moiety PM of any one of Formulae II, II-a, and II-b, is a H-phosphonate monoester moiety such as, e.g., present in any one of Formulae II-2, II-2-a, and II-2-b. However, at least in the final coupling cycle, step (f-1) or (f-2) or (f-x) may be carried out, so that any P (III) atoms are converted to P (V) atoms.

In some embodiments of the method of the invention:
- the phosphorus moiety of the building blocks B-1, B-2, and each building block B-x, e.g. the phosphorus moiety PM of any one of Formulae II, II, II-a, and II-b, is independently selected from the group consisting of a phosphoramidite moiety and a H-phosphonate monoester moiety;
- in each coupling cycle, in which said phosphorus moiety of the building block B-1, B-2 or B-x is a phosphoramidite moiety, step (f-1) or (f-2) or (f-x) is carried out; and
- at least in the final coupling cycle, step (f-1) or (f-2) or (f-x) is carried out.

In some embodiments of the method of the invention:
- the phosphorus moiety of the building blocks B-1, B-2, and each building block B-x, e.g. the phosphorus moiety PM of any one of Formulae II, II-a, and II-b, is a phosphoramidite moiety; and
- in each coupling cycle, step (f-1) or (f-2) or (f-x) is carried out.

In some embodiments of the method of the invention:
- the phosphorus moiety of the building blocks B-1, B-2, and each building block B-x, e.g. the phosphorus moiety PM of any one of Formulae II, II-a, and II-b, is a H-phosphonate monoester moiety; and
- at least in the final coupling cycle, step (f-1) or (f-2) or (f-x) is carried out.

In some embodiments of the method of the invention:
- each building block B-1, B-2, and B-x is independently a compound of any one of Formulae II-1 and II-2, preferably a compound of any one of Formulae II-1-a and II-2-a, in particular a compound of any one of Formulae II-1-b and II-2-b;
- in each coupling cycle, in which the building block B-1, B-2 or B-x is a compound of any one of Formulae II-1, 11-1-a, and II-1-b, step (f-1) or (f-2) or (f-x) is carried out; and
- at least in the final coupling cycle, step (f-1) or (f-2) or (f-x) is carried out.

In some embodiments of the method of the invention:
- each building block B-1, B-2, and B-x is independently a compound of Formula II-1, preferably of Formula II-1-a, in particular of Formula 11-1-b; and
- in each coupling cycle, step (f-1) or (f-2) or (f-x) is carried out.

In some embodiments of the method of the invention:
- each building block B-1, B-2, and B-x is a compound of Formula II-2, preferably of Formula II-2-a, in particular of Formula II-2-b; and
- at least in the final coupling cycle, step (f-1) or (f-2) or (f-x) is carried out.

The "oxidizing agent" or the "sulfurizing agent" to be used in any one of steps (f-1), (f-2), and (f-x) are not particularly limited in terms of their chemical structure as long as the respective agent is capable of converting any P (III) atoms within said first cycle oligonucleotide O-1, said second cycle oligonucleotide O-2, and said x-th cycle oligonucleotide O-x to P (V) atoms. An "oxidizing agent" and a "sulfurizing agent" may differ in the means of how P (III) atoms are converted to P (V) atoms. An "oxidizing agent" may introduce one or more covalent bonds between the P (III) atom to be oxidized and an oxygen atom. A "sulfurizing agent" may introduce one or more covalent bonds between the P (III) atom to be sulfurized and a sulfur atom. As used herein, the term "oxidizing agent" preferably refers to any agent capable of converting a phosphite triester linkage group to a phosphate triester linkage group and a H-phosphonate diester linkage group to a phosphate diester (i.e. a phosphodiester) linkage group. As used herein, the term "sulfurizing agent" preferably refers to any agent capable of converting a phosphite triester linkage group to a thiophosphate triester linkage group and a H-phosphonate diester linkage group to a thiophosphate diester linkage group (i.e. a phosphorothioate) linkage group.

Iodine may be a preferred oxidizing agent. For example, an aqueous solution of iodine may be used, preferably in combination with a co-solvent such as an ether solvent, e.g. an ether solvent S^{E} (vide infra), a nitrile solvent such as acetonitrile, a (hetero)aromatic solvent such as pyridine, or a mixture thereof. As one example, a solution of iodine (e.g. 1 mol/L) in a mixed solvent of 4-methyltetrahydropyran (MTHP), water, and, optionally, acetonitrile may be used. Alternatively, peroxides such as *tert*-butyl hydroperoxide, cumene hydroperoxides, bis-trimethylsilyl peroxide, 2-butanone peroxide, and hydrogen peroxide, or peroxy acids such as *m*-chloroperbenzoic acid (mCPBA) may, for example, be used as oxidizing agents. As one example, a solution of tert-butyl hydroperoxide in a non-polar solvent may be used. For example, a solution (e.g. 5-6 mol/L) of *tert*-butyl hydroperoxide in nonane may be used. As another example, an aqueous solution of hydrogen peroxide may be used. For example, an aqueous solution of hydrogen peroxide and, optionally, potassium iodide may be mixed with an aqueous phosphoric acid buffer (e.g. pH 6.8) to obtain an oxidizing agent. As another alternative, (1S)-(+)-(10-camphorsulfonyl)-oxaziridine (CSO) may, for example, be used as oxidizing agent, e.g. as a 0.5 M (i.e. 0.5 mol/L) solution in, e.g., acetonitrile. In general, the oxidizing agent may, for example, be applied in form of a 0.005-10.0 M (i.e. mol/L) solution, preferably a 0.01-5.0 M solution in a suitable solvent. Alternatively, neat oxidizing agent may, for example, be added (i.e. the oxidizing agent need not be dissolved in a suitable solvent).

Xanthane hydride (5-amino-3*H*-1,2,4-dithiazole-3-thione), 3*H*-1,2-benzodithiol-3-one, and 3-(*N,N*-dimethylamino-methylidene)amino)-3*H*-1,2,4-dithiazole-5-thione (CAS RN: 1192027-04-5, DDTT) may be preferred sulfurizing agents. Alternatively, 1,4-dithiothreitol (DTT), phenylacetyl disulfide (PADS) or 3*H*-1,2-benzodithiol-3-one 1,1-dioxide (Beaucage Reagent) may be used as sulfurizing agents. For example, neat sulfurizing agent (i.e. not in solution) may be added. As one example, neat DDTT may be added. As another example, neat xanthane hydride may be added. Alternatively, a solution of the sulfurizing agent in a suitable solvent may be added. The sulfurizing agent may, for example, be applied in form of a 0.005-5.0 M solution, preferably a 0.01-1.0 M solution in a suitable solvent, for example, selected from the group consisting of pyridine, acetonitrile, water, tetrahydrofuran, and mixtures thereof. As one example, a solution of xanthane hydride in pyridine may be used, optionally in combination with a co-solvent such as acetonitrile. As another example, a solution of xanthane hydride (e.g. 0.2 M) in pyridine may be used. As another example, a solution of xanthane hydride (e.g. 0.1 M) in a mixture of pyridine and acetonitrile (e.g. 1:1, v/v) may be used.

Steps (f-1), (f-2), and (f-x) may, for example, be performed at a temperature in the range of 0-90 °C, 10-70 °C, 15-60 °C, or 15-50 °C. For convenience, steps (f-1), (f-2), and (f-x) may simply be performed at room temperature. Increased temperatures may result in shorter reaction times. The reaction time may also depend on the chemical structure of the reactants and will routinely be selected by a skilled person, for example, based on reaction monitoring using, e.g., thin-layer chromatography and/or high performance liquid chromatography (HPLC), optionally coupled to mass spectrometry.

The term "incubating" in steps (f-1), (f-2), and (f-x) may be understood in the broadest sense to refer to any process of contacting the first cycle oligonucleotide O-1, the second cycle oligonucleotide O-2 or the x-th cycle oligonucleotide O-x, respectively, and the respective oxidizing or sulfurizing agent, preferably inside the same reaction vessel or reactor. Typically, the first cycle oligonucleotide O-1, the second cycle oligonucleotide O-2 or the x-th cycle oligonucleotide O-x may already be contained in a reaction vessel or reactor, to which the respective oxidizing or sulfurizing agent is then added. Alternatively, the first cycle oligonucleotide O-1, the second cycle oligonucleotide O-2 or the x-th cycle oligonucleotide O-x may be added to the reaction vessel or reactor containing the respective oxidizing or sulfurizing agent.

After said conversion of any P (III) atoms to P (V) atoms of any one of steps (f-1), (f-2), and (f-x), excess oxidizing or sulfurizing agent may typically be quenched (i.e. reduced), e.g., by treatment with a reducing agent. For example, a reducing agent such as triethylphosphite or a solution thereof may be added directly into the reaction mixture of the respective step (f-1) or (f-2) or (f-x). Alternatively, if step (g-1) or (g-2) or (g-x) is carried out, a reducing agent may also be comprised in one or more of the one or more aqueous solutions used for the one or more aqueous extractions. Examples of such water-soluble reducing agents comprise thiosulfate salts such as sodium thiosulfate.

Step (g-1) of the methods of the invention is: optionally, subjecting a solution comprising the first cycle oligonucleotide O-1 to one or more aqueous extractions, wherein the organic phase comprises the first cycle oligonucleotide O-1.

Step (g-2) of some of the methods of the invention is: optionally, subjecting a solution comprising the second cycle oligonucleotide O-2 to one or more aqueous extractions, wherein the organic phase comprises the second cycle oligonucleotide O-2.

Step (g-x) of some of the methods of the invention is: optionally, subjecting a solution comprising the x-th cycle oligonucleotide O-x to one or more aqueous extractions, wherein the organic phase comprises the x-th cycle oligonucleotide O-x.

The term "optionally" in steps (g-1), (g-2), and (g-x) denotes that the respective step may or may not be carried out in a given coupling cycle, unless indicated differently in the context of specific embodiments. Steps (g-1), (g-2), and (g-x) may, for example, be carried out to (at least partly) remove one or more side products and/or by-products and/or excess reagents and/or water-miscible solvents. In some embodiments of the method of the invention, step (g-1) is carried out (i.e. is not optional). In some embodiments of the method of the invention, in the second coupling cycle, step (g-2) is carried out (i.e. is not optional). In some embodiments of the method of the invention, in at least one, or each, coupling cycle comprising steps (b-x) to (h-x) (as far as present), step (g-x) is carried out (i.e. is not optional).

The skilled artisan will understand that said solution comprising the first cycle oligonucleotide O-1 in step (g-1) may refer to the reaction mixture obtained from carrying out step (e-1), if step (f-1) is not performed, or may refer to the reaction mixture obtained from carrying out step (f-1) (wherein one or more quenchers or reducing agents may, for example, have been added beforehand as explained above). The skilled artisan will also understand that said solution comprising the second cycle oligonucleotide O-2 in step (g-2) may refer to the reaction mixture obtained from carrying out step (e-2), if step (f-2) is not performed, or may refer to the reaction mixture obtained from carrying out step (f-2) (wherein one or more quenchers or reducing agents may, for example, have been added beforehand as explained above). The skilled artisan will also understand that said solution comprising the x-th cycle oligonucleotide O-x in step (g-x) may refer to the reaction mixture obtained from carrying out step (e-x), if step (f-x) is not performed, or may refer to the reaction mixture obtained from carrying out step (f-x) (wherein one or more quenchers or reducing agents may, for example, have been added beforehand as explained above).

Optionally, said solutions comprising the first cycle oligonucleotide O-1, the second cycle oligonucleotide O-2 or the x-th cycle oligonucleotide O-x may be obtained from the respective reaction mixture by addition of one or more non-polar solvents, for example, in order to facilitate the phase separation. Alternatively, or additionally, such non-polar solvents may also be added during or in between the one or more aqueous extractions of any one of steps (g-1), (g-2), and (g-x). Non-limiting examples of non-polar solvents comprise those listed above in the context of steps (c-1), (c-2), and (c-x).

4-Methyltetrahydropyran (MTHP) may be a preferred non-polar ether solvent which may be added prior to or during or in between the aqueous extraction(s) of any one of steps (g-1), (g-2), and (g-x). Additionally, or alternatively, one or more amide solvents S^{A} may be added prior to or during or in between the aqueous extraction(s) of any one of steps (g-1), (g-2), and (g-x).

The term "aqueous extraction" in steps (g-1), (g-2), and (g-x) of the method of the invention may be understood in the broadest sense as any liquid-liquid extraction operation during which the respective solution comprising the first cycle oligonucleotide O-1, the second cycle oligonucleotide O-2 or the x-th cycle oligonucleotide O-x is extracted with water or an aqueous solution. The term "aqueous solution" has been defined above.

As one example, an aqueous solution of acetic acid (e.g. 10 vol-%) may be used as aqueous solution for the aqueous extraction(s) of steps (g-1), (g-2), and (g-x). As another example, an aqueous solution of sodium thiosulfate (e.g. 0.5 mol/L) may be used as aqueous solution for the aqueous extraction(s) of steps (g-1), (g-2), and (g-x). As another example, a mixture of an aqueous solution of sodium thiosulfate (e.g. 0.5 mol/L) and an aqueous solution of N-methylmorpholine (e.g. 1 mol/L) (e.g. 5:1 or 4:1 or 7:3 or 2:1 or 5:3 or 4:3, v:v) may be used as aqueous solution for the aqueous extraction(s) of steps (g-1), (g-2), and (g-x). As another example, a mixture of an aqueous solution of sodium thiosulfate (e.g. 0.5 mol/L) and acetone (e.g. 2:1 or 7:5, v:v) may be used as aqueous solution for the aqueous extraction(s) of steps (g-1), (g-2), and (g-x). As another example, a mixture of an aqueous solution of sodium thiosulfate (e.g. 0.5 mol/L), an aqueous solution of sodium chloride (i.e. brine, e.g. 10 wt-%), and acetone (e.g. 2:1:1, v:v:v) may be used as aqueous solution for the aqueous extraction(s) of steps (g-1), (g-2), and (g-x). As another example, an aqueous solution of ammonium chloride (e.g. 20 wt-%) may be used as aqueous solution for the aqueous extraction(s) of steps (g-1), (g-2), and (g-x). As another example, an aqueous solution of sodium chloride (i.e. brine, e.g. 20 wt-%) may be used as aqueous solution for the aqueous extraction(s) of steps (g-1), (g-2), and (g-x). The term "v:v" is herein used to denote volume ratios. For example a mixture obtained from mixing 10 mL of water and 10 mL of acetone would be denoted as 1:1, v:v.

It will be understood that for each of the one or more aqueous extractions of a step (g-1) or (g-2) or (g-x), the aqueous solution may be the same or different (i.e. comprise the same or different components in the same or different ratios). It will also be understood that the aqueous solutions may be the same or different for different steps (g-x) of different coupling cycles.

In case of more than one organic phases (either from a single aqueous extraction or from back-extraction of one or more aqueous phase), these more than one organic phases may be combined to obtain "the organic phase" mentioned in steps (g-1), (g-2), and (g-x). In general, the means of performing aqueous extractions are well-known to the skilled person.

The expression "the organic phase comprises the first cycle oligonucleotide O-1" in step (g-1) may be understood in the broadest sense to mean that some of the molecules of said first cycle oligonucleotide O-1 are dissolved in the organic phase. The expression "the organic phase comprises the second cycle oligonucleotide O-2" in step (g-2) may be understood in the broadest sense to mean that some of the molecules of said second cycle oligonucleotide O-2 are dissolved in the organic phase. The expression "the organic phase comprises the x-th cycle oligonucleotide O-x" in step (g-x) may be understood in the broadest sense to mean that some of the molecules of said x-th cycle oligonucleotide O-x are dissolved in the organic phase. It is preferred that most molecules (e.g. more than 50 %, 60 %, 70 %, 80 %, or 90 % of the molecules) of the said first cycle oligonucleotide O-1 or said second cycle oligonucleotide O-2 or said x-th cycle oligonucleotide O-x are comprised in the organic phase (and thus not in the aqueous phase).

Step (h-1) of the methods of the invention is: if step (g-1) has been carried out, optionally reducing the water content of the organic phase comprising the first cycle oligonucleotide O-1.

Step (h-2) of some of the methods of the invention is: if step (g-2) has been carried out, optionally reducing the water content of the organic phase comprising the second cycle oligonucleotide O-2.

Step (h-x) of some of the methods of the invention is: if step (g-x) has been carried out, optionally reducing the water content of the organic phase comprising the x-th cycle oligonucleotide O-x.

The expression "if step (g-1) has been carried out" in step (h-1) means that in the first coupling cycle, step (h-1) may only be carried out, if step (g-1) has been carried out. The expression "if step (g-2) has been carried out" in step (h-2) means that in the second coupling cycle, step (h-2) may only be carried out, if step (g-2) has been carried out. The expression "if step (g-x) has been carried out" in step (h-x) means that in a given coupling cycle comprising steps (b-x) to (h-x) (as far as present), step (h-x) may only be carried out, if step (g-x) has been carried out (in the same coupling cycle).

The term "optionally" in steps (h-1), (h-2), and (h-x) denotes that, even if the respective step (g-1), (g-2) or (g-x) has been carried out, the respective step (h-1), (h-2) or (h-x) may or may not be carried, unless indicated differently in the context of specific embodiments. In some embodiments of the method of the invention, step (h-1) is carried out (i.e. is not optional), if step (g-1) has been carried out. In some embodiments of the method of the invention, in the second coupling cycle, step (h-2) is carried out (i.e. is not optional), if step (g-2) has been carried out. In some embodiments of the method of the invention, in at least one, or each, coupling cycle comprising steps (b-x) to (h-x) (as far as present), step (h-x) is carried out (i.e. is not optional), if step (g-x) has been carried out in the same coupling cycle.

The term "the organic phase" in steps (h-1), (h-2), and (h-x) refers to "the organic phase" of the respective step (g-1) or (g-2) or (g-x) of the same coupling cycle.

The term "reducing the water content" has been defined above.

In some embodiments of the method of the invention, step (h-1) is: if step (g-1) has been carried out, optionally reducing the water content of the organic phase comprising the first cycle oligonucleotide O-1, wherein the water content is adjusted to be equal to or less than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm. In some embodiments of the method of the invention, step (h-2) is: if step (g-2) has been carried out, optionally reducing the water content of the organic phase comprising the second cycle oligonucleotide O-2, wherein the water content is adjusted to be equal to or less than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm. In some embodiments of the method of the invention, step (h-x) is: if step (g-x) has been carried out, optionally reducing the water content of the organic phase comprising the x-th cycle oligonucleotide O-x, wherein the water content is adjusted to be equal to or less than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm.

In some embodiments of the method of the invention:
- step (h-1) is carried out (i.e. is not optional), if the organic phase comprising the first cycle oligonucleotide O-1 of step (g-1) comprises more than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm of water;
- step (h-2) is carried out (i.e. is not optional), if the organic phase comprising the second cycle oligonucleotide O-2 of step (g-2) comprises more than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm of water; and
- step (h-x) is carried out (i.e. is not optional), if the organic phase comprising the x-th cycle oligonucleotide O-x of step (g-x) of the same coupling cycle comprises more than 2000 ppm, 1900 ppm, 1800 ppm, 1700 ppm, 1600 ppm, 1500 ppm, 1400 ppm, 1300 ppm, 1200 ppm, 1100 ppm, 1000 ppm, 900 ppm, 800 ppm, 700 ppm, 600 ppm, 500 ppm, 400 ppm, 300 ppm, 250 ppm, 200 ppm, 150 ppm, or 100 ppm of water.

Any means of reducing the water content may be employed, preferred examples of which comprise azeotropic distillation and the use of a drying agent, both of which have been explained above.

In some embodiments of the method of the invention, in at least one or each of steps (h-1), (h-2), and (h-x), reducing the water content is achieved by means of azeotropic distillation and/or by contacting said organic phase comprising the first cycle oligonucleotide O-1, the second cycle oligonucleotide O-2 or the x-th cycle oligonucleotide O-x with a drying agent. In some embodiments of the method of the invention, in at least one or each of steps (h-1), (h-2), and (h-x), reducing the water content is achieved by means of azeotropic distillation. In some embodiments of the method of the invention, in at least one or each of steps (h-1), (h-2), and (h-x), reducing the water content is achieved by means of contacting said organic phase comprising the first cycle oligonucleotide O-1, the second cycle oligonucleotide O-2 or the x-th cycle oligonucleotide O-x with a drying agent. It will be understood that in any one of steps (h-1), (h-2), and (h-x), one or more solvents and/or further components may be added to said organic phase comprising the first cycle oligonucleotide O-1, the second cycle oligonucleotide O-2 or the x-th cycle oligonucleotide O-x prior to, during, and/or after reduction of the water content.

In some embodiments of the method of the invention, in at least one or each of steps (d-1), (d-2), (d-x), (h-1), (h-2), and (h-x), reducing the water content is achieved by means of azeotropic distillation.

In some embodiments of the method of the invention:
- the first coupling cycle further comprises a step (i-1) of reacting free hydroxyl groups with a blocking agent, wherein step (i-1) is carried out after step (e-1) or after step (f-1); and/or
- the second coupling cycle further comprises a step (i-2) of reacting free hydroxyl groups with a blocking agent, wherein step (i-2) is carried out after step (e-2) or after step (f-2); and/or
- at least one coupling cycle comprising steps (b-x) to (h-x) (as far as present) further comprises a step (i-x) of reacting free hydroxyl groups with a blocking agent, wherein step (i-x) is carried out after step (e-x) or after step (f-x).

The free backbone hydroxyl group present in the component (C-0)^{#}, the first cycle oligonucleotide (O-1)^{#}, and the (x-1)-th cycle oligonucleotide (O-(x-1))^{#} is supposed to engage in the bond forming reaction of step (e-1), (e-2) or (e-x) (of the same coupling cycle), which consumes said free backbone hydroxyl group in the sense of incorporating it into a newly-formed internucleosidic linkage group. However, a (typically very small, e.g. < 1 %, < 0.5 % or < 0.1 %) fraction of the free backbone hydroxyl groups may not engage in the bond forming reaction at the first occasion, i.e. in the same coupling cycle, in which the respective free hydroxyl group has been generated by cleaving a protecting group. Such free hydroxyl groups are herein also referred to as unreacted free hydroxyl groups. These groups would be available to participate in the bond forming reactions of the coupling steps of following coupling cycles. This may not be desirable, since it would afford an oligonucleotide product lacking one nucleoside subunit. Such oligonucleotide products may be difficult to remove from the target oligonucleotide O^{T} later on.

As used herein, the terms "blocking agent" and "capping agent" are used interchangeably to denote any chemical reagent capable of acylating, preferably acetylating, a free hydroxyl group. Capping agents for oligonucleotide synthesis form part of the common knowledge of those skilled in the art. Any blocking (i.e. capping) agents known from oligonucleotide synthesis may be used in the method of the invention. Preferred examples of such blocking agents comprise anhydrides of carboxylic acids, in particular acetic anhydride. It is known to those skilled in the art that free carboxylic acids may also be used in combination with activating agents such as carbodiimides (e.g. dicyclohexylcarbdiimide or diisopropylcarbodiimide) and additives such as, e.g., 4-dimethylaminopyridine (DMAP). For example, acetylation may be achieved by treating the growing oligonucleotide chains with neat acetic anhydride or a solution thereof. An organic base such as pyridine, lutidine (e.g. 2,6-lutidine), collidine, N-methylimidazole, or a mixture thereof may be added. For example, acetic anhydride (e.g. 4 moles of acetic anhydride per 1 mol of employed component (C-0)^{#}) may be used, preferably in combination with a base such as pyridine (e.g. 5 moles of base per 1 mol of employed component (C-0)^{#}). The blocking agents may be added directly into the reaction vessel or reactor, in which step (e-1), (e-2), (e-x), (f-1), (f-2) or (f-x) was carried out.

The term "reacting" in step (i-1), (i-2), and (i-x) may be understood in the broadest sense as any operation during which the growing oligonucleotide chains and the blocking agent are present in the same reaction vessel or reactor and engage in the blocking reaction.

In some embodiments of the method of the invention, the method further comprises
- a step (k-1) of incubating the first cycle oligonucleotide O-1 with a deprotection mixture M-(k-1), thereby cleaving the protecting group PG-1 from the first cycle oligonucleotide O-1, so as to obtain a first cycle oligonucleotide (O-1)^{#} having a free backbone hydroxyl group; and/or
- a step (m-1) of incubating the first cycle oligonucleotide O-1 or (O-1)^{#} with a base, thereby cleaving the pseudo solid-phase protecting group PG-s and, optionally, one or more further protecting groups from the first cycle oligonucleotide O-1 or (O-1)^{#} and/or
- a step (p-1) of modifying the first cycle oligonucleotide O-1 or (O-1)^{#};
wherein, if more than one of steps (k-1), (m-1), and (p-1) are performed, they may be performed in any order.

It will be understood that any one of steps (k-1), (m-1), and (p-1) may only be carried out after all steps of the first coupling cycle (optionally excluding steps indicated as optional) have been performed. It will also be understood that, if any one of steps (k-1), (m-1), and (p-1) is carried out, the second coupling cycle comprising steps (b-2) to (h-2) and thus any further coupling cycles comprising steps (b-x) to (h-x) (as far as present) may not be carried out.

In some embodiments of the method of the invention, the method further comprises
- a step (k-2) of incubating the second cycle oligonucleotide O-2 with a deprotection mixture M-(k-2), thereby cleaving the protecting group PG-2 from the second cycle oligonucleotide O-2, so as to obtain a second cycle oligonucleotide (O-2)^{#} having a free backbone hydroxyl group; and/or
- a step (m-2) of incubating the second cycle oligonucleotide O-2 or (O-2)^{#} with a base, thereby cleaving the pseudo solid-phase protecting group PG-s and, optionally, one or more further protecting groups from the second cycle oligonucleotide O-2 or (O-2)^{#}; and/or
- a step (p-2) of modifying the second cycle oligonucleotide O-2 or (O-2)^{#}; wherein, if more than one of steps (k-2), (m-2), and (p-2) are performed, they may be performed in any order.

It will be understood that any one of steps (k-2), (m-2), and (p-2) may only be carried out after all steps of the second coupling cycle (optionally excluding steps indicated as optional) have been performed. It will also be understood that, if any one of steps (k-2), (m-2), and (p-2) is carried out, further coupling cycles comprising steps (b-x) to (h-x) (as far as present) may not be carried out.

In some embodiments of the method of the invention, the method further comprises
- a step (k-n) of incubating the n-th cycle oligonucleotide O-n with a deprotection mixture M-(k-n), thereby cleaving the protecting group PG-n from the n-th cycle oligonucleotide O-n, so as to obtain a n-th cycle oligonucleotide (O-n)^{#} having a free backbone hydroxyl group; and/or
- a step (m-n) of incubating the n-th cycle oligonucleotide O-n or (O-n)^{#} with a base, thereby cleaving the pseudo solid-phase protecting group PG-s and, optionally, one or more further protecting groups from the n-th cycle oligonucleotide O-n or (O-n)^{#}; and/or
- a step (p-n) of modifying the n-th cycle oligonucleotide O-n or (O-n)^{#};
wherein, if more than one of steps (k-n), (m-n), and (p-n) are performed, they may be performed in any order.

It will be understood that any one of steps (k-n), (m-n), and (p-n) may only be carried out after all steps of the (n-2)-th iteration of the coupling cycle comprising steps (b-x) to (h-x) (optionally excluding steps indicated as optional) have been performed.

The term "deprotection mixture M-(k-1)" refers to any mixture which may be used to effect cleavage of the protecting group PG-1 from the first cycle oligonucleotide O-1. Any definitions and embodiments pertaining to the deprotection mixture M-(b-2) may also apply to the deprotection mixture M-(k-1). The term "deprotection mixture M-(k-2)" refers to any mixture which may be used to effect cleavage of the protecting group PG-2 from the second cycle oligonucleotide O-2. Any definitions and embodiments pertaining to the deprotection mixture M-(b-3) (i.e. a deprotection mixture M-(b-x) with x=3) may also apply to the deprotection mixture M-(k-2). The term "deprotection mixture M-(k-n)" refers to any mixture which may be used to effect cleavage of the protecting group PG-n from the n-th cycle oligonucleotide O-n. Any definitions and embodiments pertaining to the deprotection mixture M-(b-x) may also apply to the deprotection mixture M-(k-n).

The term "base" in steps (m-1), (m-2), and (m-n) may be understood in the broadest sense to refer to any base (a proton acceptor in the sense of the Brønsted-Lowry theory), unless indicated differently in the context of specific embodiments. Non-limiting examples of such a base comprise ammonia, a (C₁-C₆-alkyl)NH₂ monoalkylamine, in particular methylamine, ethylamine or tert-butylamine, a (C₁-C₆-alkyl)₂NH dialkylamine, in particular dimethylamine or diethylamine, a source of hydroxide ions, and mixtures thereof. As known to the skilled artisan, such bases will typically not be employed as such in a step (m-1), (m-2) or (m-n), but rather in form of a solution of the base in a suitable solvent, in particular water, i.e. as an aqueous solution of the respective base. Such an aqueous solution may further comprise one or more water-miscible organic solvents such as, e.g., acetonitrile, tetrahydrofuran, methanol, ethanol, and the like. As used herein, the term "a source of hydroxide ions" may refer to a salt which comprises hydroxide ions, wherein sodium hydroxide, potassium hydroxide, and ammonium hydroxide may be preferred examples. The person skilled in the art knows that an aqueous solution of ammonia may also be referred to as an ammonium hydroxide solution, since it comprises ammonium ions and hydroxide ions. The skilled artisan understands that a solution comprising a source of hydroxide anions may comprise said source of hydroxide ions in solvated form. In some embodiments, said base of any one of steps (m-1), (m-2), and (m-n) is an aqueous solution of one or more compounds selected from the group consisting of ammonia, a (C₁-C₆-alkyl)NH₂ monoalkylamine, a (C₁-C₆-alkyl)₂NH dialkylamine, sodium hydroxide, potassium hydroxide, and mixtures thereof. In some embodiments, said base of any one of steps (m-1), (m-2), and (m-n) is an aqueous solution of one or more compounds selected from the group consisting of ammonia, methylamine, ethylamine, tert-butylamine, sodium hydroxide, potassium hydroxide, and mixtures thereof. For example, said base of any one of steps (m-1), (m-2), and (m-n) may be an aqueous solution of ammonia, e.g. 25-30 wt-% aqueous ammonia. As another example, said base of any one of steps (m-1), (m-2), and (m-n) may be an aqueous solution of tert-butylamine, e.g. a 1:1 (v:v) mixture of water and tert-butylamine. As another example, said base of any one of steps (m-1), (m-2), and (m-n) may be an aqueous solution of methylamine, e.g. a 1:1 (v:v) mixture of water and methylamine. As another example, said base of any one of steps (m-1), (m-2), and (m-n) may be a solution of sodium hydroxide (e.g. 1 mol/L) in a mixture of water and tetrahydrofuran (e.g. 1:3, v:v). Alternatively, hydrazine (e.g. used in form of its hydrate) may, for example, be used as nucleophilic base.

In some embodiments of the method of the invention, the method comprises exactly one step selected from the group consisting of step (m-1), step (m-2), and step (m-n), and wherein
- step (m-1) is carried out by incubating the first cycle oligonucleotide O-1 or (O-1)^{#} with an aqueous solution of an organic amine, preferably an aliphatic amine, more preferably a primary or secondary aliphatic amine, in particular a primary aliphatic amine such as *tert*-butylamine, and an aliphatic alcohol, preferably an aliphatic alcohol comprising 1-6 carbon atoms, in particular methanol;
- step (m-2) is carried out by incubating the second cycle oligonucleotide O-2 or (O-2)^{#} with an aqueous solution of an organic amine, preferably an aliphatic amine, more preferably a primary or secondary aliphatic amine, in particular a primary aliphatic amine such as *tert*-butylamine, and an aliphatic alcohol, preferably an aliphatic alcohol comprising 1-6 carbon atoms, in particular methanol; and
- step (m-n) is carried out by incubating the n-th cycle oligonucleotide O-n or (O-n)^{#} with an aqueous solution of an organic amine, preferably an aliphatic amine, more preferably a primary or secondary aliphatic amine, in particular a primary aliphatic amine such as *tert*-butylamine, and an aliphatic alcohol, preferably an aliphatic alcohol comprising 1-6 carbon atoms, in particular methanol.

In such embodiments, the respective oligonucleotide, i.e., the first cycle oligonucleotide O-1 or (O-1)^{#} in a step (m-1) and the second cycle oligonucleotide O-2 or (O-2)^{#} in a step (m-2) and the n-th cycle oligonucleotide O-n or (O-n)^{#} in a step (m-n) preferably comprises at least one internucleosidic linkage group selected from the group consisting of a thiophosphate triester group and a thiophosphate diester group, preferably a linkage group of Formula B, where X¹ is S and X² is either OH or O-CH₂-CH₂-CN.

In some embodiments of the method of the invention, the method comprises exactly one step selected from the group consisting of step (m-1), step (m-2), and step (m-n), and wherein
- step (m-1) is carried out by incubating the first cycle oligonucleotide O-1 or (O-1)^{#} with an aqueous solution of a (C₁-C₆-alkyl)NH₂ monoalkylamine, in particular *tert*-butylamine, and an aliphatic alcohol comprising 1-6 carbon atoms, in particular methanol;
- step (m-2) is carried out by incubating the second cycle oligonucleotide O-2 or (O-2)^{#} with an aqueous solution of a (C₁-C₆-alkyl)NH₂ monoalkylamine, in particular *tert*-butylamine, and an aliphatic alcohol comprising 1-6 carbon atoms, in particular methanol; and
- step (m-n) is carried out by incubating the n-th cycle oligonucleotide O-n or (O-n)^{#} with an aqueous solution of a (C₁-C₆-alkyl)NH₂ monoalkylamine, in particular *tert*-butylamine, and an aliphatic alcohol comprising 1-6 carbon atoms, in particular methanol.

In such embodiments, the respective oligonucleotide, i.e., the first cycle oligonucleotide O-1 or (O-1)^{#} in a step (m-1) and the second cycle oligonucleotide O-2 or (O-2)^{#} in a step (m-2) and the n-th cycle oligonucleotide O-n or (O-n)^{#} in a step (m-n) preferably comprises at least one internucleosidic linkage group selected from the group consisting of a thiophosphate triester group and a thiophosphate diester group, preferably a linkage group of Formula B, where X¹ is S and X² is either OH or O-CH₂-CH₂-CN.

In some embodiments of the method of the invention, the method comprises exactly one step selected from the group consisting of step (m-1), step (m-2), and step (m-n), and wherein
- step (m-1) is carried out by incubating the first cycle oligonucleotide O-1 or (O-1)^{#} with an aqueous solution of a (C₁-C₆-alkyl)NH₂ monoalkylamine, in particular *tert*-butylamine, and an aliphatic alcohol comprising 1-6 carbon atoms, in particular methanol, wherein the volume-ratio of monoalkylamine to aliphatic alcohol to water is 1:1:2 (v:v:v);
- step (m-2) is carried out by incubating the second cycle oligonucleotide O-2 or (O-2)^{#} with an aqueous solution of a (C₁-C₆-alkyl)NH₂ monoalkylamine, in particular *tert*-butylamine, and an aliphatic alcohol comprising 1-6 carbon atoms, in particular methanol, wherein the volume-ratio of monoalkylamine to aliphatic alcohol to water is 1:1:2 (v:v:v); and
- step (m-n) is carried out by incubating the n-th cycle oligonucleotide O-n or (O-n)^{#} with an aqueous solution of a (C₁-C₆-alkyl)NH₂ monoalkylamine, in particular *tert*-butylamine, and an aliphatic alcohol comprising 1-6 carbon atoms, in particular methanol, wherein the volume-ratio of monoalkylamine to aliphatic alcohol to water is 1:1:2 (v:v:v).

In such embodiments, the respective oligonucleotide, i.e., the first cycle oligonucleotide O-1 or (O-1)^{#} in a step (m-1) and the second cycle oligonucleotide O-2 or (O-2)^{#} in a step (m-2) and the n-th cycle oligonucleotide O-n or (O-n)^{#} in a step (m-n) preferably comprises at least one internucleosidic linkage group selected from the group consisting of a thiophosphate triester group and a thiophosphate diester group, preferably a linkage group of Formula B, where X¹ is S and X² is either OH or O-CH₂-CH₂-CN.

The use of an aqueous solution of a (C₁-C₆-alkyl)NH₂ monoalkylamine, in particular *tert*-butylamine, and an aliphatic alcohol comprising 1-6 carbon atoms, in particular methanol is particularly preferred, if the pseudo solid-phase protecting group PG-s is a protecting group of Formula P-1-a below, in particular, with the integer a being 1.

Steps (m-1), (m-2), and (m-n) may, for example, be performed at a temperature in the range of 5-95 °C, 10-95 °C, 15-95 °C, 20-95 °C, 25-95 °C, 30-95 °C, 35-95 °C, 50-90 °C, 55-90 °C, 60-85 °C, or 60-80 °C. Lower temperatures, e.g., in the range of 45-70 °C or 50-60 °C, in particular 55 °C, may be preferred when using an aqueous solution of a (C₁-C₆-alkyl)NH₂ monoalkylamine, in particular *tert*-butylamine, and an aliphatic alcohol comprising 1-6 carbon atoms, in particular methanol. It will be understood that steps (m-1), (m-2), and (m-n) may preferably be performed in a sealed reaction vessel or reactor, e.g. in an autoclave, when operating at elevated temperatures.

Under the alkaline conditions of steps (m-1), (m-2), and (m-n), "one or more further protecting groups" (i.e. other than said pseudo solid-phase PG-s) may optionally be cleaved from the respective oligonucleotide. In fact, as will be understood by the skilled artisan, any common protecting groups removable under alkaline conditions will typically be removed during the incubation with said base in steps (m-1), (m-2), and (m-n). Such protecting groups removable under alkaline conditions, e.g. under the conditions of steps (m-1), (m-2), and (m-n), may, for example, be the typical nucleobase protecting groups, non-limiting examples of which comprise the protecting groups listed in Table T-1 above. Additionally, protecting groups at the internucleosidic linkage groups, e.g. any protecting groups R^{Z-1} and R^{Z-2}, in particular any 2-cyanoethyl protecting groups, may typically be removed under the conditions of steps (m-1), (m-2), and (m-n).

In the context of steps (p-1), (p-2), and (p-n) of the method of the invention, the term "modifying" may be understood in the broadest sense to embrace "chemically modifying" and/or "biotechnologically modifying" the respective oligonucleotides.

In the context of steps (p-1), (p-2), and (p-n), "chemically modifying" is defined as subjecting the respective oligonucleotide (which is to be chemically modified) to one or more chemical reactions. Non-limiting examples of such chemical reactions are
- the removal of one or more protecting groups (including any pseudo solid-phase protecting groups);
- conjugation with one or more compounds selected from the group consisting of a nucleoside, an oligonucleotide, a carbohydrate such as a monosaccharide or a polysaccharide, an amino acid, a peptide, a lipid, an active pharmaceutical ingredient, or the like; and
- an intramolecular bond forming reaction resulting in cyclization.

It will be understood that more than one such chemical reactions may be performed in the course of said chemical modification.

In the context of steps (p-1), (p-2), and (p-n), "biotechnologically modifying" is defined as subjecting the respective oligonucleotide (which is to be biotechnologically modified) to one or more enzymatic reactions. As used herein, the term "enzymatic reaction" refers to any reaction which is enabled by and/or catalyzed by one or more enzymes. A preferred example is enzymatic ligation.

In some embodiments, the method of the invention further comprises a step (z) of isolating the target oligonucleotide O^{T}. The means of isolating oligonucleotides upon oligonucleotide synthesis form part of the common knowledge of those skilled in the art. Typically, such a step of isolating an oligonucleotide comprises one or more purification steps and one or more steps aiming at obtaining the oligonucleotide in solid form. For oligonucleotide purification, chromatographic methods may typically be used, in particular ion exchange (especially anion exchange) chromatography and reversed phase (RP) HPLC, e.g. in form of hydrophobic interaction HPLC. These techniques are known to those skilled in the art. Additionally, a step of isolating an oligonucleotide may comprise ultrafiltration and/or desalting steps. Typically, the target oligonucleotide to be isolated may be dissolved in a reaction mixture. It may then be preferable to first obtain a crude product either by precipitating the target oligonucleotide to be purified from the respective reaction mixture or by evaporation to dryness (e.g. *in vacuo*). Precipitation may for example be achieved by (partly) removing one or more solvents and/or by addition of one or more antisolvents (in which the target oligonucleotide is not soluble or poorly soluble) and/or by cooling the reaction mixture from which the target oligonucleotide to be isolated. The so-obtained crude product may, for example be washed with one or more solvents and/or aqueous solutions. The crude product may then be dissolved, so as to obtain a solution, preferably an aqueous solution of the target oligonucleotide to be isolated. Alternatively, the reaction mixture containing the target oligonucleotide as such may be used without precipitation or evaporation to dryness. For example, a solution, preferably an aqueous solution, containing the target oligonucleotide O^{T} to be isolated may be submitted to ultrafiltration and/or desalting, ion exchange chromatography, and another round of ultrafiltration and/or desalting. Alternatively, a crude product containing the target oligonucleotide O^{T} to be isolated may be submitted to reversed phase (RP) HPLC, e.g. in form of hydrophobic interaction HPLC. The latter method may preferably be performed, if the oligonucleotides still carry the 5'-terminal hydroxyl protecting group, e.g. the DMT group. Said 5'-protecting group may even be removed on the RP-HPLC column by passing an acidic solution through the column. If the 5'-terminal protecting group has been removed prior to purification, ion exchange chromatography may be preferred. Purification is typically followed by one or more steps aiming at obtaining the oligonucleotide in solid form. Lyophilization or spray drying may, for example, be used. In some cases, it may be desirable to obtain the oligonucleotide in form of a salt with certain counter ions. In such cases, salt exchange may be performed, typically prior to lyophilization or spray drying.

In the method of the invention, during and in between steps (b-1) to (h-1) (as far as present), no solid-liquid separation is performed and steps (c-1) and (g-1) are carried out in the presence of one or more amide solvents S^{A}, wherein each amide solvent S^{A} is an amide solvent comprising one or more alkyl groups, wherein these one or more alkyl groups together comprise in total 6-48 carbon atoms. It will be understood that the expression "steps (c-1) and (g-1) are carried out in the presence of one or more amide solvents S^{A}" does not imply that step (g-1) must be carried out, i.e., is not optional. However, if step (g-1) is carried out, it is carried out in the presence of one or more amide solvents S^{A}.

If the method of the invention comprises performing a second coupling cycle comprising steps (b-2) to (h-2) (as far as present) , during and in between steps (b-1) to (h-2) (as far as present), no solid-liquid separation is performed and steps (c-1), (g-1), (c-2), and (g-2) are carried out in the presence of one or more amide solvents S^{A}, wherein each amide solvent S^{A} is an amide solvent comprising one or more alkyl groups, wherein these one or more alkyl groups together comprise in total 6-48 carbon atoms. It will be understood that the expression "steps (c-1), (g-1), (c-2), and (g-2) are carried out in the presence of one or more amide solvents S^{A}" does not imply that steps (g-1) and (g-2) must be carried out, i.e., are not optional. However, if one or both of steps (g-1) and (g-2) are carried out, they are carried out in the presence of one or more amide solvents S^{A}.

If the method of the invention comprises performing (n-2) iterations of a coupling cycle comprising the following steps (b-x) to (h-x) (as far as present), wherein n is an integer in the range of 3 to 99, which denotes the total number of coupling cycles performed to obtain to obtain the n-th cycle oligonucleotide O-n, during and in between steps (b-1) to (h-n) (as far as present), no solid-liquid separation is performed, and steps (c-1), (g-1), (c-2), and (g-2), as well as each iteration of steps (c-x) and (g-x) is carried out in the presence of one or more amide solvents S^{A}, wherein each amide solvent S^{A} is an amide solvent comprising one or more alkyl groups, wherein these one or more alkyl groups together comprise in total 6-48 carbon atoms. It will be understood that the expression "steps (c-1), (g-1), (c-2), and (g-2), as well as each iteration of steps (c-x) and (g-x) are carried out in the presence of one or more amide solvents S^{A}" does not imply that steps (g-1), (g-2), and (g-x) must be carried out, i.e., are not optional. However, if one or more of steps (g-1), (g-2) and (g-x) are carried out, they are carried out in the presence of one or more amide solvents S^{A}.

As used herein, the term "amide solvent" may be understood in the broadest sense to refer to any solvent characterized in that in its chemical structure, at least one carbonyl carbon atom (C=O) is covalently bonded directly to a nitrogen atom which may be further substituted or protected. An amide solvent S^{A} is herein further defined by comprising one or more alkyl groups, wherein these one or more alkyl groups together comprise in total 6-48 carbon atoms or 8-48 carbon atoms.

Examples and further embodiments pertaining to an amide solvent S^{A} will be laid out in a later section of this text. To state that a certain step, e.g. any one of steps (c-1), (g-1), (c-2), (g-2), (c-x), and (g-x), is carried out "in the presence of one or more amide solvents S^{A}" may be understood in the broadest sense to mean that the solvent or mixed solvent, in which or with which the respective step is carried out, comprises at least one amide solvent S^{A}. In the case of the extraction steps (c-1), (g-1), (c-2), (g-2), (c-x), and (g-x) this may mean that each of the one or more aqueous extractions is carried out in the presence of at least one amide solvent S^{A}, wherein the latter is preferably (at least partly or mostly) comprised in the organic phase.

As used herein, the term "solid-liquid separation" refers to any operation during which a solid is separated from a liquid. Most commonly, solid-liquid separation will be filtration or centrifugation, preferably filtration. It will be understood that such solid-liquid separation may typically not be complete, meaning that some residual liquid may typically be attached to the solid after solid-liquid separation, as known to those skilled in the art. It will be understood that the term "solid-liquid separation" does not embrace evaporation of the liquid. Hence, as used herein, a step of, e.g., evaporating a solution to dryness is not considered a "solid-liquid separation". In some embodiments of the present invention, the process does not comprise any solid-liquid separation during or in between at least at least 3, 4, 5, 6, 7, 8, 9 consecutive coupling cycles. In some embodiments of the present invention, the process does not comprise any solid-liquid separation of the n-th cycle oligonucleotide O-n or of any oligonucleotidic educts or intermediates involved in the synthesis of the n-th cycle oligonucleotide O-n during or in between at least at least 3, 4, 5, 6, 7, 8, 9 consecutive coupling cycles.

The term "during and in between steps (b-1) to (h-1)" means that
- none of the steps (b-1), (c-1), (d-1), (e-1), (f-1), (g-1), and (h-1) comprises a solid-liquid separation (each of steps (d-1), (f-1), (g-1), and (h-1) may or may not be carried out as explained above),
- if carried out, step (i-1) does not comprise a solid-liquid separation, and
- no solid-liquid separation is performed in between any two of these steps.

The term "during and in between steps (b-1) to (h-2)" means that
- none of the steps (b-1), (c-1), (d-1), (e-1), (f-1), (g-1), (h-1), (b-2), (c-2), (d-2), (e-2), (f-2), (g-2), and (h-2) (as far as present, each of steps (d-1), (f-1), (g-1), (h-1), (d-2), (f-2), (g-2), (h-2) may or may not be carried out as explained above), comprises a solid-liquid separation,
- if carried out, step (i-1) does not comprise a solid-liquid separation,
- if carried out, step (i-2) does not comprise a solid-liquid separation, and
- no solid-liquid separation is performed in between any two of these steps.

The term "during and in between steps (b-1) to (h-n)" means that
- none of the steps (b-1), (c-1), (d-1), (e-1), (f-1), (g-1), (h-1), (b-2), (c-2), (d-2), (e-2), (f-2), (g-2), and (h-2)(each of steps (d-1), (f-1), (g-1), (h-1), (d-2), (f-2), (g-2), (h-2) may or may not be carried out as explained above) comprises a solid-liquid separation,
- no iteration of step (b-x), no iteration of step (c-x), no iteration of step (d-x), no iteration of step (e-x), no iteration of step (f-x), no iteration of step (g-x), and no iteration of step (h-x) (each of steps (d-x), (f-x) (g-x) and (h-x) may or may not be carried out in a given coupling cycle) comprises a solid-liquid separation,
- if carried out, step (i-1) does not comprise a solid-liquid separation,
- if carried out, step (i-2) does not comprise a solid-liquid separation,
- if carried out, no iteration of step (i-x) comprises a solid-liquid separation, and
- no solid-liquid separation is performed in between any two of these steps.

In some embodiments of the method of the invention, steps (b-1) to (h-1) (i.e. any one of steps (b-1), (c-1), (d-1), (e-1), (f-1), (g-1), and (h-1) as far as present) are carried out in the presence of one or more amide solvents S^{A} and, preferably, one or more ether solvent S^{E}. It will be understood that this does not imply that any optional step must be carried out.

In some embodiments of the method of the invention, if the second coupling cycle comprising steps (b-2) to (h-2) is performed, steps (b-1) to (h-2) (i.e. any one of steps (b-1), (c-1), (d-1), (e-1), (f-1), (g-1), (h-1), (b-2), (c-2), (d-2), (e-2), (f-2), (g-2), and (h-2) as far as present) are carried out in the presence of one or more amide solvents S^{A} and, preferably, one or more ether solvent S^{E}. It will be understood that this does not imply that any optional step must be carried out.

In some embodiments of the method of the invention, if (n-2) iterations of the coupling cycle comprising steps (b-x) to (h-x) (as far as present) are performed, all of steps (b-1) to (h-n) (as far as present) (i.e. any one of steps (b-1), (c-1), (d-1), (e-1), (f-1), (g-1), (h-1), (b-2), (c-2), (d-2), (e-2), (f-2), (g-2), and (h-2), as far as present, as well as each iteration of any one of steps (b-x), (c-x), (d-x), (e-x), (f-x), (g-x), and (h-x)) are carried out in the presence of one or more amide solvents S^{A} and, preferably, one or more ether solvent S^{E}. It will be understood that this does not imply that any optional step must be carried out.

In some embodiments of the method of the invention, steps (b-1) to (h-1) (as far as present) are carried out in the presence of one or more amide solvents S^{A} and, preferably, one or more ether solvents S^{E}, and further wherein:
- if the second coupling cycle comprising steps (b-2) to (h-2) (as far as present) is performed, steps (b-1) to (h-2) (as far as present) are carried out in the presence of one or more amide solvents S^{A} and, preferably, one or more ether solvents S^{E}, and
- if (n-2) iterations of the coupling cycle comprising steps (b-x) to (h-x) (as far as present) are performed, all of steps (b-1) to (h-n) (as far as present) are carried out in the presence of one or more amide solvents S^{A} and, preferably, one or more ether solvents S^{E}.

The term "in the presence of one or more amide solvents S^{A}" has been defined above, in particular with a focus on the extraction steps. In the case of steps pertaining to a chemical reaction such as the deprotection steps (b-1), (b-2), and (b-x), the coupling steps (e-1), (e-2), and (e-x) as well as the oxidation or sulfurization steps (f-1), (f-2), and (f-x), the term "in the presence of one or more amide solvents S^{A}" may mean that the reaction mixture of the respective step(s) (i.e. the solution in which the respective reaction occurs) comprises at least one amide solvent S^{A}. In the case steps (d-1), (d-2), (d-x), (h-1), (h-2), and (h-x), the term "in the presence of one or more amide solvents S^{A}" may mean that said organic phase, whose water content is to be reduced, comprises at least one amide solvent S^{A}.

It will be understood that any explanations pertaining to the term "in the presence of one or more amide solvents S^{A}" also apply to the term "in the presence of one or more ether solvents S^{E}".

As used herein, the term "ether solvent" may be understood in the broadest sense to refer to any solvent characterized in that its chemical structure comprises at least one oxygen atom covalently bonded to exactly two residues independently selected from the group consisting of an alkyl group, an alkenyl group, an alkynyl group, a heteroalkyl group, a heteroalkenyl group, a heteroalkynyl group, an aryl group, and a heteroaryl group, wherein each of these two residues engages in a covalent chemical bond to said oxygen atom via a carbon atom (C), and wherein these two residues may optionally bond to each other to form a cyclic structure. Unless indicated differently in the context of specific embodiments, an ether solvent S^{E} may be any ether solvent. Examples and further embodiments pertaining to an ether solvent S^{E} will be laid out in a later section of this text.

In some embodiments of the method of the invention:
- each of steps (b-1), (e-1) and (f-1) is carried out in a mixed solvent comprising in total 10-90 vol-% of one or more amide solvents S^{A} and 10-90 vol-% of one or more ether solvents S^{E};
- if the second coupling cycle comprising steps (b-2) to (h-2) (as far as present) is performed, each of steps (b-1), (e-1), (f-1), (b-2), (e-2), and (f-2) is carried out in a mixed solvent comprising in total 10-90 vol-% of one or more amide solvents S^{A} and 10-90 vol-% of one or more ether solvents S^{E}; and
- if (n-2) iterations of the coupling cycle comprising steps (b-x) to (h-x) (as far as present) are performed, each of steps (b-1), (e-1), (f-1), (b-2), (e-2), and (f-2) as well as each iteration of steps (b-x), (e-x), and (f-x) is carried out in a mixed solvent comprising in total 10-90 vol-% of one or more amide solvents S^{A} and 10-90 vol-% of one or more ether solvents S^{E}.

It will be understood that this does not imply that the optional step (f-1) or (f-2) or (f-x) is performed in each coupling cycle. The term solvent in the term "carried out in a solvent" will be understood to refer to the reaction solvent, i.e. to the solvent or mixed solvent, in which the respective chemical reaction (deprotection or coupling or oxidation/sulfurization) takes place.

In some embodiments of the method of the invention:
- each of steps (b-1), (e-1) and (f-1) is carried out in a mixed solvent comprising in total 20-80 vol-% of one or more amide solvents S^{A} and 20-80 vol-% of one or more ether solvents S^{E};
- if the second coupling cycle comprising steps (b-2) to (h-2) (as far as present) is performed, each of steps (b-1), (e-1), (f-1), (b-2), (e-2), and (f-2) is carried out in a mixed solvent comprising in total 20-80 vol-% of one or more amide solvents S^{A} and 20-80 vol-% of one or more ether solvents S^{E}; and
- if (n-2) iterations of the coupling cycle comprising steps (b-x) to (h-x) (as far as present) are performed, each of steps (b-1), (e-1), (f-1), (b-2), (e-2), and (f-2) as well as each iteration of steps (b-x), (e-x), and (f-x) is carried out in a mixed solvent comprising in total 20-80 vol-% of one or more amide solvents S^{A} and 20-80 vol-% of one or more ether solvents S^{E}.

In some embodiments of the method of the invention:
- each of steps (b-1), (e-1) and (f-1) is carried out in a mixed solvent comprising in total 30-70 vol-% of one or more amide solvents S^{A} and 30-70 vol-% of one or more ether solvents S^{E};
- if the second coupling cycle comprising steps (b-2) to (h-2) (as far as present) is performed, each of steps (b-1), (e-1), (f-1), (b-2), (e-2), and (f-2) is carried out in a mixed solvent comprising in total 30-70 vol-% of one or more amide solvents S^{A} and 30-70 vol-% of one or more ether solvents S^{E}; and
- if (n-2) iterations of the coupling cycle comprising steps (b-x) to (h-x) (as far as present) are performed, each of steps (b-1), (e-1), (f-1), (b-2), (e-2), and (f-2) as well as each iteration of steps (b-x), (e-x), and (f-x) is carried out in a mixed solvent comprising in total 30-70 vol-% of one or more amide solvents S^{A} and 30-70 vol-% of one or more ether solvents S^{E}.

In some embodiments of the method of the invention:
- each of steps (b-1), (e-1) and (f-1) is carried out in a mixed solvent comprising in total 10-50 vol-% of one or more amide solvents S^{A} and 50-90 vol-% of one or more ether solvents S^{E};
- if the second coupling cycle comprising steps (b-2) to (h-2) (as far as present) is performed, each of steps (b-1), (e-1), (f-1), (b-2), (e-2), and (f-2) is carried out in a mixed solvent comprising in total 10-50 vol-% of one or more amide solvents S^{A} and 50-90 vol-% of one or more ether solvents S^{E}; and
- if (n-2) iterations of the coupling cycle comprising steps (b-x) to (h-x) (as far as present) are performed, each of steps (b-1), (e-1), (f-1), (b-2), (e-2), and (f-2) as well as each iteration of steps (b-x), (e-x), and (f-x) is carried out in a mixed solvent comprising in total 10-50 vol-% of one or more amide solvents S^{A} and 50-90 vol-% of one or more ether solvents S^{E}.

In some embodiments of the method of the invention:
- each of steps (b-1), (e-1) and (f-1) is carried out in a mixed solvent comprising in total 20-50 vol-% of one or more amide solvents S^{A} and 50-80 vol-% of one or more ether solvents S^{E};
- if the second coupling cycle comprising steps (b-2) to (h-2) (as far as present) is performed, each of steps (b-1), (e-1), (f-1), (b-2), (e-2), and (f-2) is carried out in a mixed solvent comprising in total 20-50 vol-% of one or more amide solvents S^{A} and 50-80 vol-% of one or more ether solvents S^{E}; and
- if (n-2) iterations of the coupling cycle comprising steps (b-x) to (h-x) (as far as present) are performed, each of steps (b-1), (e-1), (f-1), (b-2), (e-2), and (f-2) as well as each iteration of steps (b-x), (e-x), and (f-x) is carried out in a mixed solvent comprising in total 20-50 vol-% of one or more amide solvents S^{A} and 50-80 vol-% of one or more ether solvents S^{E}.

The composition of the solvents in which any one of steps (b-1), (e-1), (f-1), (b-2), (e-2), (f-2), (b-x), (e-x), and (f-x) is carried out, may, for example, be analyzed by means of gas chromatography. For example, the following setup may be used: Column: SH-Rxi-624Sil MS (30 m × 0.32 mmID, 1.8 µm), Injection volume: 1 µL, Inlet: Split (5/1), Injection temperature: 230 °C, Column flow: 35 cm/s Helium, Column temperature program: 40 °C (2 min)-10 °C/min-240 °C (20 min) total 42 min, Detector: FDI at 250 °C. The content of the respective solvents may be calculated by using a calibration curve. Instead of direct injection as laid out above, headspace injection may also be used, e.g., with sample thermostatting at 160 °C for 30 min, with a head space pressure of 70.0 kPa, with a sample and transfer line temperature of 165 °C, and with an injection volume of 1.0 mL.

It will be understood that one or more amide solvents S^{A} and/or one or more ether solvents S^{E} may be added to the reaction mixtures or organic phases at any time throughout the method of the invention.

In some embodiments of the method of the invention, steps (b-1) to (h-1) (i.e. any one of steps (b-1), (c-1), (d-1), (e-1), (f-1), (g-1), and (h-1) as far as present) are carried out in essentially halogen-free solvents. It will be understood that this does not imply that any optional step must be carried out.

In some embodiments of the method of the invention, if the second coupling cycle comprising steps (b-2) to (h-2) (as far as present) is performed, steps (b-1) to (h-2) (i.e. any one of steps (b-1), (c-1), (d-1), (e-1), (f-1), (g-1), (h-1), (b-2), (c-2), (d-2), (e-2), (f-2), (g-2), and (h-2) as far as present) are carried out in essentially halogen-free solvents. It will be understood that this does not imply that any optional step must be carried out.

In some embodiments of the method of the invention, if (n-2) iterations of the coupling cycle comprising steps (b-x) to (h-x) (as far as present) are performed, all of steps (b-1) to (h-n) (i.e. any one of steps (b-1), (c-1), (d-1), (e-1), (f-1), (g-1), (h-1), (b-2), (c-2), (d-2), (e-2), (f-2), (g-2), and (h-2), as far as present, as well as each iteration of any one of steps (b-x), (c-x), (d-x), (e-x), (f-x), (g-x), and (h-x)) are carried out in essentially halogen-free solvents. It will be understood that this does not imply that any optional step must be carried out.

In some embodiments of the method of the invention, steps (b-1) to (h-1) (as far as present) are carried out in essentially halogen-free solvents, and further wherein:
- if a second coupling cycle comprising steps (b-2) to (h-2) (as far as present) is performed, steps (b-1) to (h-2) (as far as present) are carried out in essentially halogen-free solvents; and
- if (n-2) iterations of the coupling cycle comprising steps (b-x) to (h-x) (as far as present) are performed, any steps (b-1) to (h-n) (as far as present) are carried out in essentially halogen-free solvents.

As used herein, the term "essentially halogen-free solvent" refers to a solvent which contains in total equal to or less than 3.0 vol-%, 2.0 vol-%, 1.0 vol-%, 0.1 vol-%, 0.01 vol-%, or 0.001 vol-% of halogenated solvents. As used, the term "halogenated solvent" refers to any solvent comprising in its chemical structure at least one halogen atom. Examples of halogenated solvents comprise dichloromethane, chloroform, and 1,1-dichloroethane, and 1,2-dichloroethane. To state that a step "is carried out in essentially halogen-free solvents" means that only essentially halogen-free solvents are used in the respective step.

In some embodiments of the method of the invention:
- said component C-0 comprises exactly one pseudo solid-phase protecting group, which is the pseudo solid-phase protecting group PG-s; and
- the building block B-1 does not comprise any pseudo solid-phase protecting groups.

In some embodiments of the method of the invention:
- said component C-0 comprises exactly one pseudo solid-phase protecting group, which is the pseudo solid-phase protecting group PG-s; and
- none of the building block B-1 and B-2 comprise any pseudo solid-phase protecting groups.

In some embodiments of the method of the invention:
- said component C-0 comprises exactly one pseudo solid-phase protecting group, which is the pseudo solid-phase protecting group PG-s; and
- none of the building block B-1, B-2, and B-x comprise any pseudo solid-phase protecting groups.

In some embodiments of the method of the invention, the sum of nucleoside subunits comprised in the component C-0 and all building blocks B-1, B-2, and B-x together is in the range of 5-100, 5-90, 5-80, 5-70, 5-60, 5-50, 5-40, 5-30, 5-20, 6-20 or 6-18. It will be understood that in the component C-0 of any one of Formulae I, I-a, I-b, the amount of nucleoside subunits is determined as 1 + m, where m is the integer m. It will be understood that in each building block B-1, B-2, and B-x of any one of Formulae II, II-a, II-b, II-1, II-1-a, II-1-b, II-2, II-2-a, and II-2-b, the amount of nucleoside subunits is determined as 1 + q, where q is the integer q.

In some embodiments of the method of the invention:
- said component C-0 comprises exactly one pseudo solid-phase protecting group, which is the pseudo solid-phase protecting group PG-s;
- none of the building blocks B-1, B-2, and B-x comprises any pseudo solid-phase protecting groups; and
- the sum of nucleoside subunits comprised in the component C-0 and all building blocks B-1, B-2, and B-x together is in the range of 5-100, 5-90, 5-80, 5-70, 5-60, 5-50, 5-40, 5-30, 5-20, 6-20 or 6-18.

In some embodiments of the method of the invention;
- the first cycle oligonucleotide O-1 comprises exactly one pseudo solid-phase protecting group, which is the pseudo solid-phase protecting group PG-s, and
- the first cycle oligonucleotide comprises in total 5-100, 5-90, 5-80, 5-70, 5-60, 5-50, 5-40, 5-30, 5-20, 6-20 or 6-18 nucleoside subunits.

In some embodiments of the method of the invention;
- the second cycle oligonucleotide O-2 comprises exactly one pseudo solid-phase protecting group, which is the pseudo solid-phase protecting group PG-s, and
- the second cycle oligonucleotide O-2 comprises in total 5-100, 5-90, 5-80, 5-70, 5-60, 5-50, 5-40, 5-30, 5-20, 6-20 or 6-18 nucleoside subunits.

In some embodiments of the method of the invention,
- the n-th cycle oligonucleotide O-n comprises exactly one pseudo solid-phase protecting group, which is the pseudo solid-phase protecting group PG-s, and
- the n-th cycle oligonucleotide O-n comprises in total 5-100, 5-90, 5-80, 5-70, 5-60, 5-50, 5-40, 5-30, 5-20, 6-20 or 6-18 nucleoside subunits.

Another aspect of the present invention pertains to a composition comprising
- an oligonucleotide which is covalently bonded to at least one pseudo solid-phase protecting group, and
- a mixed solvent which is essentially halogen-free and comprises one or more amide solvents S^{A} and, preferably, one or more ether solvents S^{E}, wherein each amide solvent S^{A} is an amide solvent comprising one or more alkyl groups, wherein these one or more alkyl groups together comprise in total 6-48 carbon atoms or 8-48 carbon atoms.

Another aspect of the present invention pertains to a composition comprising
- an oligonucleotide which is covalently bonded to at least one pseudo solid-phase protecting group, and
- a mixed solvent which is essentially halogen-free and comprises one or more amide solvents S^{A} and, preferably, one or more ether solvents S^{E}, wherein each amide solvent S^{A} is an amide solvent comprising one or more alkyl groups, wherein these one or more alkyl groups together comprise in total 6-24 carbon atoms.

Another aspect of the present invention pertains to a composition comprising
- an oligonucleotide which is covalently bonded to at least one pseudo solid-phase protecting group, and
- a mixed solvent which is essentially halogen-free and comprises one or more amide solvents S^{A} and, preferably, one or more ether solvents S^{E}, wherein each amide solvent S^{A} is an amide solvent comprising one or more alkyl groups, wherein these one or more alkyl groups together comprise in total 6-16 or 6-15 carbon atoms.

In some embodiments of the composition of the invention, said oligonucleotide is covalently bonded to exactly one pseudo solid-phase protecting group and comprises in total 5-100, 5-90, 5-80, 5-70, 5-60, 5-50, 5-40, 5-30, 5-20, 6-20 or 6-18 or 5-18 nucleoside subunits. In some preferred embodiments of the composition of the invention, said oligonucleotide is covalently bonded to exactly one pseudo solid-phase protecting group and comprises equal to or more than 5 nucleoside subunits, in particular 5-18 nucleoside subunits. In some embodiments of the composition of the invention, said oligonucleotide, which is covalently bonded to a pseudo solid-phase protecting group, has a structure of any one of the above-mentioned Formulae I, I-a, and I-b. In some embodiments of the composition of the invention, said oligonucleotide, which is covalently bonded to a pseudo solid-phase protecting group, is the component C-0 as defined above. In such embodiments, any definitions and embodiments pertaining to the component C-0 in the context of the method of the invention may likewise apply to the component C-0 in the context of the composition of the invention. In some embodiments of the composition of the invention, said oligonucleotide, which is covalently bonded to a pseudo solid-phase protecting group, is the component (C-0)^{#} as defined above. In such embodiments, any definitions and embodiments pertaining to the component (C-0)^{#} in the context of the method of the invention may likewise apply to the component (C-0)^{#} in the context of the composition of the invention.

The term "essentially halogen-free" has been defined in the context of the method of the invention. This definition likewise applies to the composition of the invention, in particular to said mixed solvent. In some embodiments of the composition of the invention, said mixed solvent comprises 10-90 vol-% of one or more amide solvents S^{A} and 10-90 vol-% of one or more ether solvents S^{E}. In some embodiments of the composition of the invention, said mixed solvent comprises 20-80 vol-% of one or more amide solvents S^{A} and 20-80 vol-% of one or more ether solvents S^{E}. In some embodiments of the composition of the invention, said mixed solvent comprises 30-70 vol-% of one or more amide solvents S^{A} and 30-70 vol-% of one or more ether solvents S^{E}. In some embodiments of the composition of the invention, said mixed solvent comprises 10-50 vol-% of one or more amide solvents S^{A} and 50-90 vol-% of one or more ether solvents S^{E}. In some embodiments of the composition of the invention, said mixed solvent comprises 20-50 vol-% of one or more amide solvents S^{A} and 50-80 vol-% of one or more ether solvents S^{E}. The composition of said mixed solvent may, for example, be analyzed by means of gas chromatography. For example, the following setup may be used: Column: SH-Rxi-624Sil MS (30 m × 0.32 mmID, 1.8 µm), Injection volume: 1 µL, Inlet: Split (5/1), Injection temperature: 230 °C, Column flow: 35 cm/s Helium, Column temperature program: 40 °C (2 min)-10 °C/min-240 °C (20 min) total 42 min, Detector: FDI at 250 °C. The content of the respective solvents may be calculated by using a calibration curve.

In some embodiments of the method and the composition of the invention, each amide solvent S^{A} is an amide solvent comprising one or more alkyl groups, wherein these one or more alkyl groups together comprise in total 6-48, 8-48, 6-47, 8-47, 6-46, 8-46, 6-45, 8-45, 6-44, 8-44, 6-43, 8-43, 6-42, 8-42, 6-41, 8-41, 6-40, 8-40, 6-39, 8-39, 6-38, 8-38, 6-37, 8-37, 6-36, 8-36, 6-35, 8-35, 6-34, 8-34, 6-33, 8-33, 6-32, 8-32, 6-31, 8-31, 6-30, 8-30, 6-29, 8-29, 6-28, 8-28, 6-27, 8-27, 6-26, 8-26, 6-25, 8-25, 6-24, 8-24, 6-23, 8-23, 6-22, 8-22, 6-21, 8-21, 6-20, 8-20, 6-19, 8-19, 6-18, 8-18, 6-17, 8-17, 6-16, 8-16, 6-15, 8-15, 6-14, 8-14, 6-13, 8-13, 6-12, or 8-12, 6-48 carbon atoms.

In some embodiments of the method and the composition of the invention, each amide solvent S^{A} is at each occurrence selected independently from the group consisting of the following Formulae S^{A}-1, S^{A}-2, and S^{A}-3: 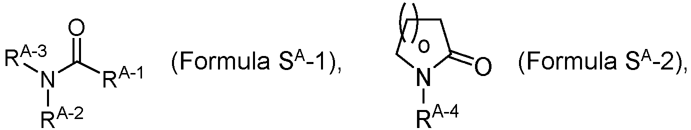 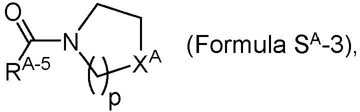 wherein in Formula S^{A}-1:
R^{A-1} is selected from the group consisting of H and a C₁-C₂₄-alkyl group, in which exactly one hydrogen residue may optionally be substituted by a C(O)O(C₁-C₅-alkyl) group; and
each of R^{A-2} and R^{A-3} is independently a C₁-C₂₄-alkyl group;
with the proviso that R^{A-1}, R^{A-2} and R^{A-3} together comprise in total 6-48 carbon atoms;
wherein in Formula S^{A}-2:
   o is an integer of 1 or 2; and
   R^{A-4} is a C₆-C₂₄-alkyl group; and
   wherein in Formula S^{A}-3:
      p is an integer of 1 or 2;
      X^{A} is selected from the group consisting of CH₂, O, and NC(O)R^{A-6}, with the proviso that, if p is 1, X^{A} is CH₂;
      R^{A-5} is a C₁-C₂₄-alkyl group; and
      R^{A-6} is a C₁-C₂₄-alkyl group;
      with the proviso that R^{A-5} and R^{A-6} together comprise in total 6-48 carbon atoms.

Herein, when denoting residues comprising heteroatoms, brackets are occasionally used for illustrative purposes, as, e.g., exemplified in the following residue: C(O)O(C₁C₅-alkyl). Such brackets are not to be construed to indicate that the group in brackets is only optionally comprised in the respective residue. If an oxygen atom of a chemical residue is depicted in brackets, this indicates that said oxygen atom is covalently bonded only to the carbon atom depicted to the left in the chemical formula of the respective residue. If a hydrocarbon group such as, e.g., a C₁-C₅-alkyl group forms part of a residue comprising further atoms, said hydrocarbon group may be presented in brackets to highlight the fact that it is a separate group within said residue. This will easily be understood based on the common general knowledge. For example, a C(O)O(C₁-C₅-alkyl) residue is an ester residue, bonded via the carboxyl carbon atom to the respective parent structure, wherein the C₁-C₅-alkyl moiety is bonded to the oxygen atom not depicted in brackets. As another example, a O(C₁-C₄₀-alkyl) residue denotes an alkoxy residue in which the alkyl moiety comprises at least one and not more than 40 carbon atoms. As another example, a C(O)(C₁-C₄₀-alkyl) residue denotes an alkanoyl residue bonded to the respective parent structure via a carbonyl carbon atom, wherein the C₁-C₄₀-alkyl moiety is bonded directly to the carbonyl carbon atom and not to the oxygen atom depicted in brackets.

In some embodiments of the method and the composition of the invention, each amide solvent S^{A} is independently of each other a solvent of any one of the aforementioned Formulae S^{A}-1, S^{A}-2, and S^{A}-3,
wherein in Formula S^{A}-1:
R^{A-1} is selected from the group consisting of H and a C₁-C₂₄-alkyl group, in which exactly one hydrogen atom may optionally be substituted by a C(O)O(C₁-C-alkyl) group; and
each of R^{A-2} and R^{A-3} is independently a C₁-C₂₄-alkyl group,
with the proviso that R^{A-1}, R^{A-2} and R^{A-3} together comprise in total 8-48 carbon atoms;
wherein in formula A-2:
   o is an integer of 1 or 2; and
   R^{A-4} is a C₈-C₂₄-alkyl group; and
   wherein in formula A-3:
      p is an integer of 1 or 2;
      X^{A} is selected from the group consisting of CH₂ (i.e. a methylene group), O, and
      NC(O)R^{A-6}, with the proviso that, if p is 1, X^{A} is CH₂;
      R^{A-5} is a C₁-C₂₄-alkyl group; and
      R^{A-6} is a C₁-C₂₄-alkyl group;
      with the proviso that R^{A-5} and R^{A-6} together comprise in total 8-48 carbon atoms.

In some embodiments of the method and the composition of the invention, each amide solvent S^{A} is independently of each other a solvent of any one of the aforementioned Formulae S^{A}-1, S^{A}-2, and S^{A}-3,
wherein in Formula S^{A}-1:
R^{A-1} is selected from the group consisting of H and a C₁-C₂₄-alkyl group, in which exactly one hydrogen residue may optionally be substituted by a C(O)O(C₁-C₅-alkyl) group; and
each of R^{A-2} and R^{A-3} is independently a C₁-C₂₄-alkyl group,
with the proviso that R^{A-1}, R^{A-2} and R^{A-3} together comprise in total 6-36 carbon atoms;
wherein in formula A-2:
   o is an integer of 1 or 2; and
   R^{A-4} is a C₆-C₂₄-alkyl group; and
   wherein in formula A-3:
      p is an integer of 1 or 2;
      X^{A} is selected from the group consisting of CH₂ (i.e. a methylene group), O, and
      NC(O)R^{A-6}, with the proviso that, if p is 1, X^{A} is CH₂;
      R^{A-5} is a C₁-C₂₄-alkyl group; and
      R^{A-6} is a C₁-C₂₄-alkyl group;
      with the proviso that R^{A-5} and R^{A-6} together comprise in total 6-36 carbon atoms.

In some embodiments of the method and the composition of the invention, each amide solvent S^{A} is independently of each other a solvent of any one of the aforementioned Formulae S^{A}-1, S^{A}-2, and S^{A}-3,
wherein in Formula S^{A}-1:
R^{A-1} is selected from the group consisting of H and a C₁-C₂₂-alkyl group, in which exactly one hydrogen residue may optionally be substituted by a C(O)O(C₁-C₅-alkyl) group; and
each of R^{A-2} and R^{A-3} is independently a C₁-C₂₃-alkyl group,
with the proviso that R^{A-1}, R^{A-2} and R^{A-3} together comprise in total 6-24 carbon atoms;
wherein in formula A-2:
   o is an integer of 1 or 2; and
   R^{A-4} is a C₆-C₂₄-alkyl group; and
   wherein in formula A-3:
      p is an integer of 1 or 2;
      X^{A} is selected from the group consisting of CH₂ (i.e. a methylene group), O, and
      NC(O)R^{A-6}, with the proviso that, if p is 1, X^{A} is CH₂;
      R^{A-5} is a C₁-C₂₄-alkyl group; and
      R^{A-6} is a C₁-C₂₃-alkyl group;
      with the proviso that R^{A-5} and R^{A-6} together comprise in total 6-24 carbon atoms.

In some embodiments of the method and the composition of the invention, each amide solvent S^{A} is independently of each other a solvent of any one of the aforementioned Formulae S^{A}-1, S^{A}-2, and S^{A}-3,
wherein in Formula S^{A}-1:
R^{A-1} is selected from the group consisting of H and a C₁-C₁₄-alkyl group, in which exactly one hydrogen residue may optionally be substituted by a C(O)O(C₁-C₅-alkyl) group; and
each of R^{A-2} and R^{A-3} is independently a C₁-C₁₅-alkyl group,
with the proviso that R^{A-1}, R^{A-2} and R^{A-3} together comprise in total 6-16 carbon atoms;
wherein in formula A-2:
   o is an integer of 1 or 2; and
   R^{A-4} is a C₆-C₁₆-alkyl group; and
   wherein in formula A-3:
      p is an integer of 1 or 2;
      X^{A} is selected from the group consisting of CH₂ (i.e. a methylene group), O, and
      NC(O)R^{A-6}, with the proviso that, if p is 1, X^{A} is CH₂;
      R^{A-5} is a C₁-C₁₆-alkyl group; and
      R^{A-6} is a C₁-C₁₅-alkyl group;
      with the proviso that R^{A-5} and R^{A-6} together comprise in total 6-16 carbon atoms.

In some embodiments of the method and the composition of the invention, each amide solvent S^{A} is independently of each other a solvent of any one of the aforementioned Formulae S^{A}-1, S^{A}-2, and S^{A}-3,
wherein in Formula S^{A}-1:
R^{A-1} is selected from the group consisting of H and a C₁-C₁₃-alkyl group, in which exactly one hydrogen residue may optionally be substituted by a C(O)O(C₁-C₅-alkyl) group; and
each of R^{A-2} and R^{A-3} is independently a C₁-C₁₄-alkyl group,
with the proviso that R^{A-1}, R^{A-2} and R^{A-3} together comprise in total 6-15 carbon atoms;
wherein in formula A-2:
   o is an integer of 1 or 2; and
   R^{A-4} is a C₆-C₁₅-alkyl group; and
   wherein in formula A-3:
      p is an integer of 1 or 2;
      X^{A} is selected from the group consisting of CH₂ (i.e. a methylene group), O, and
      NC(O)R^{A-6}, with the proviso that, if p is 1, X^{A} is CH₂;
      R^{A-5} is a C₁-C₁₅-alkyl group; and
      R^{A-6} is a C₁-C₁₄-alkyl group;
      with the proviso that R^{A-5} and R^{A-6} together comprise in total 6-15 carbon atoms.

In some embodiments of the method and the composition of the invention, each amide solvent S^{A} is independently of each other a solvent of any one of the aforementioned Formulae S^{A}-1, S^{A}-2, and S^{A}-3,
wherein in Formula S^{A}-1:
R^{A-1} is selected from the group consisting of H and a C₁-C₂₄-alkyl group, and
each of R^{A-2} and R^{A-3} is independently a C₁-C₂₄-alkyl group;
with the proviso that R^{A-1}, R^{A-2} and R^{A-3} together comprise in total 8-48 carbon atoms;
wherein in formula A-2:
   o is an integer of 1 or 2; and
   R^{A-4} is a C₈-C₂₄-alkyl group; and
   wherein in formula A-3:
      p is an integer of 1 or 2;
      X^{A} is selected from the group consisting of CH₂ (i.e. a methylene group) and O, with the proviso that, if p is 1, X^{A} is CH₂; and
      R^{A-5} is a C₈-C₂₄-alkyl group.

In some embodiments of the method and the composition of the invention, each amide solvent S^{A} is independently of each other a solvent of any one of the aforementioned Formulae S^{A}-1, S^{A}-2, and S^{A}-3,
wherein in Formula S^{A}-1:
R^{A-1} is H; and
each of R^{A-2} and R^{A-3} is independently a C₁-C₂₄-alkyl group;
with the proviso that R^{A-1}, R^{A-2} and R^{A-3} together comprise in total 8-48 carbon atoms;
wherein in formula A-2:
   o is an integer of 1 or 2; and
   R^{A-4} is a C₈-C₂₄-alkyl group; and
   wherein in formula A-3:
      p is an integer of 1 or 2;
      X^{A} is selected from the group consisting of CH₂ (i.e. a methylene group) and O, with the proviso that, if p is 1, X^{A} is CH₂; and
      R^{A-5} is a C₈-C₂₄-alkyl group.

In some embodiments of the method and the composition of the invention, each amide solvent S^{A} is independently of each other a solvent of any one of the aforementioned Formulae S^{A}-1, S^{A}-2, and S^{A}-3,
wherein in Formula S^{A}-1:
R^{A-1} is H; and
each of R^{A-2} and R^{A-3} is independently a C₄-C₁₂-alkyl group;
wherein in formula A-2:
   o is an integer of 1 or 2; and
   R^{A-4} is a C₈-C₁₂-alkyl group; and
   wherein in formula A-3:
      p is an integer of 1 or 2;
      X^{A} is selected from the group consisting of CH₂ (i.e. a methylene group) and O, with the proviso that, if p is 1, X^{A} is CH₂; and
      R^{A-5} is a C₈-C₁₂-alkyl group.

In some embodiments of the method and the composition of the invention, each amide solvent S^{A} is independently of each other a solvent of any one of the aforementioned Formulae S^{A}-1, S^{A}-2, and S^{A}-3,
wherein in Formula S^{A}-1:
R^{A-1} is H; and
each of R^{A-2} and R^{A-3} is independently a C₄-C₁₂-alkyl group;
wherein in formula A-2:
   o is an integer of 1 or 2; and
   R^{A-4} is a C₈-C₁₂-alkyl group; and
   wherein in formula A-3:
      p is an integer of 1 or 2;
      X^{A} is CH₂; and
      R^{A-5} is a C₈-C₁₂-alkyl group.

In some embodiments of the method and the composition of the invention, each amide solvent S^{A} is independently of each other a solvent of any one of the aforementioned Formulae S^{A}-1, S^{A}-2, and S^{A}-3,
wherein in Formula S^{A}-1:
R^{A-1} is selected from the group consisting of H and a C₁-C₁₂-alkyl group; and
each of R^{A-2} and R^{A-3} is independently a C₁-C₁₂-alkyl group,
with the proviso that R^{A-1}, R^{A-2} and R^{A-3} together comprise in total 6-16 carbon atoms;
wherein in formula A-2:
   o is an integer of 1 or 2; and
   R^{A-4} is a C₆-C₁₆-alkyl group; and
   wherein in formula A-3:
      p is an integer of 1 or 2;
      X^{A} is CH₂; and
      R^{A-5} is a C₆-C₁₆-alkyl group

In some embodiments of the method and the composition of the invention, each amide solvent S^{A} is independently of each other a solvent of any one of the aforementioned Formulae S^{A}-1, and S^{A}-2,
wherein in Formula S^{A}-1:
R^{A-1} is selected from the group consisting of H and a C₁-C₁₂-alkyl group; and
each of R^{A-2} and R^{A-3} is independently a C₁-C₁₂-alkyl group,
with the proviso that R^{A-1}, R^{A-2} and R^{A-3} together comprise in total 6-16 carbon atoms;
wherein in formula A-2:
   o is an integer of 1 or 2; and
   R^{A-4} is a C₆-C₁₆-alkyl group.

In some embodiments of the method and the composition of the invention, each amide solvent S^{A} is independently of each other a solvent of any one of the aforementioned Formulae S^{A}-1, and S^{A}-2. In some embodiments of the method and the composition of the invention, each amide solvent S^{A} is independently of each other a solvent of the aforementioned Formulae S^{A}-1. In some embodiments of the method and the composition of the invention, each amide solvent S^{A} is independently of each other a solvent of the aforementioned Formulae S^{A}-2.

The skilled artisan is capable of identifying and obtaining amide solvents S^{A} fulfilling the aforementioned structural requirements and such amide solvents may also be commercially available. Examples of amide solvents S^{A} of Formula S^{A}-1 comprise *N*,*N*-dibutylformamide (DBF), *N*,*N*-dibutylacetamide, methyl 5-(dimethylamino)-2-methyl-5-oxopentanoate (CAS-RN 1174627-68-9), *N,N*-dimethyloctanamide, *N*,*N*-dimethyldecanamide, *N*,*N*-diethyldodecanamide, and *N,N*-bis(1-methylpropyl)acetamide. Examples of amide solvents S^{A} of Formula S^{A}-2 comprise *N*-octyl-2-pyrrolidone (NOP), *N*-octyl-2-piperidone, and 1-cyclohexyl-2-pyrrolidone (CAS RN: 6837-24-7). Examples of amide solvents S^{A} of Formula S^{A}-3 comprise *N*-nonanoylpyrrolidine (CAS-RN 20308-70-7), *N*-nonanoylpiperidine (CAS-RN 20368-13-2), *N*-nonanoylmorpholine (CAS-RN 5299-64-9), and 1,4-dipentanoylpiperazine (CAS RN 18903-08-7). In some embodiments of the method and the composition of the invention, each amide solvent S^{A} independently of each other is selected from the group consisting of *N*,*N*-dibutylformamide (DBF) and *N*-octyl-2-pyrrolidone (NOP). In some embodiments of the method and the composition of the invention, each amide solvent S^{A} is independently of each other selected from the group consisting of, *N-*octyl-2-pyrrolidone (NOP), *N,N-*dibutylformamide (DBF), *N,N*-diethyldodecanamide, and 1-cyclohexyl-2-pyrrolidone.

In some embodiments of the method and the composition of the invention, each ether solvent S^{E} is at each occurrence selected independently from the group consisting of the following Formulae S^{E}-1 and S^{E}-2: 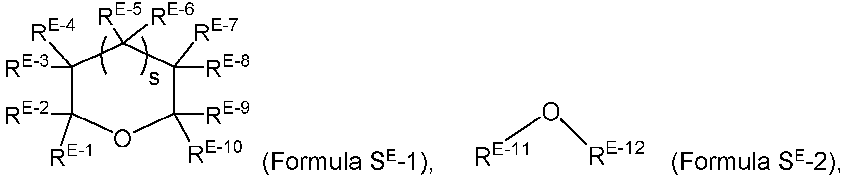 wherein in Formula S^{E}-1:
s is an integer of 0 or 1, and
each of R^{E-1}, R^{E-2}, R^{E-3}, R^{E-4}, R^{E-5}, R^{E-6}, R^{E-7}, R^{E-8} , R^{E-9}, and R^{E-10} is independently selected from the group consisting of H and a C₁-C₅-alkyl group, with the proviso that at least one of R^{E-1}, R^{E-2}, R^{E-3}, R^{E-4}, R^{E-5}, R^{E-6}, R^{E-7}, R^{E-8}, R^{E-9}, and R^{E-10} is a C₁-C₅-alkyl group; and
wherein in Formula S^{E}-2:
   each of R^{E-11} and R^{E-12} is independently a C₁-C₆-alkyl group, with the proviso that R^{E-11} and R^{E-12} together comprise in total 5-12 carbon atoms (i.e. not less than 5 and not more than 12 carbon atoms).

In some embodiments of the method and the composition of the invention, each ether solvent S^{E} is independently of each other a solvent of any one of the aforementioned Formulae S^{E}-1 and S^{E}-2,
wherein in Formula S^{E}-1:
s is an integer of 0 or 1, and
each of R^{E-1}, R^{E-2}, R^{E-3}, R^{E-4}, R^{E-5}, R^{E-6}, R^{E-7}, R^{E-8}, R^{E-9}, and R^{E-10} is independently selected from the group consisting of H and a C₁-C₅-alkyl group, with the proviso that exactly one, exactly two or exactly three of R^{E-1}, R^{E-2}, R^{E-3}, R^{E-4}, R^{E-5}, R^{E-6}, R^{E-7}, R^{E-8}, R^{E-9}, and R^{E-10} are a C₁-C₅-alkyl group; and
wherein in Formula S^{E}-2:
   each of R^{E-11} and R^{E-12} is independently a C₁-C₆-alkyl group, with the proviso that R^{E-11} and R^{E-12} together comprise in total 6-12 carbon atoms.

In some embodiments of the method and the composition of the invention, each ether solvent S^{E} is independently of each other a solvent of any one of the aforementioned Formulae S^{E}-1 and S^{E}-2,
wherein in Formula S^{E}-1:
s is an integer of 0 or 1, and
each of R^{E-1}, R^{E-2}, R^{E-3}, R^{E-4}, R^{E-5}, R^{E-6}, R^{E-7}, R^{E-8}, R^{E-9}, and R^{E-10} is independently selected from the group consisting of H and CH₃ (i.e. a methyl group, Me), with the proviso that exactly one or exactly two of R^{E-1}, R^{E-2}, R^{E-3}, R^{E-4}, R^{E-S}, R^{E-6}, R^{E-7}, R^{E-8}, R^{E-9}, and R^{E-10} are CH₃; and
wherein in Formula S^{E}-2:
   each of R^{E-11} and R^{E-12} is independently a C₁-C₆-alkyl group, with the proviso that R^{E-11} and R^{E-12} together comprise in total 6-12 carbon atoms.

In some embodiments of the method and the composition of the invention, each ether solvent S^{E} is independently of each other a solvent of any one of the aforementioned Formulae S^{E}-1 and S^{E}-2,
wherein in Formula S^{E}-1:
s is an integer of 0 or 1, and
each of R^{E-1}, R^{E-2}, R^{E-3}, R^{E-4}, R^{E-8}, R^{E-6}, R^{E-7}, R^{E-8}, R^{E-9}, and R^{E-10} is independently selected from the group consisting of H and CH₃ (i.e. a methyl group, Me), with the proviso that exactly one of R^{E-1}, R^{E-2}, R^{E-3}, R^{E-4}, R^{E-5}, R^{E-6}, R^{E-7}, R^{E-8}, R^{E-9}, and R^{E-10} is CH₃; and
wherein in Formula S^{E}-2:
   each of R^{E-11} and R^{E-12} is independently a C₁-C₆-alkyl group, with the proviso that R^{E-11} and R^{E-12} together comprise in total 6-12 carbon atoms.

In some embodiments of the method and the composition of the invention, each ether solvent S^{E} is independently of each other a solvent of the aforementioned Formula S^{E}-1,
wherein in Formula S^{E}-1:
s is an integer of 0 or 1, and
each of R^{E-1}, R^{E-2}, R^{E-3}, R^{E-4}, R^{E-8}, R^{E-6}, R^{E-7}, R^{E-8}, R^{E-9}, and R^{E-10} is independently selected from the group consisting of H and CH₃ (i.e. a methyl group, Me), with the proviso that exactly one of R^{E-1}, R^{E-2}, R^{E-3}, R^{E-4}, R^{E-5}, R^{E-6}, R^{E-7}, R^{E-3}, R^{E-9}, and R^{E-10} is CH₃.

The skilled artisan is capable of identifying and obtaining ether solvents S^{E} fulfilling the aforementioned structural requirements and such ether solvents may also be commercially available. Examples of ether solvents S^{E} of Formula S^{E}-1 comprise 4-methyltetrahydropyran (MTHP), 3-methyltetrahydropyran, 2-methyltetrahydropyran, 2-methyltetrahydrofuran, 3-methyltetrahydrofuran, 2,5-dimethyltetrahydrofuran, 2,2-dimethyltetrahydrofuran, 2,2,5,5-tetramethyltetrahydrofuran, and the like. Examples of ether solvents S^{E} of Formula S^{E}-2 comprise cyclopentyl methyl ether (CPME), cyclohexyl methyl ether, dibutyl ether, isoamyl ether, methyl tert-butyl ether (MTBE), diisopropyl ether, and the like. In some embodiments of the method and the composition of the invention each ether solvent S^{E} is independently selected from the group consisting of 4-methyltetrahydropyran (MTHP) and cyclopentyl methyl ether (CPME). In some embodiments of the method and the composition of the invention each ether solvent S^{E} is 4-methyltetrahydropyran (MTHP).

In some embodiments of the method and the composition of the invention, each pseudo solid-phase protecting group, e.g. the pseudo solid-phase protecting group PG-s, is a protecting group of the following Formula P-1: 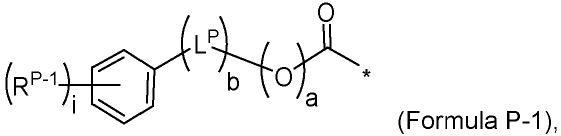 wherein in Formula P-1:
the asterisk indicates the point of attachment of the pseudo solid-phase protecting group to a hydroxyl or amine moiety of the respective nucleoside moiety (in other words: the asterisk represents the oxygen atom of the protected hydroxyl moiety or
the nitrogen atom of the protected amine moiety);
a is an integer of 0 or 1;
b is an integer of 0 or 1;
L^{P} is a linker moiety;
i is an integer of 1 to 5, preferably 1 to 3, in particular 2 to 3; and R^{P-1} is at each occurrence independently selected from the group consisting of O(C₁-C₄₀-alkyl), O(C₂-C₄₀-alkenyl), O(C₂-C₄₀-alkynyl), a C₁-C₄₀-alkyl group, a C₂-C₄₀-alkenyl group, a C₂-C₄₀-alkynyl group, C(O)(C₁-C₄₀-alkyl), C(O)(C₂-C₄₀-alkenyl), and C(O)(C₂-C₄₀-alkynyl);
wherein all present residues R^{P-1} together comprise in total 18-200 or 30-200 or 36-120 carbon atoms.

Unless indicated differently in the context of specific embodiments, a pseudo solid-phase protecting group of Formula P-1 may be bonded to any position of a nucleoside or oligonucleotide, with the proviso that said point of attachment indicated by the asterisk in Formula P-1 (i.e. the atom of the nucleoside or oligonucleotide to which said pseudo solid-phase protecting group is covalently bonded) must be the oxygen atom of a hydroxyl moiety or the nitrogen atom of an amine moiety. Preferred examples of hydroxyl moieties to which a pseudo solid-phase protecting group of Formula P-1 may be covalently bonded are hydroxyl moieties of the backbone of a nucleoside or oligonucleotide, e.g. any hydroxyl moieties available in the carbohydrate (preferably ribose or 2'-deoxyribose) moiety of a nucleoside moiety as well as any hydroxyl moieties present in an internucleosidic linkage group. Preferred examples of amine moieties to which a pseudo solid-phase protecting group of Formula P-1 may be covalently bonded are amine moieties of nucleobases, in particular the exocyclic amine moieties of adenine, guanine, cytosine, and 5-methylcytosine. The component C-0 of any one of Formulae I, I-a, and I-b comprises a pseudo solid-phase protecting group PG-s which is bonded to a hydroxyl moiety. If said protecting group PG-s in the component C-0 of any one of Formulae I, I-a, and I-b is a protecting group of the aforementioned Formula P-1, the asterisk of Formula P-1 indicates the oxygen atom in the respective Formula I, I-a, and I-b, to which the protecting group PG-s is bonded and the covalent bond between the protecting group PG-s and said oxygen atom in any one of Formulae I, I-a, and I-b is the covalent bond interconnecting the carbonyl carbon atom and the asterisk in Formula P-1 (in other words: only one and not two covalent chemical bonds interconnect the carbonyl carbon atom shown in Formula P-1 and the respective oxygen atom of the respective Formula I, I-a, and I-b).

It will be understood that, if the integer a in Formula P-1 is 0, the oxygen atom in brackets will be absent and the carbonyl carbon atom depicted to its right will be covalently bonded directly to the linker moiety L^{P}, if the integer b is 1, or to the phenyl moiety, if the integer b is 0. It will also be understood that if the integer b in Formula P-1 is 0, the linker moiety L^{P} will be absent and the phenyl moiety depicted to its left will be covalently bonded directly to the oxygen atom, if the integer a is 1, or to the carbonyl carbon atom, if the integer a is 0. This rationale is applicable to any atoms, atom groups or residues depicted in brackets having an integer which may be 0.

In some embodiments of the invention, the integer a in Formula P-1 is 0. In some embodiments of the invention, the integer a in Formula P-1 is 1. In some embodiments of the invention, the integer b in Formula P-1 is 1. In some embodiments of the invention, the integer a in Formula P-1 is 0. In some embodiments of the invention, the integer b in Formula P-1 is 0. In some embodiments of the invention, the integer a in Formula P-1 is 0 and the integer b in Formula P-1 is 1. In some embodiments of the invention, the integer a in Formula P-1 is 1 and the integer b in Formula P-1 is 1. In some embodiments of the invention, the integer a in Formula P-1 is 1 and the integer b in Formula P-1 is 0. In some embodiments of the invention, the integer a in Formula P-1 is 0 and the integer b in Formula P-1 is 0.

The linker moiety L^{P} in a pseudo solid-phase protecting group of Formula P-1 may be any chemical residue which interconnects the oxygen atom, if the integer a is 1, or the carbonyl carbon atom, if the integer a is 0, with the phenyl residue to which R^{P-1} is bonded. For example, said linker moiety may be a C₁-C₄₀-alkylene moiety, in which alkylene moiety one or more carbon atoms and/or one or more hydrogen residues may optionally be substituted for a heteroatom selected from the group consisting of O and N.

As used herein, the term "alkylene group (or moiety or residue)" may be understood in the broadest sense to be similar to an alkyl residue, with the sole difference, that an alkylene residue mandatorily comprises two or more, preferably exactly two, chemical bonds (for the avoidance of doubt: a double bond is not the same as two chemical bonds) to the parent structure comprising said alkylene group. In other words: An alkylene group may preferably be an alkanediyl residue. This will be understood by the skilled person and may be exemplified as follows: A methyl group (i.e. -CH₃) is a classical alkyl group, which bonds to the respective parent structure via exactly one covalent chemical bond (indicated via the hyphen). The corresponding alkylene group would be a methylene group (i.e. -CH₂-) and differs from the alkyl group in that is bonds to the parent structure via exactly two covalent chemical bonds (indicated by the hyphens; both bonds originate from the methylene carbon atom). Unless indicated differently in the context of specific embodiments, an alkylene group may be branched, unbranched (i.e. linear) or cyclic.

In some embodiments of the invention, the linker moiety L^{P} in Formula P-1 is a residue of the following Formula P-1-L: 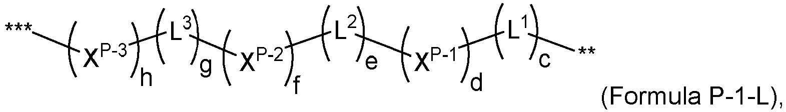 wherein in Formula P-1-L:
the two asterisks represent the oxygen atom to which L^{P} is bonded in Formula P-1, if the integer a is 1, or the carbonyl carbon atom to which L^{P} is bonded in Formula P-1, if the integer a is 0;
the three asterisks represent the carbon atom of the substituted phenyl moiety to which L^{P} is bonded in Formula P-1;
c is an integer of 0 or 1;
L¹ is a C₁-C₁₂-alkylene group;
d is an integer of 0 or 1;
X^{P-1} is selected from the group consisting of +C(O)++, +C(O)N(R^{P-B})++, +C(O)O++, and +C(O)S++, where R^{P-B} is selected from the group consisting of H and a C₁-C₆-alkyl group, and where + is the point of attachment to L¹ or ** and ++ is the point of attachment to L² or X^{P-2} or L³ or X^{P-3} or ***;
e is an integer of 0 or 1;
L² is a C₁-C₁₂-alkylene group;
f is an integer of 0 or 1;
X^{P-2} is selected from the group consisting of ++C(O)+, ++C(O)N(R^{P-C})+, ++C(O)O+, and ++C(O)S+, where R^{P-C} is selected from the group consisting of H and a C₁-C₆-alkyl group, and where + is the point of attachment to L² or X^{P-1} or L¹ or ** and ++ is the point of attachment to L³ or X^{P-3} or ***;
X^{P-1} L², and X^{P-2} may optionally together represent a structure of the following Formula P-1-L*: 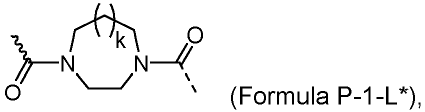 wherein in Formula P-1-L*:
   the dashed line represents the covalent bond to L¹ or **;
   the wavy line represents the covalent bond to L³ or X^{P-3} or ***; and
   k is an integer of 0 or 1 and preferably is 0;
g is an integer of 0 or 1;
L³ is a C₁-C₁₂-alkylene group;
h is an integer of 0 or 1;
X^{P-3} is selected from the group consisting of ++CH(R^{P-D})+, ++C(O)N(R^{P-E})+, and ++C(O)O+ where + denotes the point of attachment to L³ or X^{P-2} or L² or X^{P-1} or L¹ or ** and ++ denotes ***, and where R^{P-E} is selected from the group consisting of H and a C₁C₆-alkyl group, and where R^{P-D} is selected from the group consisting of H and a residue of the following Formula P-1-L**: 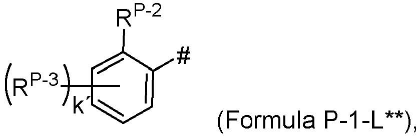 wherein in Formula P-1-L**:
   the hashtag (#) represents the carbon atom to which R^{P-D} is bonded in ++CH(R^{P-D})+,
   k is an integer of 0 to 4;
   R^{P-3} is defined as R^{P-1} (i.e. the same definitions apply to R^{P-3} of Formula P-1-L** and R^{P-1} of Formula P-1); and
   R^{P-2} is H or R^{P-2} and a residue R^{P-1} of Formula P-1 may optionally together represent a covalent chemical bond or O so as to form a fluoreny or xanthenyl moiety comprising the two phenyl residues of Formulae P-1 and P-1-L**;
   wherein:
   - all residues R^{P-1} and R^{P-3} present in a pseudo solid-phase protecting group of Formula P-1 with the linker moiety L^{P} being a liker moiety of Formula P-1-L together comprise in total 18-200 or 30-200 or 36-120 carbon atoms; and
   - if present, any residues L¹, L², and L³ together comprise in total 1-24, preferably 1-12 carbon atoms.

In some embodiments of the invention, in the linker moiety L^{P} of Formula P-1-L:
- the sum of integers c and e and g is 1 or 2 (i.e. c + e + g = 1 or 2);
- the sum of integers d and f and h is 0 or 1 or 2 (i.e. d + f + h = 0 or 1 or 2); and
- any residues L¹, L², and L³ together comprise in total 1-8 carbon atoms.

In some embodiments of the method and the composition of the invention, each pseudo solid-phase protecting group, e.g. the pseudo solid-phase protecting group PG-s, is a protecting group of any one of the following Formulae P-1-a, P-1-b, P-1-c, P-1-d, and P-1-e: 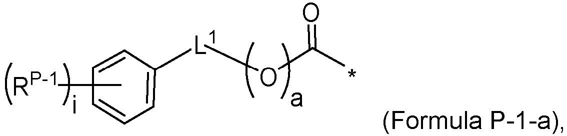 wherein in Formula P-1-a:
the integers a and i, the asterisk, and R^{P-1} are defined as for Formula P-1; and
L¹ is a C₁-C₁₂-alkylene group, preferably a C₁-C₃-alkylene group; 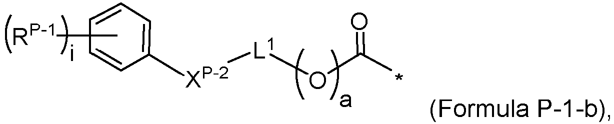
wherein in Formula P-1-b:
   the integers a and i, the asterisk, and R^{P-1} are defined as for Formula P-1;
   L¹ is a C₁-C₁₂-alkylene group, preferably a C₁-C₄-alkylene group, in particular a C₁-C₂-alkylene group; and
   X^{P-2} is selected from the group consisting of ++C(O)+, ++C(O)N(R^{P-C})+, ++C(O)O+, and ++C(O)S+, where R^{P-C} is selected from the group consisting of H and a C₁-C₆-alkyl group, and where + is the point of attachment to L¹ and ++ is the point of attachment to the phenyl moiety substituted with R^{P-1;} 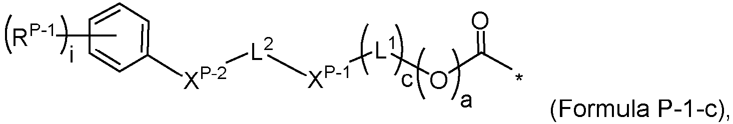 wherein in Formula P-1-c:
      the integers a and i, the asterisk, and R^{P-1} are defined as for Formula P-1;
      c is an integer of 0 or 1 and preferably is 1;
      L¹ is a C₁-C₁₂-alkylene group, preferably a C₁-C₄-alkylene group, in particular a C₂-alkylene group (i.e. an ethylene group, CH₂-CH₂);
      X^{P-1} is selected from the group consisting of +C(O)N(R^{P-B})++, +C(O)O++, and +C(O)S++, where R^{P-B} is selected from the group consisting of H and a C₁-C₆-alkyl group, and where + is the point of attachment to L¹ or O or the carbonyl carbon atom and ++ is the point of attachment to L²;
      L² is a C₁-C₁₂-alkylene group, preferably a C₁-C₄-alkylene group;
      X^{P-2} is selected from the group consisting of ++C(O)N(R^{P-C})+, ++C(O)O+, and ++C(O)S+, where R^{P-C} is selected from the group consisting of H and a C₁-C₆-alkyl group, and where + is the point of attachment to L² and ++ is the point of attachment to the phenyl moiety substituted with R^{P-1}; and
      X^{P} L², and X^{P-2} may optionally together represent the following structure 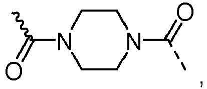 in which the dashed line represents the covalent bond to L¹ or O or the carbonyl carbon, and the wavy line represents the covalent bond to the phenyl moiety substituted with R^{P-1}; 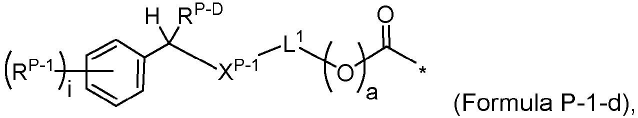
      wherein in Formula P-1-d:
         the integers a and i, the asterisk, and R^{P-1} are defined as for Formula P-1;
         L¹ is a C₁-C₁₂-alkylene group, preferably a C₁-C₄-alkylene group, in particular a C₂-alkylene group (i.e. an ethylene group, CH₂-CH₂);
         X^{P-1} is selected from the group consisting of +C(O)N(R^{P-B})++, +C(O)O++, and +C(0)S++, where R^{P-B} is selected from the group consisting of H and a C₁-C₆-alkyl group, and where + is the point of attachment to L¹ and ++ is represents the carbon atom to which R^{P-D} is bonded in CHR^{P-D}; and
         R^{P-D} is selected from the group consisting of H and a residue of the following Formula P-1-L**: 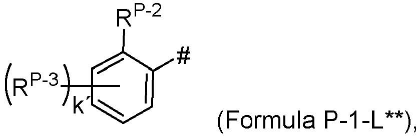 wherein in Formula P-1-L**:
            the hashtag (#) represents the carbon atom to which R^{P-D} is bonded in CHR^{P-D},
            k is an integer of 0 to 4;
            R^{P-3} is defined as R^{P-1} (i.e. the same definitions apply to R^{P-3} of Formula P-1-L** and R^{P-1} of Formula P-1); and
            R^{P-2} is H or R^{P-2} and a residue R^{P-1} may optionally together represent a covalent chemical bond or O so as to form a fluoreny or xanthenyl moiety comprising the phenyl residue to which R^{P-2} is bonded and the phenyl residue to which R^{P-1} is bonded; 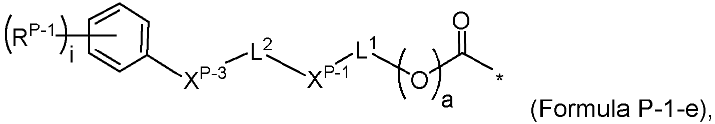
         wherein in Formula P-1-e:
            the integers a and i, the asterisk, and R^{P-1} are defined as for Formula P-1;
            L¹ is a C₁-C₁₂-alkylene group, preferably a C₁-C₄-alkylene group, in particular a C₂-alkylene group (i.e. an ethylene group, CH₂-CH₂);
            X^{P-1} is selected from the group consisting of +C(O)++, +C(O)N(R^{P-B})++, +C(O)O++, and +C(O)S++, where R^{P-B} is selected from the group consisting of H and a C₁-C₆-alkyl group, and where + is the point of attachment to L¹ and ++ is the point of attachment to L²;
            L² is a C₁-C₁₂-alkylene group, preferably a C₁-C₄-alkylene group, in particular a C₁-C₄-alkylene group; and
            X^{P-3} is selected from the group consisting of ++C(O)N(R^{P-E})+, and ++C(O)O+ where + denotes the point of attachment to L² and ++ is the point of attachment to the phenyl moiety substituted with R^{P-1}, and where R^{P-E} is selected from the group consisting of H and a C₁C₆-alkyl group; and
            wherein in any one of Formulae P-1-a, P-1-b, P-1-c and P-1-e, all present residues R^{P-1} together comprise in total 18-200 or 30-200 or 36-120 carbon atoms; and wherein in Formula P-1-d, all present residues R^{P-1} and R^{P-3} together comprise in total 18-200 or 30-200 or 36-120 carbon atoms.

In some embodiments of the method and the composition of the invention, each pseudo solid-phase protecting group, e.g. the pseudo solid-phase protecting group PG-s, is a protecting group of any one of the aforementioned Formulae P-1-a, P-1-b, P-1-c, P-1-d, and P-1-e,
wherein in Formula P-1-a:
the asterisk and R^{P-1} are defined as for Formula P-1;
a is an integer of 0 or 1;
i is an integer of 1 to 3, preferably 2 to 3; and
L¹ is a C₁-C₃-alkylene group (i.e. selected from the group consisting of CH₂, CH₂-CH₂, and CH₂-CH₂-CH₂);
wherein in Formula P-1-b:
   the asterisk and R^{P-1} are defined as for Formula P-1;
   the integer a is 0 (i.e. the oxygen atom is absent and L¹ bonds directly to the carbonyl carbon atom);
   i is an integer of 1 to 3, preferably 2 to 3;
   L¹ is a C₁-C₄-alkylene group, in particular a C₁-C₂-alkylene group; and
   X^{P-2} is selected from the group consisting of ++C(O)N(R^{P-C})+ and ++C(O)O+, where R^{P-C} is selected from the group consisting of H and a C₁-C₆-alkyl group, and where + is the point of attachment to L¹ and ++ is the point of attachment to the phenyl moiety substituted with R^{P-1} ;
   wherein in Formula P-1-c:
      the asterisk and R^{P-1} are defined as for Formula P-1;
      the integer a is 0;
      i is an integer of 1 to 3, preferably 2 to 3;
      c is an integer of 0 or 1 and preferably is 1;
      L¹ is a C₁-C₄-alkylene group, in particular a C₂-alkylene group (i.e. an ethylene group, CH₂-CH₂);
      X^{P-1} is selected from the group consisting of +C(O)N(R^{P-B})++ and +C(O)O++, where R^{P-B} is selected from the group consisting of H and a C₁-C₆-alkyl group, and where + is the point of attachment to L¹ or the carbonyl carbon atom and ++ is the point of attachment to L²;
      L² is a C₁-C₄-alkylene group;
      X^{P-2} is selected from the group consisting of ++C(O)N(R^{P-C})+ and ++C(O)O+, where R^{P-C} is selected from the group consisting of H and a C₁-C₆-alkyl group, and where + is the point of attachment to L² and ++ is the point of attachment to the phenyl moiety substituted with R^{P-1}; and
      X^{P-1}, L², and X^{P-2} may optionally together represent the following structure 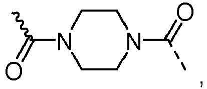 in which the dashed line represents the covalent bond to L¹ or the carbonyl carbon atom, and the wavy line represents the covalent bond to the phenyl moiety substituted with R^{P-1};
      wherein in Formula P-1-d:
         the asterisk and R^{P-1} are defined as for Formula P-1;
         the integer a is 0;
         i is an integer of 1 to 3, preferably 2 to 3;
         L¹ is a C₁-C₄-alkylene group, in particular a C₂-alkylene group;
         X^{P-1} is selected from the group consisting of +C(O)N(R^{P-B})++ and +C(O)O++, where R^{P-B} is selected from the group consisting of H and a C₁-C₆-alkyl group, and where + is the point of attachment to L¹ or the carbonyl carbon atom and ++ represents the carbon atom to which R^{P-D} is bonded in CHR^{P-D}; and
         R^{P-D} is selected from the group consisting of H and a residue of the aforementioned Formula P-1-L**,
            wherein in Formula P-1-L**:
            the hashtag (#) represents the carbon atom to which R^{P-D} is bonded in CHR^{P-D},
            k is an integer of 0 to 3;
            R^{P-3} is defined as R^{P-1} (i.e. the same definitions apply to R^{P-3} of Formula P-1-L** and R^{P-1} of Formula P-1); and
            R^{P-2} is H; and
         wherein in Formula P-1-e:
            the integer a is 0;
            i is an integer of 1 to 3, preferably 2 to 3;
            L¹ is a C₁-C₄-alkylene group, in particular a C₂-alkylene group;
            X^{P-1} is selected from the group consisting of +C(O)++, +C(O)N(R^{P-B})++, and +C(O)O++, where R^{P-B} is selected from the group consisting of H and a C₁-C₆-alkyl group, and where + is the point of attachment to L¹ and ++ is the point of attachment to L²;
            L² is a C₁-C₄-alkylene group, in particular a C₁-C₂-alkylene group; and
            X^{P-3} is selected from the group consisting of ++C(O)N(R^{P-E})+, and ++C(O)O+, where R^{P-E} is selected from the group consisting of H and a C₁-C₆-alkyl group, and where + denotes the point of attachment to L² and ++ is the point of attachment to the phenyl moiety substituted with R^{P-1}; and
            wherein in any one of Formulae P-1-a, P-1-b, P-1-c, and P-1-e, all present residues R^{P-1} together comprise in total 18-200 or 30-200 or 36-120 carbon atoms; and wherein in Formula P-1-d, all present residues R^{P-1} and R^{P-3} together comprise in total 18-200 or 30-200 or 36-120 carbon atoms.

In some embodiments of the method and the composition of the invention, each pseudo solid-phase protecting group, e.g. the pseudo solid-phase protecting group PG-s, is a protecting group of any one of the aforementioned Formulae P-1-a, P-1-b, P-1-c, P-1-d, and P-1-e, wherein in Formula P-1-a:
the asterisk and R^{P-1} are defined as for Formula P-1;
a is an integer of 0 or 1;
i is an integer of 1 to 3, preferably 2 to 3; and
L¹ is C₁-C₃-alkylene group;
wherein in Formula P-1-b:
   the asterisk and R^{P-1} are defined as for Formula P-1;
   the integer a is 0;
   i is an integer of 1 to 3, preferably 2 to 3;
   L¹ is a C₁-C₄-alkylene group, in particular a C₁-C₂-alkylene group; and
   X^{P-2} is selected from the group consisting of ++C(O)N(R^{P-C})+ and ++C(O)O+, where R^{P-C} is selected from the group consisting of H and CH₃, and where + is the point of attachment to L¹ and ++ is the point of attachment to the phenyl moiety substituted with R^{P-1};
   wherein in Formula P-1-c:
      the asterisk and R^{P-1} are defined as for Formula P-1;
      the integer a is 0;
      i is an integer of 1 to 3, preferably 2 to 3;
      the integer c is 1;
      L¹ is a C₁-C₄-alkylene group, in particular a C₂-alkylene group (i.e. an ethylene group, CH₂-CH₂);
      X^{P-1}, L², and X^{P-2} together represent the following structure 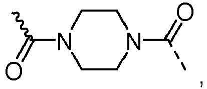 in which the dashed line represents the covalent bond to L¹ and the wavy line represents the covalent bond to the phenyl moiety substituted with R^{P-1}; and
      wherein in Formula P-1-d:
         the asterisk and R^{P-1} are defined as for Formula P-1;
         the integer a is 0;
         i is an integer of 1 to 3, preferably 2 to 3;
         L¹ is a C₁-C₄-alkylene group, in particular a C₂-alkylene group;
         X^{P-1} is selected from the group consisting of +C(O)N(R^{P-B})++ and +C(O)O++, where R^{P-B} is selected from the group consisting of H and a CH₃, and where + is the point of attachment to L¹ and ++ represents the carbon atom to which R^{P-D} is bonded in CHR^{P-D}; and
         R^{P-D} is selected from the group consisting of H and a residue of the aforementioned Formula P-1-L**,
            wherein in Formula P-1-L**:
            the hashtag (#) represents the carbon atom to which R^{P-D} is bonded in CHR^{P-D},
            k is an integer of 0 to 3;
            R^{P-3} is defined as R^{P-1} (i.e. the same definitions apply to R^{P-3} of Formula P-1-L** and R^{P-1} of Formula P-1); and
            R^{P-2} is H; and
         wherein in Formula P-1-e:
            the integer a is 0;
            i is an integer of 1 to 3, preferably 2 to 3;
            L¹ is a C₁-C₄-alkylene group, in particular a C₂-alkylene group;
            X^{P-1} is selected from the group consisting of +C(O)++, +C(O)N(R^{P-B})++, and +C(O)O++, where R^{P-B} is selected from the group consisting of H and CH₃, and where + is the point of attachment to L¹ and ++ is the point of attachment to L²;
            L² is a C₁-C₄-alkylene group, in particular a C₁-C₂-alkylene group; and X^{P-3} is selected from the group consisting of ++C(O)N(R^{P-E})+, and ++C(O)O+, where R^{P-E} is selected from the group consisting of H and a CH3, and where + denotes the point of attachment to L² and ++ is the point of attachment to the phenyl moiety substituted with R^{P-1}; and
            wherein in any one of Formulae P-1-a, P-1-b, P-1-c, and P-1-e all present residues R^{P-1} together comprise in total 36-120 carbon atoms; and
            wherein in Formula P-1-d, all present residues R^{P-1} and R^{P-3} together comprise in total 36-120 carbon atoms.

In some embodiments of the invention, R^{P-1} in any one of Formulae P-1, P-1-a, P-1-b, P-1-c, P-1-d, and P-1-e as well as R^{P-3} in any one of Formulae P-1 and P-1-d is at each occurrence independently selected from the group consisting of O(C₁-C₄₀-alkyl), O(C₂-C₄₀-alkenyl), a C₁-C₄₀-alkyl group, a C₂-C₄₀-alkenyl group, C(O)(C₁-C₄₀-alkyl), and C(O)(C₂-C₄₀-alkenyl). In some embodiments of the invention, R^{P-1} in any one of Formulae P-1, P-1-a, P-1-b, P-1-c, P-1-d, and P-1-e as well as R^{P-3} in any one of Formulae P-1 and P-1-d is at each occurrence independently selected from the group consisting of O(C₁-C₃₀-alkyl), O(C₂-C₃₀-alkenyl), a C₁-C₃₀-alkyl group, a C₂-C₃₀-alkenyl group, C(O)(C₁-C₃₀-alkyl), and C(O)(C₂-C₃₀-alkenyl). In some embodiments of the invention, R^{P-1} in any one of Formulae P-1, P-1-a, P-1-b, P-1-c, P-1-d, and P-1-e as well as R^{P-3} in any one of Formulae P-1 and P-1-d is at each occurrence independently selected from the group consisting of O(C₁-C₃₀-alkyl), O(C₂-C₃₀-alkenyl), C(O)(C₁-C₃₀-alkyl), and C(O)(C₂-C₃₀-alkenyl). In some embodiments of the invention, R^{P-1} in any one of Formulae P-1, P-1-a, P-1-b, P-1-c, P-1-d, and P-1-e as well as R^{P-3} in any one of Formulae P-1 and P-1-d is at each occurrence independently selected from the group consisting of O(C₁-C₃₀-alkyl) and C(O)(C₁-C₃₀-alkyl). In some embodiments of the invention, R^{P-1} in any one of Formulae P-1, P-1-a, P-1-b, P-1-c, P-1-d, and P-1-e as well as R^{P-3} in any one of Formulae P-1 and P-1-d is at each occurrence independently O(C₁-C₃₀-alkyl). In some embodiments of the invention, R^{P-1} in any one of Formulae P-1, P-1-a, P-1-b, P-1-c, P-1-d, and P-1-e as well as R^{P-3} in any one of Formulae P-1 and P-1-d is at each occurrence independently O(C₁-C₃₀-alkyl), wherein said C₁-C₃₀-alkyl in O(C₁-C₃₀-alkyl) is a linear (i.e. unbranched and non-cyclic) alkyl residue.

Pseudo solid-phase protecting groups of the aforementioned Formula P-1-a, methods for their preparation, introduction into nucleosides or oligonucleotides as well as cleavage from oligonucleotides are, e.g., disclosed in PCT/EP2022/059528 published as WO 2022/214692 A1, wherein reference is in particular made to Formulae II, II-a, and III to VII of said reference as well as to Examples 1 to 40 of said reference. Examples of pseudo solid-phase protecting groups of Formula P-1-a comprise protecting groups of the following structures: 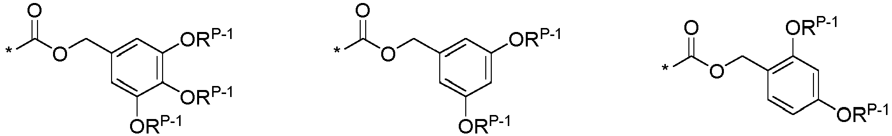 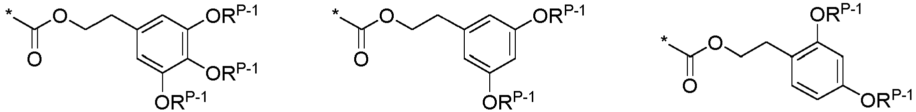 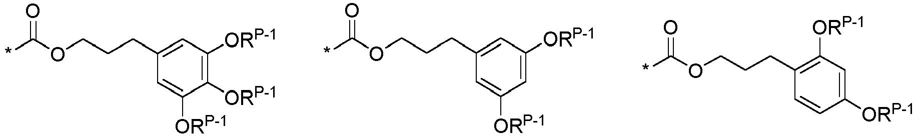 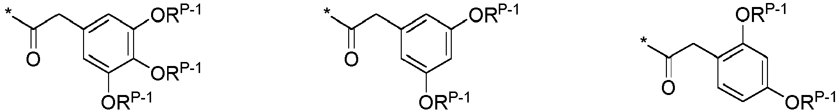 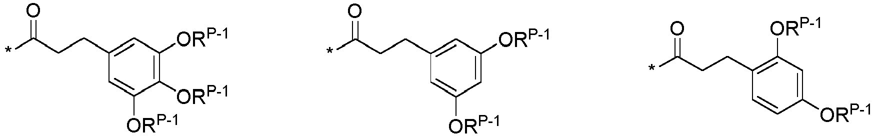 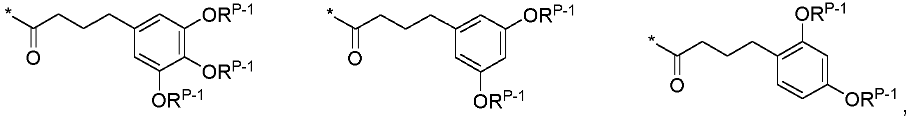 wherein in any one of these structures, the asterisk and R^{P-1} are defined as for Formula P-1. R^{P-1} may, e.g., be at each occurrence a C₂₂H₄₅-alkyl group, a C₂₁H₄₃-alkyl group, a C₂₀H₄₁-alkyl group, a C₁₉H₃₉-alkyl group or a C₁₈H₃₇-alkyl group.

Pseudo solid-phase protecting groups of the aforementioned Formula P-1-b are, e.g., disclosed in EP3825300A1. Examples of pseudo solid-phase protecting groups of Formula P-1-b comprise protecting groups of the following structures: 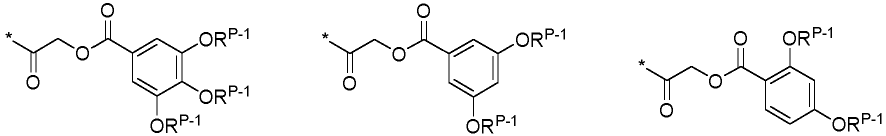 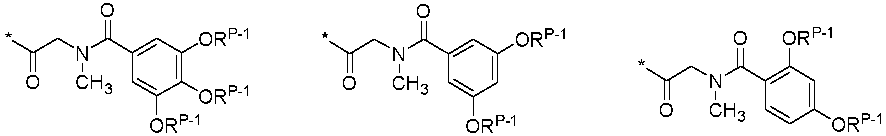 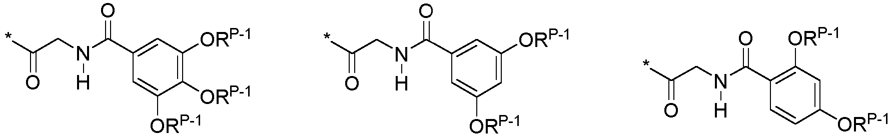 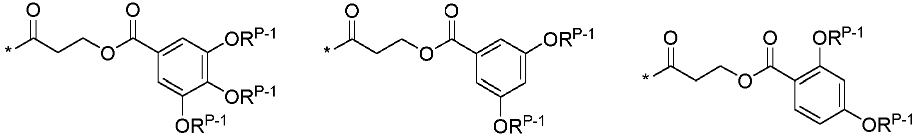 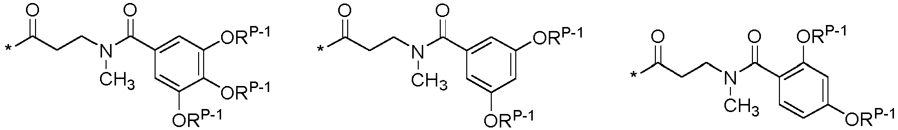 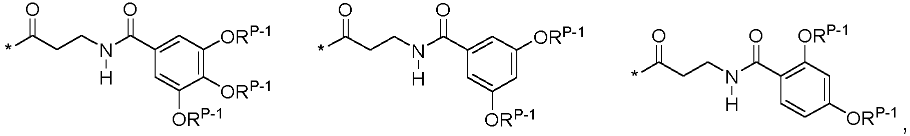 wherein in any one of these structures, the asterisk and R^{P-1} are defined as for Formula P-1. R^{P-1} may, e.g., be at each occurrence a C₂₂H₄₅-alkyl group, a C₂₁H₄₃-alkyl group, a C₂₀H₄₁-alkyl group, a C₁₉H₃₉-alkyl group or a C₁₈H₃₇-alkyl group.

Pseudo solid-phase protecting groups of the aforementioned Formula P-1-c are, e.g., disclosed in S. Kim et al., Chemistry - A European Journal 2013, 19, 8615-8620 (DOI: 10.1002/chem.201300655), wherein reference is also made to the Supporting Information belonging to said reference and comprising the experimental protocols. Particularly preferred examples of pseudo solid-phase protecting groups of Formula P-1-c comprise protecting groups of the following structures: 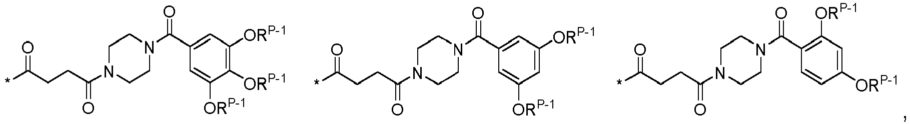 wherein in any one of these structures, the asterisk and R^{P-1} are defined as for Formula P-1. R^{P-1} may, e.g., be at each occurrence a C₂₂H₄₅-alkyl group, a C₂₁H₄₃-alkyl group, a C₂₀H₄₁-alkyl group, a C₁₉H₃₉-alkyl group or a C₁₈H₃₇-alkyl group.

Pseudo solid-phase protecting groups of the aforementioned Formula P-1-d are, e.g., disclosed in US2013267697A1, EP2711370A1, and EP3398955A1. Examples of pseudo solid-phase protecting groups of Formula P-1-d comprise protecting groups of the following structures: 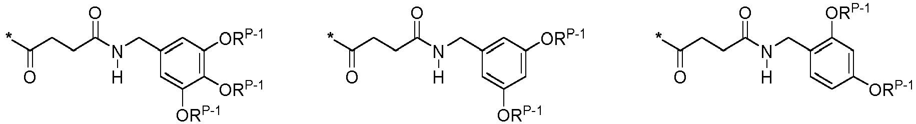 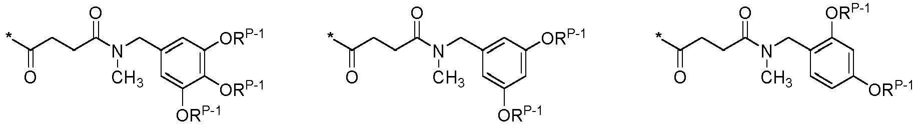 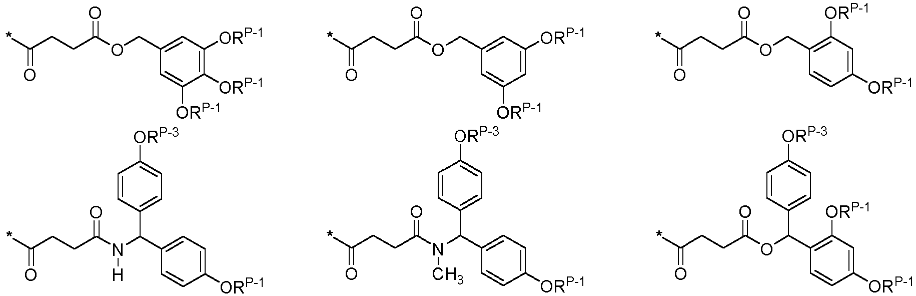 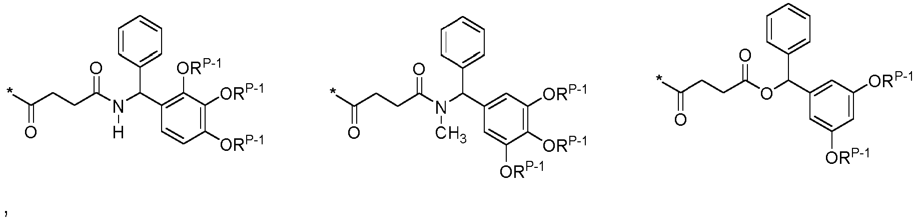 wherein in any one of these structures, the asterisk, R^{P-1}, and R^{P-3} are defined as for Formula P-1. R^{P-1} and R^{P-3} may, e.g., be at each occurrence a C₂₂H₄₅-alkyl group, a C₂₁H₄₃-alkyl group, a C₂₀H₄₁-alkyl group, a C₁₉H₃₉-alkyl group or a C₁₈H₃₇-alkyl group.

Examples of pseudo solid-phase protecting groups of Formula P-1-d comprise protecting groups of the following structures: 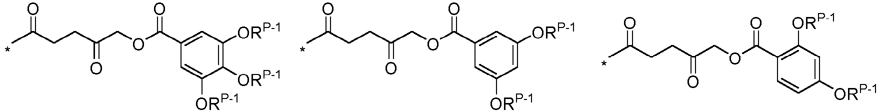 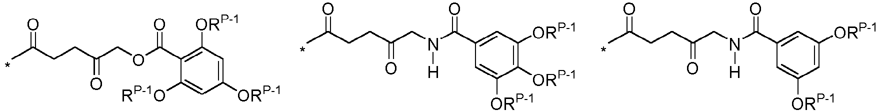 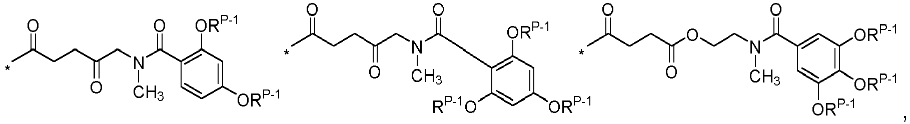 wherein in any one of these structures, the asterisk, R^{P-1}, and R^{P-3} are defined as for Formula P-1. R^{P-1} and R^{P-3} may, e.g., be at each occurrence a C₂₂H₄₅-alkyl group, a C₂₁H₄₃-alkyl group, a C₂₀H₄₁-alkyl group, a C₁₉H₃₉-alkyl group or a C₁₈H₃₇-alkyl group.

In some embodiments of the method and the composition of the invention, each pseudo solid-phase protecting group, e.g. the pseudo solid-phase protecting group PG-s, is a protecting group of the aforementioned Formula P-1-a.

The following Figures and Examples, including the experiments conducted and the results achieved, are provided for illustrative purposes only and are not to be construed as limiting the scope of the claims.

### Description of the Figure

**Figure 1** illustrates the one or more coupling cycles which may be performed as part of the method of the invention.

The method of the invention may comprise a first coupling cycle comprising steps (b-1) to (h-1) (as far as present), wherein steps (d-1), (f-1), (g-1), and (h-1) are optional, which is indicated in Figure 1 by embracing these steps in brackets. Prior to the first coupling cycle, a component C-0 is provided [step (a-1)]. In the first coupling cycle, the protecting group PG-0 is removed from said component C-0 [step (b-1)] which yields the component (C-0)^{#}. This is followed by one or more aqueous extractions [step (c-1)], optionally followed by reducing the water content of the organic phase which comprises the component (C-0)^{#} [step (d-1)]. The component (C-0)^{#} is reacted with a provided first building block B-1 [step (e-1)] which yields a first cycle oligonucleotide O-1. Optionally, any P (III) atoms within O-1 may be converted to P (V) atoms by incubation with an oxidizing or sulfurizing agent [step (f-1)], optionally followed by one or more aqueous extractions [step (g-1)], optionally followed by reducing the water content of the organic phase which comprises the first cycle oligonucleotide O-1 [step (h-1)].

The method of the invention may further comprise a second coupling cycle comprising steps (b-2) to (h-2), wherein steps (d-2), (f-2), (g-2), and (h-2) are optional, which is indicated in Figure 1 by embracing these steps in brackets. The first cycle oligonucleotide O-1 may serve as the educt of the second coupling cycle. The protecting group PG-1 is removed from the first cycle O-1 [step (b-2)] which yields the first cycle oligonucleotide (O-1)^{#}. This is followed by one or more aqueous extractions [step (c-2)], optionally followed by reducing the water content of the organic phase which comprises the first cycle oligonucleotide (O-1)^{#} [step (d-1)]. The first cycle oligonucleotide (O-1)^{#} is reacted with a provided second building block B-2 [step (e-2)] which yields a second cycle oligonucleotide O-2. Optionally, any P (III) atoms within O-2 may be converted to P (V) atoms by incubation with an oxidizing or sulfurizing agent [step (f-2)], optionally followed by one or more aqueous extractions [step (g-2)], optionally followed by reducing the water content of the organic phase which comprises the second cycle oligonucleotide O-2 [step (h-2)].

The method of the invention may further comprise (n-2) iterations of a coupling cycle comprising steps (b-x) to (h-x) (as far as present), which are referred to as "further coupling cycles" in Figure 1. Steps (d-x), (f-x), (g-x), and (h-x) are optional in each of these further coupling cycles, which is indicated in Figure 1 by embracing these steps in brackets. The second cycle oligonucleotide O-2 will then be used as educt O-(x-1), of the third coupling cycle (x=3). The protecting group PG-(x-1), i.e. PG-2, is removed [step (b-x)] which yields oligonucleotide (O-(x-1))^{#}, i.e. (O-2)^{#} (x=3). This is followed by one or more aqueous extractions [step (c-x)], optionally followed by reducing the water content of the organic phase which comprises the oligonucleotide (O-(x-1))^{#}, i.e. (O-2)^{#} (x=3) [step (d-1)]. The oligonucleotide (O-(x-1))^{#}, i.e. (O-2)^{#}, is reacted with a provided building block B-x, i.e. B-3 (x=3) [step (e-x)] which yields an oligonucleotide O-x, i.e. O-3 (x=3). Optionally, any P (III) atoms within O-3 may be converted to P (V) atoms by incubation with an oxidizing or sulfurizing agent [step (f-x)], optionally followed by one or more aqueous extractions [step (g-x)], optionally followed by reducing the water content of the organic phase which comprises the oligonucleotide O-x, i.e. O-3 (x=3) [step (h-x)].

Up to (n-2) coupling cycles comprising steps (b-x) to (h-x) (as far as present) ["further coupling cycles"] may be performed, in each of which the oligonucleotide O-x of the previous coupling cycle is used as educt O-(x-1).

The fact that the first cycle oligonucleotide O-1 need not be subjected to the second coupling cycle, the fact that the second cycle oligonucleotide O-2 need not be subjected to the third coupling cycle, and the fact that each x-th cycle oligonucleotide O-x need not be subjected to another coupling cycle are indicated in Figure 1 by the dashed "exit" arrows.

### Examples

### General information and methods

### Target oligonucleotides of the syntheses presented herein

The following Examples pertain to the synthesis of target oligonucleotides, wherein these target oligonucleotides are summarized in the following Table T-2.

**Table T-2: Target oligonucleotides of the oligonucleotide syntheses presented herein.**

| Example | name of target oligonucleotide | sequence of target oligonucleotide |
|---|---|---|
| 1 | O^{T}-a | 5'-MMeUs-MMeCs-MAs-MMeCs-MMeUs-MMeUs-MMeUs-MMeCs-MAs-MMeUs-MAs-MAs-MMeUs-MGs-MMeCs-MMeUs-MGs-MG-3' |
| 2 | | |
| 11 | | |
| 12 | | |
| 3 | O^{T}-b^{a)} | 5'-dT-dC(bz)-dA(bz)-dG(ib)-dT-(Kc-C1-carbonate) |
| 4 | | |
| 13 | | |
| 14 | | |
| 5 | O^{T}-c^{a)} | 5'-dTs-dG(ib)s-dC(bz)s-dA(bz)s-dT-(Kc-C1-carbonate) |
| 6 | O^{T}-d^{a)} | 5'-dA(bz)-dT-dC(bz)-dA(bz)-dT-(Kc-C1-carbonate) |
| 7 | O^{T}-e | 5'-MGs-MMeCs-MMeUs-MGs-MG-3' |
| 9 | | |
| 8 | O^{T}-f | 5'-MAs-MMeUs-MAs-MAs-MMeUs-MGs-MMeCs-MMeUs-MGs-MG-3' |
| 10 | | |
| 15 | | |
| 11 | O^{T}-g | 5'-MMeUs-MMeCs-MAs-MMeCs-MMeUs-MMeUs-MMeUs-MMeCs-MAs-MMeUs-MAs-MAs-MMeUs-MGs-MMeCs-3' |

a) "(Kc-C1-carbonate)" in the sequence of target oligonucleotides O^{T}-b, O^{T}-c, and O^{T}-d denotes a specific pseudo solid-phase protecting group bonded to the 3'-hydroxyl moiety of the 3'-terminal dT nucleoside subunit. The structure of this pseudo solid-phase protecting group is depicted in compound **(1)** below.

The following notation is herein used to denote oligonucleotide sequences, unless indicated differently:
A = adenosine
dA = 2'-doexyadenosine
G = guanosine
dG = 2'-doexyguanosine
C = cytidine
dC = 2'-doexycytidine
MeC or meC = 5-methylcytidine (the nucleobase is 5-methylcytosine)
T = thymidine (meaning 2'-deoxythymidine and not ribothymidine)
U = uridine
MeU or meU = 5-methyluridine (the nucleobase is 5-methyluracil, i.e. thymine)

"M" indicates that the nucleoside subunit denoted to the right of said letter "M" comprises a 2'-O-(2-methoxyethyl) residue (2'-O-MOE, i.e. the 2'-carbon atom of the carbohydrate moiety is substituted with O-CH₂-CH₂-O-CH₃).

"f" indicates that the nucleoside subunit denoted to the right of said letter "f" comprises a 2'-F residue (i.e. the 2'-carbon atom of the carbohydrate moiety is substituted with a fluorine residue).

"m" indicates that the nucleoside subunit denoted to the right of said letter "m" comprises a 2'-methoxy (Ome, OCH₃) residue (i.e. the 2'-carbon atom of the carbohydrate moiety is substituted with OCH₃).

The presence of protecting groups at the nucleobases is indicated in brackets to the right of the nucleoside symbol: "(bz)" and "(Bz)" interchangeably denote a benzoyl protecting group at the exocyclic amine moiety of the respective nucleobase; "(ac)" and "(Ac)" interchangeably denote an acetyl protecting group at the exocyclic amine moiety of the nucleobase; "(ib)" denotes an isobutyryl protecting group at the exocyclic amine moiety of the nucleobase.

Unless indicated differently, all internucleosidic linkage groups are phosphodiester linkage groups. The letter "s" to the right of a nucleoside symbol indicates that the internucleosidic linkage group in 3'-position of the respective nucleoside-subunit is a phosphorothioate linkage group instead of a phosphodiester linkage group.

Nucleoside subunits are separated by hyphens to enhance readability.

Herein, unless indicated differently, all oligonucleotides are denoted in 5' to 3' direction (i.e. the nucleoside subunit denoted to the very left is the 5'-terminal nucleoside subunit). Nonetheless, to enhance readability, the 5'- and the 3'-termnini are herein typically indicated as such.

For example, 5'-MmeUs-MmeC-3' herein denotes a dinucleotide, in which the 3'-hydroxyl moiety of a 2'-O-MOE substituted 5-methyluridine and the 5'-hydroxyl moiety of a 2'-O-MOE substituted 5-methylcytidine are interconnected by a phosphorothioate linkage group. As another example, 5'-dA(bz)-dT-3' denotes a dinucleotie, in which the 3'-hydroxyl moiety of a benzyol-protected 2'-deoxyadenosine and the 5'-hydroxyl moiety of a (2'-deoxy)thymidine are interconnected by a phosphodiester linkage group.

### HPLC methods

### Method A

Solid phase: YMC-Triart C18, 12 nm, 3.0 µm, 4.6×150 mm; Mobile phase A: 0.1 % AcOH aq.; Mobile Phase B: THF; Flow rate: 1.0 mL/min; Gradient (mobile phase B %): 0.0-10.0 min; 75 to 90 %, 10.0-15.0 min; 90 %, 15.0-15.1 min; 90 to 75 %, 15.1-20.0 min; 75 %; Column temperature: 40 °C; Detection wavelength: 220 nm.

### Method B

Solid phase: YMC-Triart C18, 12 nm, 1.9 µm, 2.1×150 mm; Mobile phase A: 10 mM dibutylamine aq. (pH adjusted to 6.0 by AcOH); Mobile phase B: THF, with Stabilizer; Flow rate: 0.3 mL/min; Gradient (mobile phase B %): 0.0-15.0 min; 65 % to 75 %, 15.0-15.1 min; 75 to 90 %, 15.1-20.1 min; 90 %, 20.0-20.1 min; 90 to 65 %, 20.1-30.0 min; 65 %; Column temperature: 60 °C; Detection wavelength: 250 nm.

### Method C

Solid phase: ACQUITY UPLC BEH C18, 1.7 µm, 2.1×100 mm; Mobile phase A: 10 mM dibutylamine aq. (pH adjusted to 6.0 by AcOH); Mobile phase B: MeOH; Flow rate: 0.2 mL/min; Gradient (mobile phase B %): 0-0.5 min; 10 %, 0.5-20.0 min; 10 to 90 %, 20.0- 22.0 min; 90 %, 22.0-22.1 min; 90 to 10 %, 22.1-30.0 min; 10 %; Column temperature: 70 °C; Detection wavelength: 260 nm. Gradient-2 (mobile phase B %): 0-5 min; 10 %, 5-30.0 min; 10 to 60 %, 30.0- 31.0 min; 60 to 90 %, 31.0-37.0 min; 90 %, 37.0-38.0 min; 90 to 10 %, 38.0-43.0 min; 10%.

### Method D

Solid phase: Triart Accura C8, 1.9 µm, 2.1×150 mm; Mobile phase A: 400 mM HFIP-15mM Triethylamine aq.; Mobile phase B: MeOH; Flow rate: 0.2 mL/min; Gradient (mobile phase B %): 0-11 min; 24 to 34 %, 11.0-13.0 min; 34 to 75 %, 13.0-13.01 min; 75 to 24 %, 13.1-20.0 min; 24 %; Column temperature: 70 °C; Detection wavelength: 260 nm.

### Method E

Solid phase: Triart Accura C8, 1.9 µm, 2.1×150 mm; Mobile phase A: 10 mM dibutylamine aq. (pH adjusted to 7.0 by AcOH); Mobile phase B: MeOH; Flow rate: 0.5 mL/min; Gradient (mobile phase B %): 0-1.0 min; 24 %, 1.0-5.0 min; 24 to 48 %, 5.0-10.0 min; 48 to 50 %, 10.0-12.0 min; 50 to 55 %, 12.0-16.0 min; 55 to 60 %, 16.0-18.0 min; 60 to 70%, 18.0 to 20.0 min; 70 to 75%, 20.0-25.0 min; 75%, 25.0-25.1 min; 75 to 24%, 25.1-30 min; 24%; Column temperature: 70 °C; Detection wavelength: 260 nm.

### Method F

Solid phase: Triart Accura C8, 1.9 µm, 2.1×150 mm; Mobile phase A: AcOH aq.; Mobile phase B: THF; Flow rate: 1.0 mL/min; Gradient (mobile phase B %): 0-10.0 min; 65 to 90 %, 10.0-15.0 min; 90 %, 15.0-15.1 min; 90 to 65 %, 15.01-20.0 min; 65 %; Column temperature: 40 °C; Detection wavelength: 220 nm.

### Method G

Solid phase: Waters ACQUITY BEH, 1.7 um, 2.1×100 mm; Mobile phase A: 50 mM di-n-buthylamminoum acetate aq.; Mobile phase B: Acetonitril:H₂O (90:10 v/v) ; Flow rate: 0.2 mL/min; Gradient (mobile phase B %): 0-30 min; 25 to 45 %, 30-31 min; 45 to 90 %, 31-33 min; 90 %, 33-33.1 min; 90 to 25 %, 33.1-45 min; 25 %; Column temperature: 60 °C; Detection wavelength: 260 nm.

### Method H

Solid phase: YMC-Triart C18, 12 nm, 1.9 µm, 2.1×150 mm; Mobile phase A: 0.1% AcOH aq.; Mobile phase B: THF; Flow rate: 0.3 mL/min; Gradient (mobile phase B %): 0.0-15.0 min; 65% to 75 %, 15.0-15.1 min; 75 to 90 %, 15.1-20.1 min; 90%, 20.0-20.1 min; 90 to 65%, 20.1-30.0 min; 65%; Detected wavelength: 260 nm.

### HPLC-MS methods

### Method A

Solid phase: YMC-Triart C18, 12 nm, 1.9 µm, 2.1×150 mm; Mobile phase A: 0.1% AcOH aq.; Mobile phase B: THF, with Stabilizer; Flow rate: 0.3 mL/min; Gradient (mobile phase B %): 0.0-15.0 min; 65% to 75 %, 15.0-15.1 min; 75 to 90 %, 15.1-20.1 min; 90%, 20.0-20.1 min; 90 to 65%, 20.1-30.0 min; 65%; Column temperature: 60°C; Detection wavelength: 250 nm.; Acquisition range: 100-3000 m/z

### Method B

Solid phase: YMC-Triart Bio C18, 30 nm, 1.9 µm, 2.1×100 mm; Mobile phase A: 0.1 % TFA aq.; Mobile phase B: THF; Flow rate: 0.48 mL/min; Gradient (mobile phase B %): 0-0.5 min; 65 %, 0.5-1.0min; 65 to 72 %, 1.0-3.0 min; 72 %, 3.0-5.0 min; 72 to 80 %, 5.0-6.0 min; 80 to 85 %, 6-6.5 min; 80 to 90 %; Column temperature: 40 °C; Detection wavelength: 220 nm; Acquisition range: 500-3000 m/z

### Method C

Solid phase: ACQUITY UPLC BEH C18, 1.7 µm, 2.1×100 mm; Mobile phase A: 200 mM HFIP-7.5 mM triethylamine aq.; Mobile phase B: MeOH; Flow rate: 0.2 mL/min; Gradient (mobile phase B %): 0-0.5 min; 10 %, 0.5-20.0 min; 10 to 90 %, 20.0- 22.0 min; 90 %, 22.0-22.1 min; 90 to 10 %, 22.1-30.0 min; 10 %; Column temperature: 70 °C; Detection wavelength: 260 nm.; Acquisition range: 500-2500 m/z

### Method D

Solid phase: ACQUITY UPLC BEH C18, 1.7 µm, 2.1×100 mm; Mobile phase A: 10mM dibutylamine (pH adjusted to 7.0) aq.; Mobile phase B: MeOH; Flow rate: 0.5 mL/min; Gradient (mobile phase B %): 0-0.5 min; 24 %, 0.5-11.0 min; 24 to 34 %, 11.0- 13.0 min; 34 to 75 %, 13.0-13.1 min; 75 to 24 %, 13.1-20.0 min; 24%; Column temperature: 70 °C; Detection wavelength: 260 nm.; Acquisition range: 500-3000 m/z.

### Method E

Solid phase: ACQUITY UPLC BEH C18, 1.7 µm, 2.1×100 mm; Mobile phase A: 400 mM HFIP-7.5 mM triethylamine aq.; Mobile phase B: MeOH; Flow rate: 0.5 mL/min; Gradient (mobile phase B %): 0-0.5 min; 24 %, 0.5-11.0 min; 24 to 34 %, 11.0- 13.0 min; 34 to 75 %, 13.0-13.1 min; 75 to 24 %, 13.1-20.0 min; 24%; Column temperature: 70 °C; Detection wavelength: 260 nm.; Acquisition range: 500-3000 m/z.

### Method F

Solid phase: YMC-Triart C18, 12 nm, 1.9 µm, 2.1×150 mm; Mobile phase A: 0.1% AcOH aq.; Mobile phase B: THF; Flow rate: 0.3 mL/min; Gradient (mobile phase B %): 0.0-15.0 min; 65% to 75 %, 15.0-15.1 min; 75 to 90 %, 15.1-20.1 min; 90%, 20.0-20.1 min; 90 to 65%, 20.1-30.0 min; 65%; Column temperature: 60°C; Detection wavelength: 260 nm.; Acquisition range: 100-3000 m/z.

### Method G

Solid phase: Waters ACQUITY BEH, 1.7 um, 2.1×100 mm; Mobile phase A: 50 mM di-n-buthylamminoum acetate aq.; Mobile phase B: Acetonitril:H₂O (90:10 v/v) ; Flow rate: 0.2 mL/min; Gradient (mobile phase B %): 0-30 min; 25 to 45 %, 30-31 min; 45 to 90 %, 31-33 min; 90 %, 33-33.1 min; 90 to 25 %, 33.1-45 min; 25 %; Column temperature: 60°C; Detection wavelength: 260 nm.; Acquisition range: 500-3000 m/z.

### MS condition (general)

Ion source: Dual ESI; Ionization mode: positive or negative; Vcap: 3500 V, 20; Gas temp: 325 °C; Gas Flow: 8 L/min; Nebulizer: 50 psi.

### Pre-treatment of N-octyl-2-pyrrolidone

N-octyl-2-pyrrolidone (NOP) was obtained commercially. Prior to use, it was treated as follows: L-Ascorbic acid (1 wt-%) and 2,6-di-tert-butyl-p-cresol (1 wt-%) were added, followed by stirring at room temperature (i.e. approximately 25 °C) for 30 min. The so-obtained solution was washed four times: first and second wash with 0.5 M NaHCO₃ aq. (1:1 v/v), third wash with 1 vol-% acetic acid aq. (1:1 v/v), and fourth wash with H₂O (1:1 v/v).

### Determination of the water content

Throughout the examples presented herein, reference is occasionally made to the water content of solutions. Unless indicated differently, the water content was determined by means of standard Karl Fischer titration at room temperature (i.e. approximately 25 °C). The titration cell was filled with HYDRANAL^{™} -Coulomat Oil for anolyte reagent, and the inner burette was filled with HYDRANAL^{™} -Coulomat CG for catholyte reagent. After pretitration for dehydration, sample (100 µL) was added to the titration sell and the measurement started. In line with common practice, the water content of solutions is herein reported in parts per million (ppm).

### Analysis of solvent compositions after azeotropic distillation

Throughout the examples presented herein, reference is occasionally made to the composition of mixed solvents after azeotropic distillation. This content is herein provided in form of volume-% (vol-%) and was, unless indicated differently, determined by gas chromatography. Samples were analyzed by the following method: Column: SH-Rxi-624Sil MS (30 m × 0.32 mmID, 1.8 µm), Injection volume: 1 µL, Inlet: Split (5/1), Injection temperature: 230 °C, Column flow: 35 cm/s Helium, Column temperature program: 40 °C(2 min)-10 °C/min-240 °C (20 min) total 42 min, Detector: FDI at 250 °C. Then, the content of solvent was calculated by using a calibration curve.

### Room temperature (rt)

As used herein, unless indicated differently, the terms room temperature (rt) and ambient temperature are used interchangeably to denote a temperature of approximately 25 °C. Unless indicated differently, all reactions were performed at rt.

### Reaction setup

Unless indicated differently, all oligonucleotide syntheses presented in the following were carried out in an eggplant flask made of glass equipped with a magnetic stir bar. All aqueous extractions were carried out in a common separatory funnel. All azeotropic distillations were carried out using a common rotary evaporator. Base treatment to effect cleavage of the pseudo solid-phase protecting groups and other base-labile protecting groups was carried out in an ACE Pressure Tupe by OSAKA CHEMICAI Co., Ltd., which is a pressure-resistant sealed glass tube.

### Synthesis and introduction of pseudo solid-phase protecting groups

### Pseudo solid-phase protecting groups of the above-mentioned Formula P-1-a, wherein the integer a is 1

Pseudo solid-phase protecting groups of the above-mentioned Formula P-1-a, where the integer a is 1, may be introduced into a nucleoside or oligonucleotide having exactly one free hydroxyl or amine group by reacting said free hydroxyl or amine group with an activated carbonic acid derivative, preferably in the presence of a base such as 1-methylimidazole, and reacting the so-obtained compound with an alcohol of the following structure: 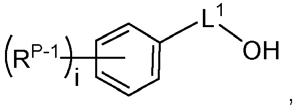 where L¹, R^{P-1}, and the integer i are defined as for Formula P-1-a. Said activated carbonic acid derivative may, for example, be selected from the group consisting of phosgene, disuccinimidyl carbonate, and 1,1'-carbonyldiimidazole.

### Exemplary synthesis of DMTr-dT-(Kc-C1-carbonate) (1)

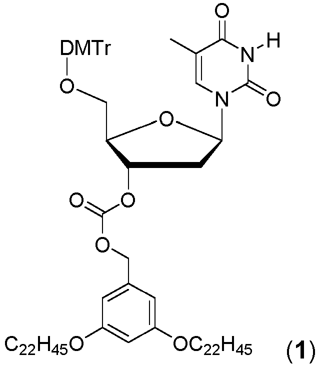

To a solution of DMTr-dT (i.e. 5'-DMTr-protected dT,14.41 g, 26.46 mmol) and 1,1'-carbonyldiimidazole (8.55 g, 52.73 mmol) in 2-methyltetrahydrofuran (300 mL) stirred at rt was added 1-methylimidazole (4.2 mL, 52.69 mmol). The resulting reaction mixture was stirred at rt for 2 h (monitored by HPLC, Method A) and subsequently washed twice with saturated NH₄Cl aq. (300 mL and 150 mL) and then with saturated NaCl aq. (75 mL). The organic layer was concentrated *in vacuo* to give DMTr-dT-1H-imidazole-1-carboxyl, which was used directly without further purification.

DBU (3.9 mL, 26.13 mmol) and 1-methylimidazole (2.1 mL, 26,35 mmol) were added to a stirred solution of DMTr-dT-1H-imidazole-1-carboxyl (approximately 26 mmol) and 3,5-bis(docosyloxy)benzenemethanol (CAS-RN: 955095-32-6, 10.00 g, 13.20 mmol) in 2-methyltetrahydrofuran (200 mL) at rt. The resulting reaction mixture was stirred at rt for 110 min, followed by addition of DMTr-dT (7.12 g, 13.07 mmol) and stirring at rt for 16 h (monitored by HPLC, Method A). Acetic anhydride (2.0 mL, 21.16 mmol) and pyridine (2.0 mL, 24.78 mmol) were added. After stirring at rt for 45 min, methanol (32 mL, 7 89 mmol) was added. After stirring 30 min at rt, the reaction mixture was concentrated *in vacuo,* followed by addition of acetonitrile (500 mL). The resulting precipitate was filtrated and washed twice with acetonitrile (200 mL × 2) to give DMTr-dT-(Kc-C1-carbonate) (19.58 g, quantitative yield) as a white solid.

### Exemplary synthesis of DMTr-MG(ib)-(Kc-C1-carbonate) (2)

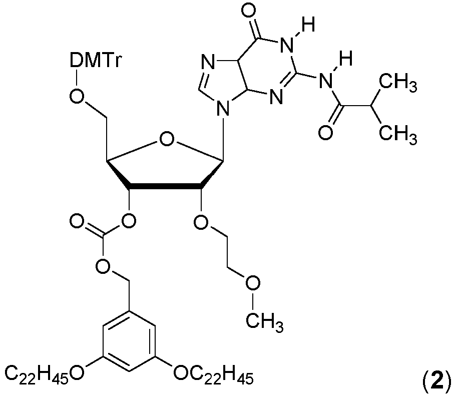

1-Methylimidazole (631 µL, 7.92 mmol) was added to a stirred solution of DMTr-MG(ib) (i.e. 5'-DMTr-protected guanosine with 2'-O-(2-methoxyethyl) residue, 2.71 g, 3.80 mmol) and 1,1'-carbonyldiimidazole (1.29 g, 7.56 mmol) in 2-methyltetrahydrofuran (60 mL) at rt. The resulting reaction mixture was stirred at rt for 40 min (monitored by HPLC, method A) and subsequently washed twice with saturated NH₄Cl aq. (300mL and 150 mL) and then with saturated NaCl aq. (75 mL). The organic phase was concentrated *in vacuo* to give DMTr-MG(ib)-1H-imidazole-1-carboxyl, which was used directly without further purification.

DBU (591 µL, 3.96 mmol) and 1-methylimidazole (316 µL, 3.91 mmol) were added to a stirred solution of DMT-MG(ib)-1H-imidazole-1-carboxyl (ca. 3.8 mmol) and 3,5-bis(docosyloxy)benzenemethanol (CAS-RN: 955095-32-6, 2.00 g, 2.64 mmol) in 2-methyltetrahydrofuran (40 mL) at rt. The resulting reaction mixture was stirred at rt for 4 h, followed by addition of DMTr-MG(ib) (1.86 g, 2.61 mmol) and stirring the reaction mixture at rt for 17 h (monitored by HPLC with Method A). Acetic anhydride (3.0 mL, 31.69 mmol), pyridine (3.2 mL, 39.61 mmol) and 1-methylimidazole (3.2 mL, 39.61 mmol) were added. After stirring at rt for 10 min, methanol (4.8 mL, 118.84 mmol) was added. After stirring 10 min at rt, the reaction mixture was concentrated *in vacuo,* followed by addition of acetonitrile (100 mL). The resulting precipitate was filtrated and washed twice with acetonitrile (40 mL × 2) to give DMTr-MG(ib)-(Kc-C1-carbonate) **(2,** 3.74 g, yield 94.6 %) as a white solid.

### Pseudo solid-phase protecting groups of the above-mentioned Formula P-1-a, wherein the integer a is 0

Pseudo solid-phase protecting groups of the above-mentioned Formula P-1-a, where the integer a is 0, may be introduced into a nucleoside or oligonucleotide having exactly one free hydroxyl or amine group by reacting said free hydroxyl or amine group with a compound of the following structure: 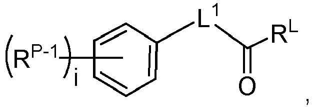 where L¹, R^{P-1}, and the integer i are defined as for Formula P-1-a and R^{L} is selected from the group consisting of a hydroxyl group (OH) and a leaving group (e.g. Cl) capable of being substituted by said hydroxyl or amine moiety of said nucleoside or oligonucleotide. If R^{L} is Cl, the reaction may preferably be conducted in the presence of a base such as pyridine, collidine, triethylamine (TEA) or diisopropylethylamine (DIPEA). If R^{L} is a hydroxyl group, the latter may be activated, e.g. towards ester bond formation, by treatment with a coupling agent such as a carbodiimide (e.g. *N*,*N*-diisopropylcarbodiimide) in combination with an additive such as 4-(dimethylamino)pyridine (DMAP), 2-cyano-2-(hydroxyimino)acetate (Oxyma), 1-hydroxybenzotriazole (HOBt) or 1-hydroxy-7-azabenzotriazole (HOAt), or by treatment with so-called aminium / uronium salts such as (benzotriazolyl)tetramethyluronium tetrafluoroborate (TBTU), N-[(7-aza-1H-benzotriazol-1-yl)(dimethylamino)-methylene]-N-methylmethanaminium tetrafluoroborate N-oxide (TATU), 2-(1H-benzotriazol-1-yl)-1,1,3,3-tetramethyluronium hexafluorophosphate (HBTU), N-[(7-aza-1H-benzotriazol-1-yl)(dimethylamino)-methylene]-Nmethylmethanaminium hexafluorophosphate N-oxide (HATU) or 1-cyano-2-ethoxy-2- oxoethylidenaminooxy)dimethylamino-morpholino-carbenium-hexafluorophosphate (COMU) in combination with a base such as diisopropylethylamine.

### Exemplary synthesis of DMTr-dT-(Kb-C2-acid) (3)

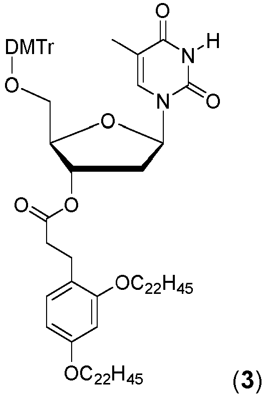

To a stirred solution of DMTr-dT (0.30 mmol, 163 mg), DIPEA (0.45 mmol, 78.6 µL), COMU (0.3 mmol, 128.5 mg) and DMAP (0.02 mmol, 2.4 mg) in CH₂Cl₂ (20 mL) was added 3-(2,4-bis(docosyloxy)phenyl)propanoic acid (0.15 mmol, 120 mg) ) at rt. The resulting reaction mixture was stirred at rt for 16 h and concentrated *in vacuo.* The precipitate was filtered and washed with MeOH to give DMTr-dT-(Kb-C2-acid) **(3,** 114 mg, yield 57 %) as white solid.

3-(2,4-bis(docosyloxy)phenyl)propanoic acid used in the synthesis of **(3)** was prepared as follows: K₂CO₃ (6 eq) was added to a stirred solution of methyl 3-(2,4-dihydroxyphenyl)propionate (5 mmol) and 1-bromodocosane (3 eq) in DMF/THF (1/1 v/v, 100 mL) at rt. The resulting reaction mixture was stirred at 80 °C for 18 h, diluted with MeCN at 60 °C, and filtered. The collected solid was washed with MeCN (60 °C) and concentrated *in vacuo* to give methyl 3-(2,4-bis(docosyloxy)phenyl)propanoate quantitatively as white solid. Aq. KOH (1 M, 6 eq) was added to a stirred solution of methyl 3-(2,4-bis(docosyloxy)phenyl)propanoate (5.0 mmol) in EtOH/THF (1/1 v/v, 50 mL) at rt. The resulting reaction mixture was stirred at 90 °C for 5 h, neutralized with 1 M aq. HCI at rt, and filtered. The collected solid was washed with water/acetone and concentrated *in vacuo* to give 3-(2,4-bis(docosyloxy)phenyl)propanoic acid (1.96 g) in 49 % yield over 2 steps from methyl 3-(2,4-dohydroxyphenyl)propionate (5 mmol) as a white solid. It is noted that such acids may be reduced with LiAlH₄ in THF to arrive at the corresponding alcohol.

### Syntheses of oligonucleotides

### Example 1: Synthesis A of target oligonucleotide O^{T}-a (see Table T-2)

### DMTr-MG(ib)s-MG(ib)-(Kc-C1-carbonate) (5)

Dichloroacetic acid (DCA, 5.0 mL) was added at rt to a stirred solution of DMTr-MG(ib)-(Kc-C1-carbonate) **(2,** 987mg, 0.659 mmol) and thiomalic acid (504 mg, 3.36 mmol) in 4-methyltetrahydropyran (MTHP, 15 mL), and the mixture was stirred at rt until reaction monitoring by HPLC (Method B was used throughout Example 1) indicated completion of the DMTr-deprotection. *N*-Octyl-2-pyrrolidone (NOP, 13 mL), *N*-methylmorpholine (NMM, 9 mL), acetone (15 mL) and 15 wt-% aqueous (aq.) sodium chloride solution (brine, 40 mL) was added, followed by extraction. The organic phase was separated and extracted twice: first with water/acetone (2:1 v/v, 30 mL), and then with a mixture of water/acetone (3:1 v/v, 20 mL) and acetic acid (150 µL). MTHP (15 mL) was added to the organic phase comprising MG(ib)-(Kc-C1-carbonate) **(4),** followed by concentration *in vacuo* (bath temperature 35-55 °C, pressure 100 hPa) to a volume of 20 mL. The MTHP (15 mL) addition and subsequent concentration was repeated three times (final volume: 20 mL, water content: 524 ppm). The so-obtained solution comprising MG(ib)-(Kc-C1-carbonate) **(4)** was used directly in the subsequent step (without any precipitation, filtration or purification steps in between). NMM (1.0 mL, 12.55 mmol), DMTr-MG(ib) phosphoramidite (CAS-RN: 251647-55-9, 1.93 g, 2.11 mmol) and 5-benzylthio-1*H-*tetrazole (BTT, 1.30 g, 6.76 mmol) were added to said solution comprising MG(ib)-(Kc-C1-carbonate) **(4)** and the mixture was stirred at rt until reaction monitoring by HPLC indicated completion of the coupling. 3-(N,N-dimethylamino-methylidene)amino)-3*H*-1,2,4-dithiazole-5-thione (CAS-RN: 1192027-04-5, DDTT, 548 µg, 2.67 mmol) was added. After stirring for 10 min at rt, triethylphosphite (226 µL, 1.32 mmol) was added. After stirring for 10 min at rt, acetic anhydride (Ac₂O, 250 µL, 2.64 mmol) and pyridine (266 µL, 3.30 mmol) were added. After stirring for 10 min at rt, the solution was washed (i.e. extracted with aqueous solutions) twice: first with a mixture of 10 vol-% acetic acid (AcOH) aq. (10 mL) and AcOH (1 mL), then with a mixture of 10 vol-% AcOH aq. (10 mL). MTHP (15 mL) was added to the organic phase, followed by concentration *in vacuo* to a volume of 20 mL (bath temperature 35-55 °C, pressure 100 hPa). This MTHP (15 mL) addition and subsequent concentration was repeated twice (final volume: 20 mL, water content: 1156 ppm). The so-obtained solution comprising DMTr-MG(ib)s-MG(ib)-(Kc-C1-carbonate) **(5)** was used directly in the subsequent step (without any precipitation, filtration or purification steps in between).

### Second coupling cycle and further coupling cycles

The second coupling cycle and all further coupling cycles were performed according to the following general protocol and as described in detail in the following Tables E-1 and E-2.

### General protocol for coupling cycle

In a typical coupling cycle, the following steps were performed in the presented order.

### DMTr-deprotection

Thiomalic acid and TFA were added to the solution obtained from the preceding azeotropic distillation and the mixture was stirred at rt until reaction monitoring by HPLC indicated complete DMTr-deprotection.

### Aqueous extraction(s)

The reaction mixture obtained from carrying out the DMTr-deprotection was extracted with one or more aqueous solutions as indicated in Table E-2.

### Azeotropic distillation

MTHP was added to the organic phase obtained from the preceding aqueous extractions, followed by concentration *in vacuo* (bath temperature 35-55 °C, pressure 100 hPa). This MTHP addition and concentration step was typically repeated as indicated in Table E-2. The water content was adjusted to be in the range of 117-524 ppm. The composition of the solution obtained after azeotropic distillation was analyzed as laid out above for the 2^{nd} to 6^{th} and for the 8^{th} to 17^{th} coupling cycle. The content of NOP was in the range of 21-44 vol-% and the content of MTHP was in the range of 56-79 vol-%.

### Coupling reaction

The respective phosphoramidite building block (obtained commercially) and BTT were added directly to the solution obtained from carrying out the preceding azeotropic distillation and the mixture was stirred at rt until reaction monitoring by HPLC indicated completion of the coupling.

### Sulfurization (including quenching with triethylphosphite)

DDTT was added and the mixture was stirred for 10 min at rt. Triethylphosphite was added and the mixture was stirred for 10 min at rt.

### Capping

Ac₂O, pyridine, and 1-methylimidazole were added and the mixture was stirred for 10 min at rt.

### Aqueous extraction(s)

The reaction mixture obtained from carrying out the capping step was extracted with one or more aqueous solutions as indicated in Table E-2.

### Azeotropic distillation

MTHP was added to the organic phase obtained from the preceding aqueous extraction(s), followed by concentration *in vacuo* (bath temperature 35-55 °C, pressure 100 hPa). This MTHP addition and concentration step was typically repeated as indicated in Table E-2. The water content was adjusted to be in the range of 1006-2340 ppm. The composition of the solution obtained after azeotropic distillation was analyzed as laid out above for the 9^{th} to 17^{th} coupling cycle. The content of NOP was in the range of 24-40 vol-% and the content of MTHP was in the range of 60-76 vol-%. The so-obtained solution was used directly in the next coupling cycle (without any precipitation, filtration or purification steps in between).

**Table E-1: Overview of iterations 2 to 17 of the coupling cycle of Examples 1 and 2 (vide infra)**

| **number of the coupling cycle** | **starting material^{a)}** | **product^{b)}** |
|---|---|---|
| 2 | **5** | DMTr-mMeUs-MG(ib)s-MG(ib)-(Kc-C1-carbonate) **(6)** |
| 3 | **6** | DMTr-mMeC(bz)s-mMeUs-MG(ib)s-MG(ib)-(Kc-C1-carbonate) **(7)** |
| 4 | **7** | DMTr-MG(ib)s-mMeC(bz)s-mMeUs-MG(ib)s-MG(ib)-(Kc-C1-carbonate) **(8)** |
| 5 | **8** | DMTr-mMeUs-MG(ib)s-mMeC(bz)s-mMeUs-MG(ib)s-MG(ib)-(Kc-C1-carbonate) **(9)** |
| 6 | **9** | DMTr-MA(bz)s-mMeUs-MG(ib)s-mMeC(bz)s-mMeUs-MG(ib)s-MG(ib)-(Kc-C1-carbonate) **(10)** |
| 7 | **10** | DMTr-MA(bz)s-MA(bz)s-mMeUs-MG(ib)s-mMeC(bz)s-mMeUs-MG(ib)s-MG(ib)-(Kc-C1-carbonate) **(11)** |
| 8 | **11** | DMTr-mMeUs-MA(bz)s-MA(bz)s-mMeUs-MG(ib)s-mMeC(bz)s-mMeUs-MG(ib)s-MG(ib)-(Kc-C1-carbonate) **(12)** |
| 9 | **12** | DMTr-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(13)** |
| 10 | **13** | DMTr-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(14)** |
| 11 | **14** | DMTr-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(15)** |
| 12 | **15** | DMTr-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(16)** |
| 13 | **16** | DMTr-mMeUs-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(17)** |
| 14 | **17** | DMTr-mMeC(bz)s-mMeUs-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(18)** |
| 15 | **18** | DMTr-MA(bz)s-mMeC(bz)s-mMeUs-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(19)** |
| 16 | **19** | DMTr-mMeC(bz)s-MA(bz)s-mMeC(bz)s-mMeUs-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(20)** |
| 17 | **20** | DMTr-mMeUs-mMeC(bz)s-MA(bz)s-mMeC(bz)s-mMeUs-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(21)** |

| | | |
|---|---|---|
| a), b): the terms "starting material" and "product" are not to be construed to indicate any precipitation and/or isolation steps in between the coupling cycles. | | |

**Table E-2: Experimental detail for the 2^{nd} to the 17^{th} coupling cycle of Example 1 as listed in Table E-1**

| **number of coupling cycle** | **step according to the general protocol** | **experimental detail** |
|---|---|---|
| 2 | DMTr-deprotection | thiomalic acid (1.49 g, 9.92 mmol) and TFA (5.0 mL) were used |
| | aqueous extractions | addition of NOP (4.0 mL), NMM (15 mL), acetone (15 mL) and 15 wt-% brine (40 mL), separation of the organic phase and extracting the latter as follows: first with a mixture of 20 wt-% brine (10 mL), acetone (10 mL), 1 M NMM (10 mL) and NMM (3 mL), second with a mixture of 1 M NMM (20 mL) and NMM (1 mL), third with a mixture of 5 wt-% brine (20 mL), acetone (15 mL) and AcOH (0.8 mL), fourth with a mixture of water (15 mL) and acetone (5 mL), fifth with 0.5 N NaHCO₃ (20 mL), sixth with a mixture of water (15 mL), acetone (5 mL), AcOH (1 mL) and NMM (0.5 mL), and seventh with a mixture of water (15 mL) and acetone (5 mL). |
| | azeotropic distillation | Five times as follows: addition of MTHP (15 mL), then concentration *in vacuo* to a volume of 20 mL |
| | coupling | 1-methylimidazole (1.0 mL, 12.54 mmol), DMTr-mMeU phosphoramidite (CAS-RN: 163878-63-5, 1.10 g, 1.34 mmol) and BTT (1.28 g, 6.66 mmol) were used |
| | sulfurization (including quenching) | DDTT (408 mg, 1.99 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15 mL), then concentration *in vacuo* to a volume of 20 mL |
| 3 | DMTr-deprotection | thiomalic acid (1.00 g, 6.66 mmol) and TFA (5.0 mL) were used |
| | aqueous extractions | addition of NOP (4.0 mL), NMM (15 mL), acetone (15 mL) and 15 wt-% brine (40 mL), separation of the organic phase and extracting the latter as follows: first with a mixture of 20 wt-% brine (10 mL), 0.5 N NaHCO₃ aq. (10 mL) and acetone (10 mL), second with a mixture of 0.5 N NaHCO₃ aq. (20 mL), acetone (30 mL) and NMM (4 mL), third with a mixture of 0.5 N NaHCO₃ aq. (20 mL), fourth with a mixture of H₂O (15 mL), acetone (5 mL) and AcOH (0.3 mL), fifth with a mixture of H₂O (15 mL) and acetone (10 mL) |
| | azeotropic distillation | 4 times as follows: addition of MTHP (15 mL), then concentration *in vacuo* to a volume of 20 mL final water content: 289 ppm |
| | coupling | 1-methylimidazole (1.0 mL, 12.54 mmol), DMTr-mMeC(Bz) phosphoramidite (CAS-RN: 163759-94-2, 1.22 g, 1.32 mmol) and BTT (1.27 g, 6.61 mmol) were used |
| | sulfurization (including quenching) | DDTT (413 mg, 2.01 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15 mL), then concentration *in vacuo* to a volume of 20 mL |
| 4 | DMTr-deprotection | thiomalic acid (1.00 g, 6.66 mmol) and TFA (5.0 mL) were used |
| | aqueous extractions | addition of NOP (4.0 mL), NMM (15 mL), acetone (15 mL) and 15 wt-% brine (40 mL), separation of the organic phase and extracting the latter as follows: first with a mixture of 10 wt-% brine (20 mL) and acetone (10 mL), second with a mixture of 1 M NMM aq. (20 mL), third with a mixture of 1 M NMM aq. (20 mL) and acetone (10 mL), fourth with a mixture of H₂O (15 mL), acetone (5 mL) and AcOH (0.4 mL), fifth with a mixture of H₂O (15 mL) and acetone (5 mL) |
| | azeotropic distillation | 3 times as follows: addition of MTHP (15mL), then concentration *in vacuo* to a volume of 20 mL |
| | coupling | 1-methylimidazole (1.0 mL, 12.54 mmol), DMTr-MG(ib) phosphoramidite (CAS-RN: 251647-55-9, 1.86 g, 2.03 mmol) and BTT (1.29 g, 6.71 mmol) were used |
| | sulfurization (including quenching) | DDTT (543 mg, 2.64 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15mL), then concentration *in vacuo* to a volume of 20 mL H₂O (17.5 µL) added |
| 5 | DMTr-deprotection | thiomalic acid (1.48 g, 9.86 mmol) and TFA (5.0 mL) were used |
| | aqueous extractions | addition of NOP (4.0 mL), NMM (15 mL), acetone (15 mL) and 15 wt-% brine (40 mL), separation of the organic phase and extracting the latter as follows: first with a mixture of 10 wt-% brine (20 mL) and acetone (10 mL), second with1 M NMM aq. (20 mL), third with a mixture of 1 M NMM aq. (20 mL) and acetone (10 mL), fourth with a mixture of H₂O (15 mL), acetone (5 mL) and AcOH (0.4 mL), fifth with a mixture of H₂O (15 mL) and acetone (15 mL) |
| | azeotropic distillation | 3 times as follows: addition of MTHP (15mL), then concentration *in vacuo* to a volume of 20 mL |
| | coupling | 1-methylimidazole (1.0 mL, 12.54 mmol), DMTr-mMeU phosphoramidite (CAS-RN: 163878-63-5, 1.62 g, 1.98 mmol) and BTT (1.27 g, 6.61 mmol) were used |
| | sulfurization (including quenching) | DDTT (545 mg, 2.65 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15mL), then concentration *in vacuo* to a volume of 20 mL |
| 6 | DMTr-deprotection | thiomalic acid (1.49 g, 9.92 mmol) and TFA (5.0 mL) were used |
| | aqueous extractions | addition of NOP (5.0 mL), NMM (17 mL), acetone (15 mL) and 15 wt-% brine (40 mL), separation of the organic phase andextracting the latter as follows: first with a mixture of 10 wt-% brine (20 mL) and acetone (10 mL), second with1 M NMM aq. (15 mL), third with a mixture of 1 M NMM aq. (15 mL), fourth with a mixture of H₂O (15 mL), acetone (5 mL) and AcOH (0.3 mL), fifth with a mixture of H₂O (15 mL) and acetone (5 mL) |
| | azeotropic distillation | 3 times as follows: addition of MTHP (15mL), then concentration *in vacuo* to a volume of 20 mL |
| | coupling | 1-methylimidazole (1.0 mL, 12.54 mmol), DMTr-MA(bz) phosphoramidite (CAS-RN: 251647-53-7, 1.85 g, 1.98 mmol) and BTT (1.27 g, 6.61 mmol) were used |
| | sulfurization (including quenching) | DDTT (544 mg, 2.65 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15mL), then concentration *in vacuo* to a volume of 20 mL |
| 7 | DMTr-deprotection | thiomalic acid (1.49 g, 9.92 mmol) and TFA (5.0 mL) were used |
| | aqueous extractions | addition of NOP (5.0 mL), NMM (17 mL), acetone (15 mL) and 15 wt-% brine (40 mL), separation of the organic phase extracting the latter as follows: first with a mixture of 10 wt-% brine (20 mL) and acetone (10 mL), second with1 M NMM aq. (20 mL), third with a mixture of 1 M NMM aq. (20 mL) and acetone (10 mL), fourth with a mixture of H₂O (15 mL), acetone (5 mL) and AcOH (0.45 mL), fifth with a mixture of H₂O (15 mL) and acetone (5 mL) |
| | azeotropic distillation | 3 times as follows: addition of MTHP (15mL), then concentration *in vacuo* to a volume of 20 mL |
| | coupling | 1-methylimidazole (1.0 mL, 12.54 mmol), DMTr-MA(bz) phosphoramidite (CAS-RN: 251647-53-7, 1.85 g, 1.98 mmol) and BTT (1.27 g, 6.61 mmol) were used |
| | sulfurization (including quenching) | DDTT (544 mg, 2.65 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions azeotropic distillation | extracted twice: first with a mixture of 10 vol-% AcOH aq. (10 mL) and AcOH (1 mL), then with 10 vol-% AcOH aq. (10 mL) twice as follows: addition of MTHP (15mL), then concentration *in vacuo* to a volume of 20 mL |
| 8 | DMTr-deprotection | thiomalic acid (1.49 g, 9.92 mmol) and TFA (5.0 mL) were used |
| | aqueous extractions | addition of NOP (5.0 mL), NMM (18 mL), acetone (15 mL) and 15 wt-% brine (40 mL), separation of the organic phase and extracting the latter as follows: first with a mixture of 10 wt-% brine (20 mL) and acetone (10 mL), second with1 M NMM aq. (20 mL), third with a mixture of 1 M NMM aq. (20 mL) and acetone (10 mL), fourth with a mixture of H₂O (15 mL), acetone (5 mL) and AcOH (0.5 mL), fifth with a mixture of H₂O (15 mL) and acetone (5 mL) |
| | azeotropic distillation | 3 times as follows: addition of MTHP (15mL), then concentration *in vacuo* to a volume of 20 mL |
| | coupling | 1-methylimidazole (1.0 mL, 12.54 mmol), DMTr-mMeU phosphoramidite (CAS-RN: 163878-63-5, 1.65 g, 2.01 mmol) and BTT (1.29 g, 6.71 mmol) were used |
| | sulfurization (including quenching) | DDTT (549 mg, 2.67 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15mL), then concentration *in vacuo* to a volume of 20 mL. |
| 9 | DMTr-deprotection | thiomalic acid (1.48 g, 9.86 mmol) and TFA (5.0 mL) were used |
| | aqueous extractions | addition of NOP (5.0 mL), NMM (15 mL), acetone (15 mL) and 15 wt-% brine (40 mL), separation of the organic phase and extracting the latter as follows: first with a mixture of 10 wt-% brine (20 mL) and acetone (10 mL), second with1 M NMM aq. (20 mL), third with a mixture of 1 M NMM aq. (20 mL) and acetone (10 mL), fourth with a mixture of H₂O (15 mL), acetone (15 mL) and AcOH (0.5 mL), fifth with a mixture of H₂O (15 mL) and acetone (15 mL) |
| | azeotropic distillation | 5 times as follows: addition of MTHP (15mL), then concentration *in vacuo* to a volume of 20 mL |
| | coupling | 1-methylimidazole (1.0 mL, 12.54 mmol), DMTr-MA(Bz) phosphoramidite (CAS-RN: 251647-53-7, 1.84 g, 1.97 mmol) and BTT (2.58 g, 13.42 mmol) were used |
| | sulfurization (including quenching) | DDTT (543 mg, 2.64 mmol) and triethylphosphite (226 µL, 1.32 mmol) were used |
| | capping | Ac₂O (499 µL, 5.26 mmol) and pyridine (533 µL, 6.60 mmol) used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15mL), then concentration *in vacuo* to a volume of 20 mL |
| 10 | DMTr-deprotection | thiomalic acid (1.49 g, 9.92 mmol) and TFA (5.0 mL) were used |
| | aqueous extractions | addition of NOP (5.0 mL), NMM (17 mL), acetone (15 mL) and 15 wt-% brine (40 mL), separation of the organic phase and extracting the latter as follows: first with a mixture of 10 wt-% brine (20 mL) and acetone (10 mL), second with1 M NMM aq. (20 mL), third with a mixture of 1 M NMM aq. (20 mL) and acetone (15 mL), fourth with a mixture of H₂O (15 mL) and AcOH (0.5 mL), fifth with a mixture of H₂O (15 mL) and acetone (15 mL) |
| | azeotropic distillation | 3 times as follows: addition of MTHP (15mL), then concentration *in vacuo* to a volume of 20 mL |
| | coupling | 1-methylimidazole (1.0 mL, 12.54 mmol), DMTr-mMeC(Bz) phosphoramidite (CAS-RN: 163759-94-2, 1.83 g, 1.98 mmol) and BTT (1.83 g, 9.52 mmol) were used |
| | sulfurization (including quenching) | DDTT (542 mg, 2.64 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (15 mL), acetone (5 mL) and AcOH (1 mL), then with 10 vol-% AcOH aq. (10 mL) |
| | azeotropic distillation | twice as follows: addition of MTHP (15mL), then concentration *in vacuo* to a volume of 20 mL |
| 11 | DMTr-deprotection | thiomalic acid (1.49 g, 9.92 mmol) and TFA (5.0 mL) were used |
| | aqueous extractions | addition of NOP (5.0 mL), NMM (15 mL), acetone (15 mL) and 15 wt-% brine (40 mL), separation of the organic phase and extracting the latter as follows: first with a mixture of 10 wt-% brine (20 mL) and acetone (10 mL), second with1 M NMM aq. (20 mL), third with a mixture of 1 M NMM aq. (20 mL) and acetone (5 mL), fourth with a mixture of H₂O (15 mL) and AcOH (0.5 mL), fifth with a mixture of H₂O (15 mL) and acetone (7.5 mL) |
| | azeotropic distillation | 3 times as follows: addition of MTHP (15mL), then concentration *in vacuo* to a volume of 20 mL |
| | coupling | 1-methylimidazole (1.0 mL, 12.54 mmol), DMTr-mMeU phosphoramidite (CAS-RN: 163878-63-5, 3.79 g, 4.62 mmol) and BTT (2.54 g, 13.22 mmol) were used |
| | sulfurization (including quenching) | DDTT (1.09 g, 5.31 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (15 mL), acetone (5 mL) and AcOH (1 mL), then with 10 vol-% AcOH aq. (10 mL) |
| | azeotropic distillation | twice as follows: addition of MTHP (15) mL, then concentration *in vacuo* to a volume of 20 mL |
| 12 | DMTr-deprotection | thiomalic acid (1.49 g, 9.92 mmol) and TFA (5.0 mL) were used |
| | | |
| | aqueous extractions | addition of NOP (5.0 mL), NMM (17 mL), acetone (15 mL) and 15 wt-% brine (40 mL), separation of the organic phase and extracting the latter as follows: first with a mixture of 10 wt-% brine (20 mL) and acetone (10 mL), second with1 M NMM aq. (20 mL), third with a mixture of 1 M NMM aq. (20 mL) and acetone (10 mL), fourth with a mixture of 10 vol-% AcOH aq. (20 mL), acetone (10 mL) and NMM (2.5 mL), fifth with a mixture of H₂O (15 mL) and acetone (5 mL) |
| | azeotropic distillation | 3 times as follows: addition of MTHP (15mL), then concentration *in vacuo* to a volume of 20 mL |
| | coupling | 1-methylimidazole (1.0 mL, 12.54 mmol), DMTr-mMeU phosphoramidite (CAS-RN: 163878-63-5, 2.21 g, 2.70 mmol) and BTT (2.58 g, 13.42 mmol) were used |
| | sulfurization (including quenching) | DDTT (678 mg, 3.30 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15) mL, then concentration *in vacuo* to a volume of 20 mL |
| 13 | DMTr-deprotection | thiomalic acid (1.51 g, 10.06 mmol) and TFA (5.0 mL) were used |
| | aqueous extractions | addition of NOP (5.0 mL), NMM (15 mL), acetone (15 mL) and 15 wt-% brine (40 mL), separation of the organic phase and extracting the latter as follows: first with a mixture of 10 wt-% brine (20 mL) and acetone (10 mL), second with a mixture of 1 M NMM aq. (20 mL), 10 wt-% brine (5 mL), acetone (10 mL) and NOP (5 mL), third with a mixture of 1 M NMM aq. (20 mL), 10 wt-% brine (5 mL) and acetone (10 mL), fourth with a mixture of H₂O (15 mL), acetone (5 mL) and AcOH (0.5 mL), fifth with a mixture of 2.5 wt-% brine (20 mL) and acetone (10 mL) |
| | azeotropic distillation | 3 times as follows: addition of MTHP (15mL), then concentration *in vacuo* to a volume of 20 mL |
| | coupling | 1-methylimidazole (1.0 mL, 12.54 mmol), DMTr-mMeU phosphoramidite (CAS-RN: 163878-63-5, 2.71 g, 3.31 mmol) and BTT (1.27 g, 6.61 mmol) were used |
| | sulfurization (including quenching) | DDTT (815 mg, 3.97 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15) mL, then concentration *in vacuo* to a volume of 20 mL |
| 14 | DMTr-deprotection | thiomalic acid (1.49 g, 9.92 mmol) and TFA (5.0 mL) were used |
| | aqueous extractions | addition of NOP (5.0 mL), NMM (16 mL), acetone (15 mL) and 15 wt-% brine (40 mL), separation of the organic phase and extracting the latter as follows: first with a mixture of 10 wt-% brine (20 mL) and acetone (10 mL), second with a mixture of 1 M NMM aq. (20 mL), 10 wt-% brine (5 mL) and acetone (5 mL), third with a mixture of 1 M NMM aq. (20 mL) and acetone (10 mL), fourth with a mixture of H₂O (15 mL), acetone (5 mL) and AcOH (0.5 mL), fifth with a mixture of 2.5 wt-% brine (20 mL) and acetone (5 mL) |
| | azeotropic distillation | twice as follows: addition of MTHP (25mL), then concentration *in vacuo* to a volume of 20 mL |
| | coupling | 1-methylimidazole (1.0 mL, 12.54 mmol), DMTr-mMeC(Bz) phosphoramidite (CAS-RN: 163759-94-2, 1.84 g, 2.00 mmol) and BTT (2.57 g, 13.37 mmol) were used |
| | sulfurization (including quenching) | DDTT (545 mg, 2.65 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15mL), then concentration *in vacuo* to a volume of 20 mL |
| 15 | DMTr-deprotection | thiomalic acid (1.49 g, 9.92 mmol) and TFA (5.0 mL) were used |
| | aqueous extractions | addition of NOP (5.0 mL), NMM (18 mL), acetone (15 mL) and 15 wt-% brine (40 mL), separation of the organic phase and extracting the latter as follows: first with a mixture of 10 wt-% brine (20 mL) and acetone (10 mL), second with a mixture of 1 M NMM aq. (20 mL) and 10 wt-% brine (5 mL), third with a mixture of 1 M NMM aq. (20 mL), 10 wt-% brine (5 mL) and acetone (15 mL), fourth with a mixture of 2.5 wt-% brine (20 mL), acetone (5 mL) and AcOH (0.5 mL), fifth with a mixture of 2.5 wt-% brine (20 mL) and acetone (5 mL) |
| | azeotropic distillation | twice as follows: addition of MTHP (20mL), then concentration *in vacuo* to a volume of 20 mL |
| | coupling | 1-methylimidazole (1.0 mL, 12.54 mmol), DMTr-MA(Bz) phosphoramidite (CAS-RN: 251647-53-7, 3.14 g, 3.37 mmol) and BTT (2.55 g, 13.26 mmol) were used |
| | sulfurization (including quenching) | DDTT (820 mg, 3.99 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15mL), then concentration *in vacuo* to a volume of 20 mL |
| 16 | DMTr-deprotection | thiomalic acid (1.49 g, 9.92 mmol) and TFA (5.0 mL) were used |
| | | |
| | aqueous extractions | addition of NOP (5.0 mL), NMM (16 mL), acetone (15 mL) and 15 wt-% brine (40 mL), separation of the organic phase and extracting the latter as follows: first with a mixture of 10 wt-% brine (20 mL) and acetone (10 mL), second with a mixture of 1 M NMM aq. (20 mL), 10 wt-% brine (5 mL) and NOP (5 mL), third with a mixture of 1 M NMM aq. (20 mL), 10 wt-% brine (5 mL) and acetone (10 mL), fourth with a mixture of 2.5 wt-% brine (20 mL), acetone (5 mL) and AcOH (0.5 mL), fifth with a mixture of 2.5 wt-% brine (20 mL) and acetone (5 mL) |
| | azeotropic distillation | three times as follows: addition of MTHP (25mL), then concentration *in vacuo* to a volume of 20 mL |
| | coupling | 1-methylimidazole (1.0 mL, 12.54 mmol), DMTr-mMeC(Bz) phosphoramidite (CAS-RN: 163759-94-2, 3.05 g, 3.31 mmol) and BTT (1.27 g, 6.61 mmol) were used |
| | sulfurization (including quenching) | DDTT (817 mg, 3.98 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15mL), then concentration *in vacuo* to a volume of 20 mL |
| 17 | DMTr-deprotection | thiomalic acid (1.49 g, 9.92 mmol) and TFA (5.0 mL) were used |
| | aqueous extractions | addition of NOP (5.0 mL), NMM (17 mL), acetone (15 mL) and 15 wt-% brine (40 mL), separation of the organic phase and extracting the latter as follows: first with a mixture of 10 wt-% brine (20 mL) and acetone (10 mL), second with a mixture of 1 M NMM aq. (20 mL), 10 wt-% brine (5 mL) and NOP (5 mL), third with a mixture of 1 M NMM aq. (20 mL), 10 wt-% brine (5 mL) and acetone (10 mL), fourth with a mixture of 2.5 wt-% brine (20 mL) and acetone (5 mL), fifth with a mixture of 2.5 wt-% brine (20 mL), acetone (5 mL) and AcOH (0.5 mL) |
| | azeotropic distillation | twice as follows: addition of MTHP (20mL), then concentration *in vacuo* to a volume of 20 mL |
| | coupling | 1-methylimidazole (1.0 mL, 12.54 mmol), DMTr-mMeU phosphoramidite (CAS-RN: 163878-63-5, 3.24 g, 3.96 mmol) and BTT (1.27 g, 6.61 mmol) were used |
| | sulfurization (including quenching) | DDTT (945 mg, 4.60 mmol) and triethylphosphite (226 µL, 1.32 mmol) were used |
| | capping | Ac₂O (499 µL, 5.26 mmol) and pyridine (533 µL, 6.60 mmol) were used |
| | aqueous extractions | extracted twice: first with a mixture of 10 vol-% AcOH aq. (10 mL) and AcOH (1 mL), then with 10 vol-% AcOH aq. (10 mL) |
| | azeotropic distillation | twice as follows: addition of MTHP (15mL), then concentration *in vacuo* to a volume of 20 mL |

Thiomalic acid (1.46 g, 9.72 mmol) and TFA (5.0 mL) were added directly to the solution comprising compound **21** obtained from the preciding azeotropic distillation, followed by by stirring at rt until reaction monitoring by HPLC (Method B was used throughout Example 1) indicated completion of the DMTr-deprotection. N-Octyl-2-pyrrolidone (NOP, 5 mL), *N*-methylmorpholine (NMM, 16 mL), acetone (15 mL) and 15 wt-% aqueous (aq.) sodium chloride solution (brine, 20 mL) was added, followed by extraction. The organic phase was separated and extracted as follows: first with a mixture of 10 wt-% brine (20 mL) and acetone (10 mL), second with a mixture of 1 M NMM aq. (20 mL), 10 wt-% brine (5 mL) and NOP (5 mL), third with a mixture of 1 M NMM aq. (20 mL), 10 wt-% brine (5 mL) and acetone (10 mL), fourth with a mixture of 2.5 wt-% brine (20 mL), acetone (5 mL) and ACOH (0.5 mL), fifth with a mixture of 2.5 wt-% brine (20 mL) and acetone (5 mL). The organic phase obtained from the final aqueous extraction was concentrated *in vacuo,* followed by addition of diisopropylether (IPE, 50 mL). The resulting precipitate was filtered and washed twice with IPE (12.5 mL × 2) to give mMeUs-mMeC(bz)s-MA(bz)s-mMeC(bz)s-mMeUs-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(22,** 2.38 g, crude).

### Target oligonucleotide O^{T}-a

A solution of the crude of compound **22** (104 mg, 5.7 nmol) in a mixture of H₂O and tert-butylamine (1:1 v/v, 2 mL) was stirred at 80 °C for 5 h. Additional tert-butylamine (2.0 mL) was added to the mixture. After stirring at 80 °C for additional 5 h, the reaction mixture was cooled to room temperature and analyzed by HPLC-MS (Method C) to find 5-mMeUs-mMeCs-mAs-mMeCs-mMeUs-mMeUs-mMeUs-mMeCs-mAs-mMeUs-mAs-mAs-mMeUs-MGs-mMeCs-mMeUs-MGs-MG-3 (O^{T}-a). MS (Method C) m/z: [M - H]³⁻ calcd for C₂₃₄H₃₃₇N₆₁O₁₂₈P₁₇S₁₇³⁻, 2373.0844, found 2373.0848.

### Example 2: Synthesis B of target oligonucleotide O^{T}-a (see Table T-2)

### DMTr-MG(ib)s-MG(ib)-(Kc-C1-carbonate) (5)

DCA (5.0 mL) was added under stirring to an ice-cooled solution (5-10 °C) of DMTr-MG(ib)-(Kc-C1-carbonate) **(2,** 0.984 g, 0.657 mmol) and thiomalic acid (0.496g, 3.30 mmol) in MTHP (15 mL). The mixture was stirred at rt until reaction monitoring by HPLC (Method B was used throughout Example 2) indicated completion of the DMTr-deprotection. NMM (7 mL), 1 M NMM aq. (10 mL), acetone (2.5 mL), MTHP (5 mL) and 10 wt-% brine (5 mL) were added. The organic phase was separated and washed (i.e. extracted) three times: first with water/acetone solution (1:1 v/v, 15 mL), then water/acetone solution (2:1 v/v, 15 mL), and lastly with a mixture of 5 wt-% brine (20 mL), acetone (7.5 mL) and AcOH (0.1 mL). *N*-Octyl-2-pyrrolidone (NOP, 10 mL) was added to the so-obtained organic phase. The mixture was dehydrated azeotropically (bath temperature 35-55 °C, pressure 100 hPa; final water content: 132 ppm) *in vacuo* with MTHP to obtain a solution comprising MG(ib)-(Kc-C1-carbonate) **(4),** which was used directly in the subsequent step (without any precipitation, filtration or purification steps in between). 1-Methylimidazole (1.0 mL, 12.55 mmol), DMTr-MG(ib) phosphoramidite (CAS-RN: 251647-55-9, 1.23 g, 1.35 mmol) and BTT (1.26 g, 6.56 mmol) were added to said solution comprising MG(ib)-(Kc-C1-carbonate) **(4).** The mixture was stirred at rt, and consumption of the starting material was monitored by HPLC. Xanthane hydride (0.299 g, 1.99 mmol) was added. After stirring for 10 min at rt, triethylphosphite (0.171 mL, 1.00 mmol) was added. After stirring for 10 min at rt, Ac₂O (250 µL, 2.64 mmol) and pyridine (266 µL, 3.30 mmol) were added, followed by stirring at rt for 10 min.

### Second coupling cycle and further coupling cycles

All further iterations of the coupling cycle were performed according to the following general protocol and as described in detail in the following Table E-3. The starting materials and products of each coupling cycle are identical to those listed in Table E-1 above. Again, the terms "starting material" and "product" are not to be construed to indicate any precipitation and/or isolation steps in between the coupling cycles.

### General protocol for coupling cycles

In a typical coupling cycle, the following steps were performed in the presented order.

### DMTr-deprotection

2-PrOH, thiomalic acid, AcOH, TMSCI, and, if indicated, Ac₂O were added under ice-cooling at a temperature in the range of 5-10 °C directly to the solution obtained from the preceding capping step. The mixture was stirred at rt for 45 min.

### Aqueous extraction(s)

The reaction mixture obtained from carrying out the DMTr-deprotection was extracted with one or more aqueous solutions as indicated in Table E-3.

### Azeotropic distillation

MTHP (15-30 mL), and if indicated, BTT and/ or NOP was added to the organic phase obtained from the preceding aqueous extractions, followed by concentration *in vacuo* (bath temperature 35-55 °C, pressure 100 hPa). This MTHP addition and concentration step was typically repeated. The water content was adjusted to be in the range of 132-424 ppm. The composition of the solution obtained after azeotropic distillation was analyzed as laid out above for the 2^{nd} to 6^{th} and for the 8^{th} to 17^{th} coupling cycle. The content of NOP was in the range of 36.7-63.5 vol-% and the content of MTHP was in the range of 36.5-63.3 vol-%.

### Coupling reaction

The respective phosphoramidite building block (obtained commercially) and BTT were added and the mixture was stirred at rt until reaction monitoring by HPLC indicated completion of the coupling.

### Sulfurization (including quenching with triethylphosphite)

Xanthane hydride was added and the mixture was stirred for 10 min at rt. Triethylphosphite was added and the mixture was stirred for 10 min at rt.

### Capping

Ac₂O, pyridine, and 1-methylimidazole were added and the mixture was stirred for 10 min at rt.

**Table E-3: Experimental detail for the 2^{nd} to the 17^{th} coupling cycle as listed in Table E-1**

| **number of coupling cycle** | **step according to the general protocol** | **experimental detail** |
|---|---|---|
| 2 | DMTr-deprotection | 2-PrOH (0.5 mL), thiomalic acid (2.97 g, 19.8 mmol) AcOH (4.0 mL) and TMSCI (5.0 mL) were used |
| | aqueous extractions | addition of 2-PrOH (5.0 mL), MTHP (7.5 mL), NMM (25 mL) and 10 wt-% brine (30 mL), separation of the organic phase and extracting the latter as follows: |
| | | 1^{st}: with 50% acetone aq. (v/v, 20 mL) |
| | | 2^{nd} and 3^{rd}: with a mixture of 10 wt-% brine (20 mL), acetone (15 mL) and MTHP (5 mL) |
| | azeotropic distillation | BTT (1.26 g, 6.57 mmol) and NOP (5 mL) were added. The solution was dehydrated azeotropically 5 times. |
| | coupling | 1-methylimidazole (1.0 mL, 12.54 mmol), DMTr-mMeU phosphoramidite (CAS-RN: 163878-63-5, 1.10 g, 1.64 g, 2.00 mmol) and BTT (1.23 g, 6.39 mmol) were used |
| | sulfurization (including quenching) | xanthane hydride (0.449 g, 2.99 mmol) and triethylphosphite (0.256 mL, 1.49 mmol) were used |
| | capping | Ac₂O (0.25 mL, 2.64 mmol) and pyridine (0.266 mL, 3.30 mmol) were used |
| 3 | DMTr-deprotection | 2-PrOH (1.0 mL), thiomalic acid (3.00 g, 20 mmol) |
| | | AcOH (4.0 mL) and TMSCI (5 mL) were used |
| | aqueous extractions | addition of 2-PrOH (5.0 mL), MTHP (7.5 mL), NMM (25 mL) and 10wt-% brine (30 mL), separation of the organic phase and extracting the latter as follows: |
| | | 1^{st}: with 50% acetone aq. (v/v, 20 mL) |
| | | 2^{nd}: with water (10 mL) |
| | | 3^{rd}, 4^{th}: with 50% acetone aq. (v/v, 20 mL) |
| | | 5^{th}: with a mixture of water (10 mL) and AcOH (0.3 mL) |
| | azeotropic distillation | BTT (1.26 g, 6.57 mmol) was added. The solution was dehydrated azeotropically 5 times. |
| | coupling | 1-methylimidazole (1.0 mL, 12.54 mmol), DMTr-mMeC(Bz) phosphoramidite (CAS-RN: 163759-94-2, 1.26 g, 1.51 mmol) and BTT (1.25 g, 6.50 mmol) were used |
| | sulfurization (including quenching) | xanthane hydride (0.300 g, 1.99 mmol) and triethylphosphite (0.171 mL, 1.00 mmol) were used |
| | capping | Ac₂O (0.25 mL, 2.64 mmol) and pyridine (0.266 mL, 3.30 mmol) were used |
| 4 | DMTr-deprotection | 2-PrOH (1.0 mL), thiomalic acid (2.97 g, 19.8 mmol) |
| | | AcOH (4.0 mL) and TMSCI (5 mL) were used |
| | aqueous extractions | additions of 2-PrOH (5.0 mL), MTHP (7.5 mL), NMM (25 mL), NOP (2 mL) and 10wt-% brine (30 mL), separation of the organic phase and extracting the latter as follows: |
| | | 1^{st}: with 50% acetone aq. (v/v, 20 mL) |
| | | 2^{nd} to 4^{th}: with water (10 mL) |
| | azeotropic distillation | BTT (0.632 g, 3.30 mmol) was added. The solution was dehydrated azeotropically 5 times. |
| | coupling | 1-methylimidazole (1.0 mL, 12.54 mmol), DMTr-MG(ib) phosphoramidite (CAS-RN: 251647-55-9, 1.84 g, 2.19 mmol) and BTT (1.92 g, 9.99 mmol) were used |
| | sulfurization (including quenching) | xanthane hydride (0.445 g, 2.96 mmol) and triethylphosphite (0.256 mL, 1.49 mmol) were used |
| | capping | Ac₂O (0.25 mL, 2.64 mmol) and pyridine (0.266 mL, 3.30 mmol) were used |
| 5 | DMTr-deprotection | 2-PrOH (0.5 mL), thiomalic acid (2.98 g, 19.9 mmol) AcOH (4.0 mL), Ac₂O (0.5 mL) and TMSCI (5 mL) were used |
| | aqueous extractions | addition of 2-PrOH (5.0 mL), MTHP (7.5 mL), NMM (25 mL), and 10wt-% brine (50 mL), separation of the organic phase and extracting the latter as follows. |
| | | 1^{st}: with 50% acetone aq. (v/v, 20 mL) |
| | | 2^{nd}: with water (10 mL) |
| | | 3^{rd}: with a mixture of water (10 mL), MTHP (2.5 vol) and acetone (10 mL) |
| | | 4^{th}: with a mixture of water (10 mL), AcOH (0.25 mL) |
| | azeotropic distillation | BTT (1.26 g, 6.58 mmol) was added. The solution was dehydrated azeotropically 4 times. |
| | coupling | 1-methylimidazole (1.0 mL, 12.54 mmol), DMTr-mMeU phosphoramidite (CAS-RN: 163878-63-5, 1.19 g, 1.45 mmol) and BTT (1.26 g, 6.53 mmol) were used |
| | sulfurization (including quenching) | xanthane hydride (0.304 g, 2.03 mmol) and triethylphosphite (0.171 mL, 1.00 mmol) were used |
| | capping | Ac₂O (0.25 mL, 2.64 mmol) and pyridine (0.266 mL, 3.30 mmol) were used |
| 6 | DMTr-deprotection | 2-PrOH (0.5 mL), thiomalic acid (3.00 g, 20 mmol) |
| | | AcOH (4.0 mL), Ac₂O (0.5 mL) and TMSCI (5 mL) were used |
| | aqueous extractions | addition of 2-PrOH (5.0 mL), MTHP (7.5 mL), NMM (26 mL), and 10wt-% brine (30 mL), separation of the organic phase and extracting the latter as follows. |
| | | 1^{st}, 2^{nd}: with 50% acetone aq. (v/v, 20 mL) |
| | | 3^{rd}: with a mixture of water (10 mL) and AcOH (0.55 mL) |
| | | 4^{th}: with water (10 mL) |
| | azeotropic distillation | NOP (0.5 mL) and BTT (1.29 g, 6.73 mmol) were added. The solution was dehydrated azeotropically 4 times. |
| | coupling | 1-methylimidazole (1.0 mL, 12.54 mmol), DMTr-MA(Bz) phosphoramidite (251647-53-7, 2.503 g, 2.68 mmol) and BTT (1.25 g, 6.50 mmol) were used |
| | sulfurization (including quenching) | xanthane hydride (0.598 g, 3.98 mmol) and triethylphosphite (0.342 mL, 1.99 mmol) were used |
| | capping | Ac₂O (0.25 mL, 2.64 mmol) and pyridine (0.266 mL, 3.30 mmol) were used |
| 7 | DMTr-deprotection | 2-PrOH (0.5 mL), thiomalic acid (3.48 g, 23.2 mmol) AcOH (4.0 mL), Ac₂O (0.5 mL) and TMSCI (5 mL) were used |
| | aqueous extractions | addition of 2-PrOH (5.0 mL), MTHP (7.5 mL), NMM (26 mL), and 10wt-% brine (30 mL), separation of the organic phase and extracting the latter as follows. |
| | | 1^{st}: with 50% acetone aq. (v/v, 20 mL) |
| | | 2^{nd}: with a mixture of 50% acetone aq. (v/v, 20 mL) and acetone (5 mL) |
| | | 3^{rd}: with a mixture of water (10 mL) and AcOH (0.4 mL) |
| | | 4^{th}: with water (10 mL) |
| | azeotropic distillation | NOP (1 mL) and BTT (1.26 g, 6.55 mmol) were added. The solution was dehydrated azeotropically 3 times. |
| | coupling | 1-methylimidazole (1.0 mL, 12.54 mmol), DMTr-MA(Bz) phosphoramidite (CAS-RN: 251647-53-7, 2.54 g, 2.72 mmol) and BTT (1.28 g, 6.66 mmol) were used |
| | sulfurization (including quenching) | xanthane hydride (0.597 g, 3.97 mmol) and triethylphosphite (0.342 mL, 1.99 mmol) wee used |
| | capping | Ac₂O (0.25 mL, 2.64 mmol) and pyridine (0.266 mL, 3.30 mmol) were used |
| 8 | DMTr-deprotection | 2-PrOH (0.5 mL), thiomalic acid (3.44 g, 22.9 mmol) |
| | | AcOH (4.0 mL), Ac₂O (0.5 mL) and TMSCI (5 mL) were used |
| | aqueous extractions | addition of 2-PrOH (5.0 mL), MTHP (7.5 mL), NMM (26 mL), and 10wt-% brine (30 mL), separation of the organic phase and extracting the lattr as follows: |
| | | 1^{st}: with 50% acetone aq. (v/v, 20 mL) |
| | | 2^{nd}: with 50% acetone aq. (v/v, 20 mL) |
| | | 3^{rd}: with a mixture of 50% acetone in 1M NMM. (v/v, 20 mL) and water (20 mL) |
| | | 4^{th}: with a mixture of 5 wt-% brine (15 mL), acetone (10 mL) and AcOH (0.5 mL) |
| | | 5^{th}: with water (10 mL) |
| | azeotropic distillation | NOP (1 mL) and BTT (1.25 g, 6.51 mmol) were added. The solution was dehydrated azeotropically 4 times. |
| | coupling | 1-methylimidazole (1.0 mL, 12.54 mmol), DMTr-mMeU phosphoramidite (CAS-RN: 163878-63-5, 2.19 g, 2.68 mmol) and BTT (1.23 g, 6.38 mmol) were used |
| | sulfurization (including quenching) | xanthane hydride (0.597 g, 3.98 mmol) and triethylphosphite (0.342 mL, 1.99 mmol) were used |
| | capping | Ac₂O (0.25 mL, 2.64 mmol) and pyridine (0.266 mL, 3.30 mmol) were used |
| 9 | DMTr-deprotection | 2-PrOH (0.5 mL), thiomalic acid (3.02 g, 20.1 mmol) |
| | | AcOH (4.0 mL), Ac₂O (0.5 mL) and TMSCI (5 mL) were used |
| | aqueous extractions | addition of 2-PrOH (5.0 mL), MTHP (7.5 mL), NMM (26 mL), and 10 wt-% brine (30 mL), separation of the organic phase and extracting the latter as follows: |
| | | 1^{st},2^{nd}: with 50% acetone aq. (v/v, 20 mL) |
| | | 3^{rd}: with a mixture of 50% acetone in 0.5 M NMM (v/v, 20 mL) and MTHP (7.5 mL) |
| | | 4^{th}: with a mixture of water (10 mL) and AcOH (0.45 mL) |
| | | 5^{th}: with water (10 mL) |
| | azeotropic distillation | NOP (2 mL) and BTT (1.27 g, 6.59 mmol) were added. The solution was dehydrated azeotropically 4 times. |
| | coupling | 1-methylimidazole (1.0 mL, 12.54 mmol), DMTr-MA(Bz) phosphoramidite (CAS-RN: 251647-53-7, 2.45 g, 2.63 mmol) and BTT (1.25 g, 6.50 mmol) were used |
| | sulfurization (including quenching) | xanthane hydride (0.601 g, 4.00 mmol) and triethylphosphite (0.342 mL, 1.99 mmol) were used |
| | capping | Ac₂O (0.25 mL, 2.64 mmol) and pyridine (0.266 mL, 3.30 mmol) were used |
| 10 | DMTr-deprotection | 2-PrOH (0.5 mL), thiomalic acid (3.000 g, 20.0 mmol), AcOH (4.0 mL), Ac₂O (0.5 mL) and TMSCI (5 mL) were used |
| | aqueous extractions | addition of 2-PrOH (5.0 mL), MTHP (7.5 mL), NMM (25 mL), acetone (5 mL) and 10wt-% brine (30 mL), separation of the organic phase and extracting the latter as follows: |
| | | 1^{st}: with a mixture of 50 % acetone aq. (v/v, 20 mL) and acetone (5 mL) |
| | | 2^{nd}: with 50 % acetone in 0.5 M NMM (v/v, 20 mL) |
| | | 3^{rd}: with 50 % acetone aq. (v/v, 20 mL) |
| | | 4^{th}: with a mixture of water (10 mL) and AcOH (0.4 mL) |
| | | 5^{th}: with water (10 mL) |
| | azeotropic distillation | NOP (1.2 mL) and BTT (1.28 g, 6.63 mmol) were added. The solution was dehydrated azeotropically 4 times. |
| | sulfurization (including quenching) | 1-methylimidazole (1.0 mL, 12.54 mmol), DMTr-mMeC(Bz) phosphoramidite (CAS-RN: 163759-94-2, 1.83 g, 1.99 mmol) and BTT (1.25 g, 6.37 mmol) were used xanthane hydride (0.447 g, 2.98 mmol) and triethylphosphite (0.257 mL, 1.50 mmol) were used |
| | capping | Ac₂O (0.25 mL, 2.64 mmol) and pyridine (0.266 mL, 3.30 mmol) were used |
| 11 | DMTr-deprotection | 2-PrOH (0.5 mL), thiomalic acid (2.974 g, 19.8 mmol), AcOH (4.0 mL), Ac₂O (0.5 mL) and TMSCI (5 mL) were used |
| | aqueous extractions | addition of 2-PrOH (5.0 mL), MTHP (7.5 mL), NMM (25 mL), NOP (3 mL) and 10wt-% brine (30 mL), separation of the organic phase and extracting the latter as follows. |
| | | 1^{st}, 2^{nd}: with 50% acetone in 0.5 M NMM (v/v, 20 mL) |
| | | 3^{rd}: with 50% acetone aq. (v/v, 20 mL) |
| | | 4^{th}: with a mixture of water (10 mL) and AcOH (0.35 mL) |
| | | 5^{th}: with water (10 mL) |
| | azeotropic distillation | BTT (1.27 g, 6.58 mmol) was added. The solution was dehydrated azeotropically 4 times. |
| | coupling | 1-methylimidazole (1.0 mL, 12.54 mmol), DMTr-mMeU phosphoramidite (CAS-RN: 163878-63-5, 1.59 g, 1.95 mmol) and BTT (1.25 g, 6.50 mmol) were used |
| | sulfurization (including quenching) | xanthane hydride (0.444 g, 2.95 mmol) and triethylphosphite (0.257 mL, 1.50 mmol) were used |
| | capping | Ac₂O (0.25 mL, 2.64 mmol) and pyridine (0.266 mL, 3.30 mmol) were used |
| 12 | DMTr-deprotection | 2-PrOH (0.5 mL), thiomalic acid (2.99 g, 19.9 mmol) |
| | | AcOH (4.0 mL), Ac₂O (0.5 mL) and TMSCI (5 mL) were used |
| | aqueous extractions | addition of 2-PrOH (5.0 mL), MTHP (7.5 mL), NMM (25 mL), acetone (5 mL) and 10wt-% brine (30 mL), separation of the organic phase and extracting the latter as follows: |
| | | 1^{st}: with MTHP (7.5 mL), 50 % acetone in 0.5 M NMM (v/v, 20 mL) |
| | | 2^{nd}: with 50% acetone aq. (v/v, 20 mL) |
| | | 3^{rd}: with a mixture of 50 % acetone aq. (v/v, 20 mL) and acetone (5 mL) |
| | | 4^{th}: with a mixture of water (10 mL) and AcOH (0.38 mL) |
| | | 5^{th}: with a mixture of water (10 mL) and NOP (3 mL) |
| | azeotropic distillation | BTT (1.27 g, 6.60 mmol) was added. The solution was dehydrated azeotropically 4 times. |
| | coupling | 1-methylimidazole (1.0 mL, 12.54 mmol), DMTr-mMeU phosphoramidite (CAS-RN: 163878-63-5, 1.64 g, 2.01 mmol) and BTT (1.27 g, 6.60 mmol) were used |
| | sulfurization (including quenching) | xanthane hydride (0.455 g, 3.03 mmol) and triethylphosphite (0.257 mL, 1.50 mmol) were used |
| | capping | Ac₂O (0.25 mL, 2.64 mmol) and pyridine (0.266 mL, 3.30 mmol) were used |
| 13 | DMTr-deprotection | 2-PrOH (0.5 mL), thiomalic acid (2.96 g, 19.7 mmol) |
| | | AcOH (4.0 mL), Ac₂O (0.5 mL) and TMSCI (5 mL) were used |
| | aqueous extractions | addition of 2-PrOH (5.0 mL), MTHP (7.5 mL), NMM (25 mL) and 10wt-% brine (30 mL), separation of the organic phase and extracting the latter as follows. |
| | | 1^{st}: with 50% acetone in 1 M NMM. (v/v, 20 mL) |
| | | 2^{nd}: with a mixture of 50 % acetone in 1 M NMM (v/v, 20 mL), acetone (5 mL) and MTHP (7.5 mL) |
| | | 3^{rd}: with a mixture of 50 % acetone aq. (v/v, 20 mL) and AcOH (0.45 mL) |
| | | 4^{th}: with water (10 mL) |
| | azeotropic distillation | NOP (3 mL) and BTT (2.54 g, 13.2 mmol) were added. The solution was dehydrated azeotropically 4 times. |
| | coupling | 1-methylimidazole (1.0 mL, 12.54 mmol), DMTr-mMeU phosphoramidite (CAS-RN: 163878-63-51.62 g, 1.98 mmol) was used |
| | sulfurization (including quenching) | xanthane hydride (0.445 g, 2.99 mmol) and triethylphosphite (0.257 mL, 1.50 mmol) were used |
| | capping | Ac₂O (0.25 mL, 2.64 mmol) and pyridine (0.266 mL, 3.30 mmol) were used |
| 14 | DMTr-deprotection | 2-PrOH (0.5 mL), thiomalic acid (2.97 g, 19.8 mmol) |
| | | AcOH (4.0 mL), Ac₂O (0.5 mL) and TMSCI (5 mL) were used |
| | aqueous extractions | addition of 2-PrOH (5.0 mL), MTHP (7.5 mL), NMM (25 mL) and 10wt-% brine (30 mL), separation of the organic phase and extracting the latter as follows: |
| | | 1^{st}: with 50 % acetone in 1 M NMM. (v/v, 20 mL) |
| | | 2^{nd}: with a mixture of 50 % acetone in 1M NMM (v/v, 20 mL), acetone (5 mL) and MTHP (7.5 mL) |
| | | 3^{rd}: with 50 % acetone aq. (v/v, 20 mL) |
| | | 4^{th}: with a mixture of 50 % acetone aq. (v/v, 20 mL) and AcOH (0.5 mL) |
| | | 5^{th}: with water (10 mL) |
| | azeotropic distillation | NOP (3 mL) and BTT (1.27 g, 6.61 mmol) were added. The solution was dehydrated azeotropically 4 times. |
| | coupling | 1-methylimidazole (1.0 mL, 12.54 mmol), DMTr-mMeC(Bz) phosphoramidite (CAS-RN: 163759-94-2, 1.83 g, 1.99 mmol) and BTT (1.27 g, 6.62 mmol) were used |
| | sulfurization (including quenching) | xanthane hydride (0.446 g, 2.97 mmol) and triethylphosphite (0.257 mL, 1.50 mmol) were used |
| | capping | Ac₂O (0.25 mL, 2.64 mmol) and pyridine (0.266 mL, 3.30 mmol) were used |
| 15 | DMTr-deprotection | 2-PrOH (0.5 mL), thiomalic acid (2.94 g, 19.6 mmol) AcOH (4.0 mL), Ac₂O (0.5 mL) and TMSCI (5 mL) were used |
| | aqueous extractions | addition of 2-PrOH (5.0 mL), MTHP (7.5 mL), NMM (25 mL), acetone (7.5 mL) and 10wt-% brine (30 mL), separation of the organic phase and extracting the latter as follows: |
| | | 1^{st},2^{nd}: with 50 % acetone in 1 M NMM. (v/v, 20 mL) |
| | | 3^{rd}: with a mixture of 50 % acetone aq. (v/v, 20 mL) and MTHP (7.5 mL) |
| | | 4^{th}: with a mixture of water (10 mL) and AcOH (0.5 mL) |
| | | 5^{th}: with a mixture of 50 % acetone aq. (v/v, 20 mL) and NOP (3 mL) |
| | azeotropic distillation | BTT (1.25 g, 6.48 mmol) was added. The solution was dehydrated azeotropically 4 times. |
| | coupling sulfurization (including quenching) | 1-methylimidazole (1.0 mL, 12.54 mmol), DMTr-MA(Bz) phosphoramidite (CAS-RN: 251647-53-7, 1.85 g, 1.99 mmol) and BTT (1.89 g, 9.86 mmol) were used xanthane hydride (0.448 g, 2.98 mmol) and triethylphosphite (0.257 mL, 1.50 mmol) were used |
| | capping | Ac₂O (0.25 mL, 2.64 mmol) and pyridine (0.266 mL, 3.30 mmol) were used |
| 16 | DMTr-deprotection | 2-PrOH (0.5 mL), thiomalic acid (2.94 g, 19.6 mmol) |
| | | AcOH (4.0 mL), Ac₂O (0.5 mL) and TMSCI (5 mL) were used |
| | aqueous extractions | addition of 2-PrOH (5.0 mL), MTHP (7.5 mL), NMM (25 mL), acetone (7.5 mL) and 10 wt-% brine (30 mL), separation of the organic phase and extracting the latter as follows. |
| | | 1^{st}: with 50 % acetone in 1 M NMM. (v/v, 20 mL) |
| | | 2^{nd}: with 50 % acetone aq. (v/v, 20 mL) |
| | | 3^{rd}: with a mixture of 10 wt-% brine (25 mL) and acetone (10 mL) |
| | | 4^{th}: with a mixture of 7.5 wt-% brine (20 mL) and AcOH (0.5 mL) |
| | | 5^{th}: with a mixture of 10 wt-% brine (5 mL), acetone (10 mL) and MTHP (5 mL) |
| | azeotropic distillation | NOP (2 mL) and BTT (0.618 g, 3.21 mmol) were added. The solution was dehydrated azeotropically 4 times. |
| | coupling | 1-methylimidazole (1.0 mL, 12.54 mmol), DMTr-mMeC(Bz) phosphoramidite (CAS-RN: 163759-94-2, 1.89 g, 2.05 mmol) and BTT (1.92 g, 9.99 mmol) were used |
| | sulfurization (including quenching) | xanthane hydride (0.443 g, 2.95 mmol) and triethylphosphite (0.257 mL, 1.50 mmol) were used |
| | capping | Ac₂O (0.25 mL, 2.64 mmol) and pyridine (0.266 mL, 3.30 mmol) were used |
| 17 | DMTr-deprotection | 2-PrOH (0.5 mL), thiomalic acid (2.97 g, 19.8 mmol) |
| | | AcOH (4.0 mL), Ac₂O (0.5 mL) and TMSCI (5 mL) were used |
| | aqueous extractions | addition of 2-PrOH (5.0 mL), MTHP (7.5 mL), NMM (22 mL), acetone (7.5 mL) and 10wt-% brine (30 mL), separation of the organic phase and extracting the latter as follows: |
| | | 1^{st}: with a mixture of 15 wt-% brine (10 mL), 1 M NMM (10 mL) and acetone (10 mL) |
| | | 2^{nd}: with a mixture of 1 M NMM (20 mL) and acetone (10 mL) |
| | | 3^{rd}: with a mixture of 15 wt-% brine (5 mL), 1 M NMM (10 mL) and acetone (10 mL) |
| | | 4^{th}: with a mixture of 5 wt-% brine (15 mL), acetone (10 mL) and AcOH (0.5 mL) |
| | | 5^{th}: with 5 wt-% brine (15 mL) |
| | azeotropic distillation | NOP (2 mL) and BTT (1.24 g, 6.42 mmol) were added. The solution was dehydrated azeotropically 4 times. |
| | coupling | 1-methylimidazole (1.0 mL, 12.54 mmol), DMTr-mMeU phosphoramidite (CAS-RN: 163878-63-5, 1.66 g, 2.03 mmol) and BTT (1.27 g, 6.63 mmol) were used |
| | sulfurization (including quenching) | xanthane hydride (0.447 g, 2.97 mmol) and triethylphosphite (0.257 mL, 1.50 mmol) were used |
| | capping | Ac₂O (0.25 mL, 2.64 mmol) and pyridine (0.266 mL, 3.30 mmol) were used |

Isoprpyl alcohol (2-PrOH, 0.5 mL), thiomalic acid (2.99 g, 19.9 mmol), AcOH (4.0 mL), Ac₂O (0.5 mL) and TMSCI (5 mL) were added directly to the solution obtained from the preceding capping step, under ice-cooling at a temperature in the range of 5-10 °C, followed by stirring at rt for 45 min. 2-PrOH (5.0 mL), MTHP (22.5 mL), NMM (24 mL), acetone (7.5 mL) and 10 wt-% brine (30 mL) were added, followed by extraction and separation of the organic phase, and extracting the latter as follows: first with a mixture of 15 wt-% brine (10 mL) and 1 M NMM (10 mL) and acetone (10 mL), next with a mixture of 15 wt-% brine (5 mL), 1 M NMM and (20 mL), acetone (10 mL), next with a mixture of 15 wt-% brine (10 mL), 1 M NMM (10 mL) and acetone (10 mL), and lastly with a mixture of 5 wt-% brine (15 mL), acetone (10 mL) and AcOH (0.5 mL). The organic phase obtained from the final aqueous extraction was concentrated *in vacuo,* followed by addition of diisopropylether (IPE, 50 mL). The resulting precipitate was filtered and washed twice with IPE (50 mL × 2) and then with 2-PrOH (50 mL) to give mMeUs-mMeC(bz)s-MA(bz)s-mMeC(bz)s-mMeUs-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) (22, 5.39 g, crude).

### Target oligonucleotide O^{T}-a

A solution of the crude of compound **22** (0.249 g, 0.030 mmol) in H₂O and *tert-*butylamine (1:1 v/v, 7.5 mL) was stirred at 60 °C for 8h. Additional *tert*-butyelamine (0.75 mL) was added to the mixture and the solution was stirred at 80 °C for additional 7 h (monitored by HPLC with Method C, Gradient-1). The reaction mixture was cooled to room temperature and analyzed by HPLC-MS (Method C) to find 5-mMeUs-mMeCs-mAs-mMeCs-mMeUs-mMeUs-mMeUs-mMeCs-mAs-mMeUs-mAs-mAs-mMeUs-MGs-mMeCs-mMeUs-MGs-MG-3 (O^{T}-a). MS (Method C) m/z: [M - H]³⁻ calcd for C₂₃₄H₃₃₇N₆₁O₁₂₈P₁₇S₁₇³⁻, 2373.0848, found 2373.0860.

### Example 3: Synthesis A of target oligonucleotide O^{T}-b (see Table T-2)

### DMTr-dG(ib)-dT-(Kc-C1-carbonate) (24)

Dichloroacetic acid (100 mL) was added to a stirred solution of DMTr-dT-(Kc-C1-carbonate) **(1,** 13.20 mmol) and thiomalic acid (9.96 g, 66.33 mmol) in cyclopentyl methyl ether (CPME, 300 mL) at rt and the mixture was stirred until reaction monitoring by HPLC (Method B) indicated completion of the DMTr-deprotection. Acetone (50 mL), 1 M *N*-methylmorpholine (NMM) aq. (200 mL) and NMM (100 mL) were added, followed by extraction. The organic phase was separated and washed (i.e. extracted) twice with water/acetone solution (1:1 v/v, 300 mL × 2). The organic phase was concentrated *in vacuo,* followed by addition of acetonitrile (300 mL). The resulting precipitate was filtrated and washed twice with acetonitrile (100 mL × 2) to give dT-(Kc-C-1-carbonate) **(23,** 12.56 g, purity 94.59 %, yield 92.7 %).

A solution of dT-(Kc-C1-carbonate) **(23,** 677 mg, 0.660 mmol) in CPME/*N*,*N-*dibutylformamide (DBF) (6:1 v/v, 35 mL) was concentrated *in vacuo* (bath temperature 35-55 °C, pressure 100 hPa) to a volume of 20 mL. The so-obtained solution comprising dT-(Kc-C1-carbonate) **(23)** was used directly in the subsequent step (without any precipitation, filtration or purification steps in between).

1-Methylimidazole (1.0 mL, 12.55 mmol), DMTr-dG(ib) phosphoramidite (CAS-RN: 93183-15-4, 1.11 g, 1.32 mmol) and BTT (637 mg, 3.31 mmol) were added to said solution comprising dT-(Kc-C1-carbonate) **(23),** and the mixture was stirred at rt until reaction monitoring by HPLC (Method B was used throughout Example 3) indicated completion of the coupling. Ac₂O (250 µL, 2.64 mmol) and pyridine (266 µL, 3.30 mmol) were added. After stirring for 10 min at rt, 1 M I₂ in CPME (1.45 mL, 1.45 mmol), H₂O (36 µL), and acetonitrile (MeCN, 1.5 mL) were added. After stirring for 10 min at rt, the reaction mixture was extracted twice: first with 0.5 N Na₂S₂O₃ aq. (10 mL), then with a mixture of 0.5 N Na₂S₂O₃ aq. (5 mL) and 1 M NMM aq. (1 mL). The so-obtained solution (organic phase) comprising DMTr-dG(ib)-dT-(Kc-C1-carbonate) **(24)** was used directly in the next step (without any precipitation, filtration or purification steps in between).

### DMTr-dA(bz)-dG(ib)-dT-(Kc-C1-carbonate) (26)

Thiomalic acid (0.991 g, 6.60 mmol) and dichloroacetic acid (DCA, 5.0 mL) were added to the so-obtained organic phase, followed by stirring at rt until reaction monitoring by HPLC indicated complete DMTr-deprotection. DBF (1.3 mL), 10 wt-% brine (12.5 mL), and NMM (10 mL) were added, followed by extraction. The organic phase was separated and extracted twice with 10 wt-% brine/*N*,*N-*dimethylformamide (DMF) solution (7:13 v/v, 25 mL × 2). CPME (15 mL) was added to the so-obtained organic phase comprising dG(ib)-dT-(Kc-C1-carbonate) **(25),** followed by concentration *in vacuo* to a volume of 20 mL (bath temperature 35-55 °C, pressure 100 hPa). The CPME (15 mL) addition and subsequent concentration was repeated three times (final volume: 20 mL). 1-Methylimidazole (1.0 mL, 12.55 mmol), DMTr-dA(bz) phosphoramidite (CAS-RN: 98796-53-3, 1.15 g, 1.32 mmol) and BTT (635 mg, 3.30 mmol) were added to said solution comprising dG(ib)-dT-(Kc-C1-carbonate) **(25),** and the mixture was stirred at rt until reaction monitoring by HPLC indicated completion of the coupling. Ac₂O (250 µL, 2.64 mmol) and pyridine (266 µL, 3.30 mmol) were added. After stirring for 10 min at rt, 1 M I₂ in CPME (1.45 mL, 1.45 mmol), H₂O (36 µL), and acetonitrile (MeCN, 1.5 mL) were added. After stirring for 10 min at rt, the reaction mixture was extracted twice: first with 0.5 N Na₂S₂O₃ aq. (10 mL), then with a mixture of 0.5 N Na₂S₂O₃ aq. (5 mL) and 1 M NMM aq. (1.25 mL). The so-obtained solution (organic phase) comprising DMTr-dA(bz)-dG(ib)-dT-(Kc-C1-carbonate) **(26)** was used directly in the next step (without any precipitation, filtration or purification steps in between).

### DMTr-dC(bz)-dA(bz)-dG(ib)-dT-(Kc-C1-carbonate) (28)

Thiomalic acid (0.992 g, 6.61 mmol) and dichloroacetic acid (DCA, 5.0 mL) were added to the so-obtained organic phase, followed by stirring at rt until reaction monitoring by HPLC indicated complete DMTr-deprotection. DBF (1.3 mL), 10 wt-% brine (12.5 mL), and NMM (11.2 mL) were added, followed by extraction. The organic phase was separated and extracted twice with 10 wt-% brine/DMF solution (7:13 v/v, 25 mL × 2). CPME (15 mL) was added to the so-obtained organic phase comprising dA(bz)-dG(ib)-dT-(Kc-C1-carbonate) **(27),** followed by concentration in *vacuo* to a volume of 20 mL (bath temperature 35-55 °C, pressure 100 hPa). The CPME (15 mL) addition and subsequent concentration was repeated three times (final volume: 20 mL). 1-Methylimidazole (1.0 mL, 12.55 mmol), DMTr-dC(bz) phosphoramidite (CAS-RN: 102212-98-6, 1.12 g, 1.32 mmol) and BTT (635 mg, 3.30 mmol) were added to the solution comprising dA(bz)-dG(ib)-dT-(Kc-C1-carbonate) **(27),** and the mixture was stirred at rt until reaction monitoring by HPLC indicated completion of the coupling. Ac₂O (250 µL, 2.64 mmol) and pyridine (266 µL, 3.30 mmol) were added. After stirring for 10 min at rt, 1 M I₂ in CPME (1.45 mL, 1.45 mmol), H₂O (36 µL), and acetonitrile (MeCN, 1.5 mL) were added. After stirring for 10 min at rt, the reaction mixture was extracted twice: first with 0.5 N Na₂S₂O₃ aq. (10 mL), then with a mixture of 0.5 N Na₂S₂O₃ aq. (5 mL) and 1 M NMM aq. (1.25 mL). The so-obtained solution (organic phase) comprising DMTr-dC(bz)-dA(bz)-dG(ib)-dT-(Kc-C1-carbonate) **(28)** was used directly in the next step (without any precipitation, filtration or purification steps in between).

### dT-dC(bz)-dA(bz)-dG(ib)-dT-(Kc-C1-carbonate) (O^{T}-b)

Thiomalic acid (0.992 g, 6.61 mmol) and dichloroacetic acid (DCA, 5.0 mL) were added to the so-obtained organic phase, followed by stirring at rt until reaction monitoring by HPLC indicated complete DMTr-deprotection. DBF (1.3 mL), 10 wt-% brine (12.5 mL), and NMM (11.6 mL) were added, followed by extraction. The organic phase was separated and extracted twice with 10 wt-% brine/DMF solution (7:13 v/v, 25 mL × 2). CPME (15 mL) was added to the so-obtained organic phase comprising dC(bz)-dA(bz)-dG(ib)-dT-(Kc-C1-carbonate) **(29),** followed by concentration *in vacuo* to a volume of 20 mL (bath temperature 35-55 °C, pressure 100 hPa). The CPME (15 mL) addition and subsequent concentration was repeated twice (final volume: 20 mL). 1-Methylimidazole (1.0 mL, 12.55 mmol), DMTr-dT phosphoramidite (CAS-RN: 98796-51-1, 1.01 g, 1.33 mmol) and BTT (636 mg, 3.31 mmol) were added to said solution comprising dC(bz)-dA(bz)-dG(ib)-dT-(Kc-C1-carbonate) **(29),** and the mixture was stirred at rt until reaction monitoring by HPLC indicated completion of the coupling. Ac₂O (250 µL, 2.64 mmol) and pyridine (266 µL, 3.30 mmol) were added. After stirring for 10 min at rt, 1 M I₂ in CPME (1.45 mL, 1.45 mmol), H₂O (36 µL), and MeCN (1.5 mL) were added. After stirring for 10 min at rt, the reaction mixture was extracted twice: first with 0.5 N Na₂S₂O₃ aq. (10 mL), then with a mixture of 0.5 N Na₂S₂O₃ aq. (5 mL) and 1 M NMM aq. (1.25 mL). The so-obtained solution (organic phase) comprising DMTr-dT-dC(bz)-dA(bz)-dG(ib)-dT-(Kc-C1-carbonate) **(30)** was used directly in the next step (without any precipitation, filtration or purification steps in between).

Thiomalic acid (0.992 g, 6.61 mmol) and dichloroacetic acid (DCA, 5.0 mL) were added to the so-obtained organic phase, followed by stirring at rt until reaction monitoring by HPLC indicated complete DMTr-deprotection. DBF (1.3 mL), 10 wt-% brine (12.5 mL), and NMM (12.0 mL) were added, followed by extraction. The organic phase was separated and extracted twice with 10 wt-% brine/DMF solution (7:13 v/v, 25 mL × 2). The so-obtained organic phase comprising dT-dC(bz)-dA(bz)-dG(ib)-dT-(Kc-C1-carbonate) (O^{T}-b) was concentrated in vacuo, followed by addition of MeOH (25 mL). The resulting precipitate was filtered and washed twice with MeOH (12.5 mL × 2) to give dT-dC(bz)-dA(bz)-dG(ib)-dT-(Kc-C1-carbonate) (O^{T}-b, 1.06 g, purity 83.152 %, yield 58.6 %) as a white solid. MS (method A) m/z: [M + 2H]²⁺ calcd for C₁₃₁H₁₈₅N₂₁O₃₆P₄⁺, 1376.1115; found 1376.1157.

### Example 4: Synthesis B of target oligonucleotide O^{T}-b (see Table T-2)

### DMTr-dG(ib)-dT-(Kc-C1-carbonate) (24)

dT-(Kc-C1-carbonate) **(23)** was synthesized as described for Example 3 above.

A solution of dT-(Kc-C1-carbonate) **(23,** 677 mg, 0.660 mmol) in 4-methyltetrahydropyran (MTHP)/*N*,*N*-dibutylformamide (DBF) (6:1 v/v, 35 mL) was concentrated *in vacuo* to a volume of 15 mL (bath temperature 35-55 °C, pressure 100 hPa). 1-Methylimidazole (1.0 mL, 12.55 mmol), DMTr-dG(ib) phosphoramidite (CAS-RN: 93183-15-4, 1.11 g, 1.32 mmol) and BTT (637 mg, 3.31 mmol) were added to said solution comprising dT-(Kc-C1-carbonate) **(23),** and the mixture was stirred at rt until reaction monitoring by HPLC (Method B was used throughout Example 4) indicated completion of the coupling. Ac₂O (250 µL, 2.64 mmol) and pyridine (266 µL, 3.30 mmol) were added. After stirring for 10 min at rt, 1 M I₂ in MTHP (1.45 mL, 1.45 mmol), H₂O (36 µL), and acetonitrile (MeCN, 1.5 mL) were added. After stirring for 10 min at rt, the reaction mixture was extracted twice: first with 0.5 N Na₂S₂O₃ aq. (10 mL), then with a mixture of 0.5 N Na₂S₂O₃ aq. (5 mL) and 1 M NMM aq. (1.3 mL). The so-obtained solution (organic phase) comprising DMTr-dG(ib)-dT-(Kc-C1-carbonate) **(24)** was used directly in the next step (without any precipitation, filtration or purification steps in between).

### DMTr-dA(bz)-dG(ib)-dT-(Kc-C1-carbonate) (26)

Thiomalic acid (0.991 g, 6.60 mmol) and dichloroacetic acid (DCA, 5.0 mL) were added to the so-obtained organic phase, followed by stirring at rt until reaction monitoring by HPLC indicated complete DMTr-deprotection. DBF (1.3 mL), 10 wt-% brine (12.5 mL), and NMM (10 mL) were added, followed by extraction. The organic phase was separated and extracted twice with 10 wt-% brine/*N*,*N-*dimethylformamide (DMF) solution (2:3 v/v, 22.5 mL × 2). MTHP (15 mL) was added to the so-obtained organic phase comprising dG(ib)-dT-(Kc-C1-carbonate) **(25),** followed by concentration *in vacuo* to a volume of 15 mL (bath temperature 35-55 °C, pressure 100 hPa). The MTHP (15 mL) addition and subsequent concentration was repeated three times (final volume: 15 mL). 1-Methylimidazole (1.0 mL, 12.55 mmol), DMTr-dA(bz) phosphoramidite (CAS-RN: 98796-53-3, 1.15 g, 1.32 mmol) and BTT (636 mg, 3.30 mmol) were added to said solution comprising dG(ib)-dT-(Kc-C1-carbonate) **(25),** and the mixture was stirred at rt until reaction monitoring by HPLC indicated completion of the coupling. Ac₂O (250 µL, 2.64 mmol) and pyridine (266 µL, 3.30 mmol) were added. After stirring for 10 min at rt, 1 M I₂ in MTHP (1.45 mL, 1.45 mmol), H₂O (36 µL), and acetonitrile (MeCN, 1.5 mL) were added. After stirring for 10 min at rt, the reaction mixture was extracted twice: first with 0.5 N Na₂S₂O₃ aq. (10 mL), then with a mixture of 0.5 N Na₂S₂O₃ aq. (5 mL) and 1 M NMM aq. (1.25 mL). MTHP (15 mL) was added to the organic phase comprising DMTr-dA(bz)-dG(ib)-dT-(Kc-C1-carbonate) **(26),** followed by concentration *in vacuo* to a volume of 15 mL (bath temperature 35-55 °C, pressure 100 hPa). This MTHP (15 mL) addition and subsequent concentration was repeated once (final volume: 15 mL). The so-obtained solution (organic phase) comprising DMTr-dA(bz)-dG(ib)-dT-(Kc-C1-carbonate) **(26)** was used directly in the next step (without any precipitation, filtration or purification steps in between).

### DMTr-dC(bz)-dA(bz)-dG(ib)-dT-(Kc-C1-carbonate) (28)

Thiomalic acid (0.992 g, 6.61 mmol) and dichloroacetic acid (DCA, 7.5 mL) were added to the so-obtained organic phase, followed by stirring at rt until reaction monitoring by HPLC indicated complete DMTr-deprotection. DBF (1.3 mL), 10 wt-% brine (12.5 mL), and NMM (12 mL) were added, followed by extraction. The organic phase was separated and extracted twice with 10 wt-% brine/DMF solution (2:3 v/v, 22.5 mL × 2). MTHP (15 mL) was added to the so-obtained organic phase comprising dA(bz)-dG(ib)-dT-(Kc-C1-carbonate) **(27),** followed by concentration in *vacuo* to a volume of 15 mL (bath temperature 35-55 °C, pressure 100 hPa). The MTHP (15 mL) addition and subsequent concentration was repeated three times (final volume: 15 mL). 1-Methylimidazole (1.0 mL, 12.55 mmol), DMTr-dC(bz) phosphoramidite (CAS-RN: 102212-98-6, 1.12 g, 1.32 mmol) and BTT (636 mg, 3.30 mmol) were added to said solution comprising dA(bz)-dG(ib)-dT-(Kc-C1-carbonate) **(27),** and the mixture was stirred at rt until reaction monitoring by HPLC indicated completion of the coupling. Ac₂O (250 µL, 2.64 mmol) and pyridine (266 µL, 3.30 mmol) were added. After stirring for 10 min at rt, 1 M I₂ in MTHP (1.45 mL, 1.45 mmol), H₂O (36 µL), and acetonitrile (MeCN, 1.5 mL) were added. After stirring for 10 min at rt, the reaction mixture was extracted twice: first with 0.5 N Na₂S₂O₃ aq. (10 mL), then with a mixture of 0.5 N Na₂S₂O₃ aq. (5 mL) and 1 M NMM aq. (1.25 mL). The so-obtained solution (organic phase) comprising DMTr-dC(bz)-dA(bz)-dG(ib)-dT-(Kc-C1-carbonate) **(28)** was used directly in the next step (without any precipitation, filtration or purification steps in between).

### dT-dC(bz)-dA(bz)-dG(ib)-dT-(Kc-C1-carbonate) (O^{T}-b)

Thiomalic acid (0.992 g, 6.61 mmol) and dichloroacetic acid (DCA, 7.5 mL) were added to the so-obtained organic phase, followed by stirring at rt until reaction monitoring by HPLC indicated complete DMTr-deprotection. DBF (1.3 mL), 10 wt-% brine (12.5 mL), and NMM (12 mL) were added, followed by extraction. The organic phase was separated and extracted twice with 10 wt-% brine/DMF solution (2:3 v/v, 22.5 mL × 2). MTHP (15 mL) was added to the so-obtained organic phase comprising dC(bz)-dA(bz)-dG(ib)-dT-(Kc-C1-carbonate) **(29),** followed by concentration *in vacuo* to a volume of 15 mL (bath temperature 35-55 °C, pressure 100 hPa). The MTHP (15 mL) addition and subsequent concentration was repeated twice (final volume: 15 mL). 1-Methylimidazole (1.0 mL, 12.55 mmol), DMTr-dT phosphoramidite (CAS-RN: 98796-51-1, 1.01 g, 1.33 mmol) and BTT (636 mg, 3.31 mmol) were added to said solution comprising dC(bz)-dA(bz)-dG(ib)-dT-(Kc-C1-carbonate) **(29),** and the mixture was stirred at rt until reaction monitoring by HPLC indicated completion of the coupling. Ac₂O (250 µL, 2.64 mmol) and pyridine (266 µL, 3.30 mmol) were added. After stirring for 10 min at rt, 1 M I₂ in MTHP (1.45 mL, 1.45 mmol), H₂O (36 µL), and MeCN (1.5 mL) were added. After stirring for 10 min at rt, the reaction mixture was extracted twice: first with 0.5 N Na₂S₂O₃ aq. (10 mL), then with a mixture of 0.5 N Na₂S₂O₃ aq. (5 mL) and 1 M NMM aq. (1.25 mL). The so-obtained solution (organic phase) comprising DMTr-dT-dC(bz)-dA(bz)-dG(ib)-dT-(Kc-C1-carbonate) **(30)** was used directly in the next step (without any precipitation, filtration or purification steps in between).

Thiomalic acid (0.992 g, 6.61 mmol) and dichloroacetic acid (DCA, 7.5 mL) were added to the so-obtained organic phase, followed by stirring at rt until reaction monitoring by HPLC indicated complete DMTr-deprotection. DBF (1.3 mL), 10 wt-% brine (12.5 mL), and NMM (11.0 mL) were added, followed by extraction. The organic phase was separated and extracted twice with 10 wt-% brine/DMF solution (2:3 v/v, 22.5 mL × 2). The so-obtained organic phase comprising dT-dC(bz)-dA(bz)-dG(ib)-dT-(Kc-C1-carbonate) (O^{T}-b) was concentrated in vacuo, followed by addition of MeOH (25 mL). The resulting precipitate was filtered and washed twice with MeOH (12.5 mL × 2) to give dT-dC(bz)-dA(bz)-dG(ib)-dT-(Kc-C1-carbonate) (O^{T}-b, 989 mg, purity 79.699 %, yield 54.7 %) as a white solid. MS (method A) m/z: [M + 2H]²⁺ calcd for C₁₃₁H₁₈₅N₂₁O₃₆P₄⁺, 1376.1115; found 1376.1157.

### Example 5: Synthesis of target oligonucleotide O^{T}-c (see Table T-2)

### DMTr-dA(bz)s-dT-(Kc-C1-carbonate) (32)

dT-(Kc-C-1-carbonate) **(23)** was synthesized as described for Example 3 above.

A solution of dT-(Kc-C1-carbonate) **(23,** 676 mg, 0.659 mmol) in 4-methyltetrahydropyran (MTHP)/*N*-octyl-2-pyrrolidone (NOP) (5:1 v/v, 30 mL) was concentrated *in vacuo* (bath temperature 35-55 °C, pressure 100 hPa) to a volume of 15 mL. 1-Methylimidazole (1.0 mL, 12.55 mmol), DMTr-dA(bz) phosphoramidite (CAS-RN: 98796-53-3, 1.72 g, 1.97 mmol) and BTT (640 mg, 3.33 mmol) were added to the so-obtained solution comprising dT-(Kc-C1-carbonate) **(23),** and the mixture was stirred at rt until reaction monitoring by HPLC (Method B was used throughout Example 5) indicated completion of the coupling. 3-(*N*,*N*-dimethylamino-methylidene)amino)-3*H*-1,2,4-dithiazole-5-thione (CAS-RN: 1192027-04-5, DDTT, 544 mg, 2.65 mmol) was added, followed by stirring at rt for 10 min. Ac₂O (250 µL, 2.64 mmol) and pyridine (266 µL, 3.30 mmol) were added. After stirring for 10 min at rt, the reaction mixture was extracted twice with 10 vol-% AcOH aq. (10 mL × 2). The so-obtained solution (organic phase) comprising DMTr-dA(bz)s-dT-(Kc-C1-carbonate) **(32)** was used directly in the next step (without any precipitation, filtration or purification steps in between).

### DMTr-dC(bz)s-dA(bz)s-dT-(Kc-C1-carbonate) (34)

Thiomalic acid (1.01 g, 6.73 mmol) and dichloroacetic acid (DCA, 5.0 mL) were added to the so-obtained organic phase, followed by stirring at rt until reaction monitoring by HPLC indicated complete DMTr-deprotection. NOP (1.3 mL), 10 wt-% brine (12.5 mL), and NMM (10 mL) were added, followed by extraction. The organic phase was separated and extracted twice with a solution of acetone and water (1:1 v/v, 20 mL × 2). MTHP (15 mL) was added to the so-obtained organic phase comprising dA(bz)s-dT-(Kc-C1-carbonate) **(33),** followed by concentration in *vacuo* to a volume of 15 mL (bath temperature 35-55 °C, pressure 100 hPa). The MTHP (15 mL) addition and subsequent concentration was repeated once (final volume: 15 mL). 1-Methylimidazole (1.0 mL, 12.55 mmol), DMTr-dC(bz) phosphoramidite (CAS-RN: 102212-98-6, 1.62 g, 1.91 mmol) and BTT (632 mg, 3.29 mmol) were added to said solution comprising dA(bz)-dT-(Kc-C1-carbonate) **(33),** and the mixture was stirred at rt until reaction monitoring by HPLC indicated completion of the coupling. DDTT (544 mg, 2.65 mmol) was added, followed by stirring at rt for 10 min. Ac₂O (250 µL, 2.64 mmol) and pyridine (266 µL, 3.30 mmol) were added. After stirring for 10 min at rt, the reaction mixture was extracted twice: first with 10 vol-% AcOH aq, then with a mixture of 10 vol-% AcOH aq. (10 mL) and acetone (10 mL). The so-obtained solution (organic phase) comprising DMTr-dC(bz)s-dA(bz)s-dT-(Kc-C1-carbonate) **(34)** was used directly in the next step (without any precipitation, filtration or purification steps in between).

### DMTr-dG(ib)s-dC(bz)s-dA(bz)s-dT-(Kc-C1-carbonate) (36)

Thiomalic acid (1.01 g, 6.73 mmol) and dichloroacetic acid (DCA, 5.0 mL) were added to the so-obtained organic phase, followed by stirring at rt until reaction monitoring by HPLC indicated complete DMTr-deprotection. NOP (1.3 mL), 10 wt-% brine (12.5 mL), and NMM (10 mL) were added, followed by extraction. The organic phase was separated and extracted eight times: twice with a solution of acetone and water (1:1 v/v, 20 mL × 2), then with a mixture of 0.5 N NaHCO₃ (5 mL), H₂O (5 mL) and acetone (10 mL), then with a mixture of 0.5 N NaHCO₃ (5 mL) 10 wt-% NaCl aq. (10 mL), and DMF (5 mL), then with a mixture of 50 vol-% aq. acetone (20mL) and acetic acid (0.5 mL), then with a mixture of 50 vol-% aq. acetone (20 mL) and NMM (1 mL), and lastly twice with 50 vol-% aq. acetone (20 mL × 2). MTHP (15 mL) was added to the so-obtained organic phase comprising dC(bz)s-dA(bz)s-dT-(Kc-C1-carbonate) **(35),** followed by concentration *in vacuo* to a volume of 15 mL (bath temperature 35-55 °C, pressure 100 hPa). The MTHP (15 mL) addition and subsequent concentration was repeated once (final volume: 15 mL). 1-Methylimidazole (1.0 mL, 12.55 mmol), DMTr-dG(ib) phosphoramidite (CAS-RN: 93183-15-4, 1.68 g, 2.00 mmol) and BTT (646 mg, 3.36 mmol) were added to said solution comprising dC(bz)s-dA(bz)s-dT-(Kc-C1-carbonate) **(35),** and the mixture was stirred at rt until reaction monitoring by HPLC indicated completion of the coupling. DDTT (544 mg, 2.65 mmol) was added, followed by stirring at rt for 10 min. Ac₂O (250 µL, 2.64 mmol) and pyridine (266 µL, 3.30 mmol) were added. After stirring for 10 min at rt, the reaction mixture was extracted twice: first with 10 vol-% AcOH aq, then with a mixture of 10 vol-% AcOH aq. (10 mL) and acetone (10 mL). The so-obtained solution (organic phase) comprising DMTr-dG(ib)s-dC(bz)s-dA(bz)s-dT-(Kc-C1-carbonate) **(36)** was used directly in the next step (without any precipitation, filtration or purification steps in between).

### dTs-dG(ib)s-dC(bz)s-dA(bz)s-dT-(Kc-C1-carbonate) (O^{T}-c)

Thiomalic acid (1.01 g, 6.73 mmol) and dichloroacetic acid (DCA, 5.0 mL) were added to the so-obtained organic phase, followed by stirring at rt until reaction monitoring by HPLC indicated complete DMTr-deprotection. NOP (1.3 mL), 10 wt-% brine (12.5 mL), and NMM (10 mL) were added, followed by extraction. The organic phase was separated and extracted eight times: twice with a solution of acetone and water (1:1 v/v, 20 mL × 2), then with a mixture of 0.5 N NaHCO₃ (5 mL), H₂O (5 mL) and acetone (10 mL), then with a mixture of 0.5 N NaHCO₃ (5 mL) 10 wt-% NaCl aq. (10mL), and DMF (5 mL), then with a mixture of 50 vol-% aq. acetone (20mL) and acetic acid (0.5 mL), then with a mixture of 50 vol-% aq. acetone (20 mL) and NMM (1 mL), and lastly twice with 50 vol-% aq. acetone (20 mL × 2). MTHP (15 mL) was added to the so-obtained organic phase comprising dG(ib)s-dC(bz)s-dA(bz)s-dT-(Kc-C1-carbonate) **(37),** followed by concentration *in vacuo* to a volume of 15 mL (bath temperature 35-55 °C, pressure 100 hPa). The MTHP (15 mL) addition and subsequent concentration was repeated once (final volume: 15 mL). 1-Methylimidazole (1.0 mL, 12.55 mmol), DMTr-dT phosphoramidite (CAS-RN: 98796-51-1, 1.50 g, 1.97 mmol) and BTT (635 mg, 3.30 mmol) were added to said solution comprising comprising dG(ib)s-dC(bz)s-dA(bz)s-dT-(Kc-C1-carbonate) **(37),** and the mixture was stirred at rt until reaction monitoring by HPLC indicated completion of the coupling. DDTT (542 mg, 2.63 mmol) was added, followed by stirring at rt for 10 min. Ac₂O (250 µL, 2.64 mmol) and pyridine (266 µL, 3.30 mmol) were added. After stirring for 10 min at rt, the reaction mixture was extracted twice: first with a mixture of 10wt% aq. NaCl (19 mL) and acetic acid (1 mL), then with a mixture of 10wt% aq. NaCl (20 mL), NOP (2.5 mL), and acetone (10 mL). The so-obtained solution (organic phase) comprising DMTr-dTs-dG(ib)s-dC(bz)s-dA(bz)s-dT-(Kc-C1-carbonate) **(38)** was used directly in the next step (without any precipitation, filtration or purification steps in between).

Thiomalic acid (1.0 g, 6.66 mmol) and dichloroacetic acid (DCA, 5.0 mL) were added to the so-obtained organic phase, followed by stirring at rt until reaction monitoring by HPLC indicated complete DMTr-deprotection. NOP (1.3 mL), 10 wt-% brine (25 mL), and NMM (11 mL) were added, followed by extraction. The organic phase was separated and extracted twice with 50 vol-% aq. acetone. The so-obtained organic phase comprising dTs-dG(ib)s-dC(bz)s-dA(bz)s-dT-(Kc-C1-carbonate) (O^{T}-c) was concentrated *in vacuo,* followed by addition of MeOH (40 mL). The resulting precipitate was filtered and washed twice, fist with MeCN (20 mL), then with MeOH (20 mL), to give dTs-dG(ib)s-dC(bz)s-dA(bz)s-dT-Kc (O^{T}-c, 1.02 g, purity 79.945%, yield 55.1%) as a white solid. MS (method A) m/z: [M + 2H]²⁺ calcd. for C₁₃₁H₁₈₅N₂₁O₃₂P₄S₄²⁺, 1408.0659; found 1408.0706.

### Example 6: Comparative Example - attempted synthesis of target oligonucleotide O^{T}-d (see Table T-2) using DMF as amide solvent

In this comparative example, elongation of an exemplary 4mer oligonucleotide to a 5mer oligonucleotide was attempted, wherein the amide solvent S^{A} as defined herein was replaced by N,N-dimethylformamide (DMF).

A solution of dT-dC(bz)-dA(bz)-dT-(Kc-C1-carbonate) **(39, 3.04** g, 1.32 mmol) in cyclopentyl methyl ether (CPME)/DMF (16:3 v/v, 95 mL) was concentrated *in vacuo* to a volume of 35 mL. DMTr-dA(Bz) phosphoramidite (CAS-RN: 98796-53-3, 2.31 g, 2.64 mmol) and BTT (2.54 g, 13.21 mmol) were added, and the mixture was stirred at rt until reaction monitoring by HPLC indicated completion of the coupling. Ac₂O (449 µL, 5.28 mmol), pyridine (533 µL, 6.60 mmol) and 1-methylimidazole (526 µL, 6.60 mmol) were added. After stirring for 10 min at rt, 1 M I₂ in CPME (2.91 mL, 2.91 mmol), H₂O (71 µL) and MeCN (3 mL) were added. After stirring for 10 min at rt, 0.5 N Na₂S₂O₃ and 1 N NMM solution (2:1 v/v, 15 mL) was added to the reaction mixture. An emulsion formed and the phase separation was not completed. The subsequent aqueous extraction and DMTr-deprotection could not to be carried out.

Thus, when replacing the amide solvent S^{A} as defined herein by the common amide solvent DMF, even a 5mer synthesis could not be completed.

### Example 7: Synthesis A of target oligonucleotide O^{T}-e (see Table T-2)

### DMTr-MG(ib)s-MG(ib)-(Kc-C1-carbonate) (5)

Dichloroacetic acid (DCA, 6.0 mL) and thiomalic acid (TMA, 0.919 mL, 5.0 eq) were added at rt to a stirred solution of DMTr-MG(ib)-(Kc-C1-carbonate) **(2,** 0.80 g, 537 µmol, 1.0 eq) in 4-methyltetrahydropyran (MTHP, 12 mL). The mixture was stirred at rt until reaction monitoring by HPLC (Method B was used throughout Example 7) indicated completion of the DMTr-deprotection. N-Octyl-2-pyrrolidone (NOP, 7 mL), N-methylmorpholine (NMM, 8 mL), Acetone (12 mL), and 15 wt-% aqueous (aq.) sodium chloride solution (brine, 30 mL) were added, followed by extraction. The organic phase was separated and extracted as follows:
- with a mixture of NMM (2 mL), 5 wt-% aqueous sodium chloride solution (brine, 9 mL), and acetone (9 mL), next
- with a mixture of 5 wt-% aqueous sodium chloride solution (brine, 12.5 mL) and acetone (12.5 mL), next
- with a mixture of water (8 mL), acetone (4 mL), and acetic acid (0.1 mL), and lastly
- with a mixture of water (15 mL), acetone (8 mL), and MTHP (6 mL).

Following this extraction procedure, the organic phase was subjected to 3 rounds of azeotropic distillation, during each of which MTHP (16-24 mL) was added, followed by concentration *in vacuo* to a volume of 12 mL (bath temperature 45-55 °C, pressure 40-100 hPa). The so-obtained solution (water content: 396.8 ppm; GC-analysis: combined content of MeCN, hexane, acetone and NMM was below 0.2 vol-%, NOP: 37 vol-%, MTHP: 63 vol-%) comprising MG(ib)-(Kc-C1-carbonate) **(4)** was used directly in the subsequent step (without any precipitation, filtration or purification steps in between). DMTr-MG(ib) phosphoramidite (CAS-RN: 251647-55-9, 1.23 g, 1.34 mmol, 2.5 eq) and a mixture of TFA, N-methylimidazole (NMI), and acetonitrile (MeCN) (9:11:30 v/v/v, 5 mL in total) was added to said solution comprising MG(ib)-(Kc-C1-carbonate) **(4),** and the mixture was stirred at rt until reaction monitoring by HPLC indicated completion of the coupling. NMI (0.6 mL), and 3-(N,N-dimethylaminomethyl idene)amino)-3*H*-1,2,4-dithiazole-5-thione (CAS-RN: 1192027-04-5, DDTT, 0.81 g, 7.5 eq) were added. After stirring for 10 min at rt, triethylphosphite (0.685 mL, 7.5 eq) was added. After stirring for 10 min at rt, pyridine (0.213 mL, 5.0 eq) and acetic anhydride (Ac₂O, 0.20 mL, 4.0 eq) was added, followed by stirring at rt for 10 min, addition of propan-2-ol (0.80 mL), and stirring for 10 min at rt. The so-obtained solution comprising DMTr-MG(ib)s-MG(ib)-(Kc-C1-carbonate) **(5)** was used directly in the subsequent step (without any precipitation, filtration or purification steps in between).

### Second coupling cycle and further coupling cycles

The second coupling cycle and all further coupling cycles were performed according to the following general protocol, unless indicated differently. The starting materials and products of each coupling cycle are identical to those listed in Table E-1 above, wherein, except for the above-mentioned first coupling cycle, only the 2^{nd}, 3^{rd}, and 4^{th} coupling cycles were performed in the present Example 7. Again, the terms "starting material" and "product" are not to be construed to indicate any precipitation and/or isolation steps in between the coupling cycles.

### General protocol for coupling cycles

In a typical coupling cycle, the following steps were performed in the presented order, unless indicated differently.

### DMTr-deprotection

At 0°C, trifluoroacetic acid (TFA, 6.0 mL) and 3-mercaptopropionic acid (MPA, 0.919 mL) were added directly to the solution obtained from the preceding capping step. The mixture was stirred at rt for 45 min.

### Aqueous extractions

N-methylmorpholine (NMM, 14 mL), n-hexane (2^{nd} and 4^{th} coupling cycle: 6 mL; 3^{rd} coupling cycle: 12 mL), MTHP (3 mL), 15 wt-% aqueous (aq.) sodium chloride solution (brine, 9 mL), and acetone (9 mL) were added, followed by extraction. The organic phase was separated and extracted as follows:
- with a mixture of NMM (2 mL), 5 wt-% aqueous sodium chloride solution (brine, 9 mL), and acetone (9 mL), next
- with a mixture of 5 wt-% aqueous sodium chloride solution (brine, 9 mL) and acetone (9 mL), next
- with a mixture of 5 wt-% aqueous sodium chloride solution (brine, 9 mL), acetone (9 mL), and acetic acid (0.15 mL), and lastly
- with a mixture of 5 wt-% aqueous sodium chloride solution (brine, 9 mL) and acetone (9 mL).

### Azeotropic distillation

MTHP (12-24 mL) and NOP (2^{nd} cycle; 3.6 mL, 3^{rd}; 4 mL) were added to the organic phase obtained from the preceding aqueous extractions, followed by concentration *in vacuo* (bath temperature 35-55 °C, pressure 100 hPa) to a volume of 12 mL. This MTHP addition and concentration step was typically repeated once or twice. The water content was adjusted to be in the range of 178.3-321.5 ppm. The composition of the solution obtained after azeotropic distillation was analyzed as laid out above. The content of NOP was in the range of 52-57 vol-% and the content of MTHP was in the range of 43-48 vol-%. The combined content of MeCN, hexane, acetone and NMM was below 0.2 vol-% in all coupling cycles.

### Coupling reaction

The respective 5'-DMTr-protected phosphoramidite (2^{nd} coupling cycle: 2.0 eq; 3^{rd} and 4^{th} coupling cycle: 3.0 eq) and a mixture of TFA, NMI, and acetonitrile (9:11:30 v/v/v, 4-5 mL in total) were added to the solution obtained from azeotropic distillation, followed by stirring at rt until reaction monitoring by HPLC indicated completion of the coupling.

### Sulfurization (including quenching with triethylphosphite)

NMI (0.6 mL), and DDTT (2^{nd} coupling cycle: 0.66 g, 6.0 eq; 3^{rd} coupling cycle: 0.82 g, 7.5 eq; 4^{th} coupling cycle: 0.98 g, 9.0 eq) were added, and the mixture was stirred at rt for 10 min. Triethylphosphite (2^{nd} coupling cycle: 0.549 mL, 6.0 eq; 3^{rd} coupling cycle: 0.687 mL, 7.5 eq; 4^{th} coupling cycle: 0.824 mL, 9.0 eq) was added, followed by stirring for 10 min at rt.

### Capping

Pyridine (0.213 mL, 5.0 eq) and Ac₂O (0.2 mL, 4.0 eq) were added, followed by stirring at rt for 10 min. Propan-2-ol (0.8 mL) was added, followed by stirring at rt for 10 min.

After completion of the 4^{th} coupling cycle, one more DMTr-deprotection, followed by aqueous extractions as laid out above in the general protocol were performed. The organic phase obtained from the final aqueous extraction was concentrated in *vacuo,* followed by addition of methanol (20 mL). The resulting precipitate was filtered and washed with MeOH (20mL, twice) to give DMTr-MG(ib)s-mMeC(bz)s-mMeUs-MG(ib)s-MG(ib)-(Kc-C1-carbonate) **(8,** 1.46 g, 66.3% crude yield).

### Target oligonucleotide O^{T}-e (see Table T-2)

A solution of the crude compound **8** (302.4 mg, 0.09 nmol) in a mixture of H₂O and tert-butylamine (1:1 v/v, 9 mL) was stirred at 80 °C for 10 h. The reaction mixture was cooled to rt and analyzed by HPLC-MS to find 5'-MGs-MMeCs-MmeUs-MGs-MG-3' (O^{T}-e). MS (Method D) m/z: [M-2H]²⁻ calcd for C₆₅H₉₁N₂₀O₃₅P₄S₄²⁻, 982.1939, found 982.1931. Purity (Method C, Gradient-2): 78.7%

### Example 8: Synthesis A of target oligonucleotide O^{T}-f (see Table T-2)

### DMTr-MG(ib)s-MG(ib)-(Kc-C1-carbonate) (5)

A solution of MG(ib)-(Kc-C1-carbonate) **(4,** 0.633 g, 0.528 mmol) in *N*-Octyl-2-pyrrolidone (NOP, 6 mL) was subjected to 3 rounds of azeotropic distillation, during each of which MTHP (12 mL) was added, followed by concentration *in vacuo* to a volume of 9 mL (bath temperature 45-55 °C, pressure 40-100 hPa). The so-obtained solution (water content: 241.1 ppm) comprising MG(ib)-(Kc-C1-carbonate) **(4)** was used directly in the subsequent step (without any precipitation, filtration or purification steps in between). DMTr-MG(ib) phosphoramidite (CAS-RN: 251647-55-9, 1.21 g, 1.36 mmol, 2.6 eq) and a mixture of TFA, N-methylimidazole (NMI), and acetonitrile (MeCN) (9:11:30 v/v/v, 4 mL in total) was added to said solution comprising MG(ib)-(Kc-C1-carbonate) **(4),** and the mixture was stirred at rt until reaction monitoring by HPLC indicated completion of the coupling. Pyridine (0.213 mL, 5.0 eq), NMI (1.8.6 mL), and 3-(N,N-dimethylaminomethylidene)amino)-3*H-*1,2,4-dithiazole-5-thione (CAS-RN: 1192027-04-5, DDTT, 0.54 g, 5.0 eq) were added. After stirring for 10 min at rt, triethylphosphite (0.453 mL, 5.0 eq) was added. After stirring for 10 min at rt, acetic anhydride (Ac₂O, 0.20 mL, 4.0 eq) was added, followed by stirring at rt for 10 min, addition of propan-2-ol (0.80 mL), and stirring for 10 min at rt. The so-obtained solution comprising DMTr-MG(ib)s-MG(ib)-(Kc-C1-carbonate) **(5)** was used directly in the subsequent step (without any precipitation, filtration or purification steps in between).

### Second coupling cycle and further coupling cycles

The second coupling cycle and all further coupling cycles were performed according to the following general protocol, unless indicated differently. The starting materials and products of each coupling cycle are identical to those listed in Table E-1 above, wherein, except for the above-mentioned first coupling cycle, only the 2^{nd} to 9^{th} coupling cycles were performed in the present Example 8. Again, the terms "starting material" and "product" are not to be construed to indicate any precipitation and/or isolation steps in between the coupling cycles.

### General protocol for coupling cycles

In a typical coupling cycle, the following steps were performed in the presented order, unless indicated differently.

### DMTr-deprotection

At 0°C, trifluoroacetic acid (TFA, 6.0 mL) and 3-mercaptopropionic acid (MPA, 0.919 mL), were added directly to the solution obtained from the preceding capping step. The mixture was stirred at rt for 45 min.

### Aqueous extractions

N-methylmorpholine (NMM, 14 mL), alkane (2^{nd} coupling cycle: cyclohexane; 3^{rd} coupling cycle: *n*-hexane; 4^{th} to 9^{th} coupling cycles: *n*-heptane; 6.0 mL in all cases), MTHP (3 mL), and 15 wt-% aqueous (aq.) sodium chloride solution (brine, 22.5 mL) were added, followed by extraction. The organic phase was separated and extracted as follows:
- with a mixture of NMM (1 mL), 5 wt-% aqueous sodium chloride solution (brine, 9-12 mL), and acetone (9-12 mL), next
- with a mixture of 5 wt-% aqueous sodium chloride solution (brine, 22.5 mL) and acetone (22.5 mL), next
- with a mixture of 5 wt-% aqueous sodium chloride solution (brine, 22.5 mL), acetone (22.5 mL), and acetic acid (0.1 mL), and lastly
- with a mixture of 5 wt-% aqueous sodium chloride solution (brine, 9-10 mL) and acetone (9-10 mL), wherein in the 4^{th} to 8^{th} coupling cycle, 2.5 wt-% brine was used instead of 5 wt-% brine and water (18 mL) was added.

### Azeotropic distillation

MTHP (12-24 mL) and NOP (2^{nd}, 8^{th} and 9^{th} coupling cycle: 2 mL; 3^{rd} and 5^{th} to 7^{th} coupling cycle: 3 mL) were added to the organic phase obtained from the preceding aqueous extractions, followed by concentration *in vacuo* (bath temperature 35-55 °C, pressure 100 hPa) to a volume of 9 mL. This MTHP addition and concentration step was typically repeated once or twice. The water content was adjusted to be in the range of 185.6-412.3 ppm. The composition of the solution obtained after azeotropic distillation was analyzed as laid out above. The content of NOP was in the range of 27-67 vol-% and the content of MTHP was in the range of 73-33 vol-%. The combined content of MeCN, the respective alkane, acetone and NMM was below 0.2 vol-% in all coupling cycles, wherein no analysis has been conducted in the 6^{th} coupling cycle.

### Coupling reaction

The respective 5'-DMTr-protected phosphoramidite (2^{nd} to 6^{th} coupling cycle: 2.0 eq; 7^{th} to 9^{th} coupling cycle: 3.0 eq) and a mixture of TFA, NMI, and acetonitrile (9:11:30 v/v/v, 4-5 mL in total) were added to the solution obtained from azeotropic distillation, followed by stirring at rt until reaction monitoring by HPLC indicated completion of the coupling.

### Sulfurization (including quenching with triethylphosphite)

Pyridine (0.213 mL, 5.0 eq), NMI (0.6 mL), and DDTT (2^{nd} to 6^{th} coupling cycle: 0.44-0.47 g, 3.0 eq; 7^{th} to 9^{th} coupling cycle: 0.66 g, 5.0 eq) were added, and the mixture was stirred at rt for 10 min. Triethylphosphite (2^{nd} to 6^{th} coupling cycle: 0.366 mL, 3.0 eq; 7^{th} to 9^{th} coupling cycle: 0.550 mL, 5.0 eq) was added, followed by stirring for 10 min at rt.

### Capping

Ac₂O (0.2 mL, 4.0 eq) was added, followed by stirring at rt for 10 min. Propan-2-ol (0.8 mL) was added, followed by stirring at rt for 10 min.

After completion of the 9^{th} coupling cycle, one more DMTr-deprotection, followed by aqueous extractions as laid out above in the general protocol were performed. The organic phase obtained from the final aqueous extraction was concentrated *in vacuo,* followed by addition of methanol (20 mL). The resulting precipitate was filtered and washed with methanol (20 mL, twice) to give DMTr-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(13,** 1.61 g, 51.8% crude yield)

### Target oligonucleotide O^{T}-f (see Table T-2)

A solution of the crude of compound **13** (305.1 mg, 0.05 mmol) in a mixture of H₂O and tert-butylamine (1:1 v/v, 9 mL) was stirred at 80 °C for 10 h. The reaction mixture was cooled to room temperature and analyzed by HPLC-MS to find 5'-MAs-MMeUs-MAs-MAs-MMeUs-MGs-MMeCs-MMeUs-MGs-MG-3' (O^{T}-f). MS (Method E) m/z: [M-2H]²⁻ calcd for C₁₃₀H₁₈₄N₃₉O₆₉P₉S₉²⁻, 1980.861, found 1980.8608. Purity (Method D): 66.6%

### Example 9: Synthesis B of target oligonucleotide O^{T}-e (see Table T-2)

### DMTr-MG(ib)s-MG(ib)-(Kc-C1-carbonate) (5)

A solution of MG(ib-(Kc-C1-carbonate) **(4,** 1.58 g, 1.32 mmol) was subjected to 2 rounds of azeotropic distillation, during each of which MTHP (15 mL) was added, followed by concentration *in vacuo* to a volume of 15 mL (bath temperature 45-55 °C, pressure 40-100 hPa). The so-obtained solution (water content: 100.2 ppm; GC-analysis: combined content of MeCN, heptane, acetone and NMM was below 0.2 vol-%, NOP: 31 vol-%, MTHP: 69 vol-%) comprising MG(ib)-(Kc-C1-carbonate) **(4)** was used directly in the subsequent step (without any precipitation, filtration or purification steps in between). DMTr-MG(ib) phosphoramidite (CAS-RN: 251647-55-9, 2.41 g, 2.64 mmol, 2.0) and a mixture of TFA, N-methylimidazole (NMI), and acetonitrile (MeCN) (9:11:30 v/v/v, 10 mL in total) were added to said solution comprising MG(ib)-(Kc-C1-carbonate) **(4),** and the mixture was stirred at rt until reaction monitoring by HPLC indicated completion of the coupling. Pyridine (0.533 mL, 5.0 eq), NMI (1.5 mL), and 3-(N,N-dimethylaminomethylidene)amino)-3*H*-1,2,4-dithiazole-5-thione (CAS-RN: 1192027-04-5, DDTT, 0.81 g, 3.0 eq) were added. After stirring for 10 min at rt, triethylphosphite (0.686 mL, 3.0 eq) was added. After stirring for 10 min at rt, acetic anhydride (Ac₂O, 0.5 mL, 4.0 eq) was added, followed by stirring at rt for 10 min, addition of propan-2-ol (2.0 mL), and stirring for 10 min at rt. The so-obtained solution comprising DMTr-MG(ib)s-MG(ib)-(Kc-C1-carbonate) **(5)** was used directly in the subsequent step (without any precipitation, filtration or purification steps in between).

### Second coupling cycle and further coupling cycles

The second coupling cycle and all further coupling cycles were performed according to the following general protocol, unless indicated differently. The starting materials and products of each coupling cycle are identical to those listed in Table E-1 above, wherein, except for the above-mentioned first coupling cycle, only the 2^{nd}, 3^{rd}, and 4^{th} coupling cycles were performed in the present Example 9. Again, the terms "starting material" and "product" are not to be construed to indicate any precipitation and/or isolation steps in between the coupling cycles.

### General protocol for coupling cycles

In a typical coupling cycle, the following steps were performed in the presented order, unless indicated differently.

### DMTr-deprotection

Methanesulfonic acid (MSA, 12.5 mL) and glutathione (2^{nd} and 3^{rd} coupling cycle: 2.03 g; 4^{th} coupling cycle: 3.02 g), were added at rt directly to the solution obtained from the preceding capping step. The mixture was stirred at rt for 45 min.

### Aqueous extractions

N-methylmorpholine (NMM, 35 mL), *n*-heptane (15 mL), MTHP (7.5 mL), and 15 wt-% aqueous (aq.) sodium chloride solution (brine, 45 mL) were added, followed by extraction. The organic phase was separated and extracted as follows:
- with a mixture of 5 wt-% aqueous sodium chloride solution (brine, 22.5 mL) and acetone (22.5 mL), and then
- with a mixture of 5 wt-% aqueous sodium chloride solution (brine, 22.5 mL), acetone (22.5 mL), and acetic acid (0.7-1.3 mL).

### Azeotropic distillation

MTHP (30 mL) and NOP (only added in the 4^{th} coupling cycle: 2mL) was added to the organic phase obtained from the preceding aqueous extractions, followed by concentration *in vacuo* (bath temperature 35-55 °C, pressure 100 hPa) to a volume of 22.5 mL. This MTHP addition and concentration step was typically repeated once or twice. The water content was adjusted to be in the range of 86.9-347.0 ppm. The composition of the solution obtained after azeotropic distillation was analyzed as laid out above. The content of NOP was in the range of 33-57 vol-% and the content of MTHP was in the range of 67-43 vol-%. The combined content of MeCN, heptane, acetone and NMM was below 0.2 vol-% in all coupling cycles.

### Coupling reaction

The respective 5'-DMTr-protected phosphoramidite (2^{nd} and 3^{rd} coupling cycle: 2.0 eq; 4^{th} coupling cycle: 3.0 eq) and a mixture of TFA, NMI, and acetonitrile (9:11:30 v/v/v, 10 mL in total) were added to the solution obtained from azeotropic distillation, followed by stirring at rt until reaction monitoring by HPLC indicated completion of the coupling.

### Sulfurization (including quenching with triethylphosphite)

Pyridine (0.533 mL, 5.0 eq), NMI (1.5 mL), and DDTT (2^{nd} and 3^{rd} coupling cycle: 0.81 g, 3.0 eq; 4^{th} coupling cycle: 1.22 g, 4.5 eq) were added, and the mixture was stirred at rt for 10 min. Triethylphosphite (0.686 mL, 3.0 eq) was added, followed by stirring for 10 min at rt.

### Capping

Ac₂O (0.5 mL, 4.0 eq) was added, followed by stirring at rt for 10 min. Propan-2-ol (2.0 mL) was added, followed by stirring at rt for 10 min.

After completion of the 4^{th} coupling cycle, the batch was divided into two equal halves by volume. A first half of the batch was treated as described in the present Example 9 below, while the other half of the batch was used in Example 10 below.

One half of the batch was subjected to one more DMTr-deprotection, followed by aqueous extractions as laid out above in the general protocol. The organic phase obtained from the final aqueous extraction was concentrated *in vacuo,* followed by addition of methanol (50 mL). The resulting precipitate was filtered and washed by MeOH (25 mL, twice) to give DMTr-MG(ib)s-mMeC(bz)s-mMeUs-MG(ib)s-MG(ib)-(Kc-C1-carbonate) **(8,** 1.21 g, 56% crude yield).

### Target oligonucleotide O^{T}-e (see Table T-2)

A solution of the crude of compound **8** (303.1 mg, 0.09 mmol) in a mixture of H₂O and tert-butylamine (1:1 v/v, 9 mL) was stirred at 80 °C for 10 h. The reaction mixture was cooled to room temperature and analyzed by HPLC-MS to find 5'-MGs-MMeCs-MMeUs-MGs-MG-3' (O^{T}-e). MS (Method D) m/z: [M-2H]²⁻ calcd for C₆₅H₉₂N₂₀O₃₅P₄S₄²⁻, 982.1939, found 982.1930. Purity (Method C, Gradient-2): 81.7%

### Example 10: Synthesis B of target oligonucleotide O^{T}-f (see Table T-2)

The second half of the batch as divided in Example 9 was subjected to the 5^{th} to 9^{th} coupling cycle, the staring materials and products of which were identical to those listed in Table E-1 above. Again, the terms "starting material" and "product" are not to be construed to indicate any precipitation and/or isolation steps in between the coupling cycles. The 5^{th} to 9^{th} coupling cycle were performed according to the following general protocol.

### General protocol for coupling cycles

In a typical coupling cycle, the following steps were performed in the presented order, unless indicated differently.

### DMTr-deprotection

Methanesulfonic acid (MSA, 6.25 mL) and glutathione (5^{th} coupling cycle: 1.22 g, 5.0 eq; 6^{th} and 7^{th} coupling cycle: 1.52 g, 7.5 eq; 8^{th} and 9^{th} coupling cycle: 2.02 g, 10.0 eq), were added at rt directly to the solution obtained from the preceding capping step. The mixture was stirred at rt for 45 min.

### Aqueous extractions

N-methylmorpholine (NMM, 17.5 mL), n-heptane (7.5 mL), MTHP (3.75 mL), and 15 wt-% aqueous (aq.) sodium chloride solution (brine, 22.5 mL) were added, followed by extraction. The organic phase was separated and extracted as follows:
- with a mixture of 5 wt-% aqueous sodium chloride solution (brine, 11.25 mL) and acetone (11.25 mL), and then
- with a mixture of 5 wt-% aqueous sodium chloride solution (brine, 22.5 mL), acetone (22.5 mL), and acetic acid (0.7-0.8 mL).

### Azeotropic distillation

MTHP (15 mL) and NOP (only added in the 6^{th} coupling cycle: 1.5 mL) was added to the organic phase obtained from the preceding aqueous extractions, followed by concentration *in vacuo* (bath temperature 35-55 °C, pressure 100 hPa) to a volume of 22.5 mL. This MTHP addition and concentration step was typically repeated once or twice. The water content was adjusted to be in the range of 59.0-154.3 ppm. The composition of the solution obtained after azeotropic distillation was analyzed as laid out above for the 5^{th} to 8^{th} coupling cycle. The content of NOP was in the range of 41-66 vol-% and the content of MTHP was in the range of 59-34 vol-%. The combined content of MeCN, heptane, acetone and NMM was below 0.2 vol-% in all coupling cycles.

### Coupling reaction

The respective 5'-DMTr-protected phosphoramidite (5^{th} coupling cycle: 2.5 eq; 6^{th} coupling cycle: 3.0 eq; 7^{th} and 9^{th} coupling cycle: 4.0 eq; 8^{th} coupling cycle: 5.0 eq) and a mixture of TFA, NMI, and acetonitrile (9:11:30 v/v/v, 5 mL in total) were added to the solution obtained from azeotropic distillation, followed by stirring at rt until reaction monitoring by HPLC indicated completion of the coupling.

### Sulfurization (including quenching with triethylphosphite)

Pyridine (0.266 mL, 5.0 eq), NMI (0.75 mL), and DDTT (5^{th} and 6^{th} coupling cycle: 0.54 g, 4.0 eq; 7^{th} and 9^{th} coupling cycle: 0.68 g, 5.0 eq; 8^{th} coupling cycle: 0.81 g, 6.0) were added, and the mixture was stirred at rt for 10 min. Triethylphosphite (0.343 mL, 3.0 eq) was added, followed by stirring for 10 min at rt.

### Capping

Ac₂O (0.25 mL, 4.0 eq) was added, followed by stirring at rt for 10 min. Propan-2-ol (1.0 mL) was added, followed by stirring at rt for 10 min.

After completion of the 9^{th} coupling cycle, one more DMTr-deprotection, followed by aqueous extractions as laid out above in the general protocol were performed. The organic phase obtained from the final aqueous extraction was concentrated in *vacuo.* Methanol (100 mL) was added, and the resulting precipitate was collected. Ethanol (100 mL) was added, and the resulting precipitate was collected. The combined precipitate was washed by Ethanol (50 mL, twice) to give DMTr-MA(bz)s-mMeUs-MA(bz)s-MA(bz)s-mMeUs-MG(ib)s-mMeC(bz)s-mMeUs-MG(ib)s-MG(ib)-(Kc-C1-carbonate) (13, 2.04 g, 52.6% crude yield starting from DMTr-MG(ib)-(Kc-C1-carbonate) **(2)).**

### Target oligonucleotide O^{T}-f (see Table T-2)

A solution of the crude of compound **13** (294.9 mg, 0.05 mmol) in a mixture of H₂O and tert-butylamine (1:1 v/v, 9 mL) was stirred at 80 °C for 10 h. The reaction mixture was cooled to rt and analyzed by HPLC-MS to find 5'-MAs-MMeUs-MAs-MAs-MMeUs-MGs-MMeCs-MMeUs-MGs-MG-3' (O^{T}-f). MS (Method E) m/z: [M-2H]²⁻calcd for C₁₃₀H₁₈₄N₃₉O₆₉P₉S₉²⁻, 1980.861 found 1980.8593. Purity (Method D): 67.0%

### Example 11: Synthesis C of target oligonucleotide O^{T}-a and synthesis of target oligonucleotide O^{T}-a (see Table T-2)

### DMTr-MG(ib)s-MG(ib)-(Kc-C1-carbonate) (5)

A solution of MG(ib)-(Kc-C1-carbonate) **(4,** 1.58 g, 1.32 mmol) in NOP (15 mL) was subjected to two rounds of azeotropic distillation, during each of which MTHP (30 mL) was added, followed by concentration *in vacuo* to a volume of 22.5 mL (bath temperature 45-55 °C, pressure 40-100 hPa). The so-obtained solution (water content: 135,0 ppm; GC-analysis: combined content of MeCN, Heptane, acetone and NMM was below 0.2 vol-%, NOP: 41 vol-%, MTHP: 59 vol-%) comprising MG(ib)-(Kc-C1-carbonate) **(4)** was used directly in the subsequent step (without any precipitation, filtration or purification steps in between). DMTr-MG(ib) phosphoramidite (CAS-RN: 251647-55-9, 2.41 g, 2.64 mmol, 2.0 eq) and a mixture of TFA, N-methylimidazole (NMI), and acetonitrile (MeCN) (11:9:30 v/v/v, 10 mL in total) was added to said solution comprising MG(ib)-(Kc-C1-carbonate) **(4)** and the mixture was stirred at rt until reaction monitoring by HPLC indicated completion of the coupling. Pyridine (0.533 mL, 5.0 eq), NMI (1.5 mL), and 3-(N,N-dimethylaminomethylidene)amino)-3*H*-1,2,4-dithiazole-5-thione (CAS-RN: 1192027-04-5, DDTT, 0.81 g, 3.0 eq) were added. After stirring for 10 min at rt, triethylphosphite (686 µL, 3.0 eq) was added. After stirring for 10 min at rt, acetic anhydride (Ac₂O, 499 µL, 4.0 eq) was added, followed by stirring at rt for 10 min, addition of propan-2-ol (2.0 mL), and stirring for 10 min at rt. The so-obtained solution comprising DMTr-MG(ib)s-MG(ib)-(Kc-C1-carbonate) **(5)** was used directly in the subsequent step (without any precipitation, filtration or purification steps in between).

### 2^{nd} to 14^{th} coupling cycle

The 2^{nd} to 14^{th} coupling cycle were performed according to the following general protocol, unless indicated differently. The starting materials and products of each coupling cycle are identical to those listed in Table E-1 above. Again, the terms "starting material" and "product" are not to be construed to indicate any precipitation and/or isolation steps in between the coupling cycles.

### General protocol for coupling cycles

In a typical coupling cycle, the following steps were performed in the presented order, unless indicated differently.

### DMTr-deprotection

Trifluoroacetic acid (TFA, 15.0 mL) and 3-mercaptopropionic acid (MPA, 2.298 mL) were added at rt directly to the solution obtained from the preceding capping step. The mixture was stirred at rt for 45 min.

### Aqueous extractions

N-methylmorpholine (NMM, 35 mL), n-heptane (15 mL), MTHP (7.5 mL), and 15 wt-% aqueous (aq.) sodium chloride solution (brine, 45 mL) were added, followed by extraction. The organic phase was separated and extracted as follows:
- with a mixture of NMM (5 mL), 5 wt-% aqueous sodium chloride solution (brine, 22.5 mL), and acetone (22.5 mL), next
- with a mixture of 5 wt-% aqueous sodium chloride solution (brine, 22.5 mL) and acetone (22.5 mL), next
- with a mixture of 5 wt-% aqueous sodium chloride solution (brine, 22.5 mL), acetone (22.5 mL), and acetic acid (0.30-0.88 mL), and lastly
- with a mixture of 5 wt-% aqueous sodium chloride solution (brine, 22.5 mL) and acetone (22.5 mL).

### Azeotropic distillation

MTHP (30 mL) and NOP (2^{nd} and 3^{rd} coupling cycle: 7.5 mL, 4^{th}, 5^{th} 7^{th} 8^{th}, 13^{th} 14^{th} coupling cycle: 5 mL, 6^{th} coupling cycle: 14 mL, 9^{th} coupling cycle: 3 mL, 10^{th} to 12^{th} coupling cycle: 4 mL) were added to the organic phase obtained from the preceding aqueous extractions, followed by concentration *in vacuo* (bath temperature 35-55 °C, pressure 40-100 hPa). This MTHP addition and concentration step was typically repeated once or twice. The water content was adjusted to be in the range of 60.4-312.4 ppm. The composition of the solution obtained after azeotropic distillation was analyzed as laid out above. The content of NOP was in the range of 27-64 vol-% and the content of MTHP was in the range of 36-73 vol-%. The combined content of MeCN, heptane, acetone and NMM was below 0.2 vol-% in all coupling cycles.

### Coupling reaction

The respective 5'-DMTr-protected phosphoramidite (2^{nd} to 5^{th} coupling cycle: 2.0 eq; 6^{th} coupling cycle: 3.0 eq; 7^{th} coupling cycle: 3.3 eq; 8^{th} coupling cycle: 3.5 eq; 9^{th} to 14^{th} coupling cycle: 3.0 eq) and a mixture of TFA, NMI, and acetonitrile (11:9:30 v/v/v, 10 mL in total) were added to the solution obtained from azeotropic distillation, followed by stirring at rt until reaction monitoring by HPLC indicated completion of the coupling.

### Sulfurization (including quenching with triethylphosphite)

Pyridine (0.533 mL, 5.0 eq), NMI (1.5 mL), and DDTT (2^{nd} and 3^{rd} coupling cycle: 0.81 g, 3.0 eq; 4^{th} to 6^{th} coupling cycle: 0.81-1.22 g, 3.0-4.5 eq; 7^{th} to 9^{th} coupling cycle: 1.09-1.22 g, 4.0-4.5 eq; 10^{th} to 14^{th} coupling cycle: 1.08-1.09 g, 4.0 eq) were added, and the mixture was stirred at rt for 10 min. Triethylphosphite (686 µL, 3.0 eq) was added, followed by stirring for 10 min at rt.

### Capping

Ac₂O (499 µL, 4.0 eq) was added, followed by stirring at rt for 10 min. Propan-2-ol (2.0 mL) was added, followed by stirring at rt for 10 min.

After completion of the 14^{th} coupling cycle, the batch was divided into two halves of equal volumes. One half batch was used for the preparation of target oligonucleotide O^{T}-g and the other half batch was subjected to the 15^{th} to 17^{th} coupling cycle to obtain target oligonucleotide O^{T}-a.

### Target oligonucleotide O^{T}-a

One half of the batch comprising compound **18** (cf. Table E-1) was subjected to one more DMTr-deprotection, followed by aqueous extractions as laid out above in the general protocol. The organic phase obtained from the final aqueous extraction was concentrated *in vacuo,* followed by addition of methanol (25 mL). The resulting precipitate was filtered and washed with methanol (25 mL, 3 times) to give mMeC(bz)s-mMeUs-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(23,** 1.97 g, crude).

A solution of the crude of compound **23** (97.9 mg, 0.01 mmol) in a mixture of H₂O and tert-butylamine (2:1 v/v, 9 mL) was stirred at 80 °C for 10 h. The reaction mixture was cooled to rt and analyzed by HPLC (Method E) and HPLC-MS (Method E) to find 5'-MMeUs-MMeCs-MAs-MMeCs-MMeUs-MMeUs-MMeUs-MMeCs-MAs-MMeUs-MAs-MAs-MMeUs-MGs-MMeCs-3' (O^{T}-g). MS(Method E) m/z: [M - 3H]³⁻calcd for C₁₉₅H₂₈₃N₅₁O₁₀₇P₁₄S₁₄³⁻, calcd. 1976.349, found 1976.3451. Purity (Method E): 49.4%.

### 15^{th} to 17^{th} coupling cycle

The half batch which was not used for the synthesis of target oligonucleotide O^{T}-g was subjected to the 15^{th} to 17^{th} coupling cycle which were performed according to the following general protocol, unless indicated differently. The starting materials and products of each coupling cycle are identical to those listed in Table E-1 above. Again, the terms "starting material" and "product" are not to be construed to indicate any precipitation and/or isolation steps in between the coupling cycles.

### General protocol for coupling cycles

In a typical coupling cycle, the following steps were performed in the presented order, unless indicated differently.

### DMTr-deprotection

Trifluoroacedtic acid (TFA, 7.5 mL) and 3-mercaptopropionic acid (MPA, 1.149 mL) were added at rt directly to the solution obtained from the preceding capping step. The mixture was stirred at rt for 45 min.

### Aqueous extractions

N-methylmorpholine (NMM, 17.5 mL), n-heptane (7.5 mL), MTHP (3.75 mL), and 15 wt-% aqueous (aq.) sodium chloride solution (brine, 22.5 mL) were added, followed by extraction. The organic phase was separated and extracted as follows:
- with a mixture of NMM (2.5 mL), 5 wt-% aqueous sodium chloride solution (brine, 11.25 mL), and acetone (11.25 mL), next
- with a mixture of 5 wt-% aqueous sodium chloride solution (brine, 11.25 mL) and acetone (11.25 mL), next
- with a mixture of 5 wt-% aqueous sodium chloride solution (brine, 11.25 mL), acetone (11.25 mL), and acetic acid (1.1 mL), and lastly
- with a mixture of 5 wt-% aqueous sodium chloride solution (brine, 11.25 mL) and acetone (11.25 mL).

### Azeotropic distillation

MTHP (30 mL) and NOP (only added in the 15^{th} coupling cycle: 5 mL) was added to the organic phase obtained from the preceding aqueous extractions, followed by concentration *in vacuo* (bath temperature 35-55 °C, pressure 100 hPa). This MTHP addition and concentration step was typically repeated once or twice. The water content was adjusted to be in the range of 261.0-312.3 ppm. The composition of the solution obtained after azeotropic distillation was analyzed as laid out above. The content of NOP was in the range of 19-42 vol-% and the content of MTHP was in the range of 58-81 vol-%. The combined content of MeCN, heptane, acetone and NMM was below 0.2 vol-% in all coupling cycles.

### Coupling reaction

The respective 5'-DMTr-protected phosphoramidite (15^{th} coupling cycle: 3.0 eq; 16^{th} coupling cycle: 3.5 eq; 17^{th} coupling cycle: 3.0 eq) and a mixture of TFA, NMI, and acetonitrile (11:9:30 v/v/v, 5 mL in total) were added to the solution obtained from azeotropic distillation, followed by stirring at rt until reaction monitoring by HPLC indicated completion of the coupling.

### Sulfurization (including quenching with triethylphosphite)

Pyridine (0.267 mL, 5.0 eq), NMI (0.75 mL), and DDTT (0.54-0.61 g, 4.0-4.5 eq) were added, and the mixture was stirred at rt for 10 min. Triethylphosphite (343 µL, 3.0 eq) was added, followed by stirring for 10 min at rt.

### Capping

Ac₂O (250 µL, 4.0 eq) was added, followed by stirring at rt for 10 min. Propan-2-ol (1.0 mL) was added, followed by stirring at rt for 10 min.

After completion of the 17^{th} coupling cycle, one more DMTr-deprotection, followed by aqueous extractions as laid out above in the general protocol were performed. The organic phase obtained from the final aqueous extraction was concentrated in *vacuo,* followed by subsequent addition of two portions of methanol (1^{st} portion: 50 mL, 2^{nd} portion: 100 mL). The resulting precipitate was filtered and washed with methanol (50 mL, twice) and then propan-2-ol (50 mL) to give mMeUs-mMeC(bz)s-MA(bz)s-mMeC(bz)s-mMeUs-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(22,** 1.75 g, crude).

### Target oligonucleotide O^{T}-a

A solution of the crude of compound **22** (98.3 mg, 0.01 mmol) in a mixture of H₂O and tert-butylamine (2:1 v/v, 9 mL) was stirred at 80 °C for 14 h. The reaction mixture was cooled to rt and analyzed by HPLC to find 5-mMeUs-mMeCs-mAs-mMeCs-mMeUs-mMeUs-mMeUs-mMeCs-mAs-mMeUs-mAs-mAs-mMeUs-MGs-mMeCs-mMeUs-MGs-MG-3 (O^{T}-a). Purity (Method E): 25.8%

### Example 12: Synthesis D of target oligonucleotide O^{T}-a (see Table T-2)

### DMTr-MG(ib)s-MG(ib)-(Kc-C1-carbonate) (5)

A solution of MG(ib)-(Kc-C1-carbonate) **(4,** 1.58 g, 1.32 mmol) in NOP (12 mL) was subjected to 2 rounds of azeotropic distillation, during each of which MTHP (15 mL) was added, followed by concentration *in vacuo* to a volume of 20 mL (bath temperature 45-55 °C, pressure 40-100 hPa). The so-obtained solution (water content: 104.2 ppm; GC-analysis: combined content of MeCN, Heptane, acetone and NMM was below 0.2 vol-%, NOP: 69 vol-%, MTHP: 31 vol-%) comprising MG(ib)-(Kc-C1-carbonate) **(4)** was used directly in the subsequent step (without any precipitation, filtration or purification steps in between). DMTr-MG(ib) phosphoramidite (CAS-RN: 251647-55-9, 2.41 g, 2.64 mmol, 2.0 eq), 5-benzylthio-1H-tetrazole (BTT, 2.54 g, 10.0 eq), N-methylimidazole (NMI, 2.0 mL), and acetonitrile (MeCN, 10 mL) were added to said solution comprising MG(ib)-(Kc-C1-carbonate) **(4)** and the mixture was stirred at rt until reaction monitoring by HPLC indicated completion of the coupling. Acetic acid (0.378 mL, 5.0 eq) was added, followed by stirring at rt for 20 min. Pyridine (0.533 mL, 5.0 eq) and 3-(N,N-dimethylaminomethylidene)amino)-3H-1,2,4-dithiazole-5-thione (CAS-RN: 1192027-04-5, DDTT, 1.02 g, 4.0 eq) were added. After stirring for 10 min at rt, triethylphosphite (914 µL, 4.0 eq) was added. After stirring for 10 min at rt, acetic anhydride (Ac₂O, 250 µL, 2.0 eq) was added, followed by stirring at rt for 10 min, addition of propan-2-ol (2.0 mL), and stirring for 10 min at rt. The so-obtained solution comprising DMTr-MG(ib)s-MG(ib)-(Kc-C1-carbonate) **(5)** was used directly in the subsequent step (without any precipitation, filtration or purification steps in between).

### 2^{nd} to 14^{th} coupling cycle

The 2^{nd} to 14^{th} coupling cycle were performed according to the following general protocol, unless indicated differently. The starting materials and products of each coupling cycle are identical to those listed in Table E-1 above. Again, the terms "starting material" and "product" are not to be construed to indicate any precipitation and/or isolation steps in between the coupling cycles.

### General protocol for coupling cycles

In a typical coupling cycle, the following steps were performed in the presented order, unless indicated differently.

### DMTr-deprotection

Trifluoroacetic acid (TFA, 15.0 mL) and 3-mercaptopropionic acid (MPA, 1.15 mL), were added at rt directly to the solution obtained from the preceding capping step. The mixture was stirred at rt for 45 min.

### Aqueous extractions

N-methylmorpholine (NMM, 35 mL), n-heptane (15 mL), MTHP (7.5-10.0 mL), and 15 wt-% aqueous (aq.) sodium chloride solution (brine, 45 mL) were added, followed by extraction. The organic phase was separated and extracted as follows:
- with a mixture of NMM (5 mL), 5 wt-% aqueous sodium chloride solution (brine, 22.5 mL), and acetone (22.5 mL), next
- with a mixture of 5 wt-% aqueous sodium chloride solution (brine, 22.5 mL) and acetone (22.5 mL), next
- with a mixture of 5 wt-% aqueous sodium chloride solution (brine, 22.5 mL), acetone (22.5 mL), and acetic acid (0.6-1.2 mL), and lastly
- with a mixture of 5 wt-% aqueous sodium chloride solution (brine, 22.5 mL) and acetone (22.5 mL).

### Azeotropic distillation

MTHP (30 mL) and NOP (2^{nd} to 4^{th} 8^{th} 10^{th} coupling cycle: 3 mL; 5^{th} and 9^{th} coupling cycle: 2 mL; 6^{th} coupling cycle; 5mL; 7^{th} coupling cycle: 1 mL; 11^{th} and 12^{th} coupling cycle: 2.5 mL) were added to the organic phase obtained from the preceding aqueous extractions, followed by concentration *in vacuo* (bath temperature 35-55 °C, pressure 100 hPa). This MTHP addition and concentration step was typically repeated once or twice. The water content was adjusted to be in the range of 58.0-231.6 ppm. The composition of the solution obtained after azeotropic distillation was analyzed as laid out above. The content of NOP was in the range of 25-58 vol-% and the content of MTHP was in the range of 42-75 vol-%. The combined content of MeCN, heptane, acetone and NMM was below 0.2 vol-% in all coupling cycles.

### Coupling reaction

The respective 5'-DMTr-protected phosphoramidite (2^{nd} to 6^{th} coupling cycle: 2.0 eq; 7^{th}, 8^{th}, 10^{th}, and 11^{th} coupling cycle: 2.5 eq; 9^{th} and 12^{th} to 14^{th} coupling cycle: 3.0 eq) 5-benzylthio-1*H*-tetrazole (BTT, 2.54 g, 10.0 eq), N-methylimidazole (NMI, 2.0 mL), and acetonitrile (MeCN, 10 mL) were added to the solution obtained from azeotropic distillation, followed by stirring at rt until reaction monitoring by HPLC indicated completion of the coupling. Acetic acid (0.378 mL, 5.0 eq) was added, followed by stirring at rt for 20 min.

### Sulfurization (including quenching with triethylphosphite)

Pyridine (0.533 mL, 5.0 eq) and DDTT (1.02-1.22 g, 4.0-5.0 eq) were added, and the mixture was stirred at rt for 10 min. Triethylphosphite (914 µL, 4.0 eq) was added, followed by stirring for 10 min at rt.

### Capping

Ac₂O (2^{nd} and 3^{rd} coupling cycle: 0.25 mL, 2.0 eq; 4^{th}, 5^{th}, and 10^{th} to 14^{th} coupling cycle: 0.013 mL, 0.1 eq; 6^{th} to 9^{th} coupling cycle: 0.062 mL, 0.5 eq) was added, followed by stirring at rt for 10 min. Propan-2-ol (2.0 mL) was added, followed by stirring at rt for 10 min.

After completion of the 14^{th} coupling cycle, the batch was divided into two halves of equal volumes, only one of which was subjected to the 15^{th} to 17^{th} coupling cycle.

### 15^{th} to 17^{th} coupling cycle

The 15^{th} to 17^{th} coupling cycle were performed according to the following general protocol, unless indicated differently. The starting materials and products of each coupling cycle are identical to those listed in Table E-1 above. Again, the terms "starting material" and "product" are not to be construed to indicate any precipitation and/or isolation steps in between the coupling cycles.

### General protocol for coupling cycles

In a typical coupling cycle, the following steps were performed in the presented order, unless indicated differently.

### DMTr-deprotection

Trifluoroacetic acid (TFA, 7.5 mL) and 3-mercaptopropionic acid (MPA, 15^{th} coupling cycle: 0.547 mL, 10.0 eq; 16^{th} and 17^{th} coupling cycle: 1.148 mL, 20.0 eq), were added at rt directly to the solution obtained from the preceding capping step. The mixture was stirred at rt for 45 min.

### Aqueous extractions

N-methylmorpholine (NMM, 17.5 mL), n-heptane (7.5 mL), NOP (1.0-2.0 mL), MTHP (3.75-7.0 mL), and 15 wt-% aqueous (aq.) sodium chloride solution (brine, 45 mL) were added, followed by extraction. The organic phase was separated and extracted as follows:
- with a mixture of NMM (2.5 mL), 5 wt-% aqueous sodium chloride solution (brine, 11.25 mL), and acetone (11.25 mL), next
- with a mixture of 5 wt-% aqueous sodium chloride solution (brine, 11.25 mL) and acetone (11.25 mL), next
- with a mixture of 5 wt-% aqueous sodium chloride solution (brine, 11.25 mL), acetone (11.25 mL), and acetic acid (1.2-1.4 mL), and lastly
- with a mixture of 5 wt-% aqueous sodium chloride solution (brine, 11.25 mL) and acetone (11.25 mL).

### Azeotropic distillation

MTHP (15 mL) was added to the organic phase obtained from the preceding aqueous extractions, followed by concentration *in vacuo* (bath temperature 35-55 °C, pressure 100 hPa). This MTHP addition and concentration step was typically repeated once or twice. The water content was adjusted to be in the range of 239.1-296.1 ppm. The composition of the solution obtained after azeotropic distillation was analyzed as laid out above. The content of NOP was in the range of 22-27 vol-% and the content of MTHP was in the range of 78-73 vol-%. The combined content of MeCN, heptane, acetone and NMM was below 0.2 vol-% in all coupling cycles.

### Coupling reaction

The respective 5'-DMTr-protected phosphoramidite (15^{th} coupling cycle: 4.0 eq; 16^{th} and 17^{th} coupling cycle: 5.0 eq), 5-benzylthio-1H-tetrazole (BTT, 1.26 g, 10.0 eq), N-methylimidazole (NMI, 1.0 mL), and acetonitrile (MeCN, 5.0 mL) were added to the solution obtained from azeotropic distillation, followed by stirring at rt until reaction monitoring by HPLC indicated completion of the coupling. Acetic acid (0.189 mL, 5.0 eq) was added, followed by stirring at rt for 20 min.

### Sulfurization (including quenching with triethylphosphite)

Pyridine (0.266 mL, 5.0 eq) and DDTT (0.68-1.36 g, 5.0-10.0 eq) were added, and the mixture was stirred at rt for 10 min. Triethylphosphite (0.457-1.14 mL, 5.0-10.0 eq) was added, followed by stirring for 10 min at rt.

### Capping

Ac₂O (15^{th} coupling cycle: 0.016 mL, 0.25 eq; 16^{th} coupling cycle: 0.031 mL, 0.5 eq; 17^{th} coupling cycle: 0.062 mL, 0.5 eq) was added, followed by stirring at rt for 10 min. Propan-2-ol (1.0 mL) was added, followed by stirring at rt for 10 min.

After completion of the 17^{th} coupling cycle, one more DMTr-deprotection, followed by aqueous extractions as laid out above in the general protocol were performed. The organic phase obtained from the final aqueous extraction was concentrated in *vacuo,* followed by addition of methanol (100 mL). The resulting precipitate was filtered and washed with methanol (50 mL, twice) to give mMeUs-mMeC(bz)s-MA(bz)s-mMeC(bz)s-mMeUs-mMeUs-mMeUs-mMeC(bz)s-MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(22,** 1.70 g, crude).

### Target oligonucleotide O^{T}-a

A solution of the crude of compound **22** (101 mg, 0.01 mmol) in a mixture of H₂O and tert-butylamine (2:1 v/v, 9 mL) was stirred at 80 °C for 11 h. The reaction mixture was cooled to room temperature and analyzed by HPLC-MS to find 5-mMeUs-mMeCs-mAs-mMeCs-mMeUs-mMeUs-mMeUs-mMeCs-mAs-mMeUs-mAs-mAs-mMeUs-MGs-mMeCs-mMeUs-MGs-MG-3 (O^{T}-a). MS (Method E) m/z: [M - 3H]³⁻calcd for C₂₃₄H₃₃₇N₆₁O₁₂₈P₁₇S₁₇³⁻, 2373.0848, found 2373.0798. Purity (Method E): 42.8%.

### Example 13: Synthesis C of target oligonucleotide O^{T}-b (see Table T-2)

### DMTr-dG(ib)-dT-(Kc-C1-carbonate) (24)

dT-(Kc-C1-carbonate) **(23)** was synthesized as described for Example 3 above. A solution of dT-(Kc-C1-carbonate) **(23,** 1.36 g, 1.33 mmol) in 4-methyltetrahydropyran (MTHP)/*N*-cyclohexyl-2-pyrrolidone (NcHP) (7:1 v/v, 80 mL) was concentrated *in vacuo* to a volume of 40 mL (bath temperature 35-55 °C, pressure 100 hPa). 1-Methylimidazole (NMI, 2.0 mL, 25.1 mmol), DMTr-dG(ib) phosphoramidite (CAS-RN: 93183-15-4, 2.22 g, 2.64 mmol) and BTT (2.54 g, 13.2 mmol) were added to said solution comprising dT-(Kc-C1-carbonate) **(23),** and the mixture was stirred at rt until reaction monitoring by HPLC (Method F was used throughout Example 13) indicated completion of the coupling. Ac₂O (499 µL, 5.28 mmol) and pyridine (533 µL, 6.60 mmol) were added. After stirring for 10 min at rt, 1 M I₂ in cyclopentyl methyl ether (CPME, 2.91 mL, 2.91 mmol), H₂O (71 µL), and acetonitrile (MeCN, 3.0 mL) were added. After stirring for 10 min at rt, the reaction mixture was extracted twice: first with 0.5 N Na₂S₂O₃ aq. (20 mL), then with a mixture of 0.5 N Na₂S₂O₃ aq. (10 mL) and 0.5 M N-methylmorpholine (NMM) aq. (5 mL). The so-obtained solution (organic phase) comprising DMTr-dG(ib)-dT-(Kc-C1-carbonate) **(24)** was used directly in the next step (without any precipitation, filtration or purification steps in between).

### DMTr-dA(bz)-dG(ib)-dT-(Kc-C1-carbonate) (26)

Thiomalic acid (1.99 g, 13.3 mmol) and dichloroacetic acid (DCA, 10.0 mL) were added to the so-obtained organic phase, followed by stirring at rt until reaction monitoring by HPLC indicated complete DMTr-deprotection. NcHP (2.5 mL), 10 wt-% brine (45 mL), NMM (20 mL) and MTHP 20 mL were added, followed by extraction. The organic phase was separated and extracted twice: first with a mixture of 10 wt-% brine/N,N-dimethylformamide (DMF) solution (45:55 v/v, 40 mL) and acetic acid (200 µL), then with 10 wt-% brine/*N*,*N*-dimethylformamide (DMF) solution (45:55 v/v, 40 mL). MTHP (15 mL) was added to the so-obtained organic phase comprising dG(ib)-dT-(Kc-C1-carbonate) **(25),** followed by concentration *in vacuo* to a volume of 40 mL (bath temperature 35-55 °C, pressure 100 hPa). The MTHP (30 mL) addition and subsequent concentration was repeated three times (final volume: 40 mL). 1-Methylimidazole (2.0 mL, 25.1 mmol), DMTr-dA(bz) phosphoramidite (CAS-RN: 98796-53-3, 2.31 g, 2.64 mmol) and BTT (2.54 g, 13.2 mmol) were added to said solution comprising dG(ib)-dT-(Kc-C1-carbonate) **(25),** and the mixture was stirred at rt until reaction monitoring by HPLC indicated completion of the coupling. Ac₂O (499 µL, 5.28 mmol) and pyridine (533 µL, 6.60 mmol) were added. After stirring for 10 min at rt, 1 M I₂ in CPME (2.91 mL, 2.91 mmol), H₂O (71 µL), and acetonitrile (MeCN, 3.0 mL) were added. After stirring for 10 min at rt, the reaction mixture was extracted twice: first with 0.5 N Na₂S₂O₃ aq. (20 mL), then with a mixture of 0.5 N Na₂S₂O₃ aq. (10 mL) and 0.5 M NMM aq. (5 mL). The so-obtained solution (organic phase) comprising DMTr-dA(bz)-dG(ib)-dT-(Kc-C1-carbonate) **(26)** was used directly in the next step (without any precipitation, filtration or purification steps in between).

### DMTr-dC(bz)-dA(bz)-dG(ib)-dT-(Kc-C1-carbonate) (28)

Thiomalic acid (1.99 g, 13.3 mmol) and dichloroacetic acid (DCA, 15 mL) were added to the so-obtained organic phase, followed by stirring at rt until reaction monitoring by HPLC indicated complete DMTr-deprotection. NcHP (2.5 mL), 10 wt-% brine (75 mL), and NMM (24 mL) were added, followed by extraction. The organic phase was separated and extracted twice: first with 10 wt-% brine/N,N-dimethylformamide (DMF) solution (45:55 v/v, 40 mL), then with a mixture of 10 wt-% brine/N,N-dimethylformamide (DMF) solution (45:55 v/v, 40 mL) and acetic acid (50 µL). MTHP (30 mL) was added to the so-obtained organic phase comprising dA(bz)-dG(ib)-dT-(Kc-C1-carbonate) **(27),** followed by concentration *in vacuo* to a volume of 30 mL (bath temperature 35-55 °C, pressure 100 hPa). The MTHP (30 mL) addition and subsequent concentration was repeated twice (final volume: 40 mL). 1-Methylimidazole (2.0 mL, 25.1 mmol), DMTr-dC(bz) phosphoramidite (CAS-RN: 102212-98-6, 2.24 g, 2.64 mmol) and BTT (2.54 mg, 13.2 mmol) were added to said solution comprising dA(bz)-dG(ib)-dT-(Kc-C1-carbonate) **(27),** and the mixture was stirred at rt until reaction monitoring by HPLC indicated completion of the coupling. Ac₂O (499 µL, 5.28 mmol) and pyridine (533 µL, 6.60 mmol) were added. After stirring for 10 min at rt, 1 M I₂ in CPME (2.91 mL, 2.91 mmol), H₂O (71 µL), and acetonitrile (MeCN, 3.0 mL) were added. After stirring for 10 min at rt, the reaction mixture was extracted twice: first with 0.5 N Na₂S₂O₃ aq. (20 mL), then with a mixture of 0.5 N Na₂S₂O₃ aq. (10 mL) and 0.5 M NMM aq. (5 mL). The so-obtained solution (organic phase) comprising DMTr-dC(bz)-dA(bz)-dG(ib)-dT-(Kc-C1-carbonate) **(28)** was used directly in the next step (without any precipitation, filtration or purification steps in between).

### dT-dC(bz)-dA(bz)-dG(ib)-dT-(Kc-C1-carbonate) (O^{T}-b)

Thiomalic acid (1.98 g, 13.2 mmol) and dichloroacetic acid (DCA, 10 mL) were added to the so-obtained organic phase, followed by stirring at rt until reaction monitoring by HPLC indicated complete DMTr-deprotection. NcHP (2.5 mL), 10 wt-% brine (75 mL), and NMM (20 mL) were added, followed by extraction. The organic phase was separated and extracted twice with 10 wt-% brine/*N*,*N-*dimethylformamide (DMF) solution (45:55 v/v, 40 mL × 2). MTHP (30 mL) was added to the so-obtained organic phase comprising dC(bz)-dA(bz)-dG(ib)-dT-(Kc-C1-carbonate) **(29),** followed by concentration *in vacuo* to a volume of 40 mL (bath temperature 35-55 °C, pressure 100 hPa). The MTHP (30 mL) addition and subsequent concentration was repeated twice (final volume: 40 mL). 1-Methylimidazole (2.0 mL, 25.1 mmol), DMTr-dT phosphoramidite (CAS-RN: 98796-51-1, 2.01 g, 2.64 mmol) and BTT (2.56 g, 13.3 mmol) were added to said solution comprising dC(bz)-dA(bz)-dG(ib)-dT-(Kc-C1-carbonate) **(29),** and the mixture was stirred at rt until reaction monitoring by HPLC indicated completion of the coupling. Ac₂O (499 µL, 5.28 mmol) and pyridine (533 µL, 6.60 mmol) were added. After stirring for 10 min at rt, 1 M I₂ in CPME (2.91 mL, 2.91 mmol), H₂O (71 µL), and MeCN (3.0 mL) were added. After stirring for 10 min at rt, the reaction mixture was extracted twice: first with 0.5 N Na₂S₂O₃ aq. (20 mL), then with a mixture of 0.5 N Na₂S₂O₃ aq. (10 mL) and 0.5 M NMM aq. (5 mL). The so-obtained solution (organic phase) comprising DMTr-dT-dC(bz)-dA(bz)-dG(ib)-dT-(Kc-C1-carbonate) **(30)** was used directly in the next step (without any precipitation, filtration or purification steps in between).

Thiomalic acid (1.99 g, 13.3 mmol) and dichloroacetic acid (DCA, 10 mL) were added to the so-obtained organic phase, followed by stirring at rt until reaction monitoring by HPLC indicated complete DMTr-deprotection. NcHP (2.5 mL), 10 wt-% brine (25 mL), and NMM (20 mL) were added, followed by extraction. The organic phase was separated and extracted twice: first with 10 wt-% brine/DMF solution (45:55 v/v, 40 mL), then with MTHP (90 mL), NcHP (5 mL) and brine/DMF solution (65:35 v/v, 65 mL). The so-obtained organic phase comprising dT-dC(bz)-dA(bz)-dG(ib)-dT-(Kc-C1-carbonate) (O^{T}-b) was concentrated in vacuo, followed by addition of MeOH (120 mL). The resulting precipitate was filtered and washed twice with MeOH (30 mL × 2) to give dT-dC(bz)-dA(bz)-dG(ib)-dT-(Kc-C1-carbonate) (O^{T}-b, 1.94 g, purity 71.647 %, yield 53.7 %) as a white solid. MS (method F) m/z: [M + 2H]²⁺ calcd for C₁₃₁H₁₈₅N₂₁O₃₆P₄⁺, 1376.1115; found 1376.1138.

### Example 14: Synthesis D of target oligonucleotide O^{T}-b (see Table T-2)

### DMTr-dG(ib)-dT-(Kc-C1-carbonate) (24)

dT-(Kc-C1-carbonate) **(23)** was synthesized as described for Example 3 above. A solution of dT-(Kc-C1-carbonate) **(23,** 1.36 g, 1.33 mmol) in 4-methyltetrahydropyran (MTHP)/*N*,*N*-diethyldodecanamide (7:1 v/v, 80 mL) was concentrated *in vacuo* to a volume of 40 mL (bath temperature 35-55 °C, pressure 100 hPa). 1-Methylimidazole (NMI, 2.0 mL, 25.1 mmol), DMTr-dG(ib) phosphoramidite (CAS-RN: 93183-15-4, 2.21 g, 2.63 mmol) and BTT (2.54 g, 13.2 mmol) were added to said solution comprising dT-(Kc-C1-carbonate) **(23),** and the mixture was stirred at rt until reaction monitoring by HPLC (Method F was used throughout Example 14) indicated completion of the coupling. Ac₂O (499 µL, 5.28 mmol) and pyridine (533 µL, 6.60 mmol) were added. After stirring for 10 min at rt, 1 M I₂ in cyclopentyl methyl ether (CPME, 2.91 mL, 2.91 mmol), H₂O (71 µL), and acetonitrile (MeCN, 3.0 mL) were added. After stirring for 10 min at rt, the reaction mixture was extracted twice: first with 0.5 N Na₂S₂O₃ aq. (20 mL), then with a mixture of 0.5 N Na₂S₂O₃ aq. (10 mL) and 0.5 M *N*-methylmorpholine (NMM) aq. (5 mL). The so-obtained solution (organic phase) comprising DMTr-dG(ib)-dT-(Kc-C1-carbonate) **(24)** was used directly in the next step (without any precipitation, filtration or purification steps in between).

### DMTr-dA(bz)-dG(ib)-dT-(Kc-C1-carbonate) (26)

Thiomalic acid (1.99 g, 13.3 mmol) and dichloroacetic acid (DCA, 10.0 mL) were added to the so-obtained organic phase, followed by stirring at rt until reaction monitoring by HPLC indicated complete DMTr-deprotection. *N*,*N-*diethyldodecanamide (2.5 mL), 10 wt-% brine (25 mL) and NMM (20 mL) were added, followed by extraction. The organic phase was separated and extracted twice: first with 10 wt-% brine/*N*,*N*-dimethylformamide (DMF) solution (45:55 v/v, 40 mL) and acetic acid (500 µL), then with 10 wt-% brine/N,N-dimethylformamide (DMF) solution (45:55 v/v, 40 mL) and acetic acid (50 µL). MTHP (30 mL) was added to the so-obtained organic phase comprising dG(ib)-dT-(Kc-C1-carbonate) **(25),** followed by concentration *in vacuo* to a volume of 40 mL (bath temperature 35-55 °C, pressure 100 hPa). The MTHP (30 mL) addition and subsequent concentration was repeated once (final volume: 40 mL). 1-Methylimidazole (2.0 mL, 25.1 mmol), DMTr-dA(bz) phosphoramidite (CAS-RN: 98796-53-3, 2.31 g, 2.64 mmol) and BTT (2.54 g, 13.2 mmol) were added to said solution comprising dG(ib)-dT-(Kc-C1-carbonate) **(25),** and the mixture was stirred at rt until reaction monitoring by HPLC indicated completion of the coupling. Ac₂O (499 µL, 5.28 mmol) and pyridine (533 µL, 6.60 mmol) were added. After stirring for 10 min at rt, 1 M I₂ in CPME (2.91 mL, 2.91 mmol), H₂O (71 µL), and acetonitrile (MeCN, 3.0 mL) were added. After stirring for 10 min at rt, the reaction mixture was extracted twice: first with 0.5 N Na₂S₂O₃ aq. (20 mL), then with a mixture of 0.5 N Na₂S₂O₃ aq. (10 mL) and 0.5 M NMM aq. (5 mL). The so-obtained solution (organic phase) comprising DMTr-dA(bz)-dG(ib)-dT-(Kc-C1-carbonate) **(26)** was used directly in the next step (without any precipitation, filtration or purification steps in between).

### DMTr-dC(bz)-dA(bz)-dG(ib)-dT-(Kc-C1-carbonate) (28)

Thiomalic acid (1.98 g, 13.2 mmol) and dichloroacetic acid (DCA, 15 mL) were added to the so-obtained organic phase, followed by stirring at rt until reaction monitoring by HPLC indicated complete DMTr-deprotection. *N,N-*diethyldodecanamide (2.5 mL), 10 wt-% brine (25 mL), and NMM (25 mL) were added, followed by extraction. The organic phase was separated and extracted twice with 10 wt-% brine/*N*,*N*-dimethylformamide (DMF) solution (45:55 v/v, 40 mL × 2). MTHP (30 mL) was added to the so-obtained organic phase comprising dA(bz)-dG(ib)-dT-(Kc-C1-carbonate) **(27),** followed by concentration *in vacuo* to a volume of 30 mL (bath temperature 35-55 °C, pressure 100 hPa). The MTHP (30 mL) addition and subsequent concentration was repeated once (final volume: 40 mL). 1-Methylimidazole (2.0 mL, 25.1 mmol), DMTr-dC(bz) phosphoramidite (CAS-RN: 102212-98-6, 2.25 g, 2.65 mmol) and BTT (2.54 mg, 13.2 mmol) were added to said solution comprising dA(bz)-dG(ib)-dT-(Kc-C1-carbonate) **(27),** and the mixture was stirred at rt until reaction monitoring by HPLC indicated completion of the coupling. Ac₂O (499 µL, 5.28 mmol) and pyridine (533 µL, 6.60 mmol) were added. After stirring for 10 min at rt, 1 M I₂ in CPME (2.91 mL, 2.91 mmol), H₂O (71 µL), and acetonitrile (MeCN, 3.0 mL) were added. After stirring for 10 min at rt, the reaction mixture was extracted twice: first with 0.5 N Na₂S₂O₃ aq. (20 mL), then with a mixture of 0.5 N Na₂S₂O₃ aq. (10 mL) and 0.5 M NMM aq. (5 mL). The so-obtained solution (organic phase) comprising DMTr-dC(bz)-dA(bz)-dG(ib)-dT-(Kc-C1-carbonate) **(28)** was used directly in the next step (without any precipitation, filtration or purification steps in between).

### dT-dC(bz)-dA(bz)-dG(ib)-dT-(Kc-C1-carbonate) (O^{T}-b)

Thiomalic acid (1.98 g, 13.2 mmol) and dichloroacetic acid (DCA, 10 mL) were added to the so-obtained organic phase, followed by stirring at rt until reaction monitoring by HPLC indicated complete DMTr-deprotection. *N,N-*diethyldodecanamide (2.5 mL), 10 wt-% brine (25 mL), and NMM (20 mL) were added, followed by extraction. The organic phase was separated and extracted twice: first with a mixture of 10 wt-% brine/*N*,*N*-dimethylformamide (DMF) solution (45:55 v/v, 40 mL) and acetic acid (650 µL), then with 10 wt-% brine/*N*,*N-*dimethylformamide (DMF) solution (45:55 v/v, 40 mL). MTHP (30 mL) was added to the so-obtained organic phase comprising dC(bz)-dA(bz)-dG(ib)-dT-(Kc-C1-carbonate) **(29),** followed by concentration *in vacuo* to a volume of 40 mL (bath temperature 35-55 °C, pressure 100 hPa). The MTHP (30 mL) addition and subsequent concentration was repeated once (final volume: 40 mL). 1-Methylimidazole (2.0 mL, 25.1 mmol), DMTr-dT phosphoramidite (CAS-RN: 98796-51-1, 2.01 g, 2.64 mmol) and BTT (2.54 g, 13.2 mmol) were added to said solution comprising dC(bz)-dA(bz)-dG(ib)-dT-(Kc-C1-carbonate) **(29),** and the mixture was stirred at rt until reaction monitoring by HPLC indicated completion of the coupling. Ac₂O (499 µL, 5.28 mmol) and pyridine (533 µL, 6.60 mmol) were added. After stirring for 10 min at rt, 1 M I₂ in CPME (2.91 mL, 2.91 mmol), H₂O (71 µL), and MeCN (3.0 mL) were added. After stirring for 10 min at rt, the reaction mixture was extracted twice: first with 0.5 N Na₂S₂O₃ aq. (20 mL), then with a mixture of 0.5 N Na₂S₂O₃ aq. (10 mL) and 0.5 M NMM aq. (5 mL). The so-obtained solution (organic phase) comprising DMTr-dT-dC(bz)-dA(bz)-dG(ib)-dT-(Kc-C1-carbonate) **(30)** was used directly in the next step (without any precipitation, filtration or purification steps in between).

Thiomalic acid (1.99 g, 13.3 mmol) and dichloroacetic acid (DCA, 10 mL) were added to the so-obtained organic phase, followed by stirring at rt until reaction monitoring by HPLC indicated complete DMTr-deprotection. *N,N-*Diethyldodecanamide (2.5 mL), 10 wt-% brine (25 mL), and NMM (20 mL) were added, followed by extraction. The organic phase was separated and extracted twice: first with a mixture of 10 wt-% brine/DMF solution (45:55 v/v, 40 mL) and acetic acid (600 µL), then with brine/DMF solution (45:55 v/v, 40 mL) and acetic acid (100 µL). The so-obtained organic phase comprising dT-dC(bz)-dA(bz)-dG(ib)-dT-(Kc-C1-carbonate) (O^{T}-b) was concentrated in vacuo, followed by addition of MeOH (120 mL). The resulting precipitate was filtered and washed twice with MeOH (30 mL × 2) to give dT-dC(bz)-dA(bz)-dG(ib)-dT-(Kc-C1-carbonate) (O^{T}-b, 2.08 g, purity 71.125 %, yield 57.5 %) as a white solid. MS (method F) m/z: [M + 2H]²⁺ calcd for C₁₃₁H₁₈₅N₂₁O₃₆P₄⁺, 1376.1115; found 1376.1111.

### Example 15: Synthesis C of target oligonucleotide O^{T}-f (see Table T-2)

### DMTr-MG(ib)s-MG(ib)-(Kc-C1-carbonate) (5)

A solution of MG(ib)-(Kc-C1-carbonate) **(4,** 1.58 g, 1.32 mmol) in *N*-octyl-2-pyrrolidone (NOP, 7.5 mL) was subjected to 5 rounds of azeotropic distillation, during each of which 4-methyltetrahydropyran (MTHP, 15 mL) was added, followed by concentration *in vacuo* to a volume of 15 mL (bath temperature 45-55 °C, pressure 40-100 hPa). DMTr-MG(ib) phosphoramidite (CAS-RN: 251647-55-9, 2.43 g, 2.66 mmol, 2.0 eq.) and MTHP (10 mL) were added to the so-obtained solution (water content: 370.8 ppm) comprising MG(ib)-(Kc-C1-carbonate) **(4),** followed by concentration *in vacuo* (bath temperature 45 °C, pressure 40 hPa) to a volume of 15 mL. 5-Ethylthio-*1H*-tetrazole (1.73 g, 13.29 mmol, 10.1 eq.) and pyridine (2.13 mL, 26.39 mmol, 20.0 eq.) were added to said solution comprising MG(ib)-(Kc-C1-carbonate) **(4),** and the mixture was stirred at rt until reaction monitoring by HPLC (Method H was used throughout Example 15) indicated completion of the coupling. Water (24 µL, 1.32 mmol, 1.0 eq.) was added, followed by stirring for 30 min. 3-(N,N-dimethylaminomethylidene)amino)-3*H*-1,2,4-dithiazole-5-thione (CAS-RN: 1192027-04-5, DDTT, 0.816 g, 3.97 mmol, 3.0 eq) was added. After stirring for 10 min at rt, triethylphosphite (0.686 mL, 3.0 eq) was added. After stirring for 10 min at rt, propan-2-ol (2.0 mL) was added, followed by stirring for 10 min at rt. The so-obtained solution comprising DMTr-MG(ib)s-MG(ib)-(Kc-C1-carbonate) **(5)** was used directly in the subsequent step (without any precipitation, filtration or purification steps in between).

### Second coupling cycle and further coupling cycles

The second coupling cycle and all further coupling cycles were performed according to the following general protocol, unless indicated differently. The starting materials and products of each coupling cycle are identical to those listed in Table E-1 above, wherein, except for the above-mentioned first coupling cycle, only the 2^{nd} to 9^{th} coupling cycles were performed in the present Example 15. Again, the terms "starting material" and "product" are not to be construed to indicate any precipitation and/or isolation steps in between the coupling cycles.

### General protocol for coupling cycles

In a typical coupling cycle, the following steps were performed in the presented order, unless indicated differently.

### DMTr-deprotection

At rt, glutathione (GSH, 2.03 g) was added directly to the solution obtained from the preceding sulfurization step, then trifluoroacetic acid (TFA, 15.0 mL) was added at 0 °C. The mixture was stirred at rt for 10 min.

### Aqueous extractions

Pyridine (25 mL), n-heptane (15.0 mL), MTHP (15 mL), and 15 wt-% aqueous (aq.) sodium chloride solution (brine, 90 mL) were added, followed by extraction. The organic phase was separated and extracted as follows:
- with a mixture of NOP (2^{nd} to 8^{th} cycle: 2.0 mL; 9^{th} cycle: 0 mL), 5 wt-% aqueous sodium chloride solution (brine, 45 mL), and acetone (45 mL), next
- with a mixture of 5 wt-% aqueous sodium chloride solution (brine, 45 mL) and acetone (45 mL).

### Azeotropic distillation

MTHP (15 mL) was added to the organic phase obtained from the preceding aqueous extractions, followed by concentration *in vacuo* (bath temperature 35-55 °C, pressure 40-200 hPa) to a volume of 15 mL. This MTHP addition and concentration step was typically repeated one to three times. The water content was adjusted to be in the range of 305.4-390.3 ppm. Then, the respective 5'-DMTr-protected phosphoramidite ( 2.0 eq.) and MTHP (10 mL) were added to the solution, followed by concentration *in vacuo* (bath temperature 45 °C, pressure 40-100 hPa) to a volume of 15 mL. The composition of the solution obtained after azeotropic distillation was analyzed as laid out above. The content of NOP was in the range of 53-72 vol-% and the content of MTHP was in the range of 47-28 vol-%. The combined content of n-heptane, acetone and pyridine was below 0.2 vol-% in all coupling cycles.

### Coupling reaction

5-Ethylthio-*1H*-tetrazole (10 eq.) and pyridine (20 eq.) were added to the solution obtained from azeotropic distillation, followed by stirring at rt until reaction monitoring by HPLC indicated completion of the coupling.

### Sulfurization (including quenching with triethylphosphite and propan-2-ol)

DDTT (3.0 eq.) was added, and the mixture was stirred at rt for 30 min. Triethylphosphite (3.0 eq) was added, followed by stirring for 10 min at rt. Then, propan-2-ol (2.0 mL) was added, followed by stirring for 10 min at rt.

After completion of the 9^{th} coupling cycle, one more DMTr-deprotection, followed by aqueous extractions as laid out above in the general protocol were performed. The organic phase obtained from the final aqueous extraction was concentrated *in vacuo,* followed by addition of methanol (240 mL). The resulting precipitate was filtered and washed with methanol (60 mL, twice) to give MA(bz)s-mMeUs-MA(bz)s-MA(bz)s-mMeUs-MG(ib)s-mMeC(bz)s-mMeUs-MG(ib)s-MG(ib)-(Kc-C1-carbonate) **(13,** 3.64 g, 47.1% crude yield)

### Target oligonucleotide O^{T}-f (see Table T-2)

A solution of the crude compound **13** (502.6 mg, 0.086 mmol) in a mixture of H₂O, methanol and tert-butylamine (2:1:1 v/v, 15 mL) was stirred at 55 °C for 17 h. The reaction mixture was cooled to rt and the precipitate was filtrated and the filtrate was concentrated *in vacuo* (bath temperature 45 °C, pressure 40 hPa) to give 5'-MAs-MMeUs-MAs-MAs-MMeUs-MGs-MMeCs-MMeUs-MGs-MG-3' (O^{T}-f, 502.0 mg, quantitative) as white solid. The so-obtained O^{T}-f (28.6 mg) was washed with mixture of acetonitrile and H₂O (99:1 v/v. 1.0 mL), followed by filtration to give 5'-MAs-MMeUs-MAs-MAs-MMeUs-MGs-MMeCs-MMeUs-MGs-MG-3' (O^{T}-f, 19.0 mg, 95.0 % crude yield from crude compound **13)** as white solid, which was analyzed by HPLC-MS to find 5'-MAs-MMeUs-MAs-MAs-MMeUs-MGs-MMeCs-MmeUs-MGs-MG-3' (O^{T}-f). MS (Method G) m/z: [M-3H]³⁻ calcd for C₁₃₀H₁₈₃N₃₉O₆₉P₉S₉³⁻,1320.2384, found 1320.2412. Purity (Method C, Gradient-2): 78.7%.

## Claims

1. A method for the synthesis of a target oligonucleotide O^{T}, said method comprising a step of subjecting a solution comprising a component C selected from the group consisting of a nucleoside and an oligonucleotide to one or more aqueous extractions, wherein the organic phase comprises the component C, and wherein
- said component C is covalently bonded to a pseudo solid-phase protecting group PG-s, and
- each of said one or more aqueous extractions is carried out in the presence of one or more amide solvents S^{A}, wherein each amide solvent S^{A} is an amide solvent comprising one or more alkyl groups, wherein these one or more alkyl groups together comprise in total 6-24 carbon atoms.

2. The method according to claim 1, wherein the target oligonucleotide O^{T} comprises a first cycle oligonucleotide O-1, and the method comprises the following step (a-1), and a first coupling cycle comprising the following steps:
| | |
|---|---|
| (a-1) | providing a component C-0 selected from the group consisting of a nucleoside and an oligonucleotide, wherein the component C-0 is covalently bonded to a pseudo solid-phase protecting group PG-s and comprises a backbone hydroxyl moiety protected by a protecting group PG-0 removable under acidic conditions; |
| (b-1) | incubating the component C-0 of step (a-1) with a deprotection mixture M-(b-1), thereby cleaving the protecting group PG-0 from the component C-0, so as to obtain a component (C-0)^{#} having a free backbone hydroxyl group; |
| (c-1) | subjecting a solution comprising the component (C-0)^{#} to one or more aqueous extractions, wherein the organic phase comprises the component (C-0)^{#}; |
| (d-1) | optionally, reducing the water content of the organic phase comprising the component (C-0)^{#}; |
| (e-1) | reacting the component (C-0)^{#} with a building block B-1, wherein said building block B-1 is selected from the group consisting of a nucleoside and an oligonucleotide and comprises |
| | - a backbone hydroxyl moiety protected by a protecting group PG-1 removable under acidic conditions, and |
| | - a phosphorus moiety covalently bonded via its phosphorus atom to an oxygen atom of the backbone of the building block B-1, |
| | under conditions suitable to form a covalent bond between said free backbone hydroxyl group of the component (C-0)^{#} and the phosphorus atom of said phosphorus moiety of the building block B-1, thereby obtaining a first cycle oligonucleotide O-1; |
| (f-1) | optionally, incubating the first cycle oligonucleotide O-1 with an oxidizing or sulfurizing agent, thereby converting any P (III) atoms within said first cycle oligonucleotide O-1 to P (V) atoms; |
| (g-1) | optionally, subjecting a solution comprising the first cycle oligonucleotide O-1 to one or more aqueous extractions, wherein the organic phase comprises the first cycle oligonucleotide O-1; |
| (h-1) | if step (g-1) has been carried out, optionally reducing the water content of the organic phase comprising the first cycle oligonucleotide O-1; |
wherein during and in between steps (b-1) to (h-1), no solid-liquid separation is performed and steps (c-1) and (g-1) are carried out in the presence of one or more amide solvents S^{A}, wherein each amide solvent S^{A} is defined as in claim 1.

3. The method according to claim 2, wherein the target oligonucleotide O^{T} comprises a second cycle oligonucleotide O-2, and the method further comprises performing a second coupling cycle comprising the following steps:
| | |
|---|---|
| (b-2) | incubating the first cycle oligonucleotide O-1 obtained in the first coupling cycle with a deprotection mixture M-(b-2), thereby cleaving the protecting group PG-1 from the first cycle oligonucleotide O-1, so as to obtain a first cycle oligonucleotide (O-1)^{#} having a free backbone hydroxyl group; |
| (c-2) | subjecting a solution comprising the first cycle oligonucleotide (O-1)^{#} to one or more aqueous extractions, wherein the organic phase comprises the first cycle oligonucleotide (O-1)^{#} |
| (d-2) | optionally, reducing the water content of the organic phase comprising the first cycle oligonucleotide (O-1)^{#}; |
| (e-2) | reacting the first cycle oligonucleotide (O-1)^{#} with a building block B-2, wherein said building block B-2 is selected from the group consisting of a nucleoside and an oligonucleotide and comprises |
| | - a backbone hydroxyl moiety protected by a protecting group PG-2 removable under acidic conditions, and |
| | - a phosphorus moiety covalently bonded via its phosphorus atom to an oxygen atom of the backbone of the building block B-2, |
| | under conditions suitable to form a covalent bond between said free backbone hydroxyl group of the first cycle oligonucleotide (O-1)^{#} and the phosphorus atom of said phosphorus moiety of the building block B-2, thereby obtaining a second cycle oligonucleotide O-2; |
| (f-2) | optionally, incubating the second cycle oligonucleotide O-2 with an oxidizing or sulfurizing agent, thereby converting any P (III) atoms within said second cycle oligonucleotide O-2 to P (V) atoms; |
| (g-2) | optionally, subjecting a solution comprising the second cycle oligonucleotide O-2 to one or more aqueous extractions, wherein the organic phase comprises the second cycle oligonucleotide O-2; |
| (h-2) | if step (g-2) has been carried out, optionally reducing the water content of the organic phase comprising the second cycle oligonucleotide O-2; |
wherein during and in between steps (b-1) to (h-2), no solid-liquid separation is performed and steps (c-1), (g-1), (c-2), and (g-2) are carried out in the presence of one or more amide solvents S^{A}, wherein each amide solvent S^{A} is defined as in claim 1.

4. The method according to claim 3, wherein the target oligonucleotide O^{T} comprises a n-th cycle oligonucleotide O-n, and the method further comprises performing (n-2) iterations of a coupling cycle comprising the following steps (b-x) to (h-x), wherein n is an integer in the range of 3 to 99, which denotes the total number of coupling cycles performed to obtain the n-th cycle oligonucleotide O-n, and each individual coupling cycle comprising the following steps (b-x) to (h-x) is identified by a serial number x, which runs in steps of 1 from 3 to n:
| | |
|---|---|
| (b-x) | incubating the (x-1)-th cycle oligonucleotide O-(x-1) obtained in the previous coupling cycle with a deprotection mixture M-(b-x), thereby cleaving the protecting group PG-(x-1) from the (x-1)-th cycle |
| | oligonucleotide O-(x-1), so as to obtain a (x-1)-th cycle oligonucleotide (O-(x-1))^{#} having a free backbone hydroxyl group; |
| (c-x) | subjecting a solution comprising the (x-1)-th cycle oligonucleotide (O-(x-1))^{#} to one or more aqueous extractions, wherein the organic phase comprises the (x-1)-th cycle oligonucleotide (O-(x-1))^{#}; |
| (d-x) | optionally, reducing the water content of the organic phase comprising the (x-1)-th cycle oligonucleotide (O-(x-1))^{#}; |
| (e-x) | reacting the (x-1)-th cycle oligonucleotide (O-(x-1))^{#} with a building block B-x, wherein said building block B-x is selected from the group consisting of a nucleoside and an oligonucleotide and comprises |
| | - a backbone hydroxyl moiety protected by a protecting group PG-x removable under acidic conditions, and |
| | - a phosphorus moiety covalently bonded via its phosphorus atom to an oxygen atom of the backbone of the building block B-x, |
| | under conditions suitable to form a covalent bond between said free backbone hydroxyl group of the (x-1)-th cycle oligonucleotide (O-(x-1))^{#} and the phosphorus atom of said phosphorus moiety of the building block B-x, thereby obtaining a x-th cycle oligonucleotide O-x; |
| (f-x) | optionally, incubating the x-th cycle oligonucleotide O-x with an oxidizing or sulfurizing agent, thereby converting any P (III) atoms within said x-th cycle oligonucleotide O-x to P (V) atoms; |
| (g-x) | optionally, subjecting a solution comprising the x-th cycle oligonucleotide O-x to one or more aqueous extractions, wherein the organic phase comprises the x-th cycle oligonucleotide O-x; |
| (h-x) | if step (g-x) has been carried out, optionally reducing the water content of the organic phase comprising the x-th cycle oligonucleotide O-x; |
wherein during and in between steps (b-1) to (h-n), no solid-liquid separation is performed, and steps (c-1), (g-1), (c-2), and (g-2), as well as each iteration of steps (c-x) and (g-x) are carried out in the presence of one or more amide solvents S^{A}, wherein each amide solvent S^{A} is defined as in claim 1.

5. The method according to any one of claims 2 to 4, wherein
- the phosphorus moiety of the building blocks B-1, B-2, and each building block B-x is independently selected from the group consisting of a phosphoramidite moiety and a H-phosphonate monoester moiety;
- in each coupling cycle, in which said phosphorus moiety of the building block B-1, B-2 or B-x is a phosphoramidite moiety, step (f-1) or (f-2) or (f-x) is carried out; and
- at least in the final coupling cycle, step (f-1) or (f-2) or (f-x) is carried out.

6. The method according to any one of claims 2 to 4, wherein the component C-0 is a compound of the following Formula I: 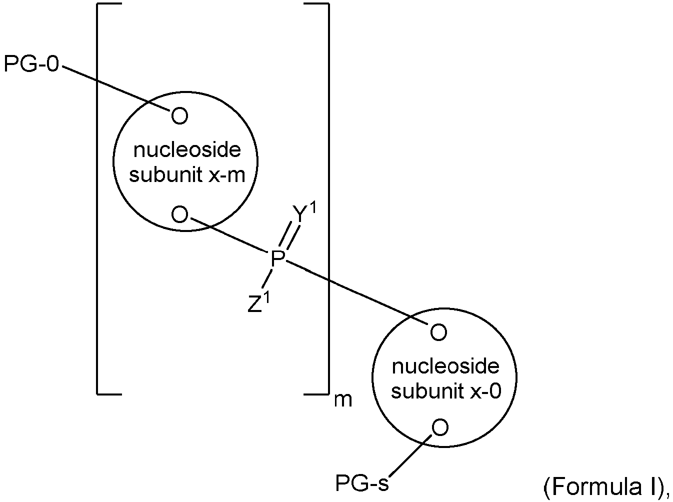 wherein in in Formula I:
each oxygen atom (O) depicted within each nucleoside subunit x-0 to x-m represents the oxygen atom of a hydroxyl moiety of the respective nucleoside subunit;
each of the nucleoside subunits x-0 to x-m may be the same or different;
m is an integer equal to or larger than 0;
PG-0 is a protecting group removable under acidic conditions;
Y¹ is selected independently for each repetitive unit m from the group consisting of O and S;
Z¹ is selected independently for each repetitive unit m from the group consisting of O-R^{z-1}, S-R^{z-1}, and H;
R^{z-1} is a protecting group, which may be the same or different for each repetitive unit m; and
PG-s is a pseudo solid-phase protecting group.

7. The method according to any one of claims 2 to 6, wherein each of the building blocks B-1, B-2, and B-x is a compound of the following Formula II-1: 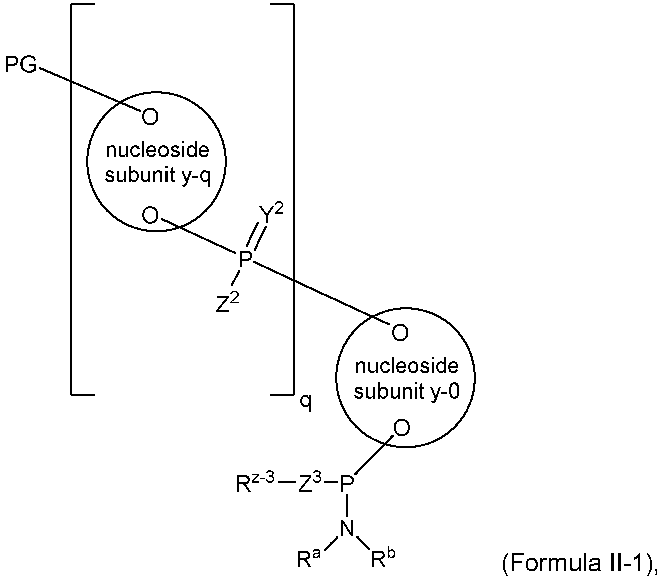 wherein in Formula II-1:
each oxygen atom (O) depicted within each nucleoside subunit y-0 to y-q represents the oxygen atom of a hydroxyl moiety of the respective nucleoside subunit;
each nucleoside subunit y-0 to y-q may be the same or different;
PG is the protecting group PG-1 or PG-2 or PG-x and is a protecting group removable under acidic conditions;
q is an integer equal to or larger than 0,
Y² is selected independently for each repetitive unit q from the group consisting of O and S;
Z² is selected independently for each repetitive unit q from the group consisting of O-R^{z-2} and S-R^{z-2};
R^{z-2} is a protecting group, which may be the same or different for each repetitive unit q;
Z³ is selected from the group consisting of O and S; and
R^{z-3} is a protecting group;
each of R^{a} and R^{b} is a C₁-C₆-alkyl group, wherein R^{a} and R^{b} may be the same or different and may also bond to each other to form a 5-membered or 6-membered aliphatic cyclic amine moiety together with the nitrogen atom to which R^{a} and R^{b} are bonded;
and wherein step (f-1) or (f-2) or (f-x) is carried out in each coupling cycle.

8. The method according to any one of claims 2 and 5 to 7, wherein the method further comprises
- a step (k-1) of incubating the first cycle oligonucleotide O-1 with a deprotection mixture M-(k-1), thereby cleaving the protecting group PG-1 from the first cycle oligonucleotide O-1, so as to obtain a first cycle oligonucleotide (O-1)^{#} having a free backbone hydroxyl group; and/or
- a step (m-1) of incubating the first cycle oligonucleotide O-1 or (O-1)^{#} with a base, thereby cleaving the pseudo solid-phase protecting group PG-s and, optionally, one or more further protecting groups from the first cycle oligonucleotide O-1 or (O-1)^{#}; and/or
- a step (p-1) of modifying the first cycle oligonucleotide O-1 or (O-1)^{#};
wherein, if more than one of steps (k-1), (m-1), and (p-1) are performed, they may be performed in any order.

9. The method according to any one of claims 3 and 5 to 7, wherein the method further comprises
- a step (k-2) of incubating the second cycle oligonucleotide O-2 with a deprotection mixture M-(k-2), thereby cleaving the protecting group PG-2 from the second cycle oligonucleotide O-2, so as to obtain a second cycle oligonucleotide (O-2)^{#} having a free backbone hydroxyl group; and/or
- a step (m-2) of incubating the second cycle oligonucleotide O-2 or (O-2)^{#} with a base, thereby cleaving the pseudo solid-phase protecting group PG-s and, optionally, one or more further protecting groups from the second cycle oligonucleotide O-2 or (O-2)^{#}; and/or
- a step (p-2) of modifying the second cycle oligonucleotide O-2 or (O-2)^{#};
wherein, if more than one of steps (k-2), (m-2), and (p-2) are performed, they may be performed in any order.

10. The method according to any one of claims 4 to 7, wherein the method further comprises
- a step (k-n) of incubating the n-th cycle oligonucleotide O-n with a deprotection mixture M-(k-n), thereby cleaving the protecting group PG-n from the n-th cycle oligonucleotide O-n, so as to obtain a n-th cycle oligonucleotide (O-n)^{#} having a free backbone hydroxyl group; and/or
- a step (m-n) of incubating the n-th cycle oligonucleotide O-n or (O-n)^{#} with a base, thereby cleaving the pseudo solid-phase protecting group PG-s and, optionally, one or more further protecting groups from the n-th cycle oligonucleotide O-n or (O-n)^{#}; and/or
- a step (p-n) of modifying the n-th cycle oligonucleotide O-n or (O-n)^{#};
wherein, if more than one of steps (k-n), (m-n), and (p-n) are performed, they may be performed in any order.

11. The method according to any one of claims 2 to 10, wherein steps (b-1) to (h-1), are carried out in the presence of one or more amide solvents S^{A} and, preferably, one or more ether solvents S^{E}, and further wherein:
- if the second coupling cycle comprising steps (b-2) to (h-2), is performed, steps (b-1) to (h-2), are carried out in the presence of one or more amide solvents S^{A} and, preferably, one or more ether solvents S^{E}; and
- if (n-2) iterations of the coupling cycle comprising steps (b-x) to (h-x) are performed, all of steps (b-1) to (h-n) are carried out in the presence of one or more amide solvents S^{A} and, preferably, one or more ether solvents S^{E}.

12. The method according to any one of claims 2 to 11, wherein steps (b-1) to (h-1) are carried out in essentially halogen-free solvents, and further wherein:
- if a second coupling cycle comprising steps (b-2) to (h-2) is performed, steps (b-1) to (h-2) are carried out in essentially halogen-free solvents; and
- if (n-2) iterations of the coupling cycle comprising steps (b-x) to (h-x) are performed, any steps (b-1) to (h-n) are carried out in essentially halogen-free solvents,
wherein an essentially halogen-free solvent is a solvent which contains in total equal to or less than 3.0 vol-% of halogenated solvents.

13. The method according to any one of claims 2 to 12, wherein in each of the one or more aqueous extractions of step (c-1), the aqueous phase has a pH-value equal to or smaller than 7, preferably in the range of 4-7, and further wherein:
- if a second coupling cycle comprising steps (b-2) to (h-2) is performed, in each of the one or more aqueous extractions of step (c-2), the aqueous phase has a pH-value equal to or smaller than 7, preferably in the range of 4-7; and
- if (n-2) iterations of the coupling cycle comprising steps (b-x) to (h-x) are performed, in each of the one or more aqueous extractions of each step (c-x), the aqueous phase has a pH-value equal to or smaller than 7, preferably in the range of 4-7.

14. The method according to any one of claims 1 to 13, wherein each amide solvent S^{A} is at each occurrence selected independently from the group consisting of the following Formulae S^{A}-1, S^{A}-2, and S^{A}-3: 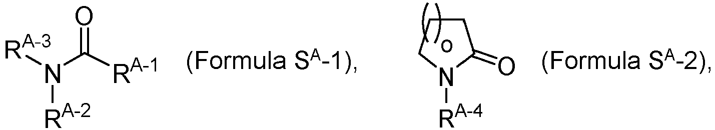 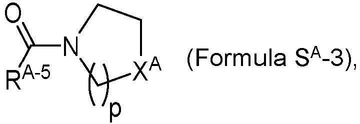 wherein in Formula S^{A}-1:
R^{A-1} is selected from the group consisting of H and a C₁-C₂₂-alkyl group, in which exactly one hydrogen residue may optionally be substituted by a C(O)O(C₁-C₅-alkyl) group; and
each of R^{A-2} and R^{A-3} is independently a C₁-C₂₃-alkyl group;
with the proviso that R^{A-1}, R^{A-2} and R^{A-3} together comprise in total 6-24 carbon atoms;
wherein in Formula S^{A}-2:
O is an integer of 1 or 2; and
R^{A-4} is a C₆-C₂₄-alkyl group; and
wherein in Formula S^{A}-3:
p is an integer of 1 or 2;
X^{A} is selected from the group consisting of CH₂, O, and NC(O)R^{A-6}, with the proviso that, if p is 1, X^{A} is CH₂;
R^{A-5} is a C₁-C₂₄-alkyl group; and
R^{A-6} is a C₁-C₂₃-alkyl group;
with the proviso that R^{A-5} and R^{A-6} together comprise in total 6-24 carbon atoms.

15. The method according to any one of claims 11 to 14, wherein each ether solvent S^{E} is at each occurrence selected independently from the group consisting of the following Formulae S^{E}-1 and S^{E}-2: 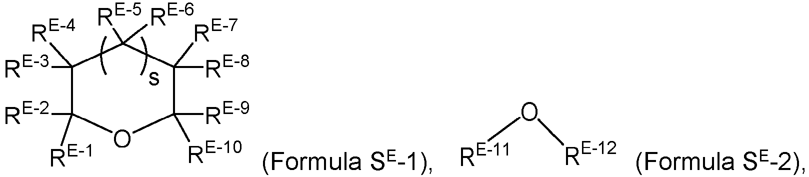 wherein in Formula S^{E}-1:
s is an integer of 0 or 1; and
each of R^{E-1}, R^{E-2}, R^{E-3}, R^{E-4}, R^{E-5}, R^{E-6}, R^{E-7}, R^{E-8}, R^{E-9}, and R^{E-10} is independently selected from the group consisting of H and a C₁-C₅-alkyl group, with the proviso that at least one of R^{E-1}, R^{E-2}, R^{E-3}, R^{E-4}, R^{E-5}, R^{E-6}, R^{E-7}, R^{E-8}, R^{E-9}, and R^{E-10} is a C₁-C₅-alkyl group; and
wherein in Formula S^{E}-2:
each of R^{E-11} and R^{E-12} is independently a C₁-C₆-alkyl group, with the proviso that R^{E-11} and R^{E-12} together comprise in total 5-12 carbon atoms.

16. The method according to any one of claims 1 to 15, wherein each pseudo solid-phase protecting group is a protecting group of the following Formula P-1: 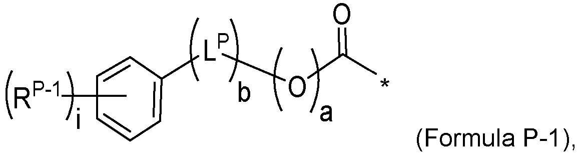 wherein in Formula P-1:
the asterisk indicates the oxygen atom of a hydroxyl moiety or the nitrogen atom of an amine moiety protected by the respective pseudo solid-phase protecting group;
a is an integer of 0 or 1;
b is an integer of 0 or 1;
L^{P} is a linker moiety;
i is an integer of 1 to 5; and
R^{P-1} is at each occurrence independently selected from the group consisting of O(C₁-C₄₀-alkyl), O(C₂-C₄₀-alkenyl), O(C₂-C₄₀-alkynyl), a C₁-C₄₀-alkyl group, a C₂-C₄₀-alkenyl group, a C₂-C₄₀-alkynyl group, C(O)(C₁-C₄₀-alkyl), C(O)(C₂-C₄₀-alkenyl), and C(O)(C₂-C₄₀-alkynyl); and
wherein all present residues R^{P-1} together comprise in total 18-200 carbon atoms.

17. A composition comprising:
- an oligonucleotide which is covalently bonded to at least one pseudo solid-phase protecting group, and
- a mixed solvent which is essentially halogen-free and comprises one or more amide solvents S^{A} and, preferably, one or more ether solvents S^{E}, wherein each amide solvent S^{A} is an amide solvent comprising one or more alkyl groups, wherein these one or more alkyl groups together comprise in total 6-24 carbon atoms, wherein an essentially halogen-free solvent is a solvent which contains in total equal to or less than 3.0 vol-% of halogenated solvents,
preferably wherein the composition and/or one or more components are defined as in one or more of the preceding claims,
in particular wherein said one or more amide solvents S^{A} are defined as in claim 14, said one or more ether solvents S^{E} are defined as in claim 15, and/or each pseudo solid-phase protecting group is defined as in claim 16.

## Patentansprüche

1. Verfahren zur Synthese eines Zieloligonukleotids O^{T}, wobei das Verfahren einen Schritt umfasst, bei dem eine Lösung umfassend eine Komponente C ausgewählt aus der Gruppe bestehend aus einem Nukleosid und einem Oligonukleotid einer oder mehreren wässrigen Extraktionen unterzogen wird, wobei die organische Phase die Komponente C umfasst, und wobei
- die Komponente C kovalent an eine pseudo-festphasige Schutzgruppe PG-s gebunden ist und
- jede der einen oder mehreren wässrigen Extraktionen in Gegenwart eines oder mehrerer Amid-Lösungsmittel S^{A} durchgeführt wird, wobei jedes Amid-Lösungsmittel S^{A} ein Amid-Lösungsmittel ist, das eine oder mehrere Alkylgruppen umfasst, wobei diese eine oder mehreren Alkylgruppen zusammen insgesamt 6 bis 24 Kohlenstoffatome umfassen.

2. Verfahren nach Anspruch 1, wobei das Zieloligonukleotid O^{T} ein Oligonukleotid O-1 eines ersten Zyklus umfasst und das Verfahren den folgenden Schritt (a-1) und einen ersten Kopplungszyklus umfassend die folgenden Schritte umfasst:
| | |
|---|---|
| (a-1) | Bereitstellen einer Komponente C-0, ausgewählt aus der Gruppe bestehend aus einem Nukleosid und einem Oligonukleotid, wobei die Komponente C-0 kovalent an eine pseudo-festphasige Schutzgruppe PG-s gebunden ist und eine Rückgrat-Hydroxylgruppe umfasst, die durch eine unter sauren Bedingungen entfernbare Schutzgruppe PG-0 geschützt ist; |
| (b-1) | Inkubieren der Komponente C-0 aus Schritt (a-1) mit einer Entschützungsmischung M-(b-1), wodurch die Schutzgruppe PG-0 von der Komponente C-0 abgespalten wird, um eine Komponente (C-0)^{#} mit einer freien Rückgrat-Hydroxylgruppe zu erhalten; |
| (c-1) | Unterziehen einer Lösung umfassend die Komponente (C-0)^{#} einer oder mehreren wässrigen Extraktionen, wobei die organische Phase die Komponente (C-0)^{#} umfasst; |
| (d-1) | optional, den Wassergehalt der organischen Phase umfassend die Komponente (C-0)^{#} reduzieren; |
| (e-1) | zur Reaktion Bringen der Komponente (C-0)^{#} mit einem Baustein B-1, wobei der Baustein B-1 ausgewählt ist aus der Gruppe bestehend aus einem Nukleosid und einem Oligonukleotid und |
| | - eine Rückgrat-Hydroxylgruppe, die durch eine unter sauren Bedingungen entfernbare Schutzgruppe PG-1 geschützt ist, und |
| | - eine Phosphorgruppe, die über ihr Phosphoratom kovalent an ein Sauerstoffatom des Rückgrats des Bausteins B-1 gebunden ist, |
| | umfasst, |
| | unter Bedingungen, die zur Bildung einer kovalenten Bindung zwischen der freien Rückgrat-Hydroxylgruppe der Komponente (C-0)^{#} und dem Phosphoratom der Phosphorgruppe des Bausteins B-1 geeignet sind, wodurch ein Oligonukleotid O-1 des ersten Zyklus erhalten wird; |
| (f-1) | optional, Inkubieren des Oligonukleotids O-1 des ersten Zyklus mit einem Oxidations- oder Sulfurierungsmittel, wodurch jegliche P(III)-Atome innerhalb des Oligonukleotids O-1 des ersten Zyklus in P(V)-Atome umgewandelt werden; |
| (g-1) | optional, Unterziehen einer Lösung umfassend das Oligonukleotid O-1 des ersten Zyklus einer oder mehreren wässrigen Extraktionen, wobei die organische Phase das Oligonukleotid O-1 des ersten Zyklus umfasst; |
| (h-1) | wenn Schritt (g-1) durchgeführt wurde, optional Reduzieren des Wassergehalts der organischen Phase umfassend das Oligonukleotid O-1 des ersten Zyklus; |
wobei während und zwischen den Schritten (b-1) bis (h-1) keine Fest-Flüssig-Trennung durchgeführt wird und die Schritte (c-1) und (g-1) in Gegenwart eines oder mehrerer Amid-Lösungsmittel S^{A} durchgeführt werden, wobei jedes Amid-Lösungsmittel S^{A} wie in Anspruch 1 definiert ist.

3. Verfahren nach Anspruch 2, wobei das Zieloligonukleotid O^{T} ein Oligonukleotid O-2 eines zweiten Zyklus umfasst und das Verfahren ferner die Durchführung eines zweiten Kopplungszyklus umfassend die folgenden Schritte umfasst:
| | |
|---|---|
| (b-2) | Inkubieren des im ersten Kopplungszyklus erhaltenen Oligonukleotids O-1 des ersten Zyklus mit einer Entschützungsmischung M-(b-2), wodurch die Schutzgruppe PG-1 vom Oligonukleotid O-1 des ersten Zyklus abgespalten wird, um ein Oligonukleotid (O-1)^{#} des ersten Zyklus mit einer freien Rückgrat-Hydroxylgruppe zu erhalten; |
| (c-2) | Unterziehen einer Lösung umfassend das Oligonukleotid (O-1)^{#} des ersten Zyklus einer oder mehreren wässrigen Extraktionen, wobei die organische Phase das Oligonukleotid (O-1)^{#} des ersten Zyklus umfasst; |
| (d-2) | optional, Reduzieren des Wassergehalts der organischen Phase umfassend das Oligonukleotid (O-1)^{#} des ersten Zyklus; |
| (e-2) | zur Reaktion Bringen des Oligonukleotids (O-1)^{#} des ersten Zyklus mit einem Baustein B-2, wobei der Baustein B-2 aus der Gruppe ausgewählt ist bestehend aus einem Nukleosid und einem Oligonukleotid und |
| | - eine Rückgrat-Hydroxylgruppe, die durch eine unter sauren Bedingungen entfernbare Schutzgruppe PG-2 geschützt ist, und |
| | - eine Phosphorgruppe, die über ihr Phosphoratom kovalent an ein Sauerstoffatom des Rückgrats des Bausteins B-2 gebunden ist, |
| | umfasst, |
| | unter Bedingungen, die zur Bildung einer kovalenten Bindung zwischen der freien Rückgrat-Hydroxylgruppe des Oligonukleotids (O-1)^{#} des ersten Zyklus und dem Phosphoratom der Phosphorgruppe des Bausteins B-2 geeignet sind, wodurch ein Oligonukleotid O-2 des zweiten Zyklus erhalten wird; |
| (f-2) | optional, Inkubieren des Oligonukleotids O-2 des zweiten Zyklus mit einem Oxidations- oder Sulfurierungsmittel, wodurch alle P(III)-Atome innerhalb des Oligonukleotids O-2 des zweiten Zyklus in P(V)-Atome umgewandelt werden; |
| (g-2) | optional, Unterziehen einer Lösung umfassend das Oligonukleotid O-2 des zweiten Zyklus einer oder mehreren wässrigen Extraktionen, wobei die organische Phase das Oligonukleotid O-2 des zweiten Zyklus umfasst; |
| (h-2) | wenn Schritt (g-2) durchgeführt wurde, optional Reduzieren des Wassergehalts der organischen Phase umfassend das Oligonukleotid O-2 des zweiten Zyklus; |
wobei während und zwischen den Schritten (b-1) bis (h-2) keine Fest-Flüssig-Trennung durchgeführt wird und die Schritte (c-1), (g-1), (c-2) und (g-2) in Gegenwart eines oder mehrerer Amid-Lösungsmittel S^{A} durchgeführt werden, wobei jedes Amid-Lösungsmittel S^{A} wie in Anspruch 1 definiert ist.

4. Verfahren nach Anspruch 3, wobei das Zieloligonukleotid O^{T} ein Oligonukleotid O-n des n-ten Zyklus umfasst, und das Verfahren ferner die Durchführung von (n-2) Iterationen eines Kopplungszyklus umfassend die folgenden Schritte (b-x) bis (h-x) umfasst, wobei n eine ganze Zahl im Bereich von 3 bis 99 ist, die die Gesamtzahl der Kopplungszyklen angibt, die durchgeführt werden, um das Oligonukleotid O-n des n-ten Zyklus zu erhalten, und jeder einzelne Kopplungszyklus umfassend die folgenden Schritte (b-x) bis (h-x) durch eine Seriennummer x identifiziert wird, die in Schritten von 1 von 3 bis n läuft:
| | | |
|---|---|---|
| (b-x) | Inkubieren des im vorherigen Kopplungszyklus erhaltenen Oligonukleotids O-(x-1) des (x-1)-ten Zyklus mit einer Entschützungsmischung M-(b-x), wodurch die Schutzgruppe PG-(x-1) vom Oligonukleotid O-(x-1) des (x-1)-ten Zyklus abgespalten wird, um ein Oligonukleotid (O-(x-1))^{#} des (x-1)-ten Zyklus mit einer freien Rückgrat-Hydroxylgruppe zu erhalten; | |
| (c-x) | Unterziehen einer Lösung umfassend das Oligonukleotid (O-(x-1))^{#} des (x-1)-ten Zyklus einer oder mehreren wässrigen Extraktionen, wobei die organische Phase das Oligonukleotid (O-(x-1))^{#} des (x-1)-ten Zyklus umfasst; | |
| (d-x) | optional, Reduzieren des Wassergehalts der organischen Phase umfassend das Oligonukleotid (O-(x-1))^{#} des (x-1)-ten Zyklus; | |
| (e-x) | zur Reaktion Bringen des Oligonukleotids (O-(x-1))^{#} des (x-1)-ten Zyklus mit einem Baustein B-x, wobei der Baustein B-x aus der | |
| | | |
| | Gruppe ausgewählt ist bestehend aus einem Nukleosid und einem Oligonukleotid und | |
| | - | eine Rückgrat-Hydroxylgruppe, die durch eine unter sauren Bedingungen entfernbare Schutzgruppe PG-x geschützt ist, und |
| | - | eine Phosphorgruppe, die über ihr Phosphoratom kovalent an ein Sauerstoffatom des Rückgrats des Bausteins B-x gebunden ist, |
| | umfasst, | |
| (f-x) | unter Bedingungen, die zur Bildung einer kovalenten Bindung zwischen der freien Rückgrat-Hydroxylgruppe des Oligonukleotids (O-(x-1))^{#} des (x-1)-ten Zyklus und dem Phosphoratom der Phosphorgruppe des Bausteins B-x geeignet sind, wodurch ein Oligonukleotid O-x des x-ten Zyklus erhalten wird; optional, Inkubieren des Oligonukleotids O-x des x-ten Zyklus mit einem Oxidations- oder Sulfurierungsmittel, wodurch alle P(III)-Atome innerhalb des Oligonukleotids O-x des x-ten Zyklus in P(V)-Atome umgewandelt werden; | |
| (g-x) | optional, Unterziehen einer Lösung, die das Oligonukleotid O-x des x-ten Zyklus umfasst, einer oder mehreren wässrigen Extraktionen, wobei die organische Phase das Oligonukleotid O-x des x-ten Zyklus umfasst; | |
| (h-x) | wenn Schritt (g-x) durchgeführt wurde, optional Reduzieren des Wassergehalts der organischen Phase umfassend das Oligonukleotid O-x des x-ten Zyklus; | |
wobei während und zwischen den Schritten (b-1) bis (h-n) keine Fest-Flüssig-Trennung durchgeführt wird und die Schritte (c-1), (g-1), (c-2) und (g-2) sowie jede Iteration der Schritte (c-x) und (g-x) in Gegenwart eines oder mehrerer Amidlösungsmittel S^{(A)} durchgeführt werden, wobei jedes Amidlösungsmittel S^{A} wie in Anspruch 1 definiert ist.

5. Verfahren nach einem der Ansprüche 2 bis 4, wobei
- die Phosphorgruppe der Bausteine B-1, B-2 und jedes Bausteins B-x unabhängig voneinander aus der Gruppe ausgewählt ist bestehend aus einer Phosphoramiditgruppe und einer H-Phosphonatmonoestergruppe;
- in jedem Kopplungszyklus, in dem die Phosphorgruppe des Bausteins B-1, B-2 oder B-x eine Phosphoramiditgruppe ist, Schritt (f-1) oder (f-2) oder (f-x) durchgeführt wird; und
- zumindest im letzten Kopplungszyklus Schritt (f-1) oder (f-2) oder (f-x) durchgeführt wird.

6. Verfahren nach einem der Ansprüche 2 bis 4, wobei die Komponente C-0 eine Verbindung der folgenden Formel I ist: 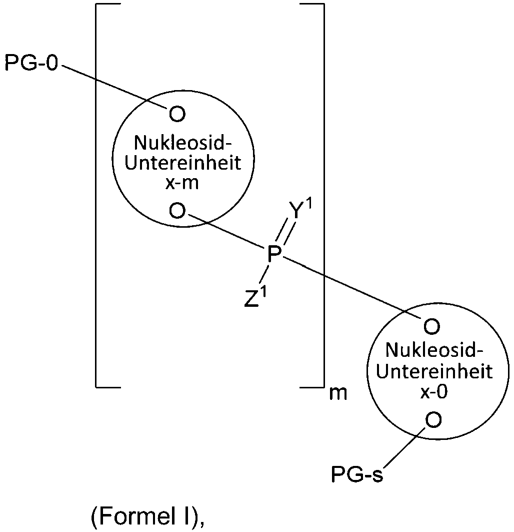 wobei in Formel I:
jedes Sauerstoffatom (O), das innerhalb jeder Nukleosid-Untereinheit x-0 bis x-m dargestellt ist, das Sauerstoffatom einer Hydroxylgruppe der jeweiligen Nukleosid-Untereinheit darstellt;
jede der Nukleosid-Untereinheiten x-0 bis x-m gleich oder unterschiedlich sein kann;
m eine ganze Zahl größer oder gleich 0 ist;
PG-0 eine unter sauren Bedingungen entfernbare Schutzgruppe ist;
Y¹ unabhängig für jede repetitive Einheit m ausgewählt ist aus der Gruppe bestehend aus O und S;
Z¹ unabhängig für jede repetitive Einheit m ausgewählt ist aus der Gruppe bestehend aus O-R^{z-1}, S-R^{z-1} und H;
R^{z-1} eine Schutzgruppe ist, die für jede repetitive Einheit m gleich oder
unterschiedlich sein kann; und
PG-s eine pseudo-festphasige Schutzgruppe ist.

7. Verfahren nach einem der Ansprüche 2 bis 6, wobei jeder der Bausteine B-1, B-2 und B-x eine Verbindung der folgenden Formel II-1 ist: 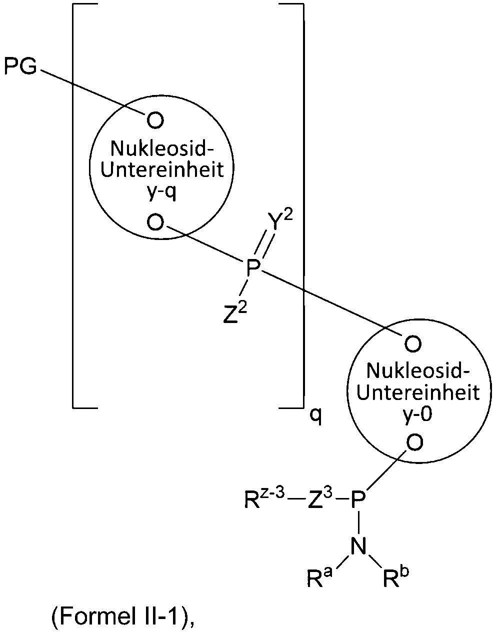 wobei in Formel II-1:
jedes Sauerstoffatom (O), das innerhalb jeder Nukleosid-Untereinheit y-0 bis y-q dargestellt ist, das Sauerstoffatom einer Hydroxylgruppe der jeweiligen Nukleosid-Untereinheit darstellt;
jede Nukleosid-Untereinheit y-0 bis y-q gleich oder unterschiedlich sein kann;
PG die Schutzgruppe PG-1 oder PG-2 oder PG-x ist und eine unter sauren Bedingungen entfernbare Schutzgruppe ist;
q eine ganze Zahl größer oder gleich 0 ist;
Y² unabhängig für jede repetitive Einheit q aus der Gruppe ausgewählt ist bestehend aus O und S;
Z² unabhängig für jede repetitive Einheit q aus der Gruppe ausgewählt ist bestehend aus O-R^{z-2} und S-R^{z-2};
R^{z-2} eine Schutzgruppe ist, die für jede repetitive Einheit q gleich oder unterschiedlich sein kann;
Z³ aus der Gruppe ausgewählt ist bestehend aus O und S; und
R^{z-3} eine Schutzgruppe ist;
jedes von R^{a} und R^{b} eine C₁-C₆-Alkylgruppe ist, wobei R^{a} und R^{b} gleich oder unterschiedlich sein können und auch miteinander verbunden sein können, um eine 5-gliedrige oder 6-gliedrige aliphatische cyclische Amin-Einheit zusammen mit dem Stickstoffatom, an das R^{a} und R^{b} gebunden sind, zu bilden;
und wobei Schritt (f-1) oder (f-2) oder (f-x) in jedem Kopplungszyklus durchgeführt wird.

8. Verfahren nach einem der Ansprüche 2 und 5 bis 7, wobei das Verfahren ferner umfasst
- einen Schritt (k-1) des Inkubierens des Oligonukleotids O-1 des ersten Zyklus mit einer Entschützungsmischung M-(k-1), wodurch die Schutzgruppe PG-1 vom Oligonukleotid O-1 des ersten Zyklus abgespalten wird, um ein Oligonukleotid (O-1)^{#} des ersten Zyklus mit einer freien Rückgrat-Hydroxylgruppe zu erhalten; und/oder
- einen Schritt (m-1) des Inkubierens des Oligonukleotids O-1 oder (O-1)^{#} des ersten Zyklus mit einer Base, wodurch die pseudo-festphasige Schutzgruppe PG-s und optional eine oder mehrere weitere Schutzgruppen vom Oligonukleotid O-1 oder (O-1)^{#} des ersten Zyklus abgespalten werden; und/oder
- einen Schritt (p-1) des Modifizierens des Oligonukleotids O-1 oder (O-1)^{#} des ersten Zyklus;
wobei, wenn mehr als einer der Schritte (k-1), (m-1) und (p-1) durchgeführt wird, diese in beliebiger Reihenfolge durchgeführt werden können.

9. Verfahren nach einem der Ansprüche 3 und 5 bis 7, wobei das Verfahren ferner umfasst
- einen Schritt (k-2) des Inkubierens des Oligonukleotids O-2 des zweiten Zyklus mit einer Entschützungsmischung M-(k-2), wodurch die Schutzgruppe PG-2 vom Oligonukleotid O-2 des zweiten Zyklus abgespalten wird, um ein Oligonukleotid (O-2)^{#} des zweiten Zyklus mit einer freien Rückgrat-Hydroxylgruppe zu erhalten; und/oder
- einen Schritt (m-2) des Inkubierens des Oligonukleotids O-2 oder (O-2)^{#} des zweiten Zyklus mit einer Base, wodurch die pseudo-festphasige Schutzgruppe PG-s und optional eine oder mehrere weitere Schutzgruppen von dem Oligonukleotid O-2 oder (O-2)^{#} des zweiten Zyklus abgespalten werden; und/oder
- einen Schritt (p-2) des Modifizierens des Oligonukleotids O-2 oder (O-2)^{#} des zweiten Zyklus;
wobei, wenn mehr als einer der Schritte (k-2), (m-2) und (p-2) durchgeführt wird, diese in beliebiger Reihenfolge durchgeführt werden können.

10. Verfahren nach einem der Ansprüche 4 bis 7, wobei das Verfahren ferner umfasst
- einen Schritt (k-n) des Inkubierens des Oligonukleotids O-n des n-ten Zyklus mit einer Entschützungsmischung M-(k-n), wodurch die Schutzgruppe PG-n vom Oligonukleotid O-n des n-ten Zyklus abgespalten wird, um ein Oligonukleotid (O-n)^{#} des n-ten Zyklus mit einer freien Rückgrat-Hydroxylgruppe zu erhalten; und/oder
- einen Schritt (m-n) des Inkubierens des Oligonukleotids O-n oder (O-n)^{#} des n-ten Zyklus mit einer Base, wodurch die pseudo-festphasige Schutzgruppe PG-s und optional eine oder mehrere weitere Schutzgruppen vom Oligonukleotid O-n oder (O-n)^{#} des n-ten Zyklus abgespalten werden; und/oder
- einen Schritt (p-n) des Modifizierens des Oligonukleotids O-n oder (O-n)^{#} des n-ten Zyklus;
wobei, wenn mehr als einer der Schritte (k-n), (m-n) und (p-n) durchgeführt wird, diese in beliebiger Reihenfolge durchgeführt werden können.

11. Verfahren nach einem der Ansprüche 2 bis 10, wobei die Schritte (b-1) bis (h-1) in Gegenwart eines oder mehrerer Amidlösungsmittel S^{A} und vorzugsweise eines oder mehrerer Etherlösungsmittel S^{E} durchgeführt werden, und wobei ferner:
- wenn der zweite Kopplungszyklus umfassend die Schritte (b-2) bis (h-2) durchgeführt wird, die Schritte (b-1) bis (h-2) in Gegenwart eines oder mehrerer Amidlösungsmittel S^{A} und bevorzugt eines oder mehrerer Etherlösungsmittel S^{E} durchgeführt werden; und
- wenn (n-2) Iterationen des Kopplungszyklus umfassend die Schritte (b-x) bis (h-x) durchgeführt werden, alle Schritte (b-1) bis (h-n) in Gegenwart eines oder mehrerer Amidlösungsmittel S^{A} und bevorzugt eines oder mehrerer Etherlösungsmittel S^{E} durchgeführt werden.

12. Verfahren nach einem der Ansprüche 2 bis 11, wobei die Schritte (b-1) bis (h-1) in im Wesentlichen halogenfreien Lösungsmitteln durchgeführt werden und wobei ferner:
- wenn ein zweiter Kopplungszyklus umfassend die Schritte (b-2) bis (h-2) durchgeführt wird, die Schritte (b-1) bis (h-2) in im Wesentlichen halogenfreien Lösungsmitteln durchgeführt werden; und
- wenn (n-2) Iterationen des Kopplungszyklus umfassend die Schritte (b-x) bis (h-x) durchgeführt werden, alle Schritte (b-1) bis (h-n) in im Wesentlichen halogenfreien Lösungsmitteln durchgeführt werden,
wobei ein im Wesentlichen halogenfreies Lösungsmittel ein Lösungsmittel ist, das insgesamt gleich oder weniger als 3,0 Vol.-% halogenierte Lösungsmittel enthält.

13. Verfahren nach einem der Ansprüche 2 bis 12, wobei in jeder der einen oder mehreren wässrigen Extraktionen des Schritts (c-1) die wässrige Phase einen pH-Wert von gleich oder kleiner als 7, bevorzugt im Bereich von 4 bis 7, aufweist, und wobei ferner:
- wenn ein zweiter Kopplungszyklus umfassend die Schritte (b-2) bis (h-2) durchgeführt wird, in jeder der einen oder mehreren wässrigen Extraktionen des Schritts (c-2) die wässrige Phase einen pH-Wert von gleich oder kleiner als 7, bevorzugt im Bereich von 4 bis 7, aufweist; und
- wenn (n-2) Iterationen des Kopplungszyklus umfassend die Schritte (b-x) bis (h-x) durchgeführt werden, in jeder der einen oder mehreren wässrigen Extraktionen jedes Schritts (c-x) die wässrige Phase einen pH-Wert von gleich oder kleiner als 7, bevorzugt im Bereich von 4 bis 7, aufweist.

14. Verfahren nach einem der Ansprüche 1 bis 13, wobei jedes Amidlösungsmittel S^{A} bei jedem Auftreten unabhängig aus der Gruppe ausgewählt ist bestehend aus den folgenden Formeln S^{A}-1, S^{A}-2 und S^{A}-3: 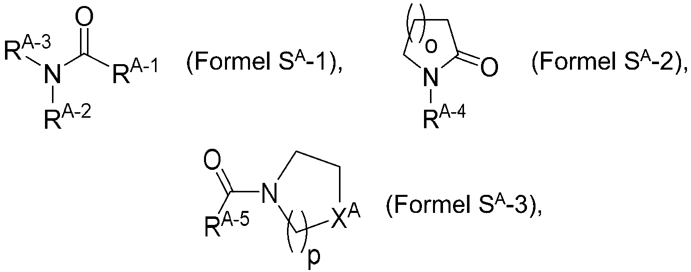 wobei in Formel S^{A}-1:
R^{A-1} aus der Gruppe ausgewählt ist bestehend aus H und einer C₁-C₂₂-Alkylgruppe, in der genau ein Wasserstoffrest optional durch eine C(O)O(C₁-C₅-Alkyl)-Gruppe ersetzt sein kann; und
jedes von R^{A-2} und R^{A-3} unabhängig voneinander eine C₁-C₂₃-Alkylgruppe ist;
mit der Maßgabe, dass R^{A-1}, R^{A-2} und R^{A-3} zusammen insgesamt 6-24 Kohlenstoffatome umfassen;
wobei in Formel S^{A}-2:
o eine ganze Zahl von 1 oder 2 ist; und
R^{A-4} eine C₆-C₂₄-Alkylgruppe ist; und
wobei in Formel S^{A}-3:
p eine ganze Zahl von 1 oder 2 ist;
X^{A} aus der Gruppe ausgewählt ist bestehend aus CH₂, O und NC(O)R^{A-6}, mit der Maßgabe, dass, wenn p 1 ist, X^{A} CH₂ ist;
R^{A-5} eine C₁-C₂₄-Alkylgruppe ist; und
R^{A-6} eine C₁-C₂₃-Alkylgruppe ist;
mit der Maßgabe, dass R^{A-5} und R^{A-6} zusammen insgesamt 6-24 Kohlenstoffatome umfassen.

15. Verfahren nach einem der Ansprüche 11 bis 14, wobei jedes Etherlösungsmittel S^{E} bei jedem Auftreten unabhängig aus der Gruppe ausgewählt ist bestehend aus den folgenden Formeln S^{E}-1 und S^{E}-2: 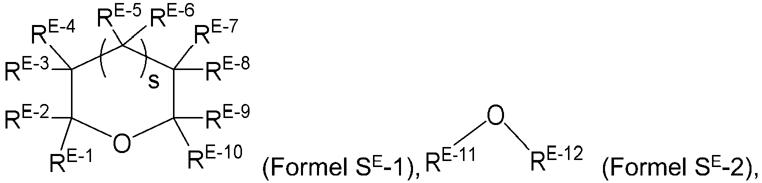 wobei in der Formel S^{E}-1:
s eine ganze Zahl von 0 oder 1 ist; und
jedes von R^{E-1}, R^{E-2}, R^{E-3}, R^{E-4}, R^{E-5}, R^{E-6}, R^{E-7}, R^{E-8}, R^{E-9} und R^{E-10} unabhängig voneinander aus der Gruppe ausgewählt ist bestehend aus H und einer C₁-C₅-Alkylgruppe, mit der Maßgabe, dass mindestens eines von R^{E-1}, R^{E-2}, R^{E-3}, R^{E-4}, R^{E-5}, R^{E-6}, R^{E-7}, R^{E-8}, R^{E-9} und R^{E-10} eine C₁-C₅-Alkylgruppe ist; und
wobei in Formel S^{E}-2:
jedes von R^{E-11} und R^{E-12} unabhängig voneinander eine C₁-C₆ -Alkylgruppe ist, mit der Maßgabe, dass R^{E-11} und R^{E-12} zusammen insgesamt 5-12 Kohlenstoffatome umfassen.

16. Verfahren nach einem der Ansprüche 1 bis 15, wobei jede pseudo-festphasige Schutzgruppe eine Schutzgruppe der folgenden Formel P-1 ist: 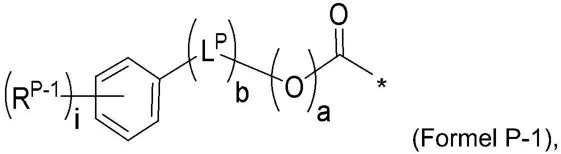 wobei in Formel P-1:
das Sternchen das Sauerstoffatom einer Hydroxylgruppe oder das Stickstoffatom einer Amingruppe bezeichnet, die durch die jeweilige pseudo-festphasige Schutzgruppe geschützt sind;
a eine ganze Zahl von 0 oder 1 ist;
b eine ganze Zahl von 0 oder 1 ist;
L^{P} eine Linkergruppe ist;
i eine ganze Zahl von 1 bis 5 ist; und
R^{P-1} bei jedem Auftreten unabhängig ausgewählt ist aus der Gruppe bestehend aus O(C₁-C₄₀-Alkyl), O(C₂-C₄₀-Alkenyl), O(C₂-C₄₀-Alkinyl), einer C₁-C₄₀-Alkylgruppe, einer C₂-C₄₀-Alkenylgruppe, einer C₂-C₄₀-Alkinylgruppe, C(O)(C₁-C₄₀-Alkyl), C(O)(C₂-C₄₀-Alkenyl) und C(O)(C₂-C₄₀-Alkinyl); und
wobei alle vorhandenen Reste R^{P-1} zusammen insgesamt 18-200 Kohlenstoffatome umfassen.

17. Zusammensetzung, umfassend:
- ein Oligonukleotid, das kovalent an mindestens eine pseudo-festphasige Schutzgruppe gebunden ist, und
- ein gemischtes Lösungsmittel, das im Wesentlichen halogenfrei ist und ein oder mehrere Amidlösungsmittel S^{A} und, bevorzugt, ein oder mehrere Etherlösungsmittel S^{E} umfasst, wobei jedes Amidlösungsmittel S^{A} ein Amidlösungsmittel umfassend eine oder mehrere Alkylgruppen ist, wobei diese eine oder mehreren Alkylgruppen zusammen insgesamt 6 bis 24 Kohlenstoffatome umfassen, wobei ein im Wesentlichen halogenfreies Lösungsmittel ein Lösungsmittel ist, das insgesamt gleich oder weniger als 3,0 Vol.-% halogenierte Lösungsmittel enthält,
bevorzugt wobei die Zusammensetzung und/oder eine oder mehrere Komponenten wie in einem oder mehreren der vorstehenden Ansprüche definiert sind,
insbesondere wobei das eine oder die mehreren Amidlösungsmittel S^{A} wie in Anspruch 14 definiert sind, das eine oder die mehreren Etherlösungsmittel S^{E} wie in Anspruch 15 definiert sind und/oder jede pseudo-festphasige Schutzgruppe wie in Anspruch 16 definiert ist.

## Revendications

1. Procédé de synthèse d'un oligonucléotide cible O^{T}, ledit procédé comprenant une étape de soumission d'une solution comprenant un composant C choisi dans le groupe constitué par un nucléoside et un oligonucléotide à une ou plusieurs extractions aqueuses, la phase organique comprenant le composant C, et dans lequel
- ledit composant C est lié de manière covalente à un groupe protecteur de type pseudo-phase solide PG-s, et
- chacune desdites extractions aqueuses est réalisée en présence d'un ou plusieurs solvants amides S^{A}, chaque solvant amide S^{A} étant un solvant amide comprenant un ou plusieurs groupes alkyle, ce groupe alkyle ou ces groupes alkyle comprenant au total de 6 à 24 atomes de carbone.

2. Procédé selon la revendication 1, dans lequel l'oligonucléotide cible O^{T} comprend un oligonucléotide de premier cycle O-1, et le procédé comprend l'étape (a-1) et un premier cycle de couplage comprenant les étapes suivantes :
(a-1) la fourniture d'un composant C-0 choisi dans le groupe constitué par un nucléoside et un oligonucléotide, le composant C-0 étant lié de manière covalente à un groupe protecteur de type pseudo-phase solide PG-s et comprenant un fragment hydroxyle du squelette protégé par un groupe protecteur PG-0 clivable dans des conditions acides ;
(b-1) l'incubation du composant C-0 de l'étape (a-1) avec un mélange de déprotection M-(b-1), séparant ainsi le groupe protecteur PG-0 du composant C-0, de façon à obtenir un composant (C-0)^{#} comportant un groupe hydroxyle libre du squelette ;
(c-1) la soumission d'une solution comprenant le composant (C-0)^{#} à une ou plusieurs extractions aqueuses, la phase organique comprenant le composant (C-0)^{#} ;
(d-1) facultativement, la réduction de la teneur en eau de la phase organique comprenant le composant (C-0)^{#} ;
(e-1) la réaction du composant (C-0)^{#} avec un bloc constitutif B-1, ledit bloc constitutif B-1 étant choisi dans le groupe constitué par un nucléoside et un oligonucléotide et comprenant
- un fragment hydroxyle du squelette protégé par un groupe protecteur PG-1 clivable dans des conditions acides, et
- un fragment phosphore lié de manière covalente, via son atome de phosphore, à un atome d'oxygène du squelette du bloc constitutif B-1, dans des conditions permettant la formation d'une liaison covalente entre ledit groupe hydroxyle libre du squelette du composant (C-0)^{#} et l'atome de phosphore dudit fragment phosphore du bloc constitutif B-1, obtenant ainsi un oligonucléotide de premier cycle O-1 ;
(f-1) facultativement, l'incubation de l'oligonucléotide de premier cycle O-1 avec un agent oxydant ou sulfurant, convertissant ainsi les atomes P(III) dudit oligonucléotide de premier cycle O-1 en atomes P(V) ;
(g-1) facultativement, la soumission d'une solution comprenant l'oligonucléotide de premier cycle O-1 à une ou plusieurs extractions aqueuses, la phase organique comprenant l'oligonucléotide de premier cycle O-1 ;
(h-1) si l'étape (g-1) a été réalisée, facultativement, la réduction de la teneur en eau de la phase organique comprenant l'oligonucléotide de premier cycle O-1 ;
dans lequel, pendant et entre les étapes (b-1) à (h-1), aucune séparation solide-liquide n'est réalisée et les étapes (c-1) et (g-1) sont réalisées en présence d'un ou plusieurs solvants amides S^{A}, chaque solvant amide S^{A} étant tel que défini dans la revendication 1.

3. Procédé selon la revendication 2, dans lequel l'oligonucléotide cible O^{T} comprend un oligonucléotide de deuxième cycle O-2, et le procédé comprend en outre la réalisation d'un deuxième cycle de couplage comprenant les étapes suivantes :
(b-2) l'incubation de l'oligonucléotide de premier cycle O-1 obtenu lors du premier cycle de couplage avec un mélange de déprotection M-(b-2), séparant ainsi le groupe protecteur PG-1 de l'oligonucléotide de premier cycle O-1, de façon à obtenir un oligonucléotide de premier cycle (O-1)^{#} comportant un groupe hydroxyle libre du squelette ;
(c-2) la soumission d'une solution comprenant l'oligonucléotide de premier cycle (O-1)^{#} à une ou plusieurs extractions aqueuses, la phase organique comprenant l'oligonucléotide de premier cycle (O-1)^{#} ;
(d-2) facultativement, la réduction de la teneur en eau de la phase organique comprenant l'oligonucléotide de premier cycle (O-1)^{#} ;
(e-2) la réaction de l'oligonucléotide de premier cycle (O-1)^{#} avec un bloc constitutif B-2, ledit bloc constitutif B-2 étant choisi dans le groupe constitué par un nucléoside et un oligonucléotide et comprenant
- un fragment hydroxyle du squelette protégé par un groupe protecteur PG-2 clivable dans des conditions acides, et
- un fragment phosphore lié de manière covalente, via son atome de phosphore, à un atome d'oxygène du squelette du bloc constitutif B-2, dans des conditions permettant la formation d'une liaison covalente entre ledit groupe hydroxyle libre du squelette de l'oligonucléotide de premier cycle (O-1)^{#} et l'atome de phosphore dudit fragment phosphore du bloc constitutif B-2, obtenant ainsi un oligonucléotide de deuxième cycle O-2 ;
(f-2) facultativement, l'incubation de l'oligonucléotide de deuxième cycle O-2 avec un agent oxydant ou sulfurant, convertissant ainsi les atomes P(III) dudit oligonucléotide de deuxième cycle O-2 en atomes P(V) ;
(g-2) facultativement, la soumission d'une solution comprenant l'oligonucléotide de deuxième cycle O-2 à une ou plusieurs extractions aqueuses, la phase organique comprenant l'oligonucléotide de deuxième cycle O-2 ;
(h-2) si l'étape (g-2) a été réalisée, facultativement, la réduction de la teneur en eau de la phase organique comprenant l'oligonucléotide de deuxième cycle O-2 ;
dans lequel, pendant et entre les étapes (b-1) à (h-2), aucune séparation solide-liquide n'est réalisée et les étapes (c-1), (g-1), (c-2) et (g-2) sont réalisées en présence d'un ou plusieurs solvants amides S^{A}, chaque solvant amide S^{A} étant tel que défini dans la revendication 1.

4. Procédé selon la revendication 3, dans lequel l'oligonucléotide cible O^{T} comprend un oligonucléotide de n-ième cycle O-n, et le procédé comprenant en outre la réalisation de (n-2) itérations d'un cycle de couplage comprenant les étapes (b-x) à (h-x) suivantes, n étant un entier compris entre 3 et 99, indiquant le nombre total de cycles de couplage réalisés pour obtenir l'oligonucléotide de n-ième cycle O-n, et chaque cycle de couplage comprenant les étapes (b-x) à (h-x) étant identifié par un numéro de série x, qui est réalisé par étapes de 1 de 3 à n :
(b-x) l'incubation de l'oligonucléotide de (x-1)-ième cycle O-(x-1) obtenu lors du cycle de couplage précédent avec un mélange de déprotection M-(b-x), séparant ainsi le groupe protecteur PG-(x-1) de l'oligonucléotide de (x-1)-ième cycle O-(x-1), de façon à obtenir un oligonucléotide de (x-1)-ième cycle (O-(x-1))^{#} comportant un groupe hydroxyle libre du squelette ;
(c-x) la soumission d'une solution comprenant l'oligonucléotide de (x-1)-ième cycle (O-(x-1))^{#} à une ou plusieurs extractions aqueuses, la phase organique comprenant l'oligonucléotide de (x-1)-ième cycle (O-(x-1))^{#} ;
(d-x) facultativement, la réduction de la teneur en eau de la phase organique comprenant l'oligonucléotide de (x-1)-ième cycle (O-(x-1))^{#} ;
(e-x) la réaction de l'oligonucléotide de (x-1)-ième cycle (O-(x-1))^{#} avec un bloc constitutif B-x, ledit bloc constitutif B-x étant choisi dans le groupe constitué par un nucléoside et un oligonucléotide et comprenant
- un fragment hydroxyle du squelette protégé par un groupe protecteur PG-x clivable dans des conditions acides, et
- un fragment phosphore lié de manière covalente, via son atome de phosphore, à un atome d'oxygène du squelette du bloc constitutif B-x, dans des conditions permettant la formation d'une liaison covalente entre ledit groupe hydroxyle libre du squelette de l'oligonucléotide de (x-1)-ième cycle (O-(x-1))^{#} et l'atome de phosphore dudit fragment phosphore du bloc constitutif B-x, obtenant ainsi un oligonucléotide de x-ième cycle O-x ;
(f-x) facultativement, l'incubation de l'oligonucléotide de x-ième cycle O-x avec un agent oxydant ou sulfurant, convertissant ainsi les atomes P(III) dudit oligonucléotide de x-ième cycle O-x en atomes P(V) ;
(g-x) facultativement, la soumission d'une solution comprenant l'oligonucléotide de x-ième cycle O-x à une ou plusieurs extractions aqueuses, la phase organique comprenant l'oligonucléotide de x-ième cycle O-x ;
(h-x) si l'étape (g-x) a été réalisée, facultativement, la réduction de la teneur en eau de la phase organique comprenant l'oligonucléotide de x-ième cycle O-x ;
dans lequel, pendant et entre les étapes (b-1) à (h-n), aucune séparation solide-liquide n'est réalisée et les étapes (c-1), (g-1), (c-2) et (g-2), ainsi que chaque itération des étapes (c-x) et (g-x), sont réalisées en présence d'un ou plusieurs solvants amides S^{A}, chaque solvant amide S^{A} étant tel que défini dans la revendication 1.

5. Procédé selon l'une quelconque des revendications 2 à 4, dans lequel
- le fragment phosphore des blocs constitutifs B-1, B-2 et de chaque bloc constitutif B-x est choisi indépendamment dans le groupe constitué par un fragment phosphoramidite et un fragment monoester de H-phosphonate ;
- dans chaque cycle de couplage dans lequel ledit fragment phosphore du bloc constitutif B-1, B-2 ou B-x est un fragment phosphoramidite, l'étape (f-1) ou (f-2) ou (f-x) est réalisée ; et
- au moins dans le cycle de couplage final, l'étape (f-1) ou (f-2) ou (f-x) est réalisée.

6. Procédé selon l'une quelconque des revendications 2 à 4, dans lequel le composant C-0 est un composé ayant la formule I suivante : 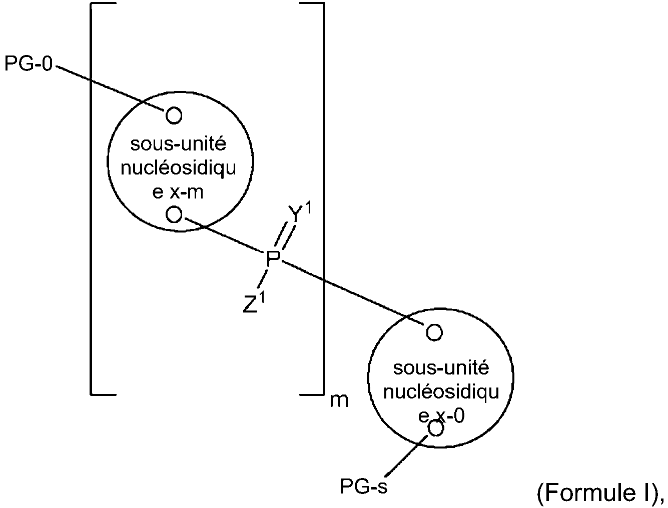 dans lequel, dans la formule I :
chaque atome d'oxygène (O) représenté dans chaque sous-unité nucléosidique x-0 à x-m représente l'atome d'oxygène d'un fragment hydroxyle de la sous-unité nucléosidique respective ;
chacune des sous-unités nucléosidiques x-0 à x-m peut être identique ou différente ;
m est un entier supérieur ou égal à 0 ;
PG-0 est un groupe protecteur clivable dans des conditions acides ;
Y¹ est choisi indépendamment pour chacun des m motifs répétitifs dans le groupe constitué par O et S ;
Z¹ est choisi indépendamment pour chacun des m motifs répétitifs dans le groupe constitué par O-R^{z-1}, S-R^{z-1} et H ;
R^{z-1} est un groupe protecteur, qui peut être identique ou différent pour chacun des m motifs répétitifs ; et
PG-s est un groupe protecteur de type pseudo-phase solide.

7. Procédé selon l'une quelconque des revendications 2 à 6, dans lequel chacun des blocs constitutifs B-1, B-2 et B-x est un composé ayant la formule II-1 suivante : 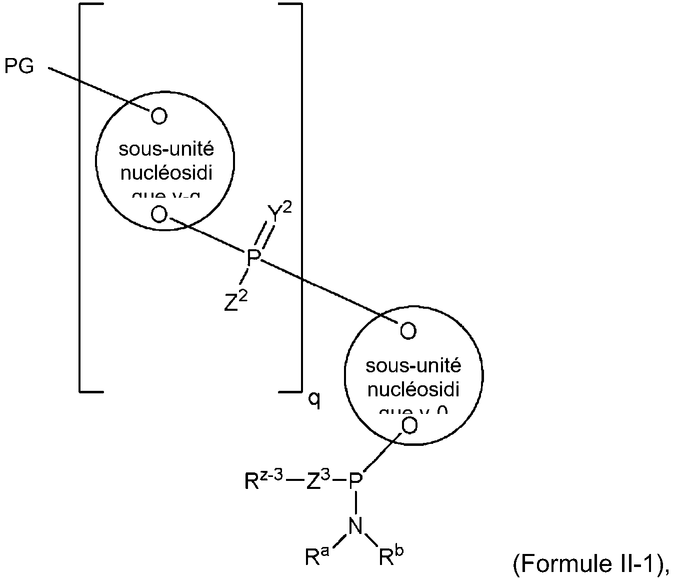 dans lequel, dans la formule II-1 :
chaque atome d'oxygène (O) représenté dans chaque sous-unité nucléosidique y-0 à y-q représente l'atome d'oxygène d'un fragment hydroxyle de la sous-unité nucléosidique respective ;
chacune des sous-unités nucléosidiques y-0 à y-q peut être identique ou différente ;
PG est le groupe protecteur PG-1, PG-2 ou PG-x et est un groupe protecteur clivable dans des conditions acides ;
q est un entier supérieur ou égal à 0 ;
Y² est choisi indépendamment pour chacun des q motifs répétitifs dans le groupe constitué par O et S ;
Z² est choisi indépendamment pour chacun des q motifs répétitifs dans le groupe constitué par O-R^{z-2} et S-R^{z-2} ;
R^{z-2} est un groupe protecteur, qui peut être identique ou différent pour chacun des q motifs répétitifs ;
Z³ est choisi dans le groupe constitué par O et S ; et
R^{z-3} est un groupe protecteur ;
chacun de R^{a} et R^{b} représente un groupe alkyle en C₁-C₆, R^{a} et R^{b} pouvant être identiques ou différents et pouvant en outre se lier l'un à l'autre pour former une fraction amine cyclique aliphatique de 5 ou 6 chaînons avec l'atome d'azote auquel R^{a} et R^{b} sont liés ;
et dans lequel l'étape (f-1), (f-2) ou (f-x) est réalisée à chaque cycle de couplage.

8. Procédé selon l'une quelconque des revendications 2 et 5 à 7, le procédé comprenant en outre
- une étape (k-1) d'incubation de l'oligonucléotide de premier cycle O-1 avec un mélange de déprotection M-(k-1), séparant ainsi le groupe protecteur PG-1 de l'oligonucléotide de premier cycle O-1, de façon à obtenir un oligonucléotide de premier cycle (O-1)^{#} comportant un groupe hydroxyle libre du squelette ; et/ou
- une étape (m-1) d'incubation de l'oligonucléotide de premier cycle O-1 ou (O-1)^{#} avec une base, séparant ainsi le groupe protecteur de type pseudo-phase solide PG-s et, facultativement, un ou plusieurs autres groupes protecteurs de l'oligonucléotide de premier cycle O-1 ou (O-1)^{#} ; et/ou
- une étape (p-1) de modification de l'oligonucléotide de premier cycle O-1 ou (O-1)^{#} ;
dans lequel, si plusieurs des étapes (k-1), (m-1) et (p-1) sont réalisées, elles peuvent être réalisées dans n'importe quel ordre.

9. Procédé selon l'une quelconque des revendications 3 et 5 à 7, le procédé comprenant en outre
- une étape (k-2) d'incubation de l'oligonucléotide de deuxième cycle O-2 avec un mélange de déprotection M-(k-2), séparant ainsi le groupe protecteur PG-2 de l'oligonucléotide de deuxième cycle O-2, de façon à obtenir un oligonucléotide de deuxième cycle (O-2)^{#} comportant un groupe hydroxyle libre du squelette ; et/ou
- une étape (m-2) d'incubation de l'oligonucléotide de deuxième cycle O-2 ou (O-2)^{#} avec une base, séparant ainsi le groupe protecteur de type pseudo-phase solide PG-s et, facultativement, un ou plusieurs autres groupes protecteurs de l'oligonucléotide de deuxième cycle O-2 ou (O-2)^{#} ; et/ou
- une étape (p-2) de modification de l'oligonucléotide de deuxième cycle O-2 ou (O-2)^{#} ;
dans lequel, si plusieurs des étapes (k-2), (m-2) et (p-2) sont réalisées, elles peuvent être réalisées dans n'importe quel ordre.

10. Procédé selon l'une quelconque des revendications 4 à 7, le procédé comprenant en outre
- une étape (k-n) d'incubation de l'oligonucléotide de n-ième cycle O-n avec un mélange de déprotection M-(k-n), séparant ainsi le groupe protecteur PG-n de l'oligonucléotide de n-ième cycle O-n, de façon à obtenir un oligonucléotide de n-ième cycle (O-n)^{#} comportant un groupe hydroxyle libre du squelette ; et/ou
- une étape (m-n) d'incubation de l'oligonucléotide de n-ième cycle On ou (O-n)^{#} avec une base, séparant ainsi le groupe protecteur de type pseudo-phase solide PG-s et facultativement, un ou plusieurs autres groupes protecteurs, de l'oligonucléotide de n-ième cycle O-n ou (O-n)^{#} ; et/ou
- une étape (p-n) de modification de l'oligonucléotide de n-ième cycle O-n ou (O-n)^{#} ;
dans lequel, si plusieurs des étapes (k-n), (m-n) et (p-n) sont réalisées, elles peuvent être réalisées dans n'importe quel ordre.

11. Procédé selon l'une quelconque des revendications 2 à 10, dans lequel les étapes (b-1) à (h-1) sont réalisées en présence d'un ou plusieurs solvants amides S^{A} et, de préférence, d'un ou plusieurs solvants éthers S^{E}, et en outre, dans lequel :
- si le deuxième cycle de couplage comprenant les étapes (b-2) à (h-2), est réalisé, les étapes (b-1) à (h-2) sont réalisées en présence d'un ou plusieurs solvants amides S^{A} et, de préférence, d'un ou plusieurs solvants éthers S^{E} ; et
- si (n-2) itérations du cycle de couplage comprenant les étapes (b-x) à (h-x) sont réalisées, toutes les étapes (b-1) à (h-n) sont réalisées en présence d'un ou plusieurs solvants amides S^{A} et, de préférence, d'un ou plusieurs solvants éthers S^{E}.

12. Procédé selon l'une quelconque des revendications 2 à 11, dans lequel les étapes (b-1) à (h-1) sont réalisées dans des solvants essentiellement exempts d'halogène, et en outre, dans lequel :
- si un deuxième cycle de couplage comprenant les étapes (b-2) à (h-2) est réalisé, les étapes (b-1) à (h-2) sont réalisées dans des solvants essentiellement exempts d'halogène ; et
- si (n-2) itérations du cycle de couplage comprenant les étapes (b-x) à (h-x) sont réalisées, les étapes (b-1) à (h-n) sont réalisées dans des solvants essentiellement exempts d'halogène,
un solvant essentiellement exempt d'halogène étant un solvant qui contient au total au plus 3,0 % en volume de solvants halogénés.

13. Procédé selon l'une quelconque des revendications 2 à 12, dans lequel, lors de chacune des extractions aqueuses de l'étape (c-1), la phase aqueuse présente un pH inférieur ou égal à 7, de préférence compris entre 4 et 7, et en outre, dans lequel :
- si un deuxième cycle de couplage comprenant les étapes (b-2) à (h-2) est réalisé, lors de chacune des extractions aqueuses de l'étape (c-2), la phase aqueuse présente un pH inférieur ou égal à 7, de préférence compris entre 4 et 7 ; et
- si (n-2) itérations du cycle de couplage comprenant les étapes (b-x) à (h-x) sont réalisées, lors de chacune des extractions aqueuses de chaque étape (c-x), la phase aqueuse présente un pH inférieur ou égal à 7, de préférence compris entre 4 et 7.

14. Procédé selon l'une quelconque des revendications 1 à 13, dans lequel chaque solvant amide S^{A} est, à chaque occurrence, choisi indépendamment dans le groupe constitué par les formules S^{A}-1, S^{A}-2 et S^{A}-3 suivantes : 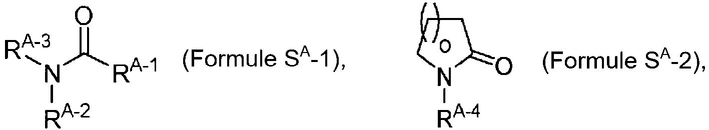 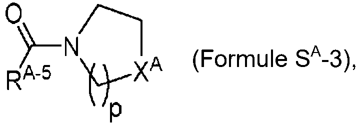 dans lequel, dans la formule S^{A}-1 :
R^{A-1} est choisi dans le groupe constitué par H et un groupe alkyle en C₁-C₂₂,
dans lequel un seul résidu hydrogène peut facultativement être substitué par un groupe C(O)O(alkyle en C₁-C₅) ; et
chacun de R^{A-2} et R^{A-3} représente indépendamment un groupe alkyle en C₁-C23 ;
à condition que R^{A-1}, R^{A-2} et R^{A-3} comprennent ensemble un total de 6 à 24 atomes de carbone ;
dans lequel, dans la formule S^{A}-2 :
o est un entier égal à 1 ou 2 ; et
R^{A-4} représente un groupe alkyle en C₆-C₂₄ ; et
dans lequel, dans la formule S^{A}-3 :
p est un entier égal à 1 ou 2 ;
X^{A} est choisi dans le groupe constitué par CH₂, O et NC(O)R^{A-6}, à condition que, si p est 1, X^{A} soit CH₂ ;
R^{A-5} représente un groupe alkyle en C₁-C₂₄ ; et
R^{A-6} représente un groupe alkyle en C₁-C₂₃ ;
à condition que R^{A-5} et R^{A-6} comprennent ensemble un total de 6 à 24 atomes de carbone.

15. Procédé selon l'une quelconque des revendications 11 à 14, dans lequel chaque solvant éther S^{R} est, à chaque occurrence, choisi indépendamment dans le groupe constitué par les formules S^{E}-1 et S^{E}-2 suivantes : 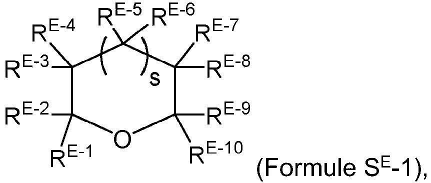 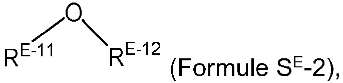 dans lequel, dans la formule S^{E}-1 :
s est un entier égal à 0 ou 1 ; et
chacun de R^{E-1}, R^{E-2}, R^{E-3}, R^{E-4}, R^{E-5}, R^{E-6}, R^{E-7}, R^{E-8}, R^{E-9}, et R^{E-10} est indépendamment choisi dans le groupe constitué par H et un groupe alkyle en C₁-C₅, à condition qu'au moins l'un de R^{E-1}, R^{E-2}, R^{E-3}, R^{E-4}, R^{E-5}, R^{E-6}, R^{E- 7}, R^{E-8}, R^{E-9}, et R^{E-10} soit un groupe alkyle en C₁-C₅ ; et
dans lequel, dans la formule S^{E}-2 :
chacun de R^{E-11} et R^{E-12} est indépendamment un groupe alkyle en C₁-C₆, à condition que R^{E-11} et R^{E-12} comprennent ensemble un total de 5 à 12 atomes de carbone.

16. Procédé selon l'une quelconque des revendications 1 à 15, dans lequel chaque groupe protecteur de type pseudo-phase solide est un groupe protecteur de formule P-1 suivante : 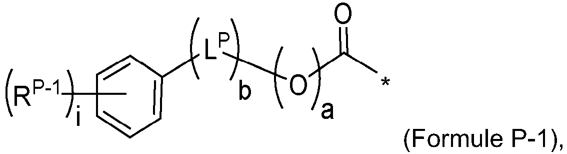 dans lequel, dans la formule P-1 :
l'astérisque indique l'atome d'oxygène d'un fragment hydroxyle ou l'atome d'azote d'un fragment amine protégé par le groupe protecteur de type pseudo-phase solide respectif ;
a est un entier égal à 0 ou 1 ;
b est un entier égal à 0 ou 1 ;
L^{P} est un fragment lieur ;
i est un entier de 1 à 5 ; et
R^{P-1} est, à chaque occurrence, indépendamment choisi dans le groupe constitué par O(alkyle en C₁-C₄₀), O(alcényle en C₂-C₄₀), O(alcynyle en C₂-C₄₀), un groupe alkyle en C₁-C₄₀, un groupe alcényle en C₂-C₄₀, un groupe alcynyle en C₂-C₄₀, C(O)(alkyle en C₁-C₄₀), C(O)(alcényle en C₂-C₄₀) et C(O)(alcynyle en C₂-C₄₀) ; et
dans lequel tous les résidus R^{P-1} présents comprennent au total 18 à 200 atomes de carbone.

17. Composition comprenant :
- un oligonucléotide qui est lié de façon covalente à au moins un groupe protecteur de type pseudo-phase solide ; et
- un solvant mixte qui est essentiellement exempt d'halogène et comprend un ou plusieurs solvants amides S^{A} et, de préférence, un ou plusieurs solvants éthers S^{E}, chaque solvant amide S^{A} étant un solvant amide comprenant un ou plusieurs groupes alkyles, ledit ou lesdits groupes alkyle comprenant ensemble un total de 6 à 24 atomes de carbone, un solvant essentiellement exempt d'halogène étant un solvant qui contient au total au plus 3,0 % en volume de solvants halogénés,
de préférence, la composition et/ou un ou plusieurs composants étant tels que définis dans une ou plusieurs des revendications précédentes,
en particulier, ledit ou lesdits solvants amides S^{A} étant tels que définis dans la revendication 14, ledit ou lesdits solvants éthers S^{E} étant tels que définis dans la revendication 15, et/ou chaque groupe protecteur de type pseudo-phase solide étant tel que défini dans la revendication 16.
